# EUROPEAN PATENT SPECIFICATION

(11) **EP 2 192 170 B1**
(45) Date of publication and mention of the grant of the patent: **15.02.2017**
(21) Application number: 08829698.3
(22) Date of filing: 03.09.2008
(51) Int. Cl.: C12N 1/21, C12N 15/09, C12P 13/06, C12P 13/08, C12P 13/22

(54) **AMINO ACID-PRODUCING MICROORGANISM AND METHOD OF PRODUCING AMINO ACID**
AMINOSÄUREPRODUZIERENDER MIKROORGANISMUS UND VERFAHREN ZUR AMINOSÄUREPRODUKTION
MICROORGANISME PRODUISANT DES ACIDES AMINÉS ET PROCÉDÉ DE PRODUCTION DES ACIDES AMINÉS

(30) Priority: 04.09.2007 JP 2007228733; 21.12.2007 RU 2007147434
(43) Date of publication of application: 02.06.2010
(73) Proprietor: Ajinomoto Co., Inc., Chuo-ku, Tokyo 104-8315 (JP)
(72) Inventor: TERASHITA, Masaru, Kawasaki-shi Kanagawa 210-8681 (JP); USUDA, Yoshihiro, Kawasaki-shi Kanagawa 210-8681 (JP); MATSUI, Kazuhiko, Kawasaki-shi Kanagawa 210-8681 (JP); YAMPOLSKAYA, Tatyana Abramovna, Moscow 123364 (RU); STOYNOVA, Natalia Viktorovna, Moscow 123479 (RU); EREMINA, Natalia Sergeevna, Moscow 129336 (RU); ALTMAN, Irina Borisovna, Moscow 119435 (RU); PTITSYN, Leonid Romanovich, Moscow 115404 (RU)
(74) Representative: Hoffmann Eitle
(86) International application number: PCT/JP2008/065834
(87) International publication number: WO 2009/031565

(56) References cited:
- EP-A1- 1 092 776
- WO-A1-02/14522
- WO-A2-02/22829
- JP-A- 2001 136 991
- HUGHES NICKY J ET AL: "Helicobacter pylori porCDAB and oorDABC genes encode distinct pyruvate:flavodoxin and 2-oxoglutarate:acceptor oxidoreductase which mediate electron transport to NADP" JOURNAL OF BACTERIOLOGY, AMERICAN SOCIETY FOR MICROBIOLOGY, WASHINGTON, DC; US, vol. 180, no. 5, 1 March 1998 (1998-03-01) , pages 1119-1128, XP002231081 ISSN: 0021-9193
- PIEULLE LAETITIA ET AL: "Isolation and analysis of the gene encoding the pyruvate-ferredoxin oxidoreductase of Desulfovibrio africanus, production of the recombinant enzyme in Escherichia coli, and effect of carboxy-terminal deletions on its stability" JOURNAL OF BACTERIOLOGY, vol. 179, no. 18, 1997, pages 5684-5692, XP002606434 ISSN: 0021-9193
- INUI H ET AL: "PURIFICATION AND CHARACTERIZATION OF PYRUVATE NADP OXIDOREDUCTASE IN EUGLENA-GRACILIS" JOURNAL OF BIOLOGICAL CHEMISTRY, AMERICAN SOCIETY FOR BIOCHEMISTRY AND MOLECULAR BIOLOGY, INC, US, vol. 262, no. 19, 1 January 1987 (1987-01-01), pages 9130-9135, XP002165256 ISSN: 0021-9258
- KRAEMER REINHARD: "Genetic and physiological approaches for the production of amino acids" JOURNAL OF BIOTECHNOLOGY, vol. 45, no. 1, 1996, pages 1-21, XP002606435 ISSN: 0168-1656
- IKEDA T. ET AL.: 'Anabolic five subunit-type pyruvate:ferredoxin oxidoreductase from Hydrogenobacter thermophilus TK-6' BIOCHEM.BIOPHYS.RES.COMMUN. vol. 340, 2006, pages 76 - 82, XP024924013
- DATABASE GENBANK [Online] 'Pyruvate flavodoxin/ferrodoxin oxidoreductase [Chlorobium tepidumm TLS]', XP009136172 Database accession no. NP_662511
- DATABASE GENBANK [Online] 'Probable pyruvate-flavodoxin oxidoreductase', XP009136250 Database accession no. P52647
- LIN, W. ET AL.: 'The anabotic pyruvate oxidoreductase from Methanococcus maripaludis' ARCH.MICROBIOL. vol. 179, no. 6, June 2003, pages 444 - 456, XP009135929
- LIN, W. AND WHITMAN, W.B.: 'The importance of porE and porF in the anabolic pyruvate oxidoreductase of Methanococcus maripaludis' ARCH.MICROBIOL. vol. 181, no. 1, January 2004, pages 68 - 73, XP009135928
- DATABASE GENBANK [Online] 18 January 2006 'Indolepyruvate ferredoxin oxidoreductase [Methanococcus maripaludis S2]', XP008135885 Database accession no. NP_988627
- DATABASE GENBANK [Online] 18 January 2006 'Pyruvate oxidoreductase (synthase) subunit delta [Methanococcus maripaludis S2]', XP009136287 Database accession no. NP_988626
- DATABASE GENBANK [Online] 18 January 2006 'Pyruvate oxidoreductase (synthase) subunit alpha [Methanococcus maripaludis S2]', XP008135886 Database accession no. NP_988625
- DATABASE GENBANK [Online] 18 January 2006 'Ferredoxin oxidoreductase beta subunit [Methanococcus maripaludis S2]' Database accession no. NP_988624
- DATABASE GENBANK [Online] 18 January 2006 'Hypothetical protein,pyruvate oxidoreductase-associated [Methanococcus maripaludis S2]', XP008135887 Database accession no. NP_988623
- DATABASE GENBANK [Online] 18 January 2006 'Hypothetical protein MMP1502 [Methanococcus maripaludis S2]', XP009136290 Database accession no. NP_988622

### SEQUENCE LISTING

<110> Ajinomoto Co., Inc.
<120> An amino acid producing bacterium and a method for producing an amino acid
<130> C985-C8187
<150> JP2007-228733
   <151> 2007-09-04
<150> RU2007147434
   <151> 2007-12-21
<160> 85
<170> PatentIn version 3.5
<210> 1
   <211> 3558
   <212> DNA
   <213> Chlorobium tepidum
<220>
   <221> CDS
   <222> (1)..(3558)
<400> 1
   atg acc cgg aca ttc aag aca atg gag ggg aat gaa gct ctt gct cat       48
   Met Thr Arg Thr Phe Lys Thr Met Glu Gly Asn Glu Ala Leu Ala His
   1               5                   10                  15
   gtc gcc tat cgc act aat gaa gtc atc tcg ata tac ccg att acc ccg       96
   Val Ala Tyr Arg Thr Asn Glu Val Ile Ser Ile Tyr Pro Ile Thr Pro
               20                  25                  30
   gca tct ccg atg gga gag tac tcc gac gca tgg gcc gct gtc gat gta      144
   Ala Ser Pro Met Gly Glu Tyr Ser Asp Ala Trp Ala Ala Val Asp Val
           35                  40                  45
   aaa aat atc tgg ggt acc gtg cca ctc gtc aat gag atg cag agc gaa      192
   Lys Asn Ile Trp Gly Thr Val Pro Leu Val Asn Glu Met Gln Ser Glu
       50                  55                  60
   gcc ggt gcc gcc gcc gcc gtt cac ggc gcg ttg cag acc ggc gcg ctg      240
   Ala Gly Ala Ala Ala Ala Val His Gly Ala Leu Gln Thr Gly Ala Leu
   65                  70                  75                  80
   acg acc acc ttc acg gcc tct cag ggt ctc tta ctg atg atc ccg aac      288
   Thr Thr Thr Phe Thr Ala Ser Gln Gly Leu Leu Leu Met Ile Pro Asn
                   85                  90                  95
   atg tac aag atc gcc ggt gaa ctg acc ccc tgc gtg att cac gtg tca      336
   Met Tyr Lys Ile Ala Gly Glu Leu Thr Pro Cys Val Ile His Val Ser
               100                 105                 110
   gcc cgt tcg ctg gcc gcg cag gcg ctc tcg ata ttc tgc gac cac ggt      384
   Ala Arg Ser Leu Ala Ala Gln Ala Leu Ser Ile Phe Cys Asp His Gly
           115                 120                 125
   gac gtg atg tcg gtc agg ggc acc ggc ttc gcg ctg ctc gct tcc tgt      432
   Asp Val Met Ser Val Arg Gly Thr Gly Phe Ala Leu Leu Ala Ser Cys
       130                 135                 140
   tcg gta cag gag gta atg gac atg gcg ctg att tcg cag gcc gca acg      480
   Ser Val Gln Glu Val Met Asp Met Ala Leu Ile Ser Gln Ala Ala Thr
   145                 150                 155                 160
   ctc gaa tcg cgc gtg cca ttc ctg cac ttc ttc gac ggc ttc cgc acg      528
   Leu Glu Ser Arg Val Pro Phe Leu His Phe Phe Asp Gly Phe Arg Thr
                   165                 170                 175
   tcg cac gaa atc tcg aaa atc gag gtg ctc tcg gac gaa cag att cgc      576
   Ser His Glu Ile Ser Lys Ile Glu Val Leu Ser Asp Glu Gln Ile Arg
               180                 185                 190
   tcg atg atc aac gac gag ctg gtc ttc gca cac cgc atg cgc cgc atg      624
   Ser Met Ile Asn Asp Glu Leu Val Phe Ala His Arg Met Arg Arg Met
           195                 200                 205
   tcg cct gat gca ccg atc atc cgc ggt acc tcg cag aat ccg gac gtc      672
   Ser Pro Asp Ala Pro Ile Ile Arg Gly Thr Ser Gln Asn Pro Asp Val
       210                 215                 220
   tat ttc cag gca cgc gag agc gtc aac aaa tat tat gag gcc tgc ccg      720
   Tyr Phe Gln Ala Arg Glu Ser Val Asn Lys Tyr Tyr Glu Ala Cys Pro
   225                 230                 235                 240
   tca atc acc cag aag gcg atg gac cag ttc gcc aaa ctg act ggg cgc      768
   Ser Ile Thr Gln Lys Ala Met Asp Gln Phe Ala Lys Leu Thr Gly Arg
                   245                 250                 255
   agc tat aaa ctt tac cag tac tac ggc gct ccg gat gcc gac cgt atc      816
   Ser Tyr Lys Leu Tyr Gln Tyr Tyr Gly Ala Pro Asp Ala Asp Arg Ile
               260                 265                 270
   atc atc atg atg ggg tca ggt gcc gag acc gct ctc gaa act gtc gaa      864
   Ile Ile Met Met Gly Ser Gly Ala Glu Thr Ala Leu Glu Thr Val Glu
           275                 280                 285
   tac ctc aac aac cac ggc gaa aag gtc ggt ctg gtc aag gta cgc ctt      912
   Tyr Leu Asn Asn His Gly Glu Lys Val Gly Leu Val Lys Val Arg Leu
       290                 295                 300
   ttc agg cca ttc gac gtt gca acc ttc atc gca tcg cta cca tcg agc      960
   Phe Arg Pro Phe Asp Val Ala Thr Phe Ile Ala Ser Leu Pro Ser Ser
   305                 310                 315                 320
   gtg aag agt atc gcg gtg ctc gac cgt gtc aag gaa cca ggc agc gct     1008
   Val Lys Ser Ile Ala Val Leu Asp Arg Val Lys Glu Pro Gly Ser Ala
                   325                 330                 335
   ggc gaa ccg ctc tat ctc gat gta gtc aac gcc gta gcc gaa tcg tac     1056
   Gly Glu Pro Leu Tyr Leu Asp Val Val Asn Ala Val Ala Glu Ser Tyr
               340                 345                 350
   cag gaa ggc aaa tgc gct tcg atg cca agc gtt ttg ggt ggg cgc tat     1104
   Gln Glu Gly Lys Cys Ala Ser Met Pro Ser Val Leu Gly Gly Arg Tyr
           355                 360                 365
   ggc ctg tcg tcg aag gag ttc act ccg gcg atg gtc aag gcg atc ttc     1152
   Gly Leu Ser Ser Lys Glu Phe Thr Pro Ala Met Val Lys Ala Ile Phe
       370                 375                 380
   gac aat atg aac gcg gaa tct cca aag aat cac ttc acc gtt ggc atc     1200
   Asp Asn Met Asn Ala Glu Ser Pro Lys Asn His Phe Thr Val Gly Ile
   385                 390                 395                 400
   gac gat gac gta acc aag aag agc ctc gcc tac gac gag acc ttc tcg     1248
   Asp Asp Asp Val Thr Lys Lys Ser Leu Ala Tyr Asp Glu Thr Phe Ser
                   405                 410                 415
   att gag ccg gac tcg gtc ttc cgc gcc ctc ttc tac ggc ctc ggt tca     1296
   Ile Glu Pro Asp Ser Val Phe Arg Ala Leu Phe Tyr Gly Leu Gly Ser
               420                 425                 430
   gac ggc acg gtc ggt gca aac aag aac tcg atc aag atc att ggc gaa     1344
   Asp Gly Thr Val Gly Ala Asn Lys Asn Ser Ile Lys Ile Ile Gly Glu
           435                 440                 445
   aac acc gac aac tac gcg cag ggc ttc ttc gtc tac gac tcc aag aaa     1392
   Asn Thr Asp Asn Tyr Ala Gln Gly Phe Phe Val Tyr Asp Ser Lys Lys
       450                 455                 460
   gcc ggt tcg atc acg acc tcg cac ctg cgg ttc ggc ccg gag cag atc     1440
   Ala Gly Ser Ile Thr Thr Ser His Leu Arg Phe Gly Pro Glu Gln Ile
   465                 470                 475                 480
   cgc tcg acc tac ctc atc acc gag gcg cag ttc gtc ggc tgc cac cac     1488
   Arg Ser Thr Tyr Leu Ile Thr Glu Ala Gln Phe Val Gly Cys His His
                   485                     490                     495
   tgg gtc ttt ctc gaa atg atc gac gtt gcc aag aac ctc aag cag ggt      1536
   Trp Val Phe Leu Glu Met Ile Asp Val Ala Lys Asn Leu Lys Gln Gly
               500                     505                     510
   ggt acg ctg ctc atc aac tcg gcc tat gcg ccg gat gtg gtg tgg agc      1584
   Gly Thr Leu Leu Ile Asn Ser Ala Tyr Ala Pro Asp Val Val Trp Ser
           515                     520                     525
   aag ctc ccg cgt ccg gtg cag cag cac ttg atc gac aag cag gcg aag      1632
   Lys Leu Pro Arg Pro Val Gln Gln His Leu Ile Asp Lys Gln Ala Lys
       530                     535                     540
   ctc tac acc atc gat gcc tac aag gtc gcc cac gaa agc ggc atg ggt      1680
   Leu Tyr Thr Ile Asp Ala Tyr Lys Val Ala His Glu Ser Gly Met Gly
   545                     550                     555                     560
   cag cgc atc aac act atc atg cag gcc tgt ttc ttc gcc att tcg ggc      1728
   Gln Arg Ile Asn Thr Ile Met Gln Ala Cys Phe Phe Ala Ile Ser Gly
                   565                     570                     575
   gtg ctg ccg cgt gaa gag gca atc gaa aag atc aag gac gcg atc cgc      1776
   Val Leu Pro Arg Glu Glu Ala Ile Glu Lys Ile Lys Asp Ala Ile Arg
               580                     585                     590
   cac acc tac ggc aaa aag ggc gat gag gtc gtt cag cag aac atc aag      1824
   His Thr Tyr Gly Lys Lys Gly Asp Glu Val Val Gln Gln Asn Ile Lys
           595                     600                     605
   gca gtt gac aac acg ctt gcc aac ctg cat gaa gtg aaa atc ggc gct      1872
   Ala Val Asp Asn Thr Leu Ala Asn Leu His Glu Val Lys Ile Gly Ala
       610                     615                     620
   gtg gca gac agc acc aag gag ctg cgc tcg ccc atc gtt ggc gac gcg      1920
   Val Ala Asp Ser Thr Lys Glu Leu Arg Ser Pro Ile Val Gly Asp Ala
   625                     630                     635                     640
   cca gag ttc gtc tgt aac gtg ctg gca aag att att gcc ggc gag ggc      1968
   Pro Glu Phe Val Cys Asn Val Leu Ala Lys Ile Ile Ala Gly Glu Gly
                   645                     650                     655
   gac tcg att ccg gtc agc aag ctg cct gcc gat gga acc tat ccg ctc      2016
   Asp Ser Ile Pro Val Ser Lys Leu Pro Ala Asp Gly Thr Tyr Pro Leu
               660                     665                     670
   ggc acc acg aag ttc gag aaa cgc aac ctc gcg cag gag att ccg gtc      2064
   Gly Thr Thr Lys Phe Glu Lys Arg Asn Leu Ala Gln Glu Ile Pro Val
           675                     680                     685
   tgg gct ccg gag ctg tgc atc gag tgt ggc aag tgc tcg atg gtc tgc      2112
   Trp Ala Pro Glu Leu Cys Ile Glu Cys Gly Lys Cys Ser Met Val Cys
       690                     695                     700
   ccg cac gct gcc atc cgc atc aag gtt tac gag ccg aag cac ctc gaa      2160
   Pro His Ala Ala Ile Arg Ile Lys Val Tyr Glu Pro Lys His Leu Glu
   705                     710                     715                     720
   aac gcc ccg gca acc ttc aag agc ctc gat gcg aaa gca aaa aac tgg      2208
   Asn Ala Pro Ala Thr Phe Lys Ser Leu Asp Ala Lys Ala Lys Asn Trp
                   725                     730                     735
   gag ggc atg cgc tat acg gtt cag att gca ccg gaa gat tgt acc ggc      2256
   Glu Gly Met Arg Tyr Thr Val Gln Ile Ala Pro Glu Asp Cys Thr Gly
               740                     745                     750
   tgc caa ctc tgc gtc aac gcc tgc ccc gca aga gac aag cag gtt gaa      2304
   Cys Gln Leu Cys Val Asn Ala Cys Pro Ala Arg Asp Lys Gln Val Glu
           755                     760                     765
   ggc cgc aaa gcg ctc aac atg cac gag cag gct ccg ctg cgc gaa acc      2352
   Gly Arg Lys Ala Leu Asn Met His Glu Gln Ala Pro Leu Arg Glu Thr
       770                     775                     780
   gaa tct gcc tgc tgg agc ttc ttc atc aat ctc ccg gaa ttc gac cgc      2400
   Glu Ser Ala Cys Trp Ser Phe Phe Ile Asn Leu Pro Glu Phe Asp Arg
   785                     790                     795                     800
   aac aag atc aac cag cgc ctc atc aaa gag cag cag ctt cag cag cca      2448
   Asn Lys Ile Asn Gln Arg Leu Ile Lys Glu Gln Gln Leu Gln Gln Pro
                   805                     810                     815
   ctc ttc gag ttc tcg ggc gca tgc tcg ggc tgc ggc gaa acg cca tac      2496
   Leu Phe Glu Phe Ser Gly Ala Cys Ser Gly Cys Gly Glu Thr Pro Tyr
                   820                 825                 830
   gtc aag ctg atg act cag ctc ttc ggt gat cgc ctc gtt atc ggc aac      2544
   Val Lys Leu Met Thr Gln Leu Phe Gly Asp Arg Leu Val Ile Gly Asn
               835                 840                 845
   gcc acc ggc tgc tcg tcg atc tac ggc ggc aac ctg ccg acc acg ccg      2592
   Ala Thr Gly Cys Ser Ser Ile Tyr Gly Gly Asn Leu Pro Thr Thr Pro
       850                 855                 860
   tat gca gcc aac ccg cag ggc ctt ggg cca acg tgg tcg aac tcg ctt      2640
   Tyr Ala Ala Asn Pro Gln Gly Leu Gly Pro Thr Trp Ser Asn Ser Leu
   865                 870                 875                 880
   ttc gag gac acg gca gag ttc gcg ctt ggt ttc cgg ata tcg atc gac      2688
   Phe Glu Asp Thr Ala Glu Phe Ala Leu Gly Phe Arg Ile Ser Ile Asp
                   885                 890                 895
   aag cag cag caa ttt gcc aaa gag ctg gtc aaa aag ctc gct ggt gac      2736
   Lys Gln Gln Gln Phe Ala Lys Glu Leu Val Lys Lys Leu Ala Gly Asp
               900                 905                 910
   atc ggt gaa aac ctt gcc acc gcc att ctc aac gcc acg cag aac agt      2784
   Ile Gly Glu Asn Leu Ala Thr Ala Ile Leu Asn Ala Thr Gln Asn Ser
           915                 920                 925
   gaa ccg gag att ttc gag cag cgt gag cgc gtg gcc gtg ctg aag gat      2832
   Glu Pro Glu Ile Phe Glu Gln Arg Glu Arg Val Ala Val Leu Lys Asp
       930                 935                 940
   aag ctc cag cag atg aaa tcc gac gat gcc aag aac ctg ctt gct gtg      2880
   Lys Leu Gln Gln Met Lys Ser Asp Asp Ala Lys Asn Leu Leu Ala Val
   945                 950                 955                 960
   gct gac atg ctg gtc aag aag agc gtg tgg gct gtc ggc ggc gac ggc      2928
   Ala Asp Met Leu Val Lys Lys Ser Val Trp Ala Val Gly Gly Asp Gly
                   965                 970                 975
   tgg gcc tac gat atc ggt tac ggg ggt ctc gac cac gtc acc gca tcg      2976
   Trp Ala Tyr Asp Ile Gly Tyr Gly Gly Leu Asp His Val Thr Ala Ser
               980                 985                 990
   ggc aag aac gtc aac atg ctc gtg  ctc gac acc gag gtc  tat tcc aat    3024
   Gly Lys Asn Val Asn Met Leu Val  Leu Asp Thr Glu Val  Tyr Ser Asn
           995                 1000                 1005
   acc ggc  ggt cag gcc tcc aag  gct acg ccg aaa gcc  gcg atc gcc       3069
   Thr Gly  Gly Gln Ala Ser Lys  Ala Thr Pro Lys Ala  Ala Ile Ala
       1010                 1015                 1020
   aag ttt  gcc gct gcg ggg cgc  atc gct acc aag aaa  gac ctt ggt       3114
   Lys Phe  Ala Ala Ala Gly Arg  Ile Ala Thr Lys Lys  Asp Leu Gly
       1025                 1030                 1035
   ctg atc  tcg atg agc tac ggc  aat gcc tat gtg gcc  agt gtt gca       3159
   Leu Ile  Ser Met Ser Tyr Gly  Asn Ala Tyr Val Ala  Ser Val Ala
       1040                 1045                 1050
   ctt ggc  gca cgt gac gag cag  aca ctc aga gct ttc  atc gaa gcc       3204
   Leu Gly  Ala Arg Asp Glu Gln  Thr Leu Arg Ala Phe  Ile Glu Ala
       1055                 1060                 1065
   gag gcg  tac gat ggc ccg tcg  att atc atc gcc tac  tcg cac tgc       3249
   Glu Ala  Tyr Asp Gly Pro Ser  Ile Ile Ile Ala Tyr  Ser His Cys
       1070                 1075                 1080
   att gca  cac ggc ttt gac ttg  tct atg ggt ctg gag  cac cag aaa       3294
   Ile Ala  His Gly Phe Asp Leu  Ser Met Gly Leu Glu  His Gln Lys
       1085                 1090                 1095
   gca gcg  gtc gat tcc ggc cac  tgg ctg ctg tat cgc  tac aat ccc       3339
   Ala Ala  Val Asp Ser Gly His  Trp Leu Leu Tyr Arg  Tyr Asn Pro
       1100                 1105                 1110
   gac aga  ctc aag gag gga ctg  aat ccg ctg cag ctc  gac tcc aaa       3384
   Asp Arg  Leu Lys Glu Gly Leu  Asn Pro Leu Gln Leu  Asp Ser Lys
       1115                 1120                 1125
   aag ccg  aaa atg ccg gtc gcg  gag ttc ctg aac atg  gag aac cgc       3429
   Lys Pro  Lys Met Pro Val Ala  Glu Phe Leu Asn Met  Glu Asn Arg
       1130                 1135                 1140
   ttc aga  ata ctg aag aag acc  cac ccc gat ctg gcc  aag aag tac        3474
   Phe Arg  Ile Leu Lys Lys Thr  His Pro Asp Leu Ala  Lys Lys Tyr
       1145                 1150                 1155
   ttc gag  gca atc cag cac gag  gtc aat gcc cgc tgg  gca cac tac        3519
   Phe Glu  Ala Ile Gln His Glu  Val Asn Ala Arg Trp  Ala His Tyr
       1160                 1165                 1170
   gaa cac  ctc gcc aac cgt tcg  att gaa ggc gaa gca  taa                3558
   Glu His  Leu Ala Asn Arg Ser  Ile Glu Gly Glu Ala
       1175                 1180                 1185
<210> 2
   <211> 1185
   <212> PRT
   <213> Chlorobium tepidum
<400> 2
   Met Thr Arg Thr Phe Lys Thr Met Glu Gly Asn Glu Ala Leu Ala His
   1               5                   10                  15
   Val Ala Tyr Arg Thr Asn Glu Val Ile Ser Ile Tyr Pro Ile Thr Pro
               20                  25                  30
   Ala Ser Pro Met Gly Glu Tyr Ser Asp Ala Trp Ala Ala Val Asp Val
           35                  40                  45
   Lys Asn Ile Trp Gly Thr Val Pro Leu Val Asn Glu Met Gln Ser Glu
       50                  55                  60
   Ala Gly Ala Ala Ala Ala Val His Gly Ala Leu Gln Thr Gly Ala Leu
   65                  70                  75                  80
   Thr Thr Thr Phe Thr Ala Ser Gln Gly Leu Leu Leu Met Ile Pro Asn
                   85                  90                  95
   Met Tyr Lys Ile Ala Gly Glu Leu Thr Pro Cys Val Ile His Val Ser
               100                 105                 110
   Ala Arg Ser Leu Ala Ala Gln Ala Leu Ser Ile Phe Cys Asp His Gly
           115                 120                 125
   Asp Val Met Ser Val Arg Gly Thr Gly Phe Ala Leu Leu Ala Ser Cys
       130                 135                 140
   Ser Val Gln Glu Val Met Asp Met Ala Leu Ile Ser Gln Ala Ala Thr
   145                 150                 155                 160
   Leu Glu Ser Arg Val Pro Phe Leu His Phe Phe Asp Gly Phe Arg Thr
                   165                 170                 175
   Ser His Glu Ile Ser Lys Ile Glu Val Leu Ser Asp Glu Gln Ile Arg
               180                 185                 190
   Ser Met Ile Asn Asp Glu Leu Val Phe Ala His Arg Met Arg Arg Met
           195                 200                 205
   Ser Pro Asp Ala Pro Ile Ile Arg Gly Thr Ser Gln Asn Pro Asp Val
       210                 215                 220
   Tyr Phe Gln Ala Arg Glu Ser Val Asn Lys Tyr Tyr Glu Ala Cys Pro
   225                 230                 235                 240
   Ser Ile Thr Gln Lys Ala Met Asp Gln Phe Ala Lys Leu Thr Gly Arg
                   245                 250                 255
   Ser Tyr Lys Leu Tyr Gln Tyr Tyr Gly Ala Pro Asp Ala Asp Arg Ile
               260                 265                 270
   Ile Ile Met Met Gly Ser Gly Ala Glu Thr Ala Leu Glu Thr Val Glu
           275                 280                 285
   Tyr Leu Asn Asn His Gly Glu Lys Val Gly Leu Val Lys Val Arg Leu
       290                 295                 300
   Phe Arg Pro Phe Asp Val Ala Thr Phe Ile Ala Ser Leu Pro Ser Ser
   305                 310                 315                 320
   Val Lys Ser Ile Ala Val Leu Asp Arg Val Lys Glu Pro Gly Ser Ala
                   325                 330                 335
   Gly Glu Pro Leu Tyr Leu Asp Val Val Asn Ala Val Ala Glu Ser Tyr
               340                 345                 350
   Gln Glu Gly Lys Cys Ala Ser Met Pro Ser Val Leu Gly Gly Arg Tyr
           355                 360                 365
   Gly Leu Ser Ser Lys Glu Phe Thr Pro Ala Met Val Lys Ala Ile Phe
       370                 375                 380
   Asp Asn Met Asn Ala Glu Ser Pro Lys Asn His Phe Thr Val Gly Ile
   385                 390                 395                 400
   Asp Asp Asp Val Thr Lys Lys Ser Leu Ala Tyr Asp Glu Thr Phe Ser
                   405                 410                 415
   Ile Glu Pro Asp Ser Val Phe Arg Ala Leu Phe Tyr Gly Leu Gly Ser
               420                 425                 430
   Asp Gly Thr Val Gly Ala Asn Lys Asn Ser Ile Lys Ile Ile Gly Glu
           435                 440                 445
   Asn Thr Asp Asn Tyr Ala Gln Gly Phe Phe Val Tyr Asp Ser Lys Lys
       450                 455                 460
   Ala Gly Ser Ile Thr Thr Ser His Leu Arg Phe Gly Pro Glu Gln Ile
   465                 470                 475                 480
   Arg Ser Thr Tyr Leu Ile Thr Glu Ala Gln Phe Val Gly Cys His His
                   485                 490                 495
   Trp Val Phe Leu Glu Met Ile Asp Val Ala Lys Asn Leu Lys Gln Gly
               500                 505                 510
   Gly Thr Leu Leu Ile Asn Ser Ala Tyr Ala Pro Asp Val Val Trp Ser
           515                 520                 525
   Lys Leu Pro Arg Pro Val Gln Gln His Leu Ile Asp Lys Gln Ala Lys
       530                 535                 540
   Leu Tyr Thr Ile Asp Ala Tyr Lys Val Ala His Glu Ser Gly Met Gly
   545                 550                 555                 560
   Gln Arg Ile Asn Thr Ile Met Gln Ala Cys Phe Phe Ala Ile Ser Gly
                   565                 570                 575
   Val Leu Pro Arg Glu Glu Ala Ile Glu Lys Ile Lys Asp Ala Ile Arg
               580                 585                 590
   His Thr Tyr Gly Lys Lys Gly Asp Glu Val Val Gln Gln Asn Ile Lys
           595                 600                 605
   Ala Val Asp Asn Thr Leu Ala Asn Leu His Glu Val Lys Ile Gly Ala
       610                 615                 620
   Val Ala Asp Ser Thr Lys Glu Leu Arg Ser Pro Ile Val Gly Asp Ala
   625                 630                 635                 640
   Pro Glu Phe Val Cys Asn Val Leu Ala Lys Ile Ile Ala Gly Glu Gly
                   645                 650                 655
   Asp Ser Ile Pro Val Ser Lys Leu Pro Ala Asp Gly Thr Tyr Pro Leu
               660                 665                 670
   Gly Thr Thr Lys Phe Glu Lys Arg Asn Leu Ala Gln Glu Ile Pro Val
           675                 680                 685
   Trp Ala Pro Glu Leu Cys Ile Glu Cys Gly Lys Cys Ser Met Val Cys
       690                 695                 700
   Pro His Ala Ala Ile Arg Ile Lys Val Tyr Glu Pro Lys His Leu Glu
   705                 710                 715                 720
   Asn Ala Pro Ala Thr Phe Lys Ser Leu Asp Ala Lys Ala Lys Asn Trp
                   725                 730                 735
   Glu Gly Met Arg Tyr Thr Val Gln Ile Ala Pro Glu Asp Cys Thr Gly
               740                 745                 750
   Cys Gln Leu Cys Val Asn Ala Cys Pro Ala Arg Asp Lys Gln Val Glu
           755                 760                 765
   Gly Arg Lys Ala Leu Asn Met His Glu Gln Ala Pro Leu Arg Glu Thr
       770                 775                 780
   Glu Ser Ala Cys Trp Ser Phe Phe Ile Asn Leu Pro Glu Phe Asp Arg
   785                 790                 795                 800
   Asn Lys Ile Asn Gln Arg Leu Ile Lys Glu Gln Gln Leu Gln Gln Pro
                   805                 810                 815
   Leu Phe Glu Phe Ser Gly Ala Cys Ser Gly Cys Gly Glu Thr Pro Tyr
               820                 825                 830
   Val Lys Leu Met Thr Gln Leu Phe Gly Asp Arg Leu Val Ile Gly Asn
           835                 840                 845
   Ala Thr Gly Cys Ser Ser Ile Tyr Gly Gly Asn Leu Pro Thr Thr Pro
       850                 855                 860
   Tyr Ala Ala Asn Pro Gln Gly Leu Gly Pro Thr Trp Ser Asn Ser Leu
   865                 870                 875                 880
   Phe Glu Asp Thr Ala Glu Phe Ala Leu Gly Phe Arg Ile Ser Ile Asp
                   885                 890                 895
   Lys Gln Gln Gln Phe Ala Lys Glu Leu Val Lys Lys Leu Ala Gly Asp
               900                 905                 910
   Ile Gly Glu Asn Leu Ala Thr Ala Ile Leu Asn Ala Thr Gln Asn Ser
           915                 920                 925
   Glu Pro Glu Ile Phe Glu Gln Arg Glu Arg Val Ala Val Leu Lys Asp
       930                 935                 940
   Lys Leu Gln Gln Met Lys Ser Asp Asp Ala Lys Asn Leu Leu Ala Val
   945                 950                 955                 960
   Ala Asp Met Leu Val Lys Lys Ser Val Trp Ala Val Gly Gly Asp Gly
                   965                 970                 975
   Trp Ala Tyr Asp Ile Gly Tyr Gly Gly Leu Asp His Val Thr Ala Ser
               980                 985                 990
   Gly Lys Asn Val Asn Met Leu Val  Leu Asp Thr Glu Val  Tyr Ser Asn
           995                 1000                1005
   Thr Gly  Gly Gln Ala Ser Lys  Ala Thr Pro Lys Ala  Ala Ile Ala
       1010                1015                1020
   Lys Phe  Ala Ala Ala Gly Arg  Ile Ala Thr Lys Lys  Asp Leu Gly
       1025                1030                1035
   Leu Ile  Ser Met Ser Tyr Gly  Asn Ala Tyr Val Ala  Ser Val Ala
       1040                1045                1050
   Leu Gly  Ala Arg Asp Glu Gln  Thr Leu Arg Ala Phe  Ile Glu Ala
       1055                1060                1065
   Glu Ala  Tyr Asp Gly Pro Ser  Ile Ile Ile Ala Tyr  Ser His Cys
       1070                1075                1080
   Ile Ala  His Gly Phe Asp Leu  Ser Met Gly Leu Glu  His Gln Lys
       1085                1090                1095
   Ala Ala  Val Asp Ser Gly His  Trp Leu Leu Tyr Arg  Tyr Asn Pro
       1100                1105                1110
   Asp Arg  Leu Lys Glu Gly Leu  Asn Pro Leu Gln Leu  Asp Ser Lys
       1115                1120                1125
   Lys Pro  Lys Met Pro Val Ala  Glu Phe Leu Asn Met  Glu Asn Arg
       1130                1135                1140
   Phe Arg  Ile Leu Lys Lys Thr  His Pro Asp Leu Ala  Lys Lys Tyr
       1145                1150                1155
   Phe Glu  Ala Ile Gln His Glu  Val Asn Ala Arg Trp  Ala His Tyr
       1160                1165                1170
   Glu His  Leu Ala Asn Arg Ser  Ile Glu Gly Glu Ala
       1175                1180                1185
<210> 3
   <211> 3525
   <212> DNA
   <213> Escherichia coli
<220>
   <221> CDS
   <222> (1)..(3525)
<400> 3
   atg att act att gac ggt aat ggc gcg gtt gct tcg gtc gca ttt cgc        48
   Met Ile Thr Ile Asp Gly Asn Gly Ala Val Ala Ser Val Ala Phe Arg
    1               5                   10                  15
   acc agt gaa gtt atc gcc atc tac cct att acc ccc agt tcc acg atg            96
   Thr Ser Glu Val Ile Ala Ile Tyr Pro Ile Thr Pro Ser Ser Thr Met
                20                  25                  30
   gca gaa cag gct gat gcc tgg gcc gga aac ggc tta aag aac gtt tgg           144
   Ala Glu Gln Ala Asp Ala Trp Ala Gly Asn Gly Leu Lys Asn Val Trp
            35                  40                  45
   gga gac aca cca cgc gtg gtt gaa atg cag tcg gaa gcg ggt gct atc           192
   Gly Asp Thr Pro Arg Val Val Glu Met Gln Ser Glu Ala Gly Ala Ile
        50                  55                  60
   gct acc gtg cat ggc gct ttg cag acg ggt gcc ctt tca aca tcg ttt           240
   Ala Thr Val His Gly Ala Leu Gln Thr Gly Ala Leu Ser Thr Ser Phe
    65                  70                  75                  80
   acg tca tcg cag ggt ttg ctg ctg atg atc ccg acg ctg tac aaa ctg           288
   Thr Ser Ser Gln Gly Leu Leu Leu Met Ile Pro Thr Leu Tyr Lys Leu
                    85                  90                  95
   gca ggc gaa cta aca ccg ttt gtc ctg cat gta gcg gca cgt acc gtt           336
   Ala Gly Glu Leu Thr Pro Phe Val Leu His Val Ala Ala Arg Thr Val
               100                 105                 110
   gcc aca cat gca ctc tct att ttt ggc gat cat tcc gac gtt atg gcg           384
   Ala Thr His Ala Leu Ser Ile Phe Gly Asp His Ser Asp Val Met Ala
           115                 120                 125
   gtg cgc cag acg ggt tgc gcg atg ttg tgt gca gca aac gtc cag gaa           432
   Val Arg Gln Thr Gly Cys Ala Met Leu Cys Ala Ala Asn Val Gln Glu
       130                 135                 140
   gcg caa gac ttt gct ctc att tcg caa atc gcg acg ctg aaa agc cgc           480
   Ala Gln Asp Phe Ala Leu Ile Ser Gln Ile Ala Thr Leu Lys Ser Arg
   145                 150                 155                 160
   gtg cca ttt att cat ttc ttt gat ggt ttc cgc acg tcc cac gaa atc           528
   Val Pro Phe Ile His Phe Phe Asp Gly Phe Arg Thr Ser His Glu Ile
                   165                 170                 175
   aat aaa att gtc ccg ctg gcc gat gac acg att ctt gat ctc atg ccg           576
   Asn Lys Ile Val Pro Leu Ala Asp Asp Thr Ile Leu Asp Leu Met Pro
               180                 185                 190
   cag gtc gaa att gat gct cat cgc gcc cgg gca ctc aac ccg gaa cat           624
   Gln Val Glu Ile Asp Ala His Arg Ala Arg Ala Leu Asn Pro Glu His
           195                 200                 205
   ccg gtg atc cgc ggt acg tcc gcc aat cct gac act tat ttc cag tct           672
   Pro Val Ile Arg Gly Thr Ser Ala Asn Pro Asp Thr Tyr Phe Gln Ser
       210                 215                 220
   cgc gaa gcc acc aac cca tgg tac aac gcg gtc tat gac cat gtt gaa           720
   Arg Glu Ala Thr Asn Pro Trp Tyr Asn Ala Val Tyr Asp His Val Glu
   225                 230                 235                 240
   cag gcg atg aat gat ttc tct gcc gcg aca ggt cgt cag tat cag ccg           768
   Gln Ala Met Asn Asp Phe Ser Ala Ala Thr Gly Arg Gln Tyr Gln Pro
                   245                 250                 255
   ttt gaa tat tac ggg cat ccg caa gcg gaa cgg gtg att atc ctg atg           816
   Phe Glu Tyr Tyr Gly His Pro Gln Ala Glu Arg Val Ile Ile Leu Met
               260                 265                 270
   ggc tct gcc att ggc acc tgt gaa gaa gtg gtt gat gaa ttg cta acc           864
   Gly Ser Ala Ile Gly Thr Cys Glu Glu Val Val Asp Glu Leu Leu Thr
           275                 280                 285
   cgt ggc gaa aaa gtt ggc gtg ctg aaa gtt cgc ctg tac cgc ccc ttc           912
   Arg Gly Glu Lys Val Gly Val Leu Lys Val Arg Leu Tyr Arg Pro Phe
       290                 295                 300
   tcc gct aaa cat tta ctg caa gct ctg ccg gga tcc gta cgc agc gtg           960
   Ser Ala Lys His Leu Leu Gln Ala Leu Pro Gly Ser Val Arg Ser Val
   305                 310                 315                 320
   gcg gta ctg gac aga acc aaa gaa ccc ggt gcc cag gca gaa ccg ctc          1008
   Ala Val Leu Asp Arg Thr Lys Glu Pro Gly Ala Gln Ala Glu Pro Leu
                   325                 330                 335
   tat ctg gat gta atg acc gca ctg gca gaa gcc ttt aat aat ggc gag      1056
   Tyr Leu Asp Val Met Thr Ala Leu Ala Glu Ala Phe Asn Asn Gly Glu
                   340                 345                 350
   cgc gaa act ctg ccc cgt gtc att ggt ggg cgc tat ggt ctt tca tcc      1104
   Arg Glu Thr Leu Pro Arg Val Ile Gly Gly Arg Tyr Gly Leu Ser Ser
           355                 360                 365
   aaa gaa ttt ggc cca gac tgt gta ctg gcg gta ttt gcc gag ctc aac      1152
   Lys Glu Phe Gly Pro Asp Cys Val Leu Ala Val Phe Ala Glu Leu Asn
       370                 375                 380
   gcg gct aaa ccg aaa gcg cgc ttt acg gtt ggt att tac gat gat gtg      1200
   Ala Ala Lys Pro Lys Ala Arg Phe Thr Val Gly Ile Tyr Asp Asp Val
   385                 390                 395                 400
   acc aat ctg tca ctg ccg ttg ccg gaa aac acc ctg cca aac tcg gcg      1248
   Thr Asn Leu Ser Leu Pro Leu Pro Glu Asn Thr Leu Pro Asn Ser Ala
                   405                 410                 415
   aaa ctg gaa gcc ttg ttt tat ggc ctt ggt agt gat ggc agc gtt tcc      1296
   Lys Leu Glu Ala Leu Phe Tyr Gly Leu Gly Ser Asp Gly Ser Val Ser
               420                 425                 430
   gcg acc aaa aac aat atc aag att atc ggt aat tcc acg ccg tgg tac      1344
   Ala Thr Lys Asn Asn Ile Lys Ile Ile Gly Asn Ser Thr Pro Trp Tyr
           435                 440                 445
   gca cag ggc tat ttt gtt tac gac tcc aaa aag gcg ggc ggc ctg acg      1392
   Ala Gln Gly Tyr Phe Val Tyr Asp Ser Lys Lys Ala Gly Gly Leu Thr
       450                 455                 460
   gtt tct cac ctt cga gtg agc gaa cag ccg att cgt tcc gct tat ctc      1440
   Val Ser His Leu Arg Val Ser Glu Gln Pro Ile Arg Ser Ala Tyr Leu
   465                 470                 475                 480
   att tcc cag gct gat ttt gtt ggc tgc cac cag ttg cag ttt atc gat      1488
   Ile Ser Gln Ala Asp Phe Val Gly Cys His Gln Leu Gln Phe Ile Asp
                   485                 490                 495
   aaa tat cag atg gct gag cgt tta aaa cct ggc ggc att ttc ctg ctc      1536
   Lys Tyr Gln Met Ala Glu Arg Leu Lys Pro Gly Gly Ile Phe Leu Leu
               500                 505                 510
   aac acg ccg tac agc gca gat gaa gtg tgg tcg cgc ttg ccg caa gaa      1584
   Asn Thr Pro Tyr Ser Ala Asp Glu Val Trp Ser Arg Leu Pro Gln Glu
           515                 520                 525
   gtt cag gcc gtg tta aac cag aaa aaa gcg cgc ttc tat gtg att aac      1632
   Val Gln Ala Val Leu Asn Gln Lys Lys Ala Arg Phe Tyr Val Ile Asn
       530                 535                 540
   gcg gcg aaa atc gcc cgc gaa tgt ggc ctg gcg gcc cgt att aat acc      1680
   Ala Ala Lys Ile Ala Arg Glu Cys Gly Leu Ala Ala Arg Ile Asn Thr
   545                 550                 555                 560
   gtc atg cag atg gct ttt ttc cat ctg acg caa att ctg cct ggc gat      1728
   Val Met Gln Met Ala Phe Phe His Leu Thr Gln Ile Leu Pro Gly Asp
                   565                 570                 575
   agc gcc ctc gca gaa ttg cag ggt gcg att gcc aaa agt tac agt agc      1776
   Ser Ala Leu Ala Glu Leu Gln Gly Ala Ile Ala Lys Ser Tyr Ser Ser
               580                 585                 590
   aaa ggc cag gat ctg gtg gaa cgc aac tgg cag gct ctg gcg ctg gcg      1824
   Lys Gly Gln Asp Leu Val Glu Arg Asn Trp Gln Ala Leu Ala Leu Ala
           595                 600                 605
   cgt gaa tcc gta gaa gaa gtt ccg ttg caa ccg gta aat ccg cac agc      1872
   Arg Glu Ser Val Glu Glu Val Pro Leu Gln Pro Val Asn Pro His Ser
       610                 615                 620
   gcc aat cga ccg cca gtg gtt tcc gat gcc gcc cct gat ttc gtg aaa      1920
   Ala Asn Arg Pro Pro Val Val Ser Asp Ala Ala Pro Asp Phe Val Lys
   625                 630                 635                 640
   acc gta acc gct gcg atg ctc gcc ggg ctt ggt gac gcc ctc ccc gtt      1968
   Thr Val Thr Ala Ala Met Leu Ala Gly Leu Gly Asp Ala Leu Pro Val
                   645                 650                 655
   tcg gcg ctg ccg cca gac ggc acc tgg ccg atg ggc act acg cgc tgg      2016
   Ser Ala Leu Pro Pro Asp Gly Thr Trp Pro Met Gly Thr Thr Arg Trp
                   660                 665                 670
   gaa aaa cgc aat atc gcc gaa gag atc ccc atc tgg aaa gag gaa ctc        2064
   Glu Lys Arg Asn Ile Ala Glu Glu Ile Pro Ile Trp Lys Glu Glu Leu
               675                 680                 685
   tgt acc caa tgt aac cac tgc gtt gcc gct tgc cca cac tca gct att        2112
   Cys Thr Gln Cys Asn His Cys Val Ala Ala Cys Pro His Ser Ala Ile
       690                 695                 700
   cgc gca aaa gtg gtg ccg cct gaa gcg atg gaa aac gcc cct gcc agc        2160
   Arg Ala Lys Val Val Pro Pro Glu Ala Met Glu Asn Ala Pro Ala Ser
   705                 710                 715                 720
   ctg cat tcg ctg gat gtg aaa tcg cgt gat atg cgc ggg cag aaa tat        2208
   Leu His Ser Leu Asp Val Lys Ser Arg Asp Met Arg Gly Gln Lys Tyr
                   725                 730                 735
   gtc ttg cag gtg gca ccg gaa gat tgc acc ggt tgt aac ctg tgc gtc        2256
   Val Leu Gln Val Ala Pro Glu Asp Cys Thr Gly Cys Asn Leu Cys Val
               740                 745                 750
   gaa gtt tgc ccg gcg aaa gac cgt cag aat cca gag att aaa gcc atc        2304
   Glu Val Cys Pro Ala Lys Asp Arg Gln Asn Pro Glu Ile Lys Ala Ile
           755                 760                 765
   aat atg atg tct cgc ctg gaa cat gtc gaa gaa gag aaa atc aat tac        2352
   Asn Met Met Ser Arg Leu Glu His Val Glu Glu Glu Lys Ile Asn Tyr
       770                 775                 780
   gat ttc ttc ctc aac ctg cca gaa atc gac cgt agc aaa ctg gaa cgt        2400
   Asp Phe Phe Leu Asn Leu Pro Glu Ile Asp Arg Ser Lys Leu Glu Arg
   785                 790                 795                 800
   att gat att cgt aca tcg cag ctg att aca ccg ctg ttt gaa tat tca        2448
   Ile Asp Ile Arg Thr Ser Gln Leu Ile Thr Pro Leu Phe Glu Tyr Ser
                   805                 810                 815
   ggt gct tgc tcc ggt tgt ggc gag acg ccg tat att aaa tta ctg act        2496
   Gly Ala Cys Ser Gly Cys Gly Glu Thr Pro Tyr Ile Lys Leu Leu Thr
               820                 825                 830
   cag ctc tat ggc gac cgg atg ttg atc gct aac gcc act ggc tgt tct        2544
   Gln Leu Tyr Gly Asp Arg Met Leu Ile Ala Asn Ala Thr Gly Cys Ser
           835                 840                 845
   tca att tat ggc ggt aac ctg ccc tct aca ccg tat acc acc gat gcc        2592
   Ser Ile Tyr Gly Gly Asn Leu Pro Ser Thr Pro Tyr Thr Thr Asp Ala
       850                 855                 860
   aac ggt cgt ggg ccg gca tgg gcg aac tct cta ttt gaa gat aat gcc        2640
   Asn Gly Arg Gly Pro Ala Trp Ala Asn Ser Leu Phe Glu Asp Asn Ala
   865                 870                 875                 880
   gaa ttt ggc ctt ggt ttc cgc ctg acg gtc gat caa cac cgt gtc cgc        2688
   Glu Phe Gly Leu Gly Phe Arg Leu Thr Val Asp Gln His Arg Val Arg
                   885                 890                 895
   gtg ctg cgt ctg ctg gat caa ttt gcc gat aaa atc ccg gcg gaa tta        2736
   Val Leu Arg Leu Leu Asp Gln Phe Ala Asp Lys Ile Pro Ala Glu Leu
               900                 905                 910
   ctg acg gcg ttg aaa tca gac gcc acg cca gag gtt cgt cgt gaa cag        2784
   Leu Thr Ala Leu Lys Ser Asp Ala Thr Pro Glu Val Arg Arg Glu Gln
           915                 920                 925
   gtt gca gct tta cgc cag caa ctc aac gat gtt gcc gaa gca cat gaa        2832
   Val Ala Ala Leu Arg Gln Gln Leu Asn Asp Val Ala Glu Ala His Glu
       930                 935                 940
   ctg cta cgt gat gca gat gca ctg gtg gaa aaa tca atc tgg ctg att        2880
   Leu Leu Arg Asp Ala Asp Ala Leu Val Glu Lys Ser Ile Trp Leu Ile
   945                 950                 955                 960
   ggt ggt gat ggc tgg gct tac gat atc ggc ttt ggc ggt ctg gat cat        2928
   Gly Gly Asp Gly Trp Ala Tyr Asp Ile Gly Phe Gly Gly Leu Asp His
                   965                 970                 975
   gta ttg agt ttg acg gaa aac gtc aac att ctg gtg ctg gat acg caa        2976
   Val Leu Ser Leu Thr Glu Asn Val Asn Ile Leu Val Leu Asp Thr Gln
               980                  985                  990
   tgc tat tcc aac acc ggt ggt cag gcg tcg aaa gcg aca ccg ctg ggt      3024
   Cys Tyr Ser Asn Thr Gly Gly Gln Ala Ser Lys Ala Thr Pro Leu Gly
           995                 1000                 1005
   gca gta act aaa ttt ggc gag cac ggc aaa cgt aaa gcg cgt aaa gat      3072
   Ala Val Thr Lys Phe Gly Glu His Gly Lys Arg Lys Ala Arg Lys Asp
      1010                 1015                 1020
   ctt ggc gtc agt atg atg atg tac ggt cat gtt tat gtg gcg cag att      3120
   Leu Gly Val Ser Met Met Met Tyr Gly His Val Tyr Val Ala Gln Ile
   1025                1030                 1035                1040
   tct ctc ggc gcg cag ctg aac cag acg gtg aaa gcg att cag gaa gcg      3168
   Ser Leu Gly Ala Gln Leu Asn Gln Thr Val Lys Ala Ile Gln Glu Ala
                  1045                 1050                 1055
   gaa gcg tat ccg ggg cca tcg ctg atc att gct tat agc ccg tgt gaa      3216
   Glu Ala Tyr Pro Gly Pro Ser Leu Ile Ile Ala Tyr Ser Pro Cys Glu
              1060                 1065                 1070
   gag cat ggt tac gat ctg gca ctc agc cac gac cag atg cgc caa ctc      3264
   Glu His Gly Tyr Asp Leu Ala Leu Ser His Asp Gln Met Arg Gln Leu
          1075                 1080                 1085
   aca gct acc ggc ttc tgg ccg cta tat cgc ttt gat ccg cgt cgt gcc      3312
   Thr Ala Thr Gly Phe Trp Pro Leu Tyr Arg Phe Asp Pro Arg Arg Ala
      1090                 1095                 1100
   gat gaa ggc aaa ctg ccg ctg gcc ttg gat tca cgc ccg ccg tca gaa      3360
   Asp Glu Gly Lys Leu Pro Leu Ala Leu Asp Ser Arg Pro Pro Ser Glu
   1105                1110                 1115                1120
   gca ccg gaa gaa acg tta ctt cac gag caa cgt ttc cgt cgg ctg aat      3408
   Ala Pro Glu Glu Thr Leu Leu His Glu Gln Arg Phe Arg Arg Leu Asn
                  1125                 1130                 1135
   tcg cag cag cca gaa gtg gca gaa cag tta tgg aaa gat gct gca gct      3456
   Ser Gln Gln Pro Glu Val Ala Glu Gln Leu Trp Lys Asp Ala Ala Ala
              1140                 1145                 1150
   gat ttg caa aaa cgc tat gac ttc ctg gca caa atg gcc gga aaa gcg      3504
   Asp Leu Gln Lys Arg Tyr Asp Phe Leu Ala Gln Met Ala Gly Lys Ala
          1155                 1160                 1165
   gaa aaa agc aac acc gat taa                                          3525
   Glu Lys Ser Asn Thr Asp
      1170
<210> 4
   <211> 1174
   <212> PRT
   <213> Escherichia coli
<400> 4
   Met Ile Thr Ile Asp Gly Asn Gly Ala Val Ala Ser Val Ala Phe Arg
   1               5                   10                  15
   Thr Ser Glu Val Ile Ala Ile Tyr Pro Ile Thr Pro Ser Ser Thr Met
               20                  25                  30
   Ala Glu Gln Ala Asp Ala Trp Ala Gly Asn Gly Leu Lys Asn Val Trp
           35                  40                  45
   Gly Asp Thr Pro Arg Val Val Glu Met Gln Ser Glu Ala Gly Ala Ile
       50                  55                  60
   Ala Thr Val His Gly Ala Leu Gln Thr Gly Ala Leu Ser Thr Ser Phe
   65                  70                  75                  80
   Thr Ser Ser Gln Gly Leu Leu Leu Met Ile Pro Thr Leu Tyr Lys Leu
                   85                  90                  95
   Ala Gly Glu Leu Thr Pro Phe Val Leu His Val Ala Ala Arg Thr Val
               100                 105                 110
   Ala Thr His Ala Leu Ser Ile Phe Gly Asp His Ser Asp Val Met Ala
           115                 120                 125
   Val Arg Gln Thr Gly Cys Ala Met Leu Cys Ala Ala Asn Val Gln Glu
       130                 135                 140
   Ala Gln Asp Phe Ala Leu Ile Ser Gln Ile Ala Thr Leu Lys Ser Arg
   145                 150                 155                 160
   Val Pro Phe Ile His Phe Phe Asp Gly Phe Arg Thr Ser His Glu Ile
                   165                 170                 175
   Asn Lys Ile Val Pro Leu Ala Asp Asp Thr Ile Leu Asp Leu Met Pro
               180                 185                 190
   Gln Val Glu Ile Asp Ala His Arg Ala Arg Ala Leu Asn Pro Glu His
           195                 200                 205
   Pro Val Ile Arg Gly Thr Ser Ala Asn Pro Asp Thr Tyr Phe Gln Ser
       210                 215                 220
   Arg Glu Ala Thr Asn Pro Trp Tyr Asn Ala Val Tyr Asp His Val Glu
   225                 230                 235                 240
   Gln Ala Met Asn Asp Phe Ser Ala Ala Thr Gly Arg Gln Tyr Gln Pro
                   245                 250                 255
   Phe Glu Tyr Tyr Gly His Pro Gln Ala Glu Arg Val Ile Ile Leu Met
               260                 265                 270
   Gly Ser Ala Ile Gly Thr Cys Glu Glu Val Val Asp Glu Leu Leu Thr
           275                 280                 285
   Arg Gly Glu Lys Val Gly Val Leu Lys Val Arg Leu Tyr Arg Pro Phe
       290                 295                 300
   Ser Ala Lys His Leu Leu Gln Ala Leu Pro Gly Ser Val Arg Ser Val
   305                 310                 315                 320
   Ala Val Leu Asp Arg Thr Lys Glu Pro Gly Ala Gln Ala Glu Pro Leu
                   325                 330                 335
   Tyr Leu Asp Val Met Thr Ala Leu Ala Glu Ala Phe Asn Asn Gly Glu
               340                 345                 350
   Arg Glu Thr Leu Pro Arg Val Ile Gly Gly Arg Tyr Gly Leu Ser Ser
           355                 360                 365
   Lys Glu Phe Gly Pro Asp Cys Val Leu Ala Val Phe Ala Glu Leu Asn
       370                 375                 380
   Ala Ala Lys Pro Lys Ala Arg Phe Thr Val Gly Ile Tyr Asp Asp Val
   385                 390                 395                 400
   Thr Asn Leu Ser Leu Pro Leu Pro Glu Asn Thr Leu Pro Asn Ser Ala
                   405                 410                 415
   Lys Leu Glu Ala Leu Phe Tyr Gly Leu Gly Ser Asp Gly Ser Val Ser
               420                 425                 430
   Ala Thr Lys Asn Asn Ile Lys Ile Ile Gly Asn Ser Thr Pro Trp Tyr
           435                 440                 445
   Ala Gln Gly Tyr Phe Val Tyr Asp Ser Lys Lys Ala Gly Gly Leu Thr
       450                 455                 460
   Val Ser His Leu Arg Val Ser Glu Gln Pro Ile Arg Ser Ala Tyr Leu
   465                 470                 475                 480
   Ile Ser Gln Ala Asp Phe Val Gly Cys His Gln Leu Gln Phe Ile Asp
                   485                 490                 495
   Lys Tyr Gln Met Ala Glu Arg Leu Lys Pro Gly Gly Ile Phe Leu Leu
               500                 505                 510
   Asn Thr Pro Tyr Ser Ala Asp Glu Val Trp Ser Arg Leu Pro Gln Glu
           515                 520                 525
   Val Gln Ala Val Leu Asn Gln Lys Lys Ala Arg Phe Tyr Val Ile Asn
       530                 535                 540
   Ala Ala Lys Ile Ala Arg Glu Cys Gly Leu Ala Ala Arg Ile Asn Thr
   545                 550                 555                 560
   Val Met Gln Met Ala Phe Phe His Leu Thr Gln Ile Leu Pro Gly Asp
                   565                 570                 575
   Ser Ala Leu Ala Glu Leu Gln Gly Ala Ile Ala Lys Ser Tyr Ser Ser
               580                 585                 590
   Lys Gly Gln Asp Leu Val Glu Arg Asn Trp Gln Ala Leu Ala Leu Ala
           595                 600                 605
   Arg Glu Ser Val Glu Glu Val Pro Leu Gln Pro Val Asn Pro His Ser
       610                 615                 620
   Ala Asn Arg Pro Pro Val Val Ser Asp Ala Ala Pro Asp Phe Val Lys
   625                 630                 635                 640
   Thr Val Thr Ala Ala Met Leu Ala Gly Leu Gly Asp Ala Leu Pro Val
                   645                 650                 655
   Ser Ala Leu Pro Pro Asp Gly Thr Trp Pro Met Gly Thr Thr Arg Trp
               660                 665                 670
   Glu Lys Arg Asn Ile Ala Glu Glu Ile Pro Ile Trp Lys Glu Glu Leu
           675                 680                 685
   Cys Thr Gln Cys Asn His Cys Val Ala Ala Cys Pro His Ser Ala Ile
       690                 695                 700
   Arg Ala Lys Val Val Pro Pro Glu Ala Met Glu Asn Ala Pro Ala Ser
   705                 710                 715                 720
   Leu His Ser Leu Asp Val Lys Ser Arg Asp Met Arg Gly Gln Lys Tyr
                   725                 730                 735
   Val Leu Gln Val Ala Pro Glu Asp Cys Thr Gly Cys Asn Leu Cys Val
               740                 745                 750
   Glu Val Cys Pro Ala Lys Asp Arg Gln Asn Pro Glu Ile Lys Ala Ile
           755                 760                 765
   Asn Met Met Ser Arg Leu Glu His Val Glu Glu Glu Lys Ile Asn Tyr
       770                 775                 780
   Asp Phe Phe Leu Asn Leu Pro Glu Ile Asp Arg Ser Lys Leu Glu Arg
   785                 790                 795                 800
   Ile Asp Ile Arg Thr Ser Gln Leu Ile Thr Pro Leu Phe Glu Tyr Ser
                   805                 810                 815
   Gly Ala Cys Ser Gly Cys Gly Glu Thr Pro Tyr Ile Lys Leu Leu Thr
               820                 825                 830
   Gln Leu Tyr Gly Asp Arg Met Leu Ile Ala Asn Ala Thr Gly Cys Ser
           835                 840                 845
   Ser Ile Tyr Gly Gly Asn Leu Pro Ser Thr Pro Tyr Thr Thr Asp Ala
       850                 855                 860
   Asn Gly Arg Gly Pro Ala Trp Ala Asn Ser Leu Phe Glu Asp Asn Ala
   865                 870                 875                 880
   Glu Phe Gly Leu Gly Phe Arg Leu Thr Val Asp Gln His Arg Val Arg
                   885                 890                 895
   Val Leu Arg Leu Leu Asp Gln Phe Ala Asp Lys Ile Pro Ala Glu Leu
               900                 905                 910
   Leu Thr Ala Leu Lys Ser Asp Ala Thr Pro Glu Val Arg Arg Glu Gln
           915                 920                 925
   Val Ala Ala Leu Arg Gln Gln Leu Asn Asp Val Ala Glu Ala His Glu
       930                 935                 940
   Leu Leu Arg Asp Ala Asp Ala Leu Val Glu Lys Ser Ile Trp Leu Ile
   945                 950                 955                 960
   Gly Gly Asp Gly Trp Ala Tyr Asp Ile Gly Phe Gly Gly Leu Asp His
                   965                 970                 975
   Val Leu Ser Leu Thr Glu Asn Val Asn Ile Leu Val Leu Asp Thr Gln
               980                 985                 990
   Cys Tyr Ser Asn Thr Gly Gly Gln  Ala Ser Lys Ala Thr  Pro Leu Gly
           995                 1000                1005
   Ala Val  Thr Lys Phe Gly Glu  His Gly Lys Arg Lys  Ala Arg Lys
       1010                1015                1020
   Asp Leu  Gly Val Ser Met Met  Met Tyr Gly His Val  Tyr Val Ala
       1025                1030                1035
   Gln Ile  Ser Leu Gly Ala Gln  Leu Asn Gln Thr Val  Lys Ala Ile
       1040                1045                1050
   Gln Glu  Ala Glu Ala Tyr Pro  Gly Pro Ser Leu Ile  Ile Ala Tyr
       1055                1060                1065
   Ser Pro  Cys Glu Glu His Gly  Tyr Asp Leu Ala Leu  Ser His Asp
       1070                1075                1080
   Gln Met  Arg Gln Leu Thr Ala  Thr Gly Phe Trp Pro  Leu Tyr Arg
       1085                1090                1095
   Phe Asp  Pro Arg Arg Ala Asp  Glu Gly Lys Leu Pro  Leu Ala Leu
       1100                 1105                 1110
   Asp Ser  Arg Pro Pro Ser Glu  Ala Pro Glu Glu Thr  Leu Leu His
       1115                 1120                 1125
   Glu Gln  Arg Phe Arg Arg Leu  Asn Ser Gln Gln Pro  Glu Val Ala
       1130                 1135                 1140
   Glu Gln  Leu Trp Lys Asp Ala  Ala Ala Asp Leu Gln  Lys Arg Tyr
       1145                 1150                 1155
   Asp Phe  Leu Ala Gln Met Ala  Gly Lys Ala Glu Lys  Ser Asn Thr
       1160                 1165                 1170
   Asp
<210> 5
   <211> 5412
   <212> DNA
   <213> Euglena gracilis
<220>
   <221> CDS
   <222> (1)..(5412)
<400> 5
   atg aag cag tct gtc cgc cca att att tcc aat gta ctg cgc aag gag        48
   Met Lys Gln Ser Val Arg Pro Ile Ile Ser Asn Val Leu Arg Lys Glu
    1               5                  10                  15
   gtt gct ctg tac tca aca atc att gga caa gac aag ggg aag gaa cca        96
   Val Ala Leu Tyr Ser Thr Ile Ile Gly Gln Asp Lys Gly Lys Glu Pro
               20                  25                  30
   act ggt cga aca tac acc agt ggc cca aaa ccg gca tct cac att gaa       144
   Thr Gly Arg Thr Tyr Thr Ser Gly Pro Lys Pro Ala Ser His Ile Glu
           35                  40                  45
   gtt ccc cat cat gtg act gtg cct gcc act gac cgc acc ccg aat ccc       192
   Val Pro His His Val Thr Val Pro Ala Thr Asp Arg Thr Pro Asn Pro
       50                  55                  60
   gat gct caa ttc ttt cag tct gta gat ggg tca caa gcc acc agt cac       240
   Asp Ala Gln Phe Phe Gln Ser Val Asp Gly Ser Gln Ala Thr Ser His
   65                  70                  75                  80
   gtt gcg tac gct ctg tct gac aca gcg ttc att tac cca att aca ccc       288
   Val Ala Tyr Ala Leu Ser Asp Thr Ala Phe Ile Tyr Pro Ile Thr Pro
                   85                  90                  95
   agt tct gtg atg ggc gag ctg gct gat gtt tgg atg gct caa ggg agg       336
   Ser Ser Val Met Gly Glu Leu Ala Asp Val Trp Met Ala Gln Gly Arg
               100                 105                 110
   aag aac gcc ttt ggt cag gtt gtg gat gtc cgt gag atg caa tct gag       384
   Lys Asn Ala Phe Gly Gln Val Val Asp Val Arg Glu Met Gln Ser Glu
           115                 120                 125
   gct gga gcc gca ggc gcc ctg cat ggg gca ctg gct gct gga gct att       432
   Ala Gly Ala Ala Gly Ala Leu His Gly Ala Leu Ala Ala Gly Ala Ile
       130                 135                 140
   gct aca acc ttc act gcc tct caa ggg ttg ttg ttg atg att ccc aac       480
   Ala Thr Thr Phe Thr Ala Ser Gln Gly Leu Leu Leu Met Ile Pro Asn
   145                 150                 155                 160
   atg tat aag att gca ggt gag ctg atg ccc tct gtc atc cac gtt gca       528
   Met Tyr Lys Ile Ala Gly Glu Leu Met Pro Ser Val Ile His Val Ala
                   165                 170                 175
   gcc cga gag ctt gca ggc cac gct ctg tcc att ttt gga gga cac gct       576
   Ala Arg Glu Leu Ala Gly His Ala Leu Ser Ile Phe Gly Gly His Ala
               180                 185                 190
   gat gtc atg gct gtc cgc caa aca gga tgg gct atg ctg tgc tcc cac       624
   Asp Val Met Ala Val Arg Gln Thr Gly Trp Ala Met Leu Cys Ser His
           195                 200                 205
   aca gtg cag cag tct cac gac atg gct ctc atc tcc cac gtg gcc acc      672
   Thr Val Gln Gln Ser His Asp Met Ala Leu Ile Ser His Val Ala Thr
       210                 215                 220
   ctc aag tcc agc atc ccc ttc gtt cac ttc ttt gat ggt ttc cgc aca      720
   Leu Lys Ser Ser Ile Pro Phe Val His Phe Phe Asp Gly Phe Arg Thr
   225                 230                 235                 240
   agc cac gaa gtg aac aaa atc aaa atg ctg cct tat gca gaa ctg aag      768
   Ser His Glu Val Asn Lys Ile Lys Met Leu Pro Tyr Ala Glu Leu Lys
                   245                 250                 255
   aaa ctg gtg cct cct ggc acc atg gaa cag cac tgg gct cgt tcg ctg      816
   Lys Leu Val Pro Pro Gly Thr Met Glu Gln His Trp Ala Arg Ser Leu
               260                 265                 270
   aac ccc atg cac ccc acc atc cga gga aca aac cag tct gca gac atc      864
   Asn Pro Met His Pro Thr Ile Arg Gly Thr Asn Gln Ser Ala Asp Ile
           275                 280                 285
   tac ttc cag aat atg gaa agt gca aac cag tac tac act gat ctg gcc      912
   Tyr Phe Gln Asn Met Glu Ser Ala Asn Gln Tyr Tyr Thr Asp Leu Ala
       290                 295                 300
   gag gtc gtt cag gag aca atg gac gaa gtt gca cca tac atc ggt cgc      960
   Glu Val Val Gln Glu Thr Met Asp Glu Val Ala Pro Tyr Ile Gly Arg
   305                 310                 315                 320
   cac tac aag atc ttt gag tat gtt ggt gca cca gat gca gaa gaa gtg     1008
   His Tyr Lys Ile Phe Glu Tyr Val Gly Ala Pro Asp Ala Glu Glu Val
                   325                 330                 335
   aca gtg ctc atg ggt tct ggt gca acc aca gtc aac gag gca gtg gac     1056
   Thr Val Leu Met Gly Ser Gly Ala Thr Thr Val Asn Glu Ala Val Asp
               340                 345                 350
   ctt ctt gtg aag cgt gga aag aag gtt ggt gca gtc ttg gtg cac ctc     1104
   Leu Leu Val Lys Arg Gly Lys Lys Val Gly Ala Val Leu Val His Leu
           355                 360                 365
   tac cga cca tgg tca aca aag gca ttt gaa aag gtc ctg ccc aag aca     1152
   Tyr Arg Pro Trp Ser Thr Lys Ala Phe Glu Lys Val Leu Pro Lys Thr
       370                 375                 380
   gtg aag cgc att gct gct ctg gat cgc tgc aag gag gtg act gca ctg     1200
   Val Lys Arg Ile Ala Ala Leu Asp Arg Cys Lys Glu Val Thr Ala Leu
   385                 390                 395                 400
   ggt gag cct ctg tat ctg gat gtg tcg gca act ctg aat ttg ttc ccg     1248
   Gly Glu Pro Leu Tyr Leu Asp Val Ser Ala Thr Leu Asn Leu Phe Pro
                   405                 410                 415
   gaa cgc cag aat gtg aaa gtc att gga gga cgt tac gga ttg ggc tca     1296
   Glu Arg Gln Asn Val Lys Val Ile Gly Gly Arg Tyr Gly Leu Gly Ser
               420                 425                 430
   aag gat ttc atc ccg gag cat gcc ctg gca att tac gcc aac ttg gcc     1344
   Lys Asp Phe Ile Pro Glu His Ala Leu Ala Ile Tyr Ala Asn Leu Ala
           435                 440                 445
   agc gag aac ccc att caa aga ttc act gtg ggt atc aca gat gat gtc     1392
   Ser Glu Asn Pro Ile Gln Arg Phe Thr Val Gly Ile Thr Asp Asp Val
       450                 455                 460
   act ggc aca tcc gtt cct ttc gtc aac gag cgt gtt gac acg ttg ccc     1440
   Thr Gly Thr Ser Val Pro Phe Val Asn Glu Arg Val Asp Thr Leu Pro
   465                 470                 475                 480
   gag ggc acc cgc cag tgt gtc ttc tgg gga att ggt tca gat gga aca     1488
   Glu Gly Thr Arg Gln Cys Val Phe Trp Gly Ile Gly Ser Asp Gly Thr
                   485                 490                 495
   gtg gga gcc aat cgc tct gcc gtg aga atc att gga gac aac agc gat     1536
   Val Gly Ala Asn Arg Ser Ala Val Arg Ile Ile Gly Asp Asn Ser Asp
               500                 505                 510
   ttg atg gtt cag gcc tac ttc caa ttt gat gct ttc aag tca ggt ggt     1584
   Leu Met Val Gln Ala Tyr Phe Gln Phe Asp Ala Phe Lys Ser Gly Gly
           515                 520                 525
   gtc act tcc tcg cat ctc cgt ttt gga cca aag ccc atc aca gcg caa        1632
   Val Thr Ser Ser His Leu Arg Phe Gly Pro Lys Pro Ile Thr Ala Gln
       530                 535                 540
   tac ctt gtt acc aat gct gac tac atc gcg tgc cac ttc cag gag tat        1680
   Tyr Leu Val Thr Asn Ala Asp Tyr Ile Ala Cys His Phe Gln Glu Tyr
   545                 550                 555                 560
   gtc aag cgc ttt gac atg ctt gat gcc atc cgt gag ggg ggc acc ttt        1728
   Val Lys Arg Phe Asp Met Leu Asp Ala Ile Arg Glu Gly Gly Thr Phe
                   565                 570                 575
   gtt ctc aat tct cgg tgg acc acg gag gac atg gag aag gag att ccg        1776
   Val Leu Asn Ser Arg Trp Thr Thr Glu Asp Met Glu Lys Glu Ile Pro
               580                 585                 590
   gct gac ttc cgg cgc aag ctg gca cag aag aag gtc cgc ttc tac aat        1824
   Ala Asp Phe Arg Arg Lys Leu Ala Gln Lys Lys Val Arg Phe Tyr Asn
           595                 600                 605
   gtg gat gct cga aag atc tgt gac agt ttt ggt ctt ggg aag cgc atc        1872
   Val Asp Ala Arg Lys Ile Cys Asp Ser Phe Gly Leu Gly Lys Arg Ile
       610                 615                 620
   aat atg ctg atg cag gct tgt ttc ttc aag ctg tct ggg gtg ctc cca        1920
   Asn Met Leu Met Gln Ala Cys Phe Phe Lys Leu Ser Gly Val Leu Pro
   625                 630                 635                 640
   ctg gcc gaa gct cag cgg ctg ctg aac gag tcc att gtg cat gag tat        1968
   Leu Ala Glu Ala Gln Arg Leu Leu Asn Glu Ser Ile Val His Glu Tyr
                   645                 650                 655
   gga aag aag ggt ggc aag gtg gtg gag atg aac caa gca gtg gtg aat        2016
   Gly Lys Lys Gly Gly Lys Val Val Glu Met Asn Gln Ala Val Val Asn
               660                 665                 670
   gct gtc ttt gct ggt gac ctg ccc cag gaa gtt caa gtc cct gcc gcc        2064
   Ala Val Phe Ala Gly Asp Leu Pro Gln Glu Val Gln Val Pro Ala Ala
           675                 680                 685
   tgg gca aac gca gtt gat aca tcc acc cgt acc ccc acc ggg att gag        2112
   Trp Ala Asn Ala Val Asp Thr Ser Thr Arg Thr Pro Thr Gly Ile Glu
       690                 695                 700
   ttt gtt gac aag atc atg cgc ccg ctg atg gat ttc aag ggt gac cag        2160
   Phe Val Asp Lys Ile Met Arg Pro Leu Met Asp Phe Lys Gly Asp Gln
   705                 710                 715                 720
   ctc cca gtc agt gtg atg act cct ggt gga acc ttc cct gtc ggg aca        2208
   Leu Pro Val Ser Val Met Thr Pro Gly Gly Thr Phe Pro Val Gly Thr
                   725                 730                 735
   aca cag tat gcc aag cgt gca att gct gct ttc att ccc cag tgg att        2256
   Thr Gln Tyr Ala Lys Arg Ala Ile Ala Ala Phe Ile Pro Gln Trp Ile
               740                 745                 750
   cct gcc aac tgc aca cag tgc aac tat tgt tcg tat gtt tgc ccc cac        2304
   Pro Ala Asn Cys Thr Gln Cys Asn Tyr Cys Ser Tyr Val Cys Pro His
           755                 760                 765
   gcc acc atc cga cct ttc gtg ctg aca gac cag gag gtg cag ctg gcc        2352
   Ala Thr Ile Arg Pro Phe Val Leu Thr Asp Gln Glu Val Gln Leu Ala
       770                 775                 780
   ccg gag agc ttt gtg aca cgc aag gcg aag ggt gat tac cag ggg atg        2400
   Pro Glu Ser Phe Val Thr Arg Lys Ala Lys Gly Asp Tyr Gln Gly Met
   785                 790                 795                 800
   aat ttc cgc atc caa gtt gct cct gag gat tgc act ggc tgc cag gtg        2448
   Asn Phe Arg Ile Gln Val Ala Pro Glu Asp Cys Thr Gly Cys Gln Val
                   805                 810                 815
   tgc gtg gag acg tgc ccc gat gat gcc ctg gag atg acc gac gct ttc        2496
   Cys Val Glu Thr Cys Pro Asp Asp Ala Leu Glu Met Thr Asp Ala Phe
               820                 825                 830
   acc gcc acc cct gtg caa cgc acc aac tgg gag ttc gcc atc aag gtg        2544
   Thr Ala Thr Pro Val Gln Arg Thr Asn Trp Glu Phe Ala Ile Lys Val
           835                 840                 845
   ccc aac cgc ggc acc atg acg gac cgc tac tcc ctg aag ggc agc cag        2592
   Pro Asn Arg Gly Thr Met Thr Asp Arg Tyr Ser Leu Lys Gly Ser Gln
       850                 855                 860
   ttc cag cag ccc ctc ctg gag ttc tcc ggg gcc tgc gag ggc tgc ggc       2640
   Phe Gln Gln Pro Leu Leu Glu Phe Ser Gly Ala Cys Glu Gly Cys Gly
   865                 870                 875                 880
   gag acc cca tat gtc aag ctg ctc acc cag ctc ttc ggc gag cgg acg       2688
   Glu Thr Pro Tyr Val Lys Leu Leu Thr Gln Leu Phe Gly Glu Arg Thr
                   885                 890                 895
   gtc atc gcc aac gcc acc ggc tgc agt tcc atc tgg ggt ggc act gcc       2736
   Val Ile Ala Asn Ala Thr Gly Cys Ser Ser Ile Trp Gly Gly Thr Ala
               900                 905                 910
   ggc ctg gcg ccg tac acc acc aac gcc aag ggc cag ggc ccg gcc tgg       2784
   Gly Leu Ala Pro Tyr Thr Thr Asn Ala Lys Gly Gln Gly Pro Ala Trp
           915                 920                 925
   ggc aac agc ctg ttc gag gac aac gcc gag ttc ggc ttt ggc att gca       2832
   Gly Asn Ser Leu Phe Glu Asp Asn Ala Glu Phe Gly Phe Gly Ile Ala
       930                 935                 940
   gtg gcc aac gcc cag aag agg tcc cgc gtg agg gac tgc atc ctg cag       2880
   Val Ala Asn Ala Gln Lys Arg Ser Arg Val Arg Asp Cys Ile Leu Gln
   945                 950                 955                 960
   gca gtg gag aag aag gtc gcc gat gag ggt ttg acc aca ttg ttg gcg       2928
   Ala Val Glu Lys Lys Val Ala Asp Glu Gly Leu Thr Thr Leu Leu Ala
                   965                 970                 975
   caa tgg ctg cag gat tgg aac aca gga gac aag acc ttg aag tac caa       2976
   Gln Trp Leu Gln Asp Trp Asn Thr Gly Asp Lys Thr Leu Lys Tyr Gln
               980                 985                 990
   gac cag atc att gca ggg ctg gca cag cag cgc agc aag gat ccc ctt       3024
   Asp Gln Ile Ile Ala Gly Leu Ala Gln Gln Arg Ser Lys Asp Pro Leu
           995                 1000                1005
   ctg gag cag atc tat ggc atg aag gac atg ctg cct aac atc agc cag       3072
   Leu Glu Gln Ile Tyr Gly Met Lys Asp Met Leu Pro Asn Ile Ser Gln
      1010                1015                1020
   tgg atc att ggt ggt gat ggc tgg gcc aac gac att ggt ttc ggt ggg       3120
   Trp Ile Ile Gly Gly Asp Gly Trp Ala Asn Asp Ile Gly Phe Gly Gly
   1025                1030                1035                1040
   ctg gac cac gtg ctg gcc tct ggg cag aac ctc aac gtc ctg gtg ctg       3168
   Leu Asp His Val Leu Ala Ser Gly Gln Asn Leu Asn Val Leu Val Leu
                  1045                1050                1055
   gac acc gag atg tac agc aac acc ggt ggg cag gcc tcc aag tcc acc       3216
   Asp Thr Glu Met Tyr Ser Asn Thr Gly Gly Gln Ala Ser Lys Ser Thr
              1060                1065                1070
   cac atg gcc tct gtg gcc aag ttt gcc ctg gga ggg aag cgc acc aac       3264
   His Met Ala Ser Val Ala Lys Phe Ala Leu Gly Gly Lys Arg Thr Asn
          1075                1080                1085
   aag aag aac ttg acg gag atg gca atg agc tat ggc aac gtc tat gtg       3312
   Lys Lys Asn Leu Thr Glu Met Ala Met Ser Tyr Gly Asn Val Tyr Val
      1090                1095                1100
   gcc acc gtc tcc cat ggc aac atg gcc cag tgc gtc aag gcg ttt gtg       3360
   Ala Thr Val Ser His Gly Asn Met Ala Gln Cys Val Lys Ala Phe Val
   1105                1110                1115                1120
   gag gct gag tct tat gat gga cct tcg ctc att gtt ggc tat gcg cca       3408
   Glu Ala Glu Ser Tyr Asp Gly Pro Ser Leu Ile Val Gly Tyr Ala Pro
                  1125                1130                1135
   tgc atc gag cat ggt ctg cgt gct ggt atg gca agg atg gtt caa gag       3456
   Cys Ile Glu His Gly Leu Arg Ala Gly Met Ala Arg Met Val Gln Glu
              1140                1145                1150
   tct gag gct gcc atc gcc acg gga tac tgg ccc ctg tac cgc ttt gac       3504
   Ser Glu Ala Ala Ile Ala Thr Gly Tyr Trp Pro Leu Tyr Arg Phe Asp
          1155                1160                1165
   ccc cgc ctg gcg acc gag ggc aag aac ccc ttc cag ctg gac tcc aag       3552
   Pro Arg Leu Ala Thr Glu Gly Lys Asn Pro Phe Gln Leu Asp Ser Lys
      1170                1175                1180
   cgc atc aag ggc aac ctg cag gag tac ctg gac cgc cag aac cgg tat         3600
   Arg Ile Lys Gly Asn Leu Gln Glu Tyr Leu Asp Arg Gln Asn Arg Tyr
   1185                1190                1195                1200
   gtc aac ctg aag aag aac aac ccg aag ggt gcg gat ctg ctg aag tct         3648
   Val Asn Leu Lys Lys Asn Asn Pro Lys Gly Ala Asp Leu Leu Lys Ser
                   1205                1210                1215
   cag atg gcc gac aac atc acc gcc cgg ttc aac cgc tac cga cgc atg         3696
   Gln Met Ala Asp Asn Ile Thr Ala Arg Phe Asn Arg Tyr Arg Arg Met
               1220                1225                1230
   ttg gag ggc ccc aat aca aaa gcc gcc gcc ccc agc ggc aac cat gtg         3744
   Leu Glu Gly Pro Asn Thr Lys Ala Ala Ala Pro Ser Gly Asn His Val
           1235                1240                1245
   acc atc ctg tac ggc tcc gaa act ggc aac agt gag ggt ctg gca aag         3792
   Thr Ile Leu Tyr Gly Ser Glu Thr Gly Asn Ser Glu Gly Leu Ala Lys
      1250                1255                1260
   gag ctg gcc acc gac ttc gag cgc cgg gag tac tcc gtc gca gtg cag         3840
   Glu Leu Ala Thr Asp Phe Glu Arg Arg Glu Tyr Ser Val Ala Val Gln
   1265                1270                1275                1280
   gct ttg gat gac atc gac gtt gct gac ttg gag aac atg ggc ttc gtg         3888
   Ala Leu Asp Asp Ile Asp Val Ala Asp Leu Glu Asn Met Gly Phe Val
                   1285                1290                1295
   gtc att gcg gtg tcc acc tgt ggg cag gga cag ttc ccc cgc aac agc         3936
   Val Ile Ala Val Ser Thr Cys Gly Gln Gly Gln Phe Pro Arg Asn Ser
               1300                1305                1310
   cag ctg ttc tgg cgg gag ctg cag cgg gac aag cct gag ggc tgg ctg         3984
   Gln Leu Phe Trp Arg Glu Leu Gln Arg Asp Lys Pro Glu Gly Trp Leu
           1315                1320                1325
   aag aac ttg aag tac act gtc ttc ggg ctg ggc gac agc aca tac tac         4032
   Lys Asn Leu Lys Tyr Thr Val Phe Gly Leu Gly Asp Ser Thr Tyr Tyr
      1330                1335                1340
   ttc tac tgc cac acc gcc aag cag atc gac gct cgc ctg gcc gcc ttg         4080
   Phe Tyr Cys His Thr Ala Lys Gln Ile Asp Ala Arg Leu Ala Ala Leu
   1345                1350                1355                1360
   ggc gct cag cgg gtg gtg ccc att ggc ttc ggc gac gat ggg gat gag         4128
   Gly Ala Gln Arg Val Val Pro Ile Gly Phe Gly Asp Asp Gly Asp Glu
                   1365                1370                1375
   gac atg ttc cac acc ggc ttc aac aac tgg atc ccc agt gtg tgg aat         4176
   Asp Met Phe His Thr Gly Phe Asn Asn Trp Ile Pro Ser Val Trp Asn
               1380                1385                1390
   gag ctc aag acc aag act ccg gag gaa gcg ctg ttc acc ccg agc atc         4224
   Glu Leu Lys Thr Lys Thr Pro Glu Glu Ala Leu Phe Thr Pro Ser Ile
           1395                1400                1405
   gcc gtg cag ctc acc ccc aac gcc acc ccg cag gat ttc cat ttc gcc         4272
   Ala Val Gln Leu Thr Pro Asn Ala Thr Pro Gln Asp Phe His Phe Ala
      1410                1415                1420
   aag tcc acc cca gtg ctg tcc atc acc ggt gcc gaa cgc atc acg ccg         4320
   Lys Ser Thr Pro Val Leu Ser Ile Thr Gly Ala Glu Arg Ile Thr Pro
   1425                1430                1435                1440
   gca gac cac acc cgc aac ttc gtc act atc cga tgg aag acc gat ttg         4368
   Ala Asp His Thr Arg Asn Phe Val Thr Ile Arg Trp Lys Thr Asp Leu
                   1445                1450                1455
   tcg tac cag gtg ggt gac tct ctt ggt gtc ttc cct gag aac acc cgg         4416
   Ser Tyr Gln Val Gly Asp Ser Leu Gly Val Phe Pro Glu Asn Thr Arg
               1460                1465                1470
   tca gtg gtg gag gag ttc ctg cag tat tac ggc ttg aac ccc aag gac         4464
   Ser Val Val Glu Glu Phe Leu Gln Tyr Tyr Gly Leu Asn Pro Lys Asp
           1475                1480                1485
   gtc atc acc atc gaa aac aag ggc agc cgg gag ttg ccc cac tgc atg         4512
   Val Ile Thr Ile Glu Asn Lys Gly Ser Arg Glu Leu Pro His Cys Met
      1490                1495                1500
   gct gtt ggg gat ctc ttc acg aag gtg ttg gac atc ttg ggc aaa ccc        4560
   Ala Val Gly Asp Leu Phe Thr Lys Val Leu Asp Ile Leu Gly Lys Pro
   1505                1510                1515                1520
   aac aac cgg ttc tac aag acc ctt tct tac ttt gca gtg gac aag gcc        4608
   Asn Asn Arg Phe Tyr Lys Thr Leu Ser Tyr Phe Ala Val Asp Lys Ala
                   1525                1530                1535
   gag aag gag cgc ttg ttg aag atc gcc gag atg ggg ccg gag tac agc        4656
   Glu Lys Glu Arg Leu Leu Lys Ile Ala Glu Met Gly Pro Glu Tyr Ser
               1540                1545                1550
   aac atc ctg tct gag acg tac cac tac gcg gac atc ttc cac atg ttc        4704
   Asn Ile Leu Ser Glu Thr Tyr His Tyr Ala Asp Ile Phe His Met Phe
           1555                1560                1565
   ccg tcc gcc cgg ccc acg ctg cag tac ctc atc gag atg atc ccc aac        4752
   Pro Ser Ala Arg Pro Thr Leu Gln Tyr Leu Ile Glu Met Ile Pro Asn
       1570                1575                1580
   atc aag ccc cgg tac tac tcc atc tcc tcc gcc ccc atc cac acc cct        4800
   Ile Lys Pro Arg Tyr Tyr Ser Ile Ser Ser Ala Pro Ile His Thr Pro
   1585                1590                1595                1600
   ggc gag gtc cac agc ctg gtg ctc atc gac acc tgg atc acg ctg tcc        4848
   Gly Glu Val His Ser Leu Val Leu Ile Asp Thr Trp Ile Thr Leu Ser
                   1605                1610                1615
   ggc aag cac cgc acg ggg ctg acc tgc acc atg ctg gag cac ctg cag        4896
   Gly Lys His Arg Thr Gly Leu Thr Cys Thr Met Leu Glu His Leu Gln
               1620                1625                1630
   gcg ggc cag gtg gtg gat ggc tgc atc cac ccc acg gcg atg gag ttc        4944
   Ala Gly Gln Val Val Asp Gly Cys Ile His Pro Thr Ala Met Glu Phe
           1635                1640                1645
   ccc gac cac gag aag ccg gtg gtg atg tgc gcc atg ggc agt ggc ctg        4992
   Pro Asp His Glu Lys Pro Val Val Met Cys Ala Met Gly Ser Gly Leu
       1650                1655                1660
   gca ccg ttc gtt gct ttc ctg cgc gac ggc tcc acg ctg cgg aag cag        5040
   Ala Pro Phe Val Ala Phe Leu Arg Asp Gly Ser Thr Leu Arg Lys Gln
   1665                1670                1675                1680
   ggc aag aag acc ggg aac atg gca ttg tac ttc ggc aac agg tat gag        5088
   Gly Lys Lys Thr Gly Asn Met Ala Leu Tyr Phe Gly Asn Arg Tyr Glu
                   1685                1690                1695
   aag acg gag ttc ctg atg aag gag gag ctg aag ggt cac atc aac gat        5136
   Lys Thr Glu Phe Leu Met Lys Glu Glu Leu Lys Gly His Ile Asn Asp
               1700                1705                1710
   ggt ttg ctg aca ctt cga tgc gct ttc agc cga gat gac ccc aag aag        5184
   Gly Leu Leu Thr Leu Arg Cys Ala Phe Ser Arg Asp Asp Pro Lys Lys
           1715                1720                1725
   aag gtg tat gtg cag gac ctt atc aag atg gac gaa aag atg atg tac        5232
   Lys Val Tyr Val Gln Asp Leu Ile Lys Met Asp Glu Lys Met Met Tyr
       1730                1735                1740
   gat tac ctc gtg gtg cag aag ggt tct atg tat tgc tgt gga tcc cgc        5280
   Asp Tyr Leu Val Val Gln Lys Gly Ser Met Tyr Cys Cys Gly Ser Arg
   1745                1750                1755                1760
   agt ttc atc aag cct gtc cag gag tca ttg aaa cat tgc ttc atg aaa        5328
   Ser Phe Ile Lys Pro Val Gln Glu Ser Leu Lys His Cys Phe Met Lys
                   1765                1770                1775
   gct ggt ggg ctg act gca gag caa gct gag aac gag gtc atc gat atg        5376
   Ala Gly Gly Leu Thr Ala Glu Gln Ala Glu Asn Glu Val Ile Asp Met
               1780                1785                1790
   ttc acg acc ggg cgg tac aat atc gag gca tgg taa                        5412
   Phe Thr Thr Gly Arg Tyr Asn Ile Glu Ala Trp
           1795                1800
<210> 6
   <211> 1803
   <212> PRT
   <213> Euglena gracilis
<400> 6
   Met Lys Gln Ser Val Arg Pro Ile Ile Ser Asn Val Leu Arg Lys Glu
    1               5                  10                  15
   Val Ala Leu Tyr Ser Thr Ile Ile Gly Gln Asp Lys Gly Lys Glu Pro
               20                  25                  30
   Thr Gly Arg Thr Tyr Thr Ser Gly Pro Lys Pro Ala Ser His Ile Glu
           35                  40                  45
   Val Pro His His Val Thr Val Pro Ala Thr Asp Arg Thr Pro Asn Pro
       50                  55                  60
   Asp Ala Gln Phe Phe Gln Ser Val Asp Gly Ser Gln Ala Thr Ser His
   65                  70                  75                  80
   Val Ala Tyr Ala Leu Ser Asp Thr Ala Phe Ile Tyr Pro Ile Thr Pro
                   85                  90                  95
   Ser Ser Val Met Gly Glu Leu Ala Asp Val Trp Met Ala Gln Gly Arg
               100                 105                 110
   Lys Asn Ala Phe Gly Gln Val Val Asp Val Arg Glu Met Gln Ser Glu
           115                 120                 125
   Ala Gly Ala Ala Gly Ala Leu His Gly Ala Leu Ala Ala Gly Ala Ile
       130                 135                 140
   Ala Thr Thr Phe Thr Ala Ser Gln Gly Leu Leu Leu Met Ile Pro Asn
   145                 150                 155                 160
   Met Tyr Lys Ile Ala Gly Glu Leu Met Pro Ser Val Ile His Val Ala
                   165                 170                 175
   Ala Arg Glu Leu Ala Gly His Ala Leu Ser Ile Phe Gly Gly His Ala
               180                 185                 190
   Asp Val Met Ala Val Arg Gln Thr Gly Trp Ala Met Leu Cys Ser His
           195                 200                 205
   Thr Val Gln Gln Ser His Asp Met Ala Leu Ile Ser His Val Ala Thr
       210                 215                 220
   Leu Lys Ser Ser Ile Pro Phe Val His Phe Phe Asp Gly Phe Arg Thr
   225                 230                 235                 240
   Ser His Glu Val Asn Lys Ile Lys Met Leu Pro Tyr Ala Glu Leu Lys
                   245                 250                 255
   Lys Leu Val Pro Pro Gly Thr Met Glu Gln His Trp Ala Arg Ser Leu
               260                 265                 270
   Asn Pro Met His Pro Thr Ile Arg Gly Thr Asn Gln Ser Ala Asp Ile
           275                 280                 285
   Tyr Phe Gln Asn Met Glu Ser Ala Asn Gln Tyr Tyr Thr Asp Leu Ala
       290                 295                 300
   Glu Val Val Gln Glu Thr Met Asp Glu Val Ala Pro Tyr Ile Gly Arg
   305                 310                 315                 320
   His Tyr Lys Ile Phe Glu Tyr Val Gly Ala Pro Asp Ala Glu Glu Val
                   325                 330                 335
   Thr Val Leu Met Gly Ser Gly Ala Thr Thr Val Asn Glu Ala Val Asp
               340                 345                 350
   Leu Leu Val Lys Arg Gly Lys Lys Val Gly Ala Val Leu Val His Leu
           355                 360                 365
   Tyr Arg Pro Trp Ser Thr Lys Ala Phe Glu Lys Val Leu Pro Lys Thr
       370                 375                 380
   Val Lys Arg Ile Ala Ala Leu Asp Arg Cys Lys Glu Val Thr Ala Leu
   385                 390                 395                 400
   Gly Glu Pro Leu Tyr Leu Asp Val Ser Ala Thr Leu Asn Leu Phe Pro
                   405                 410                 415
   Glu Arg Gln Asn Val Lys Val Ile Gly Gly Arg Tyr Gly Leu Gly Ser
               420                 425                 430
   Lys Asp Phe Ile Pro Glu His Ala Leu Ala Ile Tyr Ala Asn Leu Ala
           435                 440                 445
   Ser Glu Asn Pro Ile Gln Arg Phe Thr Val Gly Ile Thr Asp Asp Val
       450                 455                 460
   Thr Gly Thr Ser Val Pro Phe Val Asn Glu Arg Val Asp Thr Leu Pro
   465                 470                 475                 480
   Glu Gly Thr Arg Gln Cys Val Phe Trp Gly Ile Gly Ser Asp Gly Thr
                   485                 490                 495
   Val Gly Ala Asn Arg Ser Ala Val Arg Ile Ile Gly Asp Asn Ser Asp
               500                 505                 510
   Leu Met Val Gln Ala Tyr Phe Gln Phe Asp Ala Phe Lys Ser Gly Gly
           515                 520                 525
   Val Thr Ser Ser His Leu Arg Phe Gly Pro Lys Pro Ile Thr Ala Gln
       530                 535                 540
   Tyr Leu Val Thr Asn Ala Asp Tyr Ile Ala Cys His Phe Gln Glu Tyr
   545                 550                 555                 560
   Val Lys Arg Phe Asp Met Leu Asp Ala Ile Arg Glu Gly Gly Thr Phe
                   565                 570                 575
   Val Leu Asn Ser Arg Trp Thr Thr Glu Asp Met Glu Lys Glu Ile Pro
               580                 585                 590
   Ala Asp Phe Arg Arg Lys Leu Ala Gln Lys Lys Val Arg Phe Tyr Asn
           595                 600                 605
   Val Asp Ala Arg Lys Ile Cys Asp Ser Phe Gly Leu Gly Lys Arg Ile
       610                 615                 620
   Asn Met Leu Met Gln Ala Cys Phe Phe Lys Leu Ser Gly Val Leu Pro
   625                 630                 635                 640
   Leu Ala Glu Ala Gln Arg Leu Leu Asn Glu Ser Ile Val His Glu Tyr
                   645                 650                 655
   Gly Lys Lys Gly Gly Lys Val Val Glu Met Asn Gln Ala Val Val Asn
               660                 665                 670
   Ala Val Phe Ala Gly Asp Leu Pro Gln Glu Val Gln Val Pro Ala Ala
           675                 680                 685
   Trp Ala Asn Ala Val Asp Thr Ser Thr Arg Thr Pro Thr Gly Ile Glu
       690                 695                 700
   Phe Val Asp Lys Ile Met Arg Pro Leu Met Asp Phe Lys Gly Asp Gln
   705                 710                 715                 720
   Leu Pro Val Ser Val Met Thr Pro Gly Gly Thr Phe Pro Val Gly Thr
                   725                 730                 735
   Thr Gln Tyr Ala Lys Arg Ala Ile Ala Ala Phe Ile Pro Gln Trp Ile
               740                 745                 750
   Pro Ala Asn Cys Thr Gln Cys Asn Tyr Cys Ser Tyr Val Cys Pro His
           755                 760                 765
   Ala Thr Ile Arg Pro Phe Val Leu Thr Asp Gln Glu Val Gln Leu Ala
       770                 775                 780
   Pro Glu Ser Phe Val Thr Arg Lys Ala Lys Gly Asp Tyr Gln Gly Met
   785                 790                 795                 800
   Asn Phe Arg Ile Gln Val Ala Pro Glu Asp Cys Thr Gly Cys Gln Val
                   805                 810                 815
   Cys Val Glu Thr Cys Pro Asp Asp Ala Leu Glu Met Thr Asp Ala Phe
               820                 825                 830
   Thr Ala Thr Pro Val Gln Arg Thr Asn Trp Glu Phe Ala Ile Lys Val
           835                 840                 845
   Pro Asn Arg Gly Thr Met Thr Asp Arg Tyr Ser Leu Lys Gly Ser Gln
       850                 855                 860
   Phe Gln Gln Pro Leu Leu Glu Phe Ser Gly Ala Cys Glu Gly Cys Gly
   865                 870                 875                 880
   Glu Thr Pro Tyr Val Lys Leu Leu Thr Gln Leu Phe Gly Glu Arg Thr
                   885                 890                 895
   Val Ile Ala Asn Ala Thr Gly Cys Ser Ser Ile Trp Gly Gly Thr Ala
               900                 905                 910
   Gly Leu Ala Pro Tyr Thr Thr Asn Ala Lys Gly Gln Gly Pro Ala Trp
           915                 920                 925
   Gly Asn Ser Leu Phe Glu Asp Asn Ala Glu Phe Gly Phe Gly Ile Ala
       930                 935                 940
   Val Ala Asn Ala Gln Lys Arg Ser Arg Val Arg Asp Cys Ile Leu Gln
   945                 950                 955                 960
   Ala Val Glu Lys Lys Val Ala Asp Glu Gly Leu Thr Thr Leu Leu Ala
                   965                 970                 975
   Gln Trp Leu Gln Asp Trp Asn Thr Gly Asp Lys Thr Leu Lys Tyr Gln
               980                 985                 990
   Asp Gln Ile Ile Ala Gly Leu Ala Gln Gln Arg Ser Lys Asp Pro Leu
           995                 1000                1005
   Leu Glu Gln Ile Tyr Gly Met Lys Asp Met Leu Pro Asn Ile Ser Gln
      1010                1015                1020
   Trp Ile Ile Gly Gly Asp Gly Trp Ala Asn Asp Ile Gly Phe Gly Gly
   1025                1030                1035                1040
   Leu Asp His Val Leu Ala Ser Gly Gln Asn Leu Asn Val Leu Val Leu
                  1045                1050                1055
   Asp Thr Glu Met Tyr Ser Asn Thr Gly Gly Gln Ala Ser Lys Ser Thr
              1060                1065                1070
   His Met Ala Ser Val Ala Lys Phe Ala Leu Gly Gly Lys Arg Thr Asn
          1075                1080                1085
   Lys Lys Asn Leu Thr Glu Met Ala Met Ser Tyr Gly Asn Val Tyr Val
      1090                1095                1100
   Ala Thr Val Ser His Gly Asn Met Ala Gln Cys Val Lys Ala Phe Val
   1105                1110                1115                1120
   Glu Ala Glu Ser Tyr Asp Gly Pro Ser Leu Ile Val Gly Tyr Ala Pro
                  1125                1130                1135
   Cys Ile Glu His Gly Leu Arg Ala Gly Met Ala Arg Met Val Gln Glu
              1140                1145                1150
   Ser Glu Ala Ala Ile Ala Thr Gly Tyr Trp Pro Leu Tyr Arg Phe Asp
          1155                1160                1165
   Pro Arg Leu Ala Thr Glu Gly Lys Asn Pro Phe Gln Leu Asp Ser Lys
      1170                1175                1180
   Arg Ile Lys Gly Asn Leu Gln Glu Tyr Leu Asp Arg Gln Asn Arg Tyr
   1185                1190                1195                1200
   Val Asn Leu Lys Lys Asn Asn Pro Lys Gly Ala Asp Leu Leu Lys Ser
                  1205                1210                1215
   Gln Met Ala Asp Asn Ile Thr Ala Arg Phe Asn Arg Tyr Arg Arg Met
              1220                1225                1230
   Leu Glu Gly Pro Asn Thr Lys Ala Ala Ala Pro Ser Gly Asn His Val
          1235                1240                1245
   Thr Ile Leu Tyr Gly Ser Glu Thr Gly Asn Ser Glu Gly Leu Ala Lys
      1250                1255                1260
   Glu Leu Ala Thr Asp Phe Glu Arg Arg Glu Tyr Ser Val Ala Val Gln
   1265                1270                1275                1280
   Ala Leu Asp Asp Ile Asp Val Ala Asp Leu Glu Asn Met Gly Phe Val
                  1285                1290                1295
   Val Ile Ala Val Ser Thr Cys Gly Gln Gly Gln Phe Pro Arg Asn Ser
              1300                1305                1310
   Gln Leu Phe Trp Arg Glu Leu Gln Arg Asp Lys Pro Glu Gly Trp Leu
          1315                1320                1325
   Lys Asn Leu Lys Tyr Thr Val Phe Gly Leu Gly Asp Ser Thr Tyr Tyr
      1330                1335                1340
   Phe Tyr Cys His Thr Ala Lys Gln Ile Asp Ala Arg Leu Ala Ala Leu
   1345                1350                1355                1360
   Gly Ala Gln Arg Val Val Pro Ile Gly Phe Gly Asp Asp Gly Asp Glu
                  1365                1370                1375
   Asp Met Phe His Thr Gly Phe Asn Asn Trp Ile Pro Ser Val Trp Asn
              1380                1385                1390
   Glu Leu Lys Thr Lys Thr Pro Glu Glu Ala Leu Phe Thr Pro Ser Ile
          1395                1400                1405
   Ala Val Gln Leu Thr Pro Asn Ala Thr Pro Gln Asp Phe His Phe Ala
      1410                1415                1420
   Lys Ser Thr Pro Val Leu Ser Ile Thr Gly Ala Glu Arg Ile Thr Pro
   1425                1430                1435                1440
   Ala Asp His Thr Arg Asn Phe Val Thr Ile Arg Trp Lys Thr Asp Leu
                   1445                1450                1455
   Ser Tyr Gln Val Gly Asp Ser Leu Gly Val Phe Pro Glu Asn Thr Arg
               1460                1465                1470
   Ser Val Val Glu Glu Phe Leu Gln Tyr Tyr Gly Leu Asn Pro Lys Asp
           1475                1480                1485
   Val Ile Thr Ile Glu Asn Lys Gly Ser Arg Glu Leu Pro His Cys Met
       1490                1495                1500
   Ala Val Gly Asp Leu Phe Thr Lys Val Leu Asp Ile Leu Gly Lys Pro
   1505                1510                1515                1520
   Asn Asn Arg Phe Tyr Lys Thr Leu Ser Tyr Phe Ala Val Asp Lys Ala
                   1525                1530                1535
   Glu Lys Glu Arg Leu Leu Lys Ile Ala Glu Met Gly Pro Glu Tyr Ser
               1540                1545                1550
   Asn Ile Leu Ser Glu Thr Tyr His Tyr Ala Asp Ile Phe His Met Phe
           1555                1560                1565
   Pro Ser Ala Arg Pro Thr Leu Gln Tyr Leu Ile Glu Met Ile Pro Asn
       1570                1575                1580
   Ile Lys Pro Arg Tyr Tyr Ser Ile Ser Ser Ala Pro Ile His Thr Pro
   1585                1590                1595                1600
   Gly Glu Val His Ser Leu Val Leu Ile Asp Thr Trp Ile Thr Leu Ser
                   1605                1610                1615
   Gly Lys His Arg Thr Gly Leu Thr Cys Thr Met Leu Glu His Leu Gln
               1620                1625                1630
   Ala Gly Gln Val Val Asp Gly Cys Ile His Pro Thr Ala Met Glu Phe
           1635                1640                1645
   Pro Asp His Glu Lys Pro Val Val Met Cys Ala Met Gly Ser Gly Leu
       1650                1655                1660
   Ala Pro Phe Val Ala Phe Leu Arg Asp Gly Ser Thr Leu Arg Lys Gln
   1665                1670                1675                1680
   Gly Lys Lys Thr Gly Asn Met Ala Leu Tyr Phe Gly Asn Arg Tyr Glu
                   1685                1690                1695
   Lys Thr Glu Phe Leu Met Lys Glu Glu Leu Lys Gly His Ile Asn Asp
               1700                1705                1710
   Gly Leu Leu Thr Leu Arg Cys Ala Phe Ser Arg Asp Asp Pro Lys Lys
           1715                1720                1725
   Lys Val Tyr Val Gln Asp Leu Ile Lys Met Asp Glu Lys Met Met Tyr
       1730                1735                1740
   Asp Tyr Leu Val Val Gln Lys Gly Ser Met Tyr Cys Cys Gly Ser Arg
   1745                1750                1755                1760
   Ser Phe Ile Lys Pro Val Gln Glu Ser Leu Lys His Cys Phe Met Lys
                   1765                1770                1775
   Ala Gly Gly Leu Thr Ala Glu Gln Ala Glu Asn Glu Val Ile Asp Met
               1780                1785                1790
   Phe Thr Thr Gly Arg Tyr Asn Ile Glu Ala Trp
           1795                1800
<210> 7
   <211> 747
   <212> DNA
   <213> Escherichia coli
<220>
   <221> CDS
   <222> (1)..(747)
<400> 7
   atg gct gat tgg gta aca ggc aaa gtc act aaa gtg cag aac tgg acc       48
   Met Ala Asp Trp Val Thr Gly Lys Val Thr Lys Val Gln Asn Trp Thr
   1               5                   10                  15
   gac gcc ctg ttt agt ctc acc gtt cac gcc ccc gtg ctt ccg ttt acc       96
   Asp Ala Leu Phe Ser Leu Thr Val His Ala Pro Val Leu Pro Phe Thr
               20                  25                  30
   gcc ggg caa ttt acc aag ctt ggc ctt gaa atc gac ggc gaa cgc gtc      144
   Ala Gly Gln Phe Thr Lys Leu Gly Leu Glu Ile Asp Gly Glu Arg Val
           35                  40                  45
   cag cgc gcc tac tcc tat gta aac tcg ccc gat aat ccc gat ctg gag      192
   Gln Arg Ala Tyr Ser Tyr Val Asn Ser Pro Asp Asn Pro Asp Leu Glu
       50                  55                  60
   ttt tac ctg gtc acc gtc ccc gat ggc aaa tta agc cca cga ctg gcg      240
   Phe Tyr Leu Val Thr Val Pro Asp Gly Lys Leu Ser Pro Arg Leu Ala
   65                  70                  75                  80
   gca ctg aaa cca ggc gat gaa gtg cag gtg gtt agc gaa gcg gca gga      288
   Ala Leu Lys Pro Gly Asp Glu Val Gln Val Val Ser Glu Ala Ala Gly
                   85                  90                  95
   ttc ttt gtg ctc gat gaa gtg ccg cac tgc gaa acg cta tgg atg ctg      336
   Phe Phe Val Leu Asp Glu Val Pro His Cys Glu Thr Leu Trp Met Leu
               100                 105                 110
   gca acc ggt aca gcg att ggc cct tat tta tcg att ctg caa cta ggt      384
   Ala Thr Gly Thr Ala Ile Gly Pro Tyr Leu Ser Ile Leu Gln Leu Gly
           115                 120                 125
   aaa gat tta gat cgc ttc aaa aat ctg gtc ctg gtg cac gcc gca cgt      432
   Lys Asp Leu Asp Arg Phe Lys Asn Leu Val Leu Val His Ala Ala Arg
       130                 135                 140
   tat gcc gcc gac tta agc tat ttg cca ctg atg cag gaa ctg gaa aaa      480
   Tyr Ala Ala Asp Leu Ser Tyr Leu Pro Leu Met Gln Glu Leu Glu Lys
   145                 150                 155                 160
   cgc tac gaa gga aaa ctg cgc att cag acg gtg gtc agt cgg gaa acg      528
   Arg Tyr Glu Gly Lys Leu Arg Ile Gln Thr Val Val Ser Arg Glu Thr
                   165                 170                 175
   gca gcg ggg tcg ctc acc gga cgg ata ccg gca tta att gaa agt ggg      576
   Ala Ala Gly Ser Leu Thr Gly Arg Ile Pro Ala Leu Ile Glu Ser Gly
               180                 185                 190
   gaa ctg gaa agc acg att ggc ctg ccg atg aat aaa gaa acc agc cat      624
   Glu Leu Glu Ser Thr Ile Gly Leu Pro Met Asn Lys Glu Thr Ser His
           195                 200                 205
   gtg atg ctg tgc ggc aat cca cag atg gtg cgc gat aca caa cag ttg      672
   Val Met Leu Cys Gly Asn Pro Gln Met Val Arg Asp Thr Gln Gln Leu
       210                 215                 220
   ctg aaa gag acc cgg cag atg acg aaa cat tta cgt cgc cga ccg ggc      720
   Leu Lys Glu Thr Arg Gln Met Thr Lys His Leu Arg Arg Arg Pro Gly
   225                 230                 235                 240
   cat atg aca gcg gag cat tac tgg taa                                  747
   His Met Thr Ala Glu His Tyr Trp
                   245
<210> 8
   <211> 248
   <212> PRT
   <213> Escherichia coli
<400> 8
   Met Ala Asp Trp Val Thr Gly Lys Val Thr Lys Val Gln Asn Trp Thr
   1               5                   10                  15
   Asp Ala Leu Phe Ser Leu Thr Val His Ala Pro Val Leu Pro Phe Thr
               20                  25                  30
   Ala Gly Gln Phe Thr Lys Leu Gly Leu Glu Ile Asp Gly Glu Arg Val
           35                  40                  45
   Gln Arg Ala Tyr Ser Tyr Val Asn Ser Pro Asp Asn Pro Asp Leu Glu
       50                  55                  60
   Phe Tyr Leu Val Thr Val Pro Asp Gly Lys Leu Ser Pro Arg Leu Ala
   65                  70                  75                  80
   Ala Leu Lys Pro Gly Asp Glu Val Gln Val Val Ser Glu Ala Ala Gly
                   85                  90                  95
   Phe Phe Val Leu Asp Glu Val Pro His Cys Glu Thr Leu Trp Met Leu
               100                 105                 110
   Ala Thr Gly Thr Ala Ile Gly Pro Tyr Leu Ser Ile Leu Gln Leu Gly
           115                 120                 125
   Lys Asp Leu Asp Arg Phe Lys Asn Leu Val Leu Val His Ala Ala Arg
       130                 135                 140
   Tyr Ala Ala Asp Leu Ser Tyr Leu Pro Leu Met Gln Glu Leu Glu Lys
   145                 150                 155                 160
   Arg Tyr Glu Gly Lys Leu Arg Ile Gln Thr Val Val Ser Arg Glu Thr
                   165                 170                 175
   Ala Ala Gly Ser Leu Thr Gly Arg Ile Pro Ala Leu Ile Glu Ser Gly
               180                 185                 190
   Glu Leu Glu Ser Thr Ile Gly Leu Pro Met Asn Lys Glu Thr Ser His
           195                 200                 205
   Val Met Leu Cys Gly Asn Pro Gln Met Val Arg Asp Thr Gln Gln Leu
       210                 215                 220
   Leu Lys Glu Thr Arg Gln Met Thr Lys His Leu Arg Arg Arg Pro Gly
   225                 230                 235                 240
   His Met Thr Ala Glu His Tyr Trp
                   245
<210> 9
   <211> 336
   <212> DNA
   <213> Escherichia coli
<220>
   <221> CDS
   <222> (1)..(336)
<400> 9
   atg cca aag att gtt att ttg cct cat cag gat ctc tgc cct gat ggc      48
   Met Pro Lys Ile Val Ile Leu Pro His Gln Asp Leu Cys Pro Asp Gly
   1               5                   10                  15
   gct gtt ctg gaa gct aat agc ggt gaa acc att ctc gac gca gct ctg      96
   Ala Val Leu Glu Ala Asn Ser Gly Glu Thr Ile Leu Asp Ala Ala Leu
               20                  25                  30
   cgt aac ggt atc gag att gaa cac gcc tgt gaa aaa tcc tgt gct tgc     144
   Arg Asn Gly Ile Glu Ile Glu His Ala Cys Glu Lys Ser Cys Ala Cys
           35                  40                  45
   acc acc tgc cac tgc atc gtt cgt gaa ggt ttt gac tca ctg ccg gaa     192
   Thr Thr Cys His Cys Ile Val Arg Glu Gly Phe Asp Ser Leu Pro Glu
       50                  55                  60
   agc tca gag cag gaa gac gac atg ctg gac aaa gcc tgg gga ctg gag     240
   Ser Ser Glu Gln Glu Asp Asp Met Leu Asp Lys Ala Trp Gly Leu Glu
   65                  70                  75                  80
   ccg gaa agc cgt tta agc tgc cag gcg cgc gtt acc gac gaa gat tta     288
   Pro Glu Ser Arg Leu Ser Cys Gln Ala Arg Val Thr Asp Glu Asp Leu
                   85                  90                  95
   gta gtc gaa atc ccg cgt tac act atc aac cat gcg cgt gag cat taa     336
   Val Val Glu Ile Pro Arg Tyr Thr Ile Asn His Ala Arg Glu His
               100                 105                 110
<210> 10
   <211> 111
   <212> PRT
   <213> Escherichia coli
<400> 10
   Met Pro Lys Ile Val Ile Leu Pro His Gln Asp Leu Cys Pro Asp Gly
   1               5                   10                  15
   Ala Val Leu Glu Ala Asn Ser Gly Glu Thr Ile Leu Asp Ala Ala Leu
               20                  25                  30
   Arg Asn Gly Ile Glu Ile Glu His Ala Cys Glu Lys Ser Cys Ala Cys
           35                  40                  45
   Thr Thr Cys His Cys Ile Val Arg Glu Gly Phe Asp Ser Leu Pro Glu
       50                  55                  60
   Ser Ser Glu Gln Glu Asp Asp Met Leu Asp Lys Ala Trp Gly Leu Glu
   65                  70                  75                  80
   Pro Glu Ser Arg Leu Ser Cys Gln Ala Arg Val Thr Asp Glu Asp Leu
                   85                  90                  95
   Val Val Glu Ile Pro Arg Tyr Thr Ile Asn His Ala Arg Glu His
               100                 105                 110
<210> 11
   <211> 261
   <212> DNA
   <213> Escherichia coli
<220>
   <221> CDS
   <222> (1)..(261)
<400> 11
   atg gcg ttg tta atc act aaa aaa tgc atc aat tgt gat atg tgt gaa       48
   Met Ala Leu Leu Ile Thr Lys Lys Cys Ile Asn Cys Asp Met Cys Glu
   1               5                   10                  15
   ccc gaa tgc ccg aat gag gcg att tca atg gga gat cat atc tac gag       96
   Pro Glu Cys Pro Asn Glu Ala Ile Ser Met Gly Asp His Ile Tyr Glu
               20                  25                  30
   att aac agc gat aag tgt acc gaa tgc gta ggg cac tac gag aca cca      144
   Ile Asn Ser Asp Lys Cys Thr Glu Cys Val Gly His Tyr Glu Thr Pro
           35                  40                  45
   acc tgc cag aag gtg tgc ccg atc ccc aat act att gtg aaa gat ccg      192
   Thr Cys Gln Lys Val Cys Pro Ile Pro Asn Thr Ile Val Lys Asp Pro
       50                  55                  60
   gcg cat gtc gag aca gaa gaa cag ttg tgg gat aaa ttt gtg ctg atg      240
   Ala His Val Glu Thr Glu Glu Gln Leu Trp Asp Lys Phe Val Leu Met
   65                  70                  75                  80
   cac cac gcg gat aaa att taa                                          261
   His His Ala Asp Lys Ile
                   85
<210> 12
   <211> 86
   <212> PRT
   <213> Escherichia coli
<400> 12
   Met Ala Leu Leu Ile Thr Lys Lys Cys Ile Asn Cys Asp Met Cys Glu
   1               5                   10                  15
   Pro Glu Cys Pro Asn Glu Ala Ile Ser Met Gly Asp His Ile Tyr Glu
               20                  25                  30
   Ile Asn Ser Asp Lys Cys Thr Glu Cys Val Gly His Tyr Glu Thr Pro
           35                  40                  45
   Thr Cys Gln Lys Val Cys Pro Ile Pro Asn Thr Ile Val Lys Asp Pro
       50                  55                  60
   Ala His Val Glu Thr Glu Glu Gln Leu Trp Asp Lys Phe Val Leu Met
   65                  70                  75                  80
   His His Ala Asp Lys Ile
                   85
<210> 13
   <211> 531
   <212> DNA
   <213> Escherichia coli
<220>
   <221> CDS
   <222> (1)..(531)
<400> 13
   atg gct atc act ggc atc ttt ttc ggc agc gac acc ggt aat acc gaa      48
   Met Ala Ile Thr Gly Ile Phe Phe Gly Ser Asp Thr Gly Asn Thr Glu
   1               5                   10                  15
   aat atc gca aaa atg att caa aaa cag ctt ggt aaa gac gtt gcc gat      96
   Asn Ile Ala Lys Met Ile Gln Lys Gln Leu Gly Lys Asp Val Ala Asp
               20                  25                  30
   gtc cat gac att gca aaa agc agc aaa gaa gat ctg gaa gct tat gac     144
   Val His Asp Ile Ala Lys Ser Ser Lys Glu Asp Leu Glu Ala Tyr Asp
           35                  40                  45
   att ctg ctg ctg ggc atc cca acc tgg tat tac ggc gaa gcg cag tgt     192
   Ile Leu Leu Leu Gly Ile Pro Thr Trp Tyr Tyr Gly Glu Ala Gln Cys
       50                  55                  60
   gac tgg gat gac ttc ttc ccg act ctc gaa gag att gat ttc aac ggc     240
   Asp Trp Asp Asp Phe Phe Pro Thr Leu Glu Glu Ile Asp Phe Asn Gly
   65                  70                  75                  80
   aaa ctg gtt gcg ctg ttt ggt tgt ggt gac cag gaa gat tac gcc gaa     288
   Lys Leu Val Ala Leu Phe Gly Cys Gly Asp Gln Glu Asp Tyr Ala Glu
                   85                  90                  95
   tat ttc tgc gac gca ttg ggc acc atc cgc gac atc att gaa ccg cgc     336
   Tyr Phe Cys Asp Ala Leu Gly Thr Ile Arg Asp Ile Ile Glu Pro Arg
               100                 105                 110
   ggt gca acc atc gtt ggt cac tgg cca act gcg ggc tat cat ttc gaa     384
   Gly Ala Thr Ile Val Gly His Trp Pro Thr Ala Gly Tyr His Phe Glu
           115                 120                 125
   gca tca aaa ggt ctg gca gat gac gac cac ttt gtc ggt ctg gct atc     432
   Ala Ser Lys Gly Leu Ala Asp Asp Asp His Phe Val Gly Leu Ala Ile
       130                 135                 140
   gac gaa gac cgt cag ccg gaa ctg acc gct gaa cgt gta gaa aaa tgg     480
   Asp Glu Asp Arg Gln Pro Glu Leu Thr Ala Glu Arg Val Glu Lys Trp
   145                 150                 155                 160
   gtt aaa cag att tct gaa gag ttg cat ctc gac gaa att ctc aat gcc     528
   Val Lys Gln Ile Ser Glu Glu Leu His Leu Asp Glu Ile Leu Asn Ala
                   165                 170                 175
   tga                                                                 531
<210> 14
   <211> 176
   <212> PRT
   <213> Escherichia coli
<400> 14
   Met Ala Ile Thr Gly Ile Phe Phe Gly Ser Asp Thr Gly Asn Thr Glu
   1               5                   10                  15
   Asn Ile Ala Lys Met Ile Gln Lys Gln Leu Gly Lys Asp Val Ala Asp
               20                  25                  30
   Val His Asp Ile Ala Lys Ser Ser Lys Glu Asp Leu Glu Ala Tyr Asp
           35                  40                  45
   Ile Leu Leu Leu Gly Ile Pro Thr Trp Tyr Tyr Gly Glu Ala Gln Cys
       50                  55                  60
   Asp Trp Asp Asp Phe Phe Pro Thr Leu Glu Glu Ile Asp Phe Asn Gly
   65                  70                  75                  80
   Lys Leu Val Ala Leu Phe Gly Cys Gly Asp Gln Glu Asp Tyr Ala Glu
                   85                  90                  95
   Tyr Phe Cys Asp Ala Leu Gly Thr Ile Arg Asp Ile Ile Glu Pro Arg
               100                 105                 110
   Gly Ala Thr Ile Val Gly His Trp Pro Thr Ala Gly Tyr His Phe Glu
           115                 120                 125
   Ala Ser Lys Gly Leu Ala Asp Asp Asp His Phe Val Gly Leu Ala Ile
       130                 135                 140
   Asp Glu Asp Arg Gln Pro Glu Leu Thr Ala Glu Arg Val Glu Lys Trp
   145                 150                 155                 160
   Val Lys Gln Ile Ser Glu Glu Leu His Leu Asp Glu Ile Leu Asn Ala
                   165                 170                 175
<210> 15
   <211> 522
   <212> DNA
   <213> Escherichia coli
<220>
   <221> CDS
   <222> (1)..(522)
<400> 15
   atg aat atg ggt ctt ttt tac ggt tcc agc acc tgt tac acc gaa atg        48
   Met Asn Met Gly Leu Phe Tyr Gly Ser Ser Thr Cys Tyr Thr Glu Met
   1               5                   10                  15
   gcg gca gaa aaa atc cgc gat att atc ggc cca gaa ctg gtg acc tta        96
   Ala Ala Glu Lys Ile Arg Asp Ile Ile Gly Pro Glu Leu Val Thr Leu
               20                  25                  30
   cat aac ctc aag gac gac tcc ccg aaa tta atg gag cag tac gat gtg       144
   His Asn Leu Lys Asp Asp Ser Pro Lys Leu Met Glu Gln Tyr Asp Val
           35                  40                  45
   ctc att ctg ggt atc ccg acc tgg gat ttt ggt gaa atc cag gaa gac       192
   Leu Ile Leu Gly Ile Pro Thr Trp Asp Phe Gly Glu Ile Gln Glu Asp
       50                  55                  60
   tgg gaa gcc gtc tgg gat cag ctc gac gac ctg aac ctt gaa ggt aaa       240
   Trp Glu Ala Val Trp Asp Gln Leu Asp Asp Leu Asn Leu Glu Gly Lys
   65                  70                  75                  80
   att gtt gcg ctg tat ggg ctt ggc gat caa ctg gga tac ggc gag tgg       288
   Ile Val Ala Leu Tyr Gly Leu Gly Asp Gln Leu Gly Tyr Gly Glu Trp
                   85                  90                  95
   ttc ctc gat gcg ctc ggt atg ctg cat gac aaa ctc tcg acc aaa ggc       336
   Phe Leu Asp Ala Leu Gly Met Leu His Asp Lys Leu Ser Thr Lys Gly
               100                 105                 110
   gtg aag ttc gtc ggc tac tgg cca acg gaa gga tat gaa ttt acc agc       384
   Val Lys Phe Val Gly Tyr Trp Pro Thr Glu Gly Tyr Glu Phe Thr Ser
           115                 120                 125
   ccg aaa ccg gtg att gct gac ggg caa ctg ttc gtg ggt ctg gcg ctg       432
   Pro Lys Pro Val Ile Ala Asp Gly Gln Leu Phe Val Gly Leu Ala Leu
       130                 135                 140
   gat gaa act aac cag tat gac ctt agc gac gag cgt att cag agc tgg       480
   Asp Glu Thr Asn Gln Tyr Asp Leu Ser Asp Glu Arg Ile Gln Ser Trp
   145                 150                 155                 160
   tgc gag caa atc ctc aac gaa atg gca gag cat tac gcc tga               522
   Cys Glu Gln Ile Leu Asn Glu Met Ala Glu His Tyr Ala
                   165                 170
<210> 16
   <211> 173
   <212> PRT
   <213> Escherichia coli
<400> 16
   Met Asn Met Gly Leu Phe Tyr Gly Ser Ser Thr Cys Tyr Thr Glu Met
   1               5                   10                  15
   Ala Ala Glu Lys Ile Arg Asp Ile Ile Gly Pro Glu Leu Val Thr Leu
               20                  25                  30
   His Asn Leu Lys Asp Asp Ser Pro Lys Leu Met Glu Gln Tyr Asp Val
           35                  40                  45
   Leu Ile Leu Gly Ile Pro Thr Trp Asp Phe Gly Glu Ile Gln Glu Asp
       50                  55                  60
   Trp Glu Ala Val Trp Asp Gln Leu Asp Asp Leu Asn Leu Glu Gly Lys
   65                  70                  75                  80
   Ile Val Ala Leu Tyr Gly Leu Gly Asp Gln Leu Gly Tyr Gly Glu Trp
                   85                  90                  95
   Phe Leu Asp Ala Leu Gly Met Leu His Asp Lys Leu Ser Thr Lys Gly
               100                 105                 110
   Val Lys Phe Val Gly Tyr Trp Pro Thr Glu Gly Tyr Glu Phe Thr Ser
           115                 120                 125
   Pro Lys Pro Val Ile Ala Asp Gly Gln Leu Phe Val Gly Leu Ala Leu
       130                 135                 140
   Asp Glu Thr Asn Gln Tyr Asp Leu Ser Asp Glu Arg Ile Gln Ser Trp
   145                 150                 155                 160
   Cys Glu Gln Ile Leu Asn Glu Met Ala Glu His Tyr Ala
                   165                 170
<210> 17
   <211> 522
   <212> DNA
   <213> Chlorobium tepidum
<220>
   <221> CDS
   <222> (1)..(522)
<400> 17
   atg aat atg ggt ctt ttt tac ggt tcc agc acc tgt tac acc gaa atg       48
   Met Asn Met Gly Leu Phe Tyr Gly Ser Ser Thr Cys Tyr Thr Glu Met
   1               5                   10                  15
   gcg gca gaa aaa atc cgc gat att atc ggc cca gaa ctg gtg acc tta       96
   Ala Ala Glu Lys Ile Arg Asp Ile Ile Gly Pro Glu Leu Val Thr Leu
               20                  25                  30
   cat aac ctc aag gac gac tcc ccg aaa tta atg gag cag tac gat gtg      144
   His Asn Leu Lys Asp Asp Ser Pro Lys Leu Met Glu Gln Tyr Asp Val
           35                  40                  45
   ctc att ctg ggt atc ccg acc tgg gat ttt ggt gaa atc cag gaa gac      192
   Leu Ile Leu Gly Ile Pro Thr Trp Asp Phe Gly Glu Ile Gln Glu Asp
       50                  55                  60
   tgg gaa gcc gtc tgg gat cag ctc gac gac ctg aac ctt gaa ggt aaa      240
   Trp Glu Ala Val Trp Asp Gln Leu Asp Asp Leu Asn Leu Glu Gly Lys
   65                  70                  75                  80
   att gtt gcg ctg tat ggg ctt ggc gat caa ctg gga tac ggc gag tgg      288
   Ile Val Ala Leu Tyr Gly Leu Gly Asp Gln Leu Gly Tyr Gly Glu Trp
                   85                  90                  95
   ttc ctc gat gcg ctc ggt atg ctg cat gac aaa ctc tcg acc aaa ggc      336
   Phe Leu Asp Ala Leu Gly Met Leu His Asp Lys Leu Ser Thr Lys Gly
               100                 105                 110
   gtg aag ttc gtc ggc tac tgg cca acg gaa gga tat gaa ttt acc agc      384
   Val Lys Phe Val Gly Tyr Trp Pro Thr Glu Gly Tyr Glu Phe Thr Ser
           115                 120                 125
   ccg aaa ccg gtg att gct gac ggg caa ctg ttc gtg ggt ctg gcg ctg      432
   Pro Lys Pro Val Ile Ala Asp Gly Gln Leu Phe Val Gly Leu Ala Leu
       130                 135                 140
   gat gaa act aac cag tat gac ctt agc gac gag cgt att cag agc tgg      480
   Asp Glu Thr Asn Gln Tyr Asp Leu Ser Asp Glu Arg Ile Gln Ser Trp
   145                 150                 155                 160
   tgc gag caa atc ctc aac gaa atg gca gag cat tac gcc tga              522
   Cys Glu Gln Ile Leu Asn Glu Met Ala Glu His Tyr Ala
                   165                 170
<210> 18
   <211> 173
   <212> PRT
   <213> Chlorobium tepidum
<400> 18
   Met Asn Met Gly Leu Phe Tyr Gly Ser Ser Thr Cys Tyr Thr Glu Met
   1               5                   10                  15
   Ala Ala Glu Lys Ile Arg Asp Ile Ile Gly Pro Glu Leu Val Thr Leu
               20                  25                  30
   His Asn Leu Lys Asp Asp Ser Pro Lys Leu Met Glu Gln Tyr Asp Val
           35                  40                  45
   Leu Ile Leu Gly Ile Pro Thr Trp Asp Phe Gly Glu Ile Gln Glu Asp
       50                  55                  60
   Trp Glu Ala Val Trp Asp Gln Leu Asp Asp Leu Asn Leu Glu Gly Lys
   65                  70                  75                  80
   Ile Val Ala Leu Tyr Gly Leu Gly Asp Gln Leu Gly Tyr Gly Glu Trp
                   85                  90                  95
   Phe Leu Asp Ala Leu Gly Met Leu His Asp Lys Leu Ser Thr Lys Gly
               100                 105                 110
   Val Lys Phe Val Gly Tyr Trp Pro Thr Glu Gly Tyr Glu Phe Thr Ser
           115                 120                 125
   Pro Lys Pro Val Ile Ala Asp Gly Gln Leu Phe Val Gly Leu Ala Leu
       130                 135                 140
   Asp Glu Thr Asn Gln Tyr Asp Leu Ser Asp Glu Arg Ile Gln Ser Trp
   145                 150                 155                 160
   Cys Glu Gln Ile Leu Asn Glu Met Ala Glu His Tyr Ala
                   165                 170
<210> 19
   <211> 189
   <212> DNA
   <213> Chlorobium tepidum
<220>
   <221> CDS
   <222> (1)..(189)
<400> 19
   atg gca ctg tat atc acc gaa gaa tgc acc tac tgc ggt gct tgc gaa        48
   Met Ala Leu Tyr Ile Thr Glu Glu Cys Thr Tyr Cys Gly Ala Cys Glu
   1               5                   10                  15
   ccc gaa tgc ccg acc aac gct atc tcc gct ggc agc gag atc tac gtt        96
   Pro Glu Cys Pro Thr Asn Ala Ile Ser Ala Gly Ser Glu Ile Tyr Val
               20                  25                  30
   atc gat gcc gca tcc tgc aac gag tgc gcc ggt ttt gct gac tct cct       144
   Ile Asp Ala Ala Ser Cys Asn Glu Cys Ala Gly Phe Ala Asp Ser Pro
           35                  40                  45
   gct tgc gtt gct gtc tgc ccg gca gag tgc atc gtt cag ggc tga           189
   Ala Cys Val Ala Val Cys Pro Ala Glu Cys Ile Val Gln Gly
       50                  55                  60
<210> 20
   <211> 62
   <212> PRT
   <213> Chlorobium tepidum
<400> 20
   Met Ala Leu Tyr Ile Thr Glu Glu Cys Thr Tyr Cys Gly Ala Cys Glu
   1               5                   10                  15
   Pro Glu Cys Pro Thr Asn Ala Ile Ser Ala Gly Ser Glu Ile Tyr Val
               20                  25                  30
   Ile Asp Ala Ala Ser Cys Asn Glu Cys Ala Gly Phe Ala Asp Ser Pro
           35                  40                  45
   Ala Cys Val Ala Val Cys Pro Ala Glu Cys Ile Val Gln Gly
       50                  55                  60
<210> 21
   <211> 2676
   <212> DNA
   <213> Escherichia coli
<220>
   <221> CDS
   <222> (1)..(2676)
<400> 21
   atg gct gtt act aat gtc gct gaa ctt aac gca ctc gta gag cgt gta        48
   Met Ala Val Thr Asn Val Ala Glu Leu Asn Ala Leu Val Glu Arg Val
    1               5                  10                  15
   aaa aaa gcc cag cgt gaa tat gcc agt ttc act caa gag caa gta gac        96
   Lys Lys Ala Gln Arg Glu Tyr Ala Ser Phe Thr Gln Glu Gln Val Asp
               20                  25                  30
   aaa atc ttc cgc gcc gcc gct ctg gct gct gca gat gct cga atc cca       144
   Lys Ile Phe Arg Ala Ala Ala Leu Ala Ala Ala Asp Ala Arg Ile Pro
           35                  40                  45
   ctc gcg aaa atg gcc gtt gcc gaa tcc ggc atg ggt atc gtc gaa gat       192
   Leu Ala Lys Met Ala Val Ala Glu Ser Gly Met Gly Ile Val Glu Asp
       50                  55                  60
   aaa gtg atc aaa aac cac ttt gct tct gaa tat atc tac aac gcc tat       240
   Lys Val Ile Lys Asn His Phe Ala Ser Glu Tyr Ile Tyr Asn Ala Tyr
   65                  70                  75                  80
   aaa gat gaa aaa acc tgt ggt gtt ctg tct gaa gac gac act ttt ggt       288
   Lys Asp Glu Lys Thr Cys Gly Val Leu Ser Glu Asp Asp Thr Phe Gly
                   85                  90                  95
   acc atc act atc gct gaa cca atc ggt att att tgc ggt atc gtt ccg       336
   Thr Ile Thr Ile Ala Glu Pro Ile Gly Ile Ile Cys Gly Ile Val Pro
               100                 105                 110
   acc act aac ccg act tca act gct atc ttc aaa tcg ctg atc agt ctg            384
   Thr Thr Asn Pro Thr Ser Thr Ala Ile Phe Lys Ser Leu Ile Ser Leu
           115                 120                 125
   aag acc cgt aac gcc att atc ttc tcc ccg cac ccg cgt gca aaa gat            432
   Lys Thr Arg Asn Ala Ile Ile Phe Ser Pro His Pro Arg Ala Lys Asp
       130                 135                 140
   gcc acc aac aaa gcg gct gat atc gtt ctg cag gct gct atc gct gcc            480
   Ala Thr Asn Lys Ala Ala Asp Ile Val Leu Gln Ala Ala Ile Ala Ala
   145                 150                 155                 160
   ggt gct ccg aaa gat ctg atc ggc tgg atc gat caa cct tct gtt gaa            528
   Gly Ala Pro Lys Asp Leu Ile Gly Trp Ile Asp Gln Pro Ser Val Glu
                   165                 170                 175
   ctg tct aac gca ctg atg cac cac cca gac atc aac ctg atc ctc gcg            576
   Leu Ser Asn Ala Leu Met His His Pro Asp Ile Asn Leu Ile Leu Ala
               180                 185                 190
   act ggt ggt ccg ggc atg gtt aaa gcc gca tac agc tcc ggt aaa cca            624
   Thr Gly Gly Pro Gly Met Val Lys Ala Ala Tyr Ser Ser Gly Lys Pro
           195                 200                 205
   gct atc ggt gta ggc gcg ggc aac act cca gtt gtt atc gat gaa act            672
   Ala Ile Gly Val Gly Ala Gly Asn Thr Pro Val Val Ile Asp Glu Thr
       210                 215                 220
   gct gat atc aaa cgt gca gtt gca tct gta ctg atg tcc aaa acc ttc            720
   Ala Asp Ile Lys Arg Ala Val Ala Ser Val Leu Met Ser Lys Thr Phe
   225                 230                 235                 240
   gac aac ggc gta atc tgt gct tct gaa cag tct gtt gtt gtt gtt gac            768
   Asp Asn Gly Val Ile Cys Ala Ser Glu Gln Ser Val Val Val Val Asp
                   245                 250                 255
   tct gtt tat gac gct gta cgt gaa cgt ttt gca acc cac ggc ggc tat            816
   Ser Val Tyr Asp Ala Val Arg Glu Arg Phe Ala Thr His Gly Gly Tyr
               260                 265                 270
   ctg ttg cag ggt aaa gag ctg aaa gct gtt cag gat gtt atc ctg aaa            864
   Leu Leu Gln Gly Lys Glu Leu Lys Ala Val Gln Asp Val Ile Leu Lys
           275                 280                 285
   aac ggt gcg ctg aac gcg gct atc gtt ggt cag cca gcc tat aaa att            912
   Asn Gly Ala Leu Asn Ala Ala Ile Val Gly Gln Pro Ala Tyr Lys Ile
       290                 295                 300
   gct gaa ctg gca ggc ttc tct gta cca gaa aac acc aag att ctg atc            960
   Ala Glu Leu Ala Gly Phe Ser Val Pro Glu Asn Thr Lys Ile Leu Ile
   305                 310                 315                 320
   ggt gaa gtg acc gtt gtt gat gaa agc gaa ccg ttc gca cat gaa aaa           1008
   Gly Glu Val Thr Val Val Asp Glu Ser Glu Pro Phe Ala His Glu Lys
                   325                 330                 335
   ctg tcc ccg act ctg gca atg tac cgc gct aaa gat ttc gaa gac gcg           1056
   Leu Ser Pro Thr Leu Ala Met Tyr Arg Ala Lys Asp Phe Glu Asp Ala
               340                 345                 350
   gta gaa aaa gca gag aaa ctg gtt gct atg ggc ggt atc ggt cat acc           1104
   Val Glu Lys Ala Glu Lys Leu Val Ala Met Gly Gly Ile Gly His Thr
           355                 360                 365
   tct tgc ctg tac act gac cag gat aac caa ccg gct cgc gtt tct tac           1152
   Ser Cys Leu Tyr Thr Asp Gln Asp Asn Gln Pro Ala Arg Val Ser Tyr
       370                 375                 380
   ttc ggt cag aaa atg aaa acg gcg cgt atc ctg att aac acc cca gcg           1200
   Phe Gly Gln Lys Met Lys Thr Ala Arg Ile Leu Ile Asn Thr Pro Ala
   385                 390                 395                 400
   tct cag ggt ggt atc ggt gac ctg tat aac ttc aaa ctc gca cct tcc           1248
   Ser Gln Gly Gly Ile Gly Asp Leu Tyr Asn Phe Lys Leu Ala Pro Ser
                   405                 410                 415
   ctg act ctg ggt tgt ggt tct tgg ggt ggt aac tcc atc tct gaa aac           1296
   Leu Thr Leu Gly Cys Gly Ser Trp Gly Gly Asn Ser Ile Ser Glu Asn
               420                     425                     430
   gtt ggt ccg aaa cac ctg atc aac aag aaa acc gtt gct aag cga gct        1344
   Val Gly Pro Lys His Leu Ile Asn Lys Lys Thr Val Ala Lys Arg Ala
           435                     440                     445
   gaa aac atg ttg tgg cac aaa ctt ccg aaa tct atc tac ttc cgc cgt        1392
   Glu Asn Met Leu Trp His Lys Leu Pro Lys Ser Ile Tyr Phe Arg Arg
       450                     455                     460
   ggc tcc ctg cca atc gcg ctg gat gaa gtg att act gat ggc cac aaa        1440
   Gly Ser Leu Pro Ile Ala Leu Asp Glu Val Ile Thr Asp Gly His Lys
   465                     470                     475                     480
   cgt gcg ctc atc gtg act gac cgc ttc ctg ttc aac aat ggt tat gct        1488
   Arg Ala Leu Ile Val Thr Asp Arg Phe Leu Phe Asn Asn Gly Tyr Ala
                   485                     490                     495
   gat cag atc act tcc gta ctg aaa gca gca ggc gtt gaa act gaa gtc        1536
   Asp Gln Ile Thr Ser Val Leu Lys Ala Ala Gly Val Glu Thr Glu Val
               500                     505                     510
   ttc ttc gaa gta gaa gcg gac ccg acc ctg agc atc gtt cgt aaa ggt        1584
   Phe Phe Glu Val Glu Ala Asp Pro Thr Leu Ser Ile Val Arg Lys Gly
           515                     520                     525
   gca gaa ctg gca aac tcc ttc aaa cca gac gtg att atc gcg ctg ggt        1632
   Ala Glu Leu Ala Asn Ser Phe Lys Pro Asp Val Ile Ile Ala Leu Gly
       530                     535                     540
   ggt ggt tcc ccg atg gac gcc gcg aag atc atg tgg gtt atg tac gaa        1680
   Gly Gly Ser Pro Met Asp Ala Ala Lys Ile Met Trp Val Met Tyr Glu
   545                     550                     555                     560
   cat ccg gaa act cac ttc gaa gag ctg gcg ctg cgc ttt atg gat atc        1728
   His Pro Glu Thr His Phe Glu Glu Leu Ala Leu Arg Phe Met Asp Ile
                   565                     570                     575
   cgt aaa cgt atc tac aag ttc ccg aaa atg ggc gtg aaa gcg aaa atg        1776
   Arg Lys Arg Ile Tyr Lys Phe Pro Lys Met Gly Val Lys Ala Lys Met
               580                     585                     590
   atc gct gtc acc acc act tct ggt aca ggt tct gaa gtc act ccg ttt        1824
   Ile Ala Val Thr Thr Thr Ser Gly Thr Gly Ser Glu Val Thr Pro Phe
           595                     600                     605
   gcg gtt gta act gac gac gct act ggt cag aaa tat ccg ctg gca gac        1872
   Ala Val Val Thr Asp Asp Ala Thr Gly Gln Lys Tyr Pro Leu Ala Asp
       610                     615                     620
   tat gcg ctg act ccg gat atg gcg att gtc gac gcc aac ctg gtt atg        1920
   Tyr Ala Leu Thr Pro Asp Met Ala Ile Val Asp Ala Asn Leu Val Met
   625                     630                     635                     640
   gac atg ccg aag tcc ctg tgt gct ttc ggt ggt ctg gac gca gta act        1968
   Asp Met Pro Lys Ser Leu Cys Ala Phe Gly Gly Leu Asp Ala Val Thr
                   645                     650                     655
   cac gcc atg gaa gct tat gtt tct gta ctg gca tct gag ttc tct gat        2016
   His Ala Met Glu Ala Tyr Val Ser Val Leu Ala Ser Glu Phe Ser Asp
               660                     665                     670
   ggt cag gct ctg cag gca ctg aaa ctg ctg aaa gaa tat ctg cca gcg        2064
   Gly Gln Ala Leu Gln Ala Leu Lys Leu Leu Lys Glu Tyr Leu Pro Ala
           675                     680                     685
   tcc tac cac gaa ggg tct aaa aat ccg gta gcg cgt gaa cgt gtt cac        2112
   Ser Tyr His Glu Gly Ser Lys Asn Pro Val Ala Arg Glu Arg Val His
       690                     695                     700
   agt gca gcg act atc gcg ggt atc gcg ttt gcg aac gcc ttc ctg ggt        2160
   Ser Ala Ala Thr Ile Ala Gly Ile Ala Phe Ala Asn Ala Phe Leu Gly
   705                     710                     715                     720
   gta tgt cac tca atg gcg cac aaa ctg ggt tcc cag ttc cat att ccg        2208
   Val Cys His Ser Met Ala His Lys Leu Gly Ser Gln Phe His Ile Pro
                   725                     730                     735
   cac ggt ctg gca aac gcc ctg ctg att tgt aac gtt att cgc tac aat        2256
   His Gly Leu Ala Asn Ala Leu Leu Ile Cys Asn Val Ile Arg Tyr Asn
               740                     745                     750
   gcg aac gac aac ccg acc aag cag act gca ttc agc cag tat gac cgt        2304
   Ala Asn Asp Asn Pro Thr Lys Gln Thr Ala Phe Ser Gln Tyr Asp Arg
           755                 760                 765
   ccg cag gct cgc cgt cgt tat gct gaa att gcc gac cac ttg ggt ctg        2352
   Pro Gln Ala Arg Arg Arg Tyr Ala Glu Ile Ala Asp His Leu Gly Leu
       770                 775                 780
   agc gca ccg ggc gac cgt act gct gct aag atc gag aaa ctg ctg gca        2400
   Ser Ala Pro Gly Asp Arg Thr Ala Ala Lys Ile Glu Lys Leu Leu Ala
   785                 790                 795                 800
   tgg ctg gaa acg ctg aaa gct gaa ctg ggt att ccg aaa tct atc cgt        2448
   Trp Leu Glu Thr Leu Lys Ala Glu Leu Gly Ile Pro Lys Ser Ile Arg
                   805                 810                 815
   gaa gct ggc gtt cag gaa gca gac ttc ctg gcg aac gtg gat aaa ctg        2496
   Glu Ala Gly Val Gln Glu Ala Asp Phe Leu Ala Asn Val Asp Lys Leu
               820                 825                 830
   tct gaa gat gca ttc gat gac cag tgc acc ggc gct aac ccg cgt tac        2544
   Ser Glu Asp Ala Phe Asp Asp Gln Cys Thr Gly Ala Asn Pro Arg Tyr
           835                 840                 845
   ccg ctg atc tcc gag ctg aaa cag att ctg ctg gat acc tac tac ggt        2592
   Pro Leu Ile Ser Glu Leu Lys Gln Ile Leu Leu Asp Thr Tyr Tyr Gly
       850                 855                 860
   cgt gat tat gta gaa ggt gaa act gca gcg aag aaa gaa gct gct ccg        2640
   Arg Asp Tyr Val Glu Gly Glu Thr Ala Ala Lys Lys Glu Ala Ala Pro
   865                 870                 875                 880
   gct aaa gct gag aaa aaa gcg aaa aaa tcc gct taa                        2676
   Ala Lys Ala Glu Lys Lys Ala Lys Lys Ser Ala
                   885                 890
<210> 22
   <211> 891
   <212> PRT
   <213> Escherichia coli
<400> 22
   Met Ala Val Thr Asn Val Ala Glu Leu Asn Ala Leu Val Glu Arg Val
    1               5                  10                  15
   Lys Lys Ala Gln Arg Glu Tyr Ala Ser Phe Thr Gln Glu Gln Val Asp
               20                  25                  30
   Lys Ile Phe Arg Ala Ala Ala Leu Ala Ala Ala Asp Ala Arg Ile Pro
           35                  40                  45
   Leu Ala Lys Met Ala Val Ala Glu Ser Gly Met Gly Ile Val Glu Asp
       50                  55                  60
   Lys Val Ile Lys Asn His Phe Ala Ser Glu Tyr Ile Tyr Asn Ala Tyr
   65                  70                  75                  80
   Lys Asp Glu Lys Thr Cys Gly Val Leu Ser Glu Asp Asp Thr Phe Gly
                   85                  90                  95
   Thr Ile Thr Ile Ala Glu Pro Ile Gly Ile Ile Cys Gly Ile Val Pro
               100                 105                 110
   Thr Thr Asn Pro Thr Ser Thr Ala Ile Phe Lys Ser Leu Ile Ser Leu
           115                 120                 125
   Lys Thr Arg Asn Ala Ile Ile Phe Ser Pro His Pro Arg Ala Lys Asp
       130                 135                 140
   Ala Thr Asn Lys Ala Ala Asp Ile Val Leu Gln Ala Ala Ile Ala Ala
   145                 150                 155                 160
   Gly Ala Pro Lys Asp Leu Ile Gly Trp Ile Asp Gln Pro Ser Val Glu
                   165                 170                 175
   Leu Ser Asn Ala Leu Met His His Pro Asp Ile Asn Leu Ile Leu Ala
               180                 185                 190
   Thr Gly Gly Pro Gly Met Val Lys Ala Ala Tyr Ser Ser Gly Lys Pro
           195                 200                 205
   Ala Ile Gly Val Gly Ala Gly Asn Thr Pro Val Val Ile Asp Glu Thr
       210                 215                 220
   Ala Asp Ile Lys Arg Ala Val Ala Ser Val Leu Met Ser Lys Thr Phe
   225                 230                 235                 240
   Asp Asn Gly Val Ile Cys Ala Ser Glu Gln Ser Val Val Val Val Asp
                   245                 250                 255
   Ser Val Tyr Asp Ala Val Arg Glu Arg Phe Ala Thr His Gly Gly Tyr
               260                 265                 270
   Leu Leu Gln Gly Lys Glu Leu Lys Ala Val Gln Asp Val Ile Leu Lys
           275                 280                 285
   Asn Gly Ala Leu Asn Ala Ala Ile Val Gly Gln Pro Ala Tyr Lys Ile
       290                 295                 300
   Ala Glu Leu Ala Gly Phe Ser Val Pro Glu Asn Thr Lys Ile Leu Ile
   305                 310                 315                 320
   Gly Glu Val Thr Val Val Asp Glu Ser Glu Pro Phe Ala His Glu Lys
                   325                 330                 335
   Leu Ser Pro Thr Leu Ala Met Tyr Arg Ala Lys Asp Phe Glu Asp Ala
               340                 345                 350
   Val Glu Lys Ala Glu Lys Leu Val Ala Met Gly Gly Ile Gly His Thr
           355                 360                 365
   Ser Cys Leu Tyr Thr Asp Gln Asp Asn Gln Pro Ala Arg Val Ser Tyr
       370                 375                 380
   Phe Gly Gln Lys Met Lys Thr Ala Arg Ile Leu Ile Asn Thr Pro Ala
   385                 390                 395                 400
   Ser Gln Gly Gly Ile Gly Asp Leu Tyr Asn Phe Lys Leu Ala Pro Ser
                   405                 410                 415
   Leu Thr Leu Gly Cys Gly Ser Trp Gly Gly Asn Ser Ile Ser Glu Asn
               420                 425                 430
   Val Gly Pro Lys His Leu Ile Asn Lys Lys Thr Val Ala Lys Arg Ala
           435                 440                 445
   Glu Asn Met Leu Trp His Lys Leu Pro Lys Ser Ile Tyr Phe Arg Arg
       450                 455                 460
   Gly Ser Leu Pro Ile Ala Leu Asp Glu Val Ile Thr Asp Gly His Lys
   465                 470                 475                 480
   Arg Ala Leu Ile Val Thr Asp Arg Phe Leu Phe Asn Asn Gly Tyr Ala
                   485                 490                 495
   Asp Gln Ile Thr Ser Val Leu Lys Ala Ala Gly Val Glu Thr Glu Val
               500                 505                 510
   Phe Phe Glu Val Glu Ala Asp Pro Thr Leu Ser Ile Val Arg Lys Gly
           515                 520                 525
   Ala Glu Leu Ala Asn Ser Phe Lys Pro Asp Val Ile Ile Ala Leu Gly
       530                 535                 540
   Gly Gly Ser Pro Met Asp Ala Ala Lys Ile Met Trp Val Met Tyr Glu
   545                 550                 555                 560
   His Pro Glu Thr His Phe Glu Glu Leu Ala Leu Arg Phe Met Asp Ile
                   565                 570                 575
   Arg Lys Arg Ile Tyr Lys Phe Pro Lys Met Gly Val Lys Ala Lys Met
               580                 585                 590
   Ile Ala Val Thr Thr Thr Ser Gly Thr Gly Ser Glu Val Thr Pro Phe
           595                 600                 605
   Ala Val Val Thr Asp Asp Ala Thr Gly Gln Lys Tyr Pro Leu Ala Asp
       610                 615                 620
   Tyr Ala Leu Thr Pro Asp Met Ala Ile Val Asp Ala Asn Leu Val Met
   625                 630                 635                 640
   Asp Met Pro Lys Ser Leu Cys Ala Phe Gly Gly Leu Asp Ala Val Thr
                   645                 650                 655
   His Ala Met Glu Ala Tyr Val Ser Val Leu Ala Ser Glu Phe Ser Asp
               660                 665                 670
   Gly Gln Ala Leu Gln Ala Leu Lys Leu Leu Lys Glu Tyr Leu Pro Ala
           675                 680                 685
   Ser Tyr His Glu Gly Ser Lys Asn Pro Val Ala Arg Glu Arg Val His
       690                 695                 700
   Ser Ala Ala Thr Ile Ala Gly Ile Ala Phe Ala Asn Ala Phe Leu Gly
   705                 710                 715                 720
   Val Cys His Ser Met Ala His Lys Leu Gly Ser Gln Phe His Ile Pro
                   725                 730                 735
   His Gly Leu Ala Asn Ala Leu Leu Ile Cys Asn Val Ile Arg Tyr Asn
               740                 745                 750
   Ala Asn Asp Asn Pro Thr Lys Gln Thr Ala Phe Ser Gln Tyr Asp Arg
           755                 760                 765
   Pro Gln Ala Arg Arg Arg Tyr Ala Glu Ile Ala Asp His Leu Gly Leu
       770                 775                 780
   Ser Ala Pro Gly Asp Arg Thr Ala Ala Lys Ile Glu Lys Leu Leu Ala
   785                 790                 795                 800
   Trp Leu Glu Thr Leu Lys Ala Glu Leu Gly Ile Pro Lys Ser Ile Arg
                   805                 810                 815
   Glu Ala Gly Val Gln Glu Ala Asp Phe Leu Ala Asn Val Asp Lys Leu
               820                 825                 830
   Ser Glu Asp Ala Phe Asp Asp Gln Cys Thr Gly Ala Asn Pro Arg Tyr
           835                 840                 845
   Pro Leu Ile Ser Glu Leu Lys Gln Ile Leu Leu Asp Thr Tyr Tyr Gly
       850                 855                 860
   Arg Asp Tyr Val Glu Gly Glu Thr Ala Ala Lys Lys Glu Ala Ala Pro
   865                 870                 875                 880
   Ala Lys Ala Glu Lys Lys Ala Lys Lys Ser Ala
                   885                 890
<210> 23
   <211> 69
   <212> DNA
   <213> Artificial sequence
<220>
   <223> primer
<400> 23
   cgttattgtt atctagttgt gcaaaacatg ctaatgtagc attacgcccc gccctgccac     60
   tcatcgcag                                                              69
<210> 24
   <211> 58
   <212> DNA
   <213> Artificial sequence
<220>
   <223> primer
<400> 24
   attagtaaca gccataatgc tctcctgata atgttaaacc gctcacaatt ccacacat 58
<210> 25
   <211> 183
   <212> DNA
   <213> Artificial sequence
<220>
   <223> hybrid promoter
<400> 25
   ctagatctct cacctaccaa acaatgcccc cctgcaaaaa ataaattcat aaaaaacata     60
   cagataacca tctgcggtga taaattatct ctggcggtgt tgacaattaa tcatcggctc    120
   gtataatgtg tggaattgtg agcggtttaa cattatcagg agagcattat ggctgttact    180
   aat                                                                  183
<210> 26
   <211> 24
   <212> DNA
   <213> Artificial sequence
<220>
   <223> primer
<400> 26
   acttgttctt gagtgaaact ggca 24
<210> 27
   <211> 22
   <212> DNA
   <213> Artificial sequence
<220>
   <223> primer
<400> 27
   aagacgcgct gacaatacgc ct 22
<210> 28
   <211> 69
   <212> DNA
   <213> Artificial sequence
<220>
   <223> primer
<400> 28
   cgttattgtt atctagttgt gcaaaacatg ctaatgtagc atcagaaaaa ctcatcgagc     60
   atcaaatga                                                             69
<210> 29
   <211> 65
   <212> DNA
   <213> Artificial sequence
<220>
   <223> primer
<400> 29
   agccggagca gcttctttct tcgctgcagt ttcaccttct acgttgtgtc tcaaaatctc     60
   tgatg                                                                 65
<210> 30
   <211> 24
   <212> DNA
   <213> Artificial sequence
<220>
   <223> primer
<400> 30
   aagacgcgct gacaatacgc cttt 24
<210> 31
   <211> 24
   <212> DNA
   <213> Artificial sequence
<220>
   <223> primer
<400> 31
   aaggggccgt ttatgttgcc agac 24
<210> 32
   <211> 69
   <212> DNA
   <213> Artificial sequence
<220>
   <223> primer
<400> 32
   catgtgggtt atgtacgaac atccggaaac tcacttcgaa aagctggcgc tgcgctttat 60
   ggatatccg 69
<210> 33
   <211> 24
   <212> DNA
   <213> Artificial sequence
<220>
   <223> primer
<400> 33
   aaggggccgt ttatgttgcc agac 24
<210> 34
   <211> 24
   <212> DNA
   <213> Artificial sequence
<220>
   <223> primer
<400> 34
   aagacgcgct gacaatacgc cttt 24
<210> 35
   <211> 51
   <212> DNA
   <213> Artificial sequence
<220>
   <223> primer
<400> 35
   ttttctagat aaggaggaat gacgtatgac ccggacattc aagacaatgg a 51
<210> 36
   <211> 39
   <212> DNA
   <213> Artificial sequence
<220>
   <223> primer
<400> 36
   aaatctagat tcagcttatg cttcgccttc aatcgaacg 39
<210> 37
   <211> 344
   <212> DNA
   <213> Escherichia coli
<400> 37
   aagctttacg cgaacgagcc atgacattgc tgacgactct ggcagtggca gatgacataa     60
   aactggtcga ctggttacaa caacgcctgg ggcttttaga gcaacgagac acggcaatgt    120
   tgcaccgttt gctgcatgat attgaaaaaa atatcaccaa ataaaaaacg ccttagtaag    180
   tatttttcag cttttcattc tgactgcaac gggcaatatg tctctgtgtg gattaaaaaa    240
   agagtgtctg atagcagctt ctgaactggt tacctgccgt gagtaaatta aaattttatt    300
   gacttaggtc actaaatact ttaaccaata taggcgactc taga                     344
<210> 38
   <211> 53
   <212> DNA
   <213> Artificial sequence
<220>
   <223> primer
<400> 38
   ttttctggta cctaaggagg aatgacgtat gattactatt gacggtaatg gcg 53
<210> 39
   <211> 40
   <212> DNA
   <213> Artificial sequence
<220>
   <223> primer
<400> 39
   gagagaccgg gtaccttaat cggtgttgct tttttccgct 40
<210> 40
   <211> 49
   <212> DNA
   <213> Artificial sequence
<220>
   <223> primer
<400> 40
   aattggtacc taaggaggaa tgacgtatga ccagtggccc aaaaccggc 49
<210> 41
   <211> 42
   <212> DNA
   <213> Artificial sequence
<220>
   <223> primer
<400> 41
   gcgacgccta gcttagggta ccttaccatg cctcgatatt gt 42
<210> 42
   <211> 35
   <212> DNA
   <213> Artificial sequence
<220>
   <223> primer
<400> 42
   ctctctggcc ccgggctgat tgatttgatc gattg 35
<210> 43
   <211> 33
   <212> DNA
   <213> Artificial sequence
<220>
   <223> primer
<400> 43
   gagagacccc gggttaccag taatgctccg ctg 33
<210> 44
   <211> 30
   <212> DNA
   <213> Artificial sequence
<220>
   <223> primer
<400> 44
   gcgcgaattc gggcgatgat gttgacgcca 30
<210> 45
   <211> 30
   <212> DNA
   <213> Artificial sequence
<220>
   <223> primer
<400> 45
   gcgcgaattc gtcccatact aacctctgtt 30
<210> 46
   <211> 2664
   <212> DNA
   <213> Escherichia coli
<220>
   <221> CDS
   <222> (1)..(2664)
<400> 46
   atg tca gaa cgt ttc cca aat gac gtg gat ccg atc gaa act cgc gac       48
   Met Ser Glu Arg Phe Pro Asn Asp Val Asp Pro Ile Glu Thr Arg Asp
   1               5                   10                  15
   tgg ctc cag gcg atc gaa tcg gtc atc cgt gaa gaa ggt gtt gag cgt       96
   Trp Leu Gln Ala Ile Glu Ser Val Ile Arg Glu Glu Gly Val Glu Arg
               20                  25                  30
   gct cag tat ctg atc gac caa ctg ctt gct gaa gcc cgc aaa ggc ggt      144
   Ala Gln Tyr Leu Ile Asp Gln Leu Leu Ala Glu Ala Arg Lys Gly Gly
           35                  40                  45
   gta aac gta gcc gca ggc aca ggt atc agc aac tac atc aac acc atc      192
   Val Asn Val Ala Ala Gly Thr Gly Ile Ser Asn Tyr Ile Asn Thr Ile
       50                  55                  60
   ccc gtt gaa gaa caa ccg gag tat ccg ggt aat ctg gaa ctg gaa cgc      240
   Pro Val Glu Glu Gln Pro Glu Tyr Pro Gly Asn Leu Glu Leu Glu Arg
   65                  70                  75                  80
   cgt att cgt tca gct atc cgc tgg aac gcc atc atg acg gtg ctg cgt      288
   Arg Ile Arg Ser Ala Ile Arg Trp Asn Ala Ile Met Thr Val Leu Arg
                   85                  90                  95
   gcg tcg aaa aaa gac ctc gaa ctg ggc ggc cat atg gcg tcc ttc cag      336
   Ala Ser Lys Lys Asp Leu Glu Leu Gly Gly His Met Ala Ser Phe Gln
               100                 105                 110
   tct tcc gca acc att tat gat gtg tgc ttt aac cac ttc ttc cgt gca      384
   Ser Ser Ala Thr Ile Tyr Asp Val Cys Phe Asn His Phe Phe Arg Ala
           115                 120                 125
   cgc aac gag cag gat ggc ggc gac ctg gtt tac ttc cag ggc cac atc      432
   Arg Asn Glu Gln Asp Gly Gly Asp Leu Val Tyr Phe Gln Gly His Ile
       130                 135                 140
   tcc ccg ggc gtg tac gct cgt gct ttc ctg gaa ggt cgt ctg act cag      480
   Ser Pro Gly Val Tyr Ala Arg Ala Phe Leu Glu Gly Arg Leu Thr Gln
   145                 150                 155                 160
   gag cag ctg gat aac ttc cgt cag gaa gtt cac ggc aat ggc ctc tct      528
   Glu Gln Leu Asp Asn Phe Arg Gln Glu Val His Gly Asn Gly Leu Ser
                   165                 170                 175
   tcc tat ccg cac ccg aaa ctg atg ccg gaa ttc tgg cag ttc ccg acc      576
   Ser Tyr Pro His Pro Lys Leu Met Pro Glu Phe Trp Gln Phe Pro Thr
               180                 185                 190
   gta tct atg ggt ctg ggt ccg att ggt gct att tac cag gct aaa ttc      624
   Val Ser Met Gly Leu Gly Pro Ile Gly Ala Ile Tyr Gln Ala Lys Phe
           195                 200                 205
   ctg aaa tat ctg gaa cac cgt ggc ctg aaa gat acc tct aaa caa acc      672
   Leu Lys Tyr Leu Glu His Arg Gly Leu Lys Asp Thr Ser Lys Gln Thr
       210                 215                 220
   gtt tac gcg ttc ctc ggt gac ggt gaa atg gac gaa ccg gaa tcc aaa      720
   Val Tyr Ala Phe Leu Gly Asp Gly Glu Met Asp Glu Pro Glu Ser Lys
   225                 230                 235                 240
   ggt gcg atc acc atc gct acc cgt gaa aaa ctg gat aac ctg gtc ttc      768
   Gly Ala Ile Thr Ile Ala Thr Arg Glu Lys Leu Asp Asn Leu Val Phe
                   245                 250                 255
   gtt atc aac tgt aac ctg cag cgt ctt gac ggc ccg gtc acc ggt aac      816
   Val Ile Asn Cys Asn Leu Gln Arg Leu Asp Gly Pro Val Thr Gly Asn
               260                 265                 270
   ggc aag atc atc aac gaa ctg gaa ggc atc ttc gaa ggt gct ggc tgg      864
   Gly Lys Ile Ile Asn Glu Leu Glu Gly Ile Phe Glu Gly Ala Gly Trp
           275                 280                 285
   aac gtg atc aaa gtg atg tgg ggt agc cgt tgg gat gaa ctg ctg cgt      912
   Asn Val Ile Lys Val Met Trp Gly Ser Arg Trp Asp Glu Leu Leu Arg
       290                 295                 300
   aag gat acc agc ggt aaa ctg atc cag ctg atg aac gaa acc gtt gac      960
   Lys Asp Thr Ser Gly Lys Leu Ile Gln Leu Met Asn Glu Thr Val Asp
   305                 310                 315                 320
   ggc gac tac cag acc ttc aaa tcg aaa gat ggt gcg tac gtt cgt gaa     1008
   Gly Asp Tyr Gln Thr Phe Lys Ser Lys Asp Gly Ala Tyr Val Arg Glu
                   325                 330                 335
   cac ttc ttc ggt aaa tat cct gaa acc gca gca ctg gtt gca gac tgg     1056
   His Phe Phe Gly Lys Tyr Pro Glu Thr Ala Ala Leu Val Ala Asp Trp
               340                 345                 350
   act gac gag cag atc tgg gca ctg aac cgt ggt ggt cac gat ccg aag     1104
   Thr Asp Glu Gln Ile Trp Ala Leu Asn Arg Gly Gly His Asp Pro Lys
           355                 360                 365
   aaa atc tac gct gca ttc aag aaa gcg cag gaa acc aaa ggc aaa gcg     1152
   Lys Ile Tyr Ala Ala Phe Lys Lys Ala Gln Glu Thr Lys Gly Lys Ala
       370                 375                 380
   aca gta atc ctt gct cat acc att aaa ggt tac ggc atg ggc gac gcg     1200
   Thr Val Ile Leu Ala His Thr Ile Lys Gly Tyr Gly Met Gly Asp Ala
   385                 390                 395                 400
   gct gaa ggt aaa aac atc gcg cac cag gtt aag aaa atg aac atg gac     1248
   Ala Glu Gly Lys Asn Ile Ala His Gln Val Lys Lys Met Asn Met Asp
                   405                 410                 415
   ggt gtg cgt cat atc cgc gac cgt ttc aat gtg ccg gtg tct gat gca     1296
   Gly Val Arg His Ile Arg Asp Arg Phe Asn Val Pro Val Ser Asp Ala
               420                 425                 430
   gat atc gaa aaa ctg ccg tac atc acc ttc ccg gaa ggt tct gaa gag     1344
   Asp Ile Glu Lys Leu Pro Tyr Ile Thr Phe Pro Glu Gly Ser Glu Glu
           435                 440                 445
   cat acc tat ctg cac gct cag cgt cag aaa ctg cac ggt tat ctg cca     1392
   His Thr Tyr Leu His Ala Gln Arg Gln Lys Leu His Gly Tyr Leu Pro
       450                 455                 460
   agc cgt cag ccg aac ttc acc gag aag ctt gag ctg ccg agc ctg caa     1440
   Ser Arg Gln Pro Asn Phe Thr Glu Lys Leu Glu Leu Pro Ser Leu Gln
   465                 470                 475                 480
   gac ttc ggc gcg ctg ttg gaa gag cag agc aaa gag atc tct acc act     1488
   Asp Phe Gly Ala Leu Leu Glu Glu Gln Ser Lys Glu Ile Ser Thr Thr
                   485                 490                 495
   atc gct ttc gtt cgt gct ctg aac gtg atg ctg aag aac aag tcg atc     1536
   Ile Ala Phe Val Arg Ala Leu Asn Val Met Leu Lys Asn Lys Ser Ile
               500                 505                 510
   aaa gat cgt ctg gta ccg atc atc gcc gac gaa gcg cgt act ttc ggt     1584
   Lys Asp Arg Leu Val Pro Ile Ile Ala Asp Glu Ala Arg Thr Phe Gly
           515                 520                 525
   atg gaa ggt ctg ttc cgt cag att ggt att tac agc ccg aac ggt cag     1632
   Met Glu Gly Leu Phe Arg Gln Ile Gly Ile Tyr Ser Pro Asn Gly Gln
       530                 535                 540
   cag tac acc ccg cag gac cgc gag cag gtt gct tac tat aaa gaa gac     1680
   Gln Tyr Thr Pro Gln Asp Arg Glu Gln Val Ala Tyr Tyr Lys Glu Asp
   545                 550                 555                 560
   gag aaa ggt cag att ctg cag gaa ggg atc aac gag ctg ggc gca ggt      1728
   Glu Lys Gly Gln Ile Leu Gln Glu Gly Ile Asn Glu Leu Gly Ala Gly
                   565                 570                 575
   tgt tcc tgg ctg gca gcg gcg acc tct tac agc acc aac aat ctg ccg      1776
   Cys Ser Trp Leu Ala Ala Ala Thr Ser Tyr Ser Thr Asn Asn Leu Pro
               580                 585                 590
   atg atc ccg ttc tac atc tat tac tcg atg ttc ggc ttc cag cgt att      1824
   Met Ile Pro Phe Tyr Ile Tyr Tyr Ser Met Phe Gly Phe Gln Arg Ile
           595                 600                 605
   ggc gat ctg tgc tgg gcg gct ggc gac cag caa gcg cgt ggc ttc ctg      1872
   Gly Asp Leu Cys Trp Ala Ala Gly Asp Gln Gln Ala Arg Gly Phe Leu
       610                 615                 620
   atc ggc ggt act tcc ggt cgt acc acc ctg aac ggc gaa ggt ctg cag      1920
   Ile Gly Gly Thr Ser Gly Arg Thr Thr Leu Asn Gly Glu Gly Leu Gln
   625                 630                 635                 640
   cac gaa gat ggt cac agc cac att cag tcg ctg act atc ccg aac tgt      1968
   His Glu Asp Gly His Ser His Ile Gln Ser Leu Thr Ile Pro Asn Cys
                   645                 650                 655
   atc tct tac gac ccg gct tac gct tac gaa gtt gct gtc atc atg cat      2016
   Ile Ser Tyr Asp Pro Ala Tyr Ala Tyr Glu Val Ala Val Ile Met His
               660                 665                 670
   gac ggt ctg gag cgt atg tac ggt gaa aaa caa gag aac gtt tac tac      2064
   Asp Gly Leu Glu Arg Met Tyr Gly Glu Lys Gln Glu Asn Val Tyr Tyr
           675                 680                 685
   tac atc act acg ctg aac gaa aac tac cac atg ccg gca atg ccg gaa      2112
   Tyr Ile Thr Thr Leu Asn Glu Asn Tyr His Met Pro Ala Met Pro Glu
       690                 695                 700
   ggt gct gag gaa ggt atc cgt aaa ggt atc tac aaa ctc gaa act att      2160
   Gly Ala Glu Glu Gly Ile Arg Lys Gly Ile Tyr Lys Leu Glu Thr Ile
   705                 710                 715                 720
   gaa ggt agc aaa ggt aaa gtt cag ctg ctc ggc tcc ggt tct atc ctg      2208
   Glu Gly Ser Lys Gly Lys Val Gln Leu Leu Gly Ser Gly Ser Ile Leu
                   725                 730                 735
   cgt cac gtc cgt gaa gca gct gag atc ctg gcg aaa gat tac ggc gta      2256
   Arg His Val Arg Glu Ala Ala Glu Ile Leu Ala Lys Asp Tyr Gly Val
               740                 745                 750
   ggt tct gac gtt tat agc gtg acc tcc ttc acc gag ctg gcg cgt gat      2304
   Gly Ser Asp Val Tyr Ser Val Thr Ser Phe Thr Glu Leu Ala Arg Asp
           755                 760                 765
   ggt cag gat tgt gaa cgc tgg aac atg ctg cac ccg ctg gaa act ccg      2352
   Gly Gln Asp Cys Glu Arg Trp Asn Met Leu His Pro Leu Glu Thr Pro
       770                 775                 780
   cgc gtt ccg tat atc gct cag gtg atg aac gac gct ccg gca gtg gca      2400
   Arg Val Pro Tyr Ile Ala Gln Val Met Asn Asp Ala Pro Ala Val Ala
   785                 790                 795                 800
   tct acc gac tat atg aaa ctg ttc gct gag cag gtc cgt act tac gta      2448
   Ser Thr Asp Tyr Met Lys Leu Phe Ala Glu Gln Val Arg Thr Tyr Val
                   805                 810                 815
   ccg gct gac gac tac cgc gta ctg ggt act gat ggc ttc ggt cgt tcc      2496
   Pro Ala Asp Asp Tyr Arg Val Leu Gly Thr Asp Gly Phe Gly Arg Ser
               820                 825                 830
   gac agc cgt gag aac ctg cgt cac cac ttc gaa gtt gat gct tct tat      2544
   Asp Ser Arg Glu Asn Leu Arg His His Phe Glu Val Asp Ala Ser Tyr
           835                 840                 845
   gtc gtg gtt gcg gcg ctg ggc gaa ctg gct aaa cgt ggc gaa atc gat      2592
   Val Val Val Ala Ala Leu Gly Glu Leu Ala Lys Arg Gly Glu Ile Asp
       850                 855                 860
   aag aaa gtg gtt gct gac gca atc gcc aaa ttc aac atc gat gca gat      2640
   Lys Lys Val Val Ala Asp Ala Ile Ala Lys Phe Asn Ile Asp Ala Asp
   865                 870                 875                 880
   aaa gtt aac ccg cgt ctg gcg taa                                      2664
   Lys Val Asn Pro Arg Leu Ala
   885
<210> 47
   <211> 887
   <212> PRT
   <213> Escherichia coli
<400> 47
   Met Ser Glu Arg Phe Pro Asn Asp Val Asp Pro Ile Glu Thr Arg Asp
   1               5                   10                  15
   Trp Leu Gln Ala Ile Glu Ser Val Ile Arg Glu Glu Gly Val Glu Arg
               20                  25                  30
   Ala Gln Tyr Leu Ile Asp Gln Leu Leu Ala Glu Ala Arg Lys Gly Gly
           35                  40                  45
   Val Asn Val Ala Ala Gly Thr Gly Ile Ser Asn Tyr Ile Asn Thr Ile
       50                  55                  60
   Pro Val Glu Glu Gln Pro Glu Tyr Pro Gly Asn Leu Glu Leu Glu Arg
   65                  70                  75                  80
   Arg Ile Arg Ser Ala Ile Arg Trp Asn Ala Ile Met Thr Val Leu Arg
                   85                  90                  95
   Ala Ser Lys Lys Asp Leu Glu Leu Gly Gly His Met Ala Ser Phe Gln
               100                 105                 110
   Ser Ser Ala Thr Ile Tyr Asp Val Cys Phe Asn His Phe Phe Arg Ala
           115                 120                 125
   Arg Asn Glu Gln Asp Gly Gly Asp Leu Val Tyr Phe Gln Gly His Ile
       130                 135                 140
   Ser Pro Gly Val Tyr Ala Arg Ala Phe Leu Glu Gly Arg Leu Thr Gln
   145                 150                 155                 160
   Glu Gln Leu Asp Asn Phe Arg Gln Glu Val His Gly Asn Gly Leu Ser
                   165                 170                 175
   Ser Tyr Pro His Pro Lys Leu Met Pro Glu Phe Trp Gln Phe Pro Thr
               180                 185                 190
   Val Ser Met Gly Leu Gly Pro Ile Gly Ala Ile Tyr Gln Ala Lys Phe
           195                 200                 205
   Leu Lys Tyr Leu Glu His Arg Gly Leu Lys Asp Thr Ser Lys Gln Thr
       210                 215                 220
   Val Tyr Ala Phe Leu Gly Asp Gly Glu Met Asp Glu Pro Glu Ser Lys
   225                 230                 235                 240
   Gly Ala Ile Thr Ile Ala Thr Arg Glu Lys Leu Asp Asn Leu Val Phe
                   245                 250                 255
   Val Ile Asn Cys Asn Leu Gln Arg Leu Asp Gly Pro Val Thr Gly Asn
               260                 265                 270
   Gly Lys Ile Ile Asn Glu Leu Glu Gly Ile Phe Glu Gly Ala Gly Trp
           275                 280                 285
   Asn Val Ile Lys Val Met Trp Gly Ser Arg Trp Asp Glu Leu Leu Arg
       290                 295                 300
   Lys Asp Thr Ser Gly Lys Leu Ile Gln Leu Met Asn Glu Thr Val Asp
   305                 310                 315                 320
   Gly Asp Tyr Gln Thr Phe Lys Ser Lys Asp Gly Ala Tyr Val Arg Glu
                   325                 330                 335
   His Phe Phe Gly Lys Tyr Pro Glu Thr Ala Ala Leu Val Ala Asp Trp
               340                 345                 350
   Thr Asp Glu Gln Ile Trp Ala Leu Asn Arg Gly Gly His Asp Pro Lys
           355                 360                 365
   Lys Ile Tyr Ala Ala Phe Lys Lys Ala Gln Glu Thr Lys Gly Lys Ala
       370                 375                 380
   Thr Val Ile Leu Ala His Thr Ile Lys Gly Tyr Gly Met Gly Asp Ala
   385                 390                 395                 400
   Ala Glu Gly Lys Asn Ile Ala His Gln Val Lys Lys Met Asn Met Asp
                   405                 410                 415
   Gly Val Arg His Ile Arg Asp Arg Phe Asn Val Pro Val Ser Asp Ala
               420                 425                 430
   Asp Ile Glu Lys Leu Pro Tyr Ile Thr Phe Pro Glu Gly Ser Glu Glu
           435                 440                 445
   His Thr Tyr Leu His Ala Gln Arg Gln Lys Leu His Gly Tyr Leu Pro
       450                 455                 460
   Ser Arg Gln Pro Asn Phe Thr Glu Lys Leu Glu Leu Pro Ser Leu Gln
   465                 470                 475                 480
   Asp Phe Gly Ala Leu Leu Glu Glu Gln Ser Lys Glu Ile Ser Thr Thr
                   485                 490                 495
   Ile Ala Phe Val Arg Ala Leu Asn Val Met Leu Lys Asn Lys Ser Ile
               500                 505                 510
   Lys Asp Arg Leu Val Pro Ile Ile Ala Asp Glu Ala Arg Thr Phe Gly
           515                 520                 525
   Met Glu Gly Leu Phe Arg Gln Ile Gly Ile Tyr Ser Pro Asn Gly Gln
       530                 535                 540
   Gln Tyr Thr Pro Gln Asp Arg Glu Gln Val Ala Tyr Tyr Lys Glu Asp
   545                 550                 555                 560
   Glu Lys Gly Gln Ile Leu Gln Glu Gly Ile Asn Glu Leu Gly Ala Gly
                   565                 570                 575
   Cys Ser Trp Leu Ala Ala Ala Thr Ser Tyr Ser Thr Asn Asn Leu Pro
               580                 585                 590
   Met Ile Pro Phe Tyr Ile Tyr Tyr Ser Met Phe Gly Phe Gln Arg Ile
           595                 600                 605
   Gly Asp Leu Cys Trp Ala Ala Gly Asp Gln Gln Ala Arg Gly Phe Leu
       610                 615                 620
   Ile Gly Gly Thr Ser Gly Arg Thr Thr Leu Asn Gly Glu Gly Leu Gln
   625                 630                 635                 640
   His Glu Asp Gly His Ser His Ile Gln Ser Leu Thr Ile Pro Asn Cys
                   645                 650                 655
   Ile Ser Tyr Asp Pro Ala Tyr Ala Tyr Glu Val Ala Val Ile Met His
               660                 665                 670
   Asp Gly Leu Glu Arg Met Tyr Gly Glu Lys Gln Glu Asn Val Tyr Tyr
           675                 680                 685
   Tyr Ile Thr Thr Leu Asn Glu Asn Tyr His Met Pro Ala Met Pro Glu
       690                 695                 700
   Gly Ala Glu Glu Gly Ile Arg Lys Gly Ile Tyr Lys Leu Glu Thr Ile
   705                 710                 715                 720
   Glu Gly Ser Lys Gly Lys Val Gln Leu Leu Gly Ser Gly Ser Ile Leu
                   725                 730                 735
   Arg His Val Arg Glu Ala Ala Glu Ile Leu Ala Lys Asp Tyr Gly Val
               740                 745                 750
   Gly Ser Asp Val Tyr Ser Val Thr Ser Phe Thr Glu Leu Ala Arg Asp
           755                 760                 765
   Gly Gln Asp Cys Glu Arg Trp Asn Met Leu His Pro Leu Glu Thr Pro
       770                 775                 780
   Arg Val Pro Tyr Ile Ala Gln Val Met Asn Asp Ala Pro Ala Val Ala
   785                 790                 795                 800
   Ser Thr Asp Tyr Met Lys Leu Phe Ala Glu Gln Val Arg Thr Tyr Val
                   805                 810                 815
   Pro Ala Asp Asp Tyr Arg Val Leu Gly Thr Asp Gly Phe Gly Arg Ser
               820                 825                 830
   Asp Ser Arg Glu Asn Leu Arg His His Phe Glu Val Asp Ala Ser Tyr
           835                 840                 845
   Val Val Val Ala Ala Leu Gly Glu Leu Ala Lys Arg Gly Glu Ile Asp
       850                 855                 860
   Lys Lys Val Val Ala Asp Ala Ile Ala Lys Phe Asn Ile Asp Ala Asp
   865                 870                 875                 880
   Lys Val Asn Pro Arg Leu Ala
                   885
<210> 48
   <211> 1893
   <212> DNA
   <213> Escherichia coli
<220>
   <221> CDS
   <222> (1)..(1893)
<400> 48
   atg gct atc gaa atc aaa gta ccg gac atc ggg gct gat gaa gtt gaa        48
   Met Ala Ile Glu Ile Lys Val Pro Asp Ile Gly Ala Asp Glu Val Glu
   1               5                   10                  15
   atc acc gag atc ctg gtc aaa gtg ggc gac aaa gtt gaa gcc gaa cag        96
   Ile Thr Glu Ile Leu Val Lys Val Gly Asp Lys Val Glu Ala Glu Gln
               20                  25                  30
   tcg ctg atc acc gta gaa ggc gac aaa gcc tct atg gaa gtt ccg tct       144
   Ser Leu Ile Thr Val Glu Gly Asp Lys Ala Ser Met Glu Val Pro Ser
           35                  40                  45
   ccg cag gcg ggt atc gtt aaa gag atc aaa gtc tct gtt ggc gat aaa       192
   Pro Gln Ala Gly Ile Val Lys Glu Ile Lys Val Ser Val Gly Asp Lys
       50                  55                  60
   acc cag acc ggc gca ctg att atg att ttc gat tcc gcc gac ggt gca       240
   Thr Gln Thr Gly Ala Leu Ile Met Ile Phe Asp Ser Ala Asp Gly Ala
   65                  70                  75                  80
   gca gac gct gca cct gct cag gca gaa gag aag aaa gaa gca gct ccg       288
   Ala Asp Ala Ala Pro Ala Gln Ala Glu Glu Lys Lys Glu Ala Ala Pro
                   85                  90                  95
   gca gca gca cca gcg gct gcg gcg gca aaa gac gtt aac gtt ccg gat       336
   Ala Ala Ala Pro Ala Ala Ala Ala Ala Lys Asp Val Asn Val Pro Asp
               100                 105                 110
   atc ggc agc gac gaa gtt gaa gtg acc gaa atc ctg gtg aaa gtt ggc       384
   Ile Gly Ser Asp Glu Val Glu Val Thr Glu Ile Leu Val Lys Val Gly
           115                 120                 125
   gat aaa gtt gaa gct gaa cag tcg ctg atc acc gta gaa ggc gac aag       432
   Asp Lys Val Glu Ala Glu Gln Ser Leu Ile Thr Val Glu Gly Asp Lys
       130                 135                 140
   gct tct atg gaa gtt ccg gct ccg ttt gct ggc acc gtg aaa gag atc       480
   Ala Ser Met Glu Val Pro Ala Pro Phe Ala Gly Thr Val Lys Glu Ile
   145                 150                 155                 160
   aaa gtg aac gtg ggt gac aaa gtg tct acc ggc tcg ctg att atg gtc       528
   Lys Val Asn Val Gly Asp Lys Val Ser Thr Gly Ser Leu Ile Met Val
                   165                 170                 175
   ttc gaa gtc gcg ggt gaa gca ggc gcg gca gct ccg gcc gct aaa cag       576
   Phe Glu Val Ala Gly Glu Ala Gly Ala Ala Ala Pro Ala Ala Lys Gln
               180                 185                 190
   gaa gca gct ccg gca gcg gcc cct gca cca gcg gct ggc gtg aaa gaa       624
   Glu Ala Ala Pro Ala Ala Ala Pro Ala Pro Ala Ala Gly Val Lys Glu
           195                 200                 205
   gtt aac gtt ccg gat atc ggc ggt gac gaa gtt gaa gtg act gaa gtg       672
   Val Asn Val Pro Asp Ile Gly Gly Asp Glu Val Glu Val Thr Glu Val
       210                 215                 220
   atg gtg aaa gtg ggc gac aaa gtt gcc gct gaa cag tca ctg atc acc       720
   Met Val Lys Val Gly Asp Lys Val Ala Ala Glu Gln Ser Leu Ile Thr
   225                 230                 235                 240
   gta gaa ggc gac aaa gct tct atg gaa gtt ccg gcg ccg ttt gca ggc       768
   Val Glu Gly Asp Lys Ala Ser Met Glu Val Pro Ala Pro Phe Ala Gly
                   245                 250                 255
   gtc gtg aag gaa ctg aaa gtc aac gtt ggc gat aaa gtg aaa act ggc       816
   Val Val Lys Glu Leu Lys Val Asn Val Gly Asp Lys Val Lys Thr Gly
                   260                 265                 270
   tcg ctg att atg atc ttc gaa gtt gaa ggc gca gcg cct gcg gca gct       864
   Ser Leu Ile Met Ile Phe Glu Val Glu Gly Ala Ala Pro Ala Ala Ala
               275                 280                 285
   cct gcg aaa cag gaa gcg gca gcg ccg gca ccg gca gca aaa gct gaa       912
   Pro Ala Lys Gln Glu Ala Ala Ala Pro Ala Pro Ala Ala Lys Ala Glu
       290                 295                 300
   gcc ccg gca gca gca cca gct gcg aaa gcg gaa ggc aaa tct gaa ttt       960
   Ala Pro Ala Ala Ala Pro Ala Ala Lys Ala Glu Gly Lys Ser Glu Phe
   305                 310                 315                 320
   gct gaa aac gac gct tat gtt cac gcg act ccg ctg atc cgc cgt ctg      1008
   Ala Glu Asn Asp Ala Tyr Val His Ala Thr Pro Leu Ile Arg Arg Leu
                   325                 330                 335
   gca cgc gag ttt ggt gtt aac ctt gcg aaa gtg aag ggc act ggc cgt      1056
   Ala Arg Glu Phe Gly Val Asn Leu Ala Lys Val Lys Gly Thr Gly Arg
               340                 345                 350
   aaa ggt cgt atc ctg cgc gaa gac gtt cag gct tac gtg aaa gaa gct      1104
   Lys Gly Arg Ile Leu Arg Glu Asp Val Gln Ala Tyr Val Lys Glu Ala
           355                 360                 365
   atc aaa cgt gca gaa gca gct ccg gca gcg act ggc ggt ggt atc cct      1152
   Ile Lys Arg Ala Glu Ala Ala Pro Ala Ala Thr Gly Gly Gly Ile Pro
       370                 375                 380
   ggc atg ctg ccg tgg ccg aag gtg gac ttc agc aag ttt ggt gaa atc      1200
   Gly Met Leu Pro Trp Pro Lys Val Asp Phe Ser Lys Phe Gly Glu Ile
   385                 390                 395                 400
   gaa gaa gtg gaa ctg ggc cgc atc cag aaa atc tct ggt gcg aac ctg      1248
   Glu Glu Val Glu Leu Gly Arg Ile Gln Lys Ile Ser Gly Ala Asn Leu
                   405                 410                 415
   agc cgt aac tgg gta atg atc ccg cat gtt act cac ttc gac aaa acc      1296
   Ser Arg Asn Trp Val Met Ile Pro His Val Thr His Phe Asp Lys Thr
               420                 425                 430
   gat atc acc gag ttg gaa gcg ttc cgt aaa cag cag aac gaa gaa gcg      1344
   Asp Ile Thr Glu Leu Glu Ala Phe Arg Lys Gln Gln Asn Glu Glu Ala
           435                 440                 445
   gcg aaa cgt aag ctg gat gtg aag atc acc ccg gtt gtc ttc atc atg      1392
   Ala Lys Arg Lys Leu Asp Val Lys Ile Thr Pro Val Val Phe Ile Met
       450                 455                 460
   aaa gcc gtt gct gca gct ctt gag cag atg cct cgc ttc aat agt tcg      1440
   Lys Ala Val Ala Ala Ala Leu Glu Gln Met Pro Arg Phe Asn Ser Ser
   465                 470                 475                 480
   ctg tcg gaa gac ggt cag cgt ctg acc ctg aag aaa tac atc aac atc      1488
   Leu Ser Glu Asp Gly Gln Arg Leu Thr Leu Lys Lys Tyr Ile Asn Ile
                   485                 490                 495
   ggt gtg gcg gtg gat acc ccg aac ggt ctg gtt gtt ccg gta ttc aaa      1536
   Gly Val Ala Val Asp Thr Pro Asn Gly Leu Val Val Pro Val Phe Lys
               500                 505                 510
   gac gtc aac aag aaa ggc atc atc gag ctg tct cgc gag ctg atg act      1584
   Asp Val Asn Lys Lys Gly Ile Ile Glu Leu Ser Arg Glu Leu Met Thr
           515                 520                 525
   att tct aag aaa gcg cgt gac ggt aag ctg act gcg ggc gaa atg cag      1632
   Ile Ser Lys Lys Ala Arg Asp Gly Lys Leu Thr Ala Gly Glu Met Gln
       530                 535                 540
   ggc ggt tgc ttc acc atc tcc agc atc ggc ggc ctg ggt act acc cac      1680
   Gly Gly Cys Phe Thr Ile Ser Ser Ile Gly Gly Leu Gly Thr Thr His
   545                 550                 555                 560
   ttc gcg ccg att gtg aac gcg ccg gaa gtg gct atc ctc ggc gtt tcc      1728
   Phe Ala Pro Ile Val Asn Ala Pro Glu Val Ala Ile Leu Gly Val Ser
                   565                 570                 575
   aag tcc gcg atg gag ccg gtg tgg aat ggt aaa gag ttc gtg ccg cgt      1776
   Lys Ser Ala Met Glu Pro Val Trp Asn Gly Lys Glu Phe Val Pro Arg
                580                 585                 590
   ctg atg ctg ccg att tct ctc tcc ttc gac cac cgc gtg atc gac ggt      1824
   Leu Met Leu Pro Ile Ser Leu Ser Phe Asp His Arg Val Ile Asp Gly
            595                 600                 605
   gct gat ggt gcc cgt ttc att acc atc att aac aac acg ctg tct gac      1872
   Ala Asp Gly Ala Arg Phe Ile Thr Ile Ile Asn Asn Thr Leu Ser Asp
       610                 615                 620
   att cgc cgt ctg gtg atg taa                                          1893
   Ile Arg Arg Leu Val Met
   625                 630
<210> 49
   <211> 630
   <212> PRT
   <213> Escherichia coli
<400> 49
   Met Ala Ile Glu Ile Lys Val Pro Asp Ile Gly Ala Asp Glu Val Glu
   1               5                   10                  15
   Ile Thr Glu Ile Leu Val Lys Val Gly Asp Lys Val Glu Ala Glu Gln
               20                  25                  30
   Ser Leu Ile Thr Val Glu Gly Asp Lys Ala Ser Met Glu Val Pro Ser
           35                  40                  45
   Pro Gln Ala Gly Ile Val Lys Glu Ile Lys Val Ser Val Gly Asp Lys
       50                  55                  60
   Thr Gln Thr Gly Ala Leu Ile Met Ile Phe Asp Ser Ala Asp Gly Ala
   65                  70                  75                  80
   Ala Asp Ala Ala Pro Ala Gln Ala Glu Glu Lys Lys Glu Ala Ala Pro
                   85                  90                  95
   Ala Ala Ala Pro Ala Ala Ala Ala Ala Lys Asp Val Asn Val Pro Asp
               100                 105                 110
   Ile Gly Ser Asp Glu Val Glu Val Thr Glu Ile Leu Val Lys Val Gly
           115                 120                 125
   Asp Lys Val Glu Ala Glu Gln Ser Leu Ile Thr Val Glu Gly Asp Lys
       130                 135                 140
   Ala Ser Met Glu Val Pro Ala Pro Phe Ala Gly Thr Val Lys Glu Ile
   145                 150                 155                 160
   Lys Val Asn Val Gly Asp Lys Val Ser Thr Gly Ser Leu Ile Met Val
                   165                 170                 175
   Phe Glu Val Ala Gly Glu Ala Gly Ala Ala Ala Pro Ala Ala Lys Gln
               180                 185                 190
   Glu Ala Ala Pro Ala Ala Ala Pro Ala Pro Ala Ala Gly Val Lys Glu
           195                 200                 205
   Val Asn Val Pro Asp Ile Gly Gly Asp Glu Val Glu Val Thr Glu Val
       210                 215                 220
   Met Val Lys Val Gly Asp Lys Val Ala Ala Glu Gln Ser Leu Ile Thr
   225                 230                 235                 240
   Val Glu Gly Asp Lys Ala Ser Met Glu Val Pro Ala Pro Phe Ala Gly
                   245                 250                 255
   Val Val Lys Glu Leu Lys Val Asn Val Gly Asp Lys Val Lys Thr Gly
               260                 265                 270
   Ser Leu Ile Met Ile Phe Glu Val Glu Gly Ala Ala Pro Ala Ala Ala
           275                 280                 285
   Pro Ala Lys Gln Glu Ala Ala Ala Pro Ala Pro Ala Ala Lys Ala Glu
       290                 295                 300
   Ala Pro Ala Ala Ala Pro Ala Ala Lys Ala Glu Gly Lys Ser Glu Phe
   305                 310                 315                 320
   Ala Glu Asn Asp Ala Tyr Val His Ala Thr Pro Leu Ile Arg Arg Leu
                   325                 330                 335
   Ala Arg Glu Phe Gly Val Asn Leu Ala Lys Val Lys Gly Thr Gly Arg
               340                 345                 350
   Lys Gly Arg Ile Leu Arg Glu Asp Val Gln Ala Tyr Val Lys Glu Ala
           355                 360                 365
   Ile Lys Arg Ala Glu Ala Ala Pro Ala Ala Thr Gly Gly Gly Ile Pro
       370                 375                 380
   Gly Met Leu Pro Trp Pro Lys Val Asp Phe Ser Lys Phe Gly Glu Ile
   385                 390                 395                 400
   Glu Glu Val Glu Leu Gly Arg Ile Gln Lys Ile Ser Gly Ala Asn Leu
                   405                 410                 415
   Ser Arg Asn Trp Val Met Ile Pro His Val Thr His Phe Asp Lys Thr
               420                 425                 430
   Asp Ile Thr Glu Leu Glu Ala Phe Arg Lys Gln Gln Asn Glu Glu Ala
           435                 440                 445
   Ala Lys Arg Lys Leu Asp Val Lys Ile Thr Pro Val Val Phe Ile Met
       450                 455                 460
   Lys Ala Val Ala Ala Ala Leu Glu Gln Met Pro Arg Phe Asn Ser Ser
   465                 470                 475                 480
   Leu Ser Glu Asp Gly Gln Arg Leu Thr Leu Lys Lys Tyr Ile Asn Ile
                   485                 490                 495
   Gly Val Ala Val Asp Thr Pro Asn Gly Leu Val Val Pro Val Phe Lys
               500                 505                 510
   Asp Val Asn Lys Lys Gly Ile Ile Glu Leu Ser Arg Glu Leu Met Thr
           515                 520                 525
   Ile Ser Lys Lys Ala Arg Asp Gly Lys Leu Thr Ala Gly Glu Met Gln
       530                 535                 540
   Gly Gly Cys Phe Thr Ile Ser Ser Ile Gly Gly Leu Gly Thr Thr His
   545                 550                 555                 560
   Phe Ala Pro Ile Val Asn Ala Pro Glu Val Ala Ile Leu Gly Val Ser
                   565                 570                 575
   Lys Ser Ala Met Glu Pro Val Trp Asn Gly Lys Glu Phe Val Pro Arg
               580                 585                 590
   Leu Met Leu Pro Ile Ser Leu Ser Phe Asp His Arg Val Ile Asp Gly
           595                 600                 605
   Ala Asp Gly Ala Arg Phe Ile Thr Ile Ile Asn Asn Thr Leu Ser Asp
       610                 615                 620
   Ile Arg Arg Leu Val Met
   625                 630
<210> 50
   <211> 1425
   <212> DNA
   <213> Escherichia coli
<220>
   <221> CDS
   <222> (1)..(1425)
<400> 50
   atg agt act gaa atc aaa act cag gtc gtg gta ctt ggg gca ggc ccc        48
   gca ggt tac tcc gct gcc ttc cgt tgc gct gat tta ggt ctg gaa acc        96
   gta atc gta gaa cgt tac aac acc ctt ggc ggt gtt tgc ctg aac gtc       144
   ggc tgt atc cct tct aaa gca ctg ctg cac gta gca aaa gtt atc gaa       192
   gaa gcc aaa gcg ctg gct gaa cac ggt atc gtc ttc ggc gaa ccg aaa      240
   acc gat atc gac aag att cgt acc tgg aaa gag aaa gtg atc aat cag      288
   ctg acc ggt ggt ctg gct ggt atg gcg aaa ggc cgc aaa gtc aaa gtg      336
   gtc aac ggt ctg ggt aaa ttc acc ggg gct aac acc ctg gaa gtt gaa      384
   ggt gag aac ggc aaa acc gtg atc aac ttc gac aac gcg atc att gca      432
   gcg ggt tct cgc ccg atc caa ctg ccg ttt att ccg cat gaa gat ccg      480
   cgt atc tgg gac tcc act gac gcg ctg gaa ctg aaa gaa gta cca gaa      528
   cgc ctg ctg gta atg ggt ggc ggt atc atc ggt ctg gaa atg ggc acc      576
   gtt tac cac gcg ctg ggt tca cag att gac gtg gtt gaa atg ttc gac      624
   cag gtt atc ccg gca gct gac aaa gac atc gtt aaa gtc ttc acc aag      672
   cgt atc agc aag aaa ttc aac ctg atg ctg gaa acc aaa gtt acc gcc      720
   gtt gaa gcg aaa gaa gac ggc att tat gtg acg atg gaa ggc aaa aaa      768
   gca ccc gct gaa ccg cag cgt tac gac gcc gtg ctg gta gcg att ggt      816
   cgt gtg ccg aac ggt aaa aac ctc gac gca ggc aaa gca ggc gtg gaa      864
   gtt gac gac cgt ggt ttc atc cgc gtt gac aaa cag ctg cgt acc aac      912
   gta ccg cac atc ttt gct atc ggc gat atc gtc ggt caa ccg atg ctg      960
   gca cac aaa ggt gtt cac gaa ggt cac gtt gcc gct gaa gtt atc gcc     1008
   ggt aag aaa cac tac ttc gat ccg aaa gtt atc ccg tcc atc gcc tat     1056
   acc gaa cca gaa gtt gca tgg gtg ggt ctg act gag aaa gaa gcg aaa     1104
   gag aaa ggc atc agc tat gaa acc gcc acc ttc ccg tgg gct gct tct     1152
   ggt cgt gct atc gct tcc gac tgc gca gac ggt atg acc aag ctg att     1200
   ttc gac aaa gaa tct cac cgt gtg atc ggt ggt gcg att gtc ggt act      1248
   aac ggc ggc gag ctg ctg ggt gaa atc ggc ctg gca atc gaa atg ggt      1296
   tgt gat gct gaa gac atc gca ctg acc atc cac gcg cac ccg act ctg      1344
   cac gag tct gtg ggc ctg gcg gca gaa gtg ttc gaa ggt agc att acc      1392
   gac ctg ccg aac ccg aaa gcg aag aag aag taa                          1425
<210> 51
   <211> 474
   <212> PRT
   <213> Escherichia coli
<400> 51
<210> 52
   <211> 1698
   <212> DNA
   <213> Escherichia coli
<220>
   <221> CDS
   <222> (1)..(1698)
<400> 52
   atg gaa cca aaa aca aaa aaa cag cgt tcg ctt tat atc cct tac gct      48
   Met Glu Pro Lys Thr Lys Lys Gln Arg Ser Leu Tyr Ile Pro Tyr Ala
   1               5                   10                  15
   ggc cct gta ctg ctg gaa ttt ccg ttg ttg aat aaa ggc agt gcc ttc      96
   Gly Pro Val Leu Leu Glu Phe Pro Leu Leu Asn Lys Gly Ser Ala Phe
               20                  25                  30
   agc atg gaa gaa cgc cgt aac ttc aac ctg ctg ggg tta ctg ccg gaa     144
   Ser Met Glu Glu Arg Arg Asn Phe Asn Leu Leu Gly Leu Leu Pro Glu
           35                  40                  45
   gtg gtc gaa acc atc gaa gaa caa gcg gaa cga gca tgg atc cag tat     192
   Val Val Glu Thr Ile Glu Glu Gln Ala Glu Arg Ala Trp Ile Gln Tyr
       50                  55                  60
   cag gga ttc aaa acc gaa atc gac aaa cac atc tac ctg cgt aac atc     240
   Gln Gly Phe Lys Thr Glu Ile Asp Lys His Ile Tyr Leu Arg Asn Ile
   65                  70                  75                  80
   cag gac act aac gaa acc ctc ttc tac cgt ctg gta aac aat cat ctt     288
   Gln Asp Thr Asn Glu Thr Leu Phe Tyr Arg Leu Val Asn Asn His Leu
                   85                  90                  95
   gat gag atg atg cct gtt att tat acc cca acc gtc ggc gca gcc tgt     336
   Asp Glu Met Met Pro Val Ile Tyr Thr Pro Thr Val Gly Ala Ala Cys
               100                 105                 110
   gag cgt ttt tct gag atc tac cgc cgt tca cgc ggc gtg ttt atc tct     384
   Glu Arg Phe Ser Glu Ile Tyr Arg Arg Ser Arg Gly Val Phe Ile Ser
           115                 120                 125
   tac cag aac cgg cac aat atg gac gat att ctg caa aac gtg ccg aac     432
   Tyr Gln Asn Arg His Asn Met Asp Asp Ile Leu Gln Asn Val Pro Asn
       130                 135                 140
   cat aat att aaa gtg att gtg gtg act gac ggt gaa cgc att ctg ggg      480
   His Asn Ile Lys Val Ile Val Val Thr Asp Gly Glu Arg Ile Leu Gly
   145                 150                 155                 160
   ctt ggt gac cag ggc atc ggc ggg atg ggc att ccg atc ggt aaa ctg      528
   Leu Gly Asp Gln Gly Ile Gly Gly Met Gly Ile Pro Ile Gly Lys Leu
                   165                 170                 175
   tcg ctc tat acc gcc tgt ggc ggc atc agc ccg gcg tat acc ctt ccg      576
   Ser Leu Tyr Thr Ala Cys Gly Gly Ile Ser Pro Ala Tyr Thr Leu Pro
               180                 185                 190
   gtg gtg ctg gat gtc gga acg aac aac caa cag ctg ctt aac gat ccg      624
   Val Val Leu Asp Val Gly Thr Asn Asn Gln Gln Leu Leu Asn Asp Pro
           195                 200                 205
   ctg tat atg ggc tgg cgt aat ccg cgt atc act gac gac gaa tac tat      672
   Leu Tyr Met Gly Trp Arg Asn Pro Arg Ile Thr Asp Asp Glu Tyr Tyr
       210                 215                 220
   gaa ttc gtt gat gaa ttt atc cag gct gtg aaa caa cgc tgg cca gac      720
   Glu Phe Val Asp Glu Phe Ile Gln Ala Val Lys Gln Arg Trp Pro Asp
   225                 230                 235                 240
   gtg ctg ttg cag ttt gaa gac ttt gct caa aaa aat gcg atg ccg tta      768
   Val Leu Leu Gln Phe Glu Asp Phe Ala Gln Lys Asn Ala Met Pro Leu
                   245                 250                 255
   ctt aac cgc tat cgc aat gaa att tgt tct ttt aac gat gac att cag      816
   Leu Asn Arg Tyr Arg Asn Glu Ile Cys Ser Phe Asn Asp Asp Ile Gln
               260                 265                 270
   ggc act gcg gcg gta aca gtc ggc aca ctg atc gca gca agc cgc gcg      864
   Gly Thr Ala Ala Val Thr Val Gly Thr Leu Ile Ala Ala Ser Arg Ala
           275                 280                 285
   gca ggt ggt cag tta agc gag aaa aaa atc gtc ttc ctt ggc gca ggt      912
   Ala Gly Gly Gln Leu Ser Glu Lys Lys Ile Val Phe Leu Gly Ala Gly
       290                 295                 300
   tca gcg gga tgc ggc att gcc gaa atg atc atc tcc cag acc cag cgc      960
   Ser Ala Gly Cys Gly Ile Ala Glu Met Ile Ile Ser Gln Thr Gln Arg
   305                 310                 315                 320
   gaa gga tta agc gag gaa gcg gcg cgg cag aaa gtc ttt atg gtc gat     1008
   Glu Gly Leu Ser Glu Glu Ala Ala Arg Gln Lys Val Phe Met Val Asp
                   325                 330                 335
   cgc ttt ggc ttg ctg act gac aag atg ccg aac ctg ctg cct ttc cag     1056
   Arg Phe Gly Leu Leu Thr Asp Lys Met Pro Asn Leu Leu Pro Phe Gln
               340                 345                 350
   acc aaa ctg gtg cag aag cgc gaa aac ctc agt gac tgg gat acc gac     1104
   Thr Lys Leu Val Gln Lys Arg Glu Asn Leu Ser Asp Trp Asp Thr Asp
           355                 360                 365
   agc gat gtg ctg tca ctg ctg gat gtg gtg cgc aat gta aaa cca gat     1152
   Ser Asp Val Leu Ser Leu Leu Asp Val Val Arg Asn Val Lys Pro Asp
       370                 375                 380
   att ctg att ggc gtc tca gga cag acc ggg ctg ttt acg gaa gag atc     1200
   Ile Leu Ile Gly Val Ser Gly Gln Thr Gly Leu Phe Thr Glu Glu Ile
   385                 390                 395                 400
   atc cgt gag atg cat aaa cac tgt ccg cgt ccg atc gtg atg ccg ctg     1248
   Ile Arg Glu Met His Lys His Cys Pro Arg Pro Ile Val Met Pro Leu
                   405                 410                 415
   tct aac ccg acg tca cgc gtg gaa gcc aca ccg cag gac att atc gcc     1296
   Ser Asn Pro Thr Ser Arg Val Glu Ala Thr Pro Gln Asp Ile Ile Ala
               420                 425                 430
   tgg acc gaa ggt aac gcg ctg gtc gcc acg ggc agc ccg ttt aat cca     1344
   Trp Thr Glu Gly Asn Ala Leu Val Ala Thr Gly Ser Pro Phe Asn Pro
           435                 440                 445
   gtg gta tgg aaa gat aaa atc tac cct atc gcc cag tgt aac aac gcc     1392
   Val Val Trp Lys Asp Lys Ile Tyr Pro Ile Ala Gln Cys Asn Asn Ala
       450                 455                 460
   ttt att ttc ccg ggc atc ggc ctg ggt gtt att gct tcc ggc gcg tca      1440
   Phe Ile Phe Pro Gly Ile Gly Leu Gly Val Ile Ala Ser Gly Ala Ser
   465                 470                 475                 480
   cgt atc acc gat gag atg ctg atg tcg gca agt gaa acg ctg gcg cag      1488
   Arg Ile Thr Asp Glu Met Leu Met Ser Ala Ser Glu Thr Leu Ala Gln
                   485                 490                 495
   tat tca cca ttg gtg ctg aac ggc gaa ggt atg gta ctg ccg gaa ctg      1536
   Tyr Ser Pro Leu Val Leu Asn Gly Glu Gly Met Val Leu Pro Glu Leu
               500                 505                 510
   aaa gat att cag aaa gtc tcc cgc gca att gcg ttt gcg gtt ggc aaa      1584
   Lys Asp Ile Gln Lys Val Ser Arg Ala Ile Ala Phe Ala Val Gly Lys
           515                 520                 525
   atg gcg cag cag caa ggc gtg gcg gtg aaa acc tct gcc gaa gcc ctg      1632
   Met Ala Gln Gln Gln Gly Val Ala Val Lys Thr Ser Ala Glu Ala Leu
       530                 535                 540
   caa cag gcc att gac gat aat ttc tgg caa gcc gaa tac cgc gac tac      1680
   Gln Gln Ala Ile Asp Asp Asn Phe Trp Gln Ala Glu Tyr Arg Asp Tyr
   545                 550                 555                 560
   cgc cgt acc tcc atc taa                                              1698
   Arg Arg Thr Ser Ile
                   565
<210> 53
   <211> 565
   <212> PRT
   <213> Escherichia coli
<400> 53
   Met Glu Pro Lys Thr Lys Lys Gln Arg Ser Leu Tyr Ile Pro Tyr Ala
   1               5                   10                  15
   Gly Pro Val Leu Leu Glu Phe Pro Leu Leu Asn Lys Gly Ser Ala Phe
               20                  25                  30
   Ser Met Glu Glu Arg Arg Asn Phe Asn Leu Leu Gly Leu Leu Pro Glu
           35                  40                  45
   Val Val Glu Thr Ile Glu Glu Gln Ala Glu Arg Ala Trp Ile Gln Tyr
       50                  55                  60
   Gln Gly Phe Lys Thr Glu Ile Asp Lys His Ile Tyr Leu Arg Asn Ile
   65                  70                  75                  80
   Gln Asp Thr Asn Glu Thr Leu Phe Tyr Arg Leu Val Asn Asn His Leu
                   85                  90                  95
   Asp Glu Met Met Pro Val Ile Tyr Thr Pro Thr Val Gly Ala Ala Cys
               100                 105                 110
   Glu Arg Phe Ser Glu Ile Tyr Arg Arg Ser Arg Gly Val Phe Ile Ser
           115                 120                 125
   Tyr Gln Asn Arg His Asn Met Asp Asp Ile Leu Gln Asn Val Pro Asn
       130                 135                 140
   His Asn Ile Lys Val Ile Val Val Thr Asp Gly Glu Arg Ile Leu Gly
   145                 150                 155                 160
   Leu Gly Asp Gln Gly Ile Gly Gly Met Gly Ile Pro Ile Gly Lys Leu
                   165                 170                 175
   Ser Leu Tyr Thr Ala Cys Gly Gly Ile Ser Pro Ala Tyr Thr Leu Pro
               180                 185                 190
   Val Val Leu Asp Val Gly Thr Asn Asn Gln Gln Leu Leu Asn Asp Pro
           195                 200                 205
   Leu Tyr Met Gly Trp Arg Asn Pro Arg Ile Thr Asp Asp Glu Tyr Tyr
       210                 215                 220
   Glu Phe Val Asp Glu Phe Ile Gln Ala Val Lys Gln Arg Trp Pro Asp
   225                 230                 235                 240
   Val Leu Leu Gln Phe Glu Asp Phe Ala Gln Lys Asn Ala Met Pro Leu
                   245                 250                 255
   Leu Asn Arg Tyr Arg Asn Glu Ile Cys Ser Phe Asn Asp Asp Ile Gln
               260                 265                 270
   Gly Thr Ala Ala Val Thr Val Gly Thr Leu Ile Ala Ala Ser Arg Ala
           275                 280                 285
   Ala Gly Gly Gln Leu Ser Glu Lys Lys Ile Val Phe Leu Gly Ala Gly
       290                 295                 300
   Ser Ala Gly Cys Gly Ile Ala Glu Met Ile Ile Ser Gln Thr Gln Arg
   305                 310                 315                 320
   Glu Gly Leu Ser Glu Glu Ala Ala Arg Gln Lys Val Phe Met Val Asp
                   325                 330                 335
   Arg Phe Gly Leu Leu Thr Asp Lys Met Pro Asn Leu Leu Pro Phe Gln
               340                 345                 350
   Thr Lys Leu Val Gln Lys Arg Glu Asn Leu Ser Asp Trp Asp Thr Asp
           355                 360                 365
   Ser Asp Val Leu Ser Leu Leu Asp Val Val Arg Asn Val Lys Pro Asp
       370                 375                 380
   Ile Leu Ile Gly Val Ser Gly Gln Thr Gly Leu Phe Thr Glu Glu Ile
   385                 390                 395                 400
   Ile Arg Glu Met His Lys His Cys Pro Arg Pro Ile Val Met Pro Leu
                   405                 410                 415
   Ser Asn Pro Thr Ser Arg Val Glu Ala Thr Pro Gln Asp Ile Ile Ala
               420                 425                 430
   Trp Thr Glu Gly Asn Ala Leu Val Ala Thr Gly Ser Pro Phe Asn Pro
           435                 440                 445
   Val Val Trp Lys Asp Lys Ile Tyr Pro Ile Ala Gln Cys Asn Asn Ala
       450                 455                 460
   Phe Ile Phe Pro Gly Ile Gly Leu Gly Val Ile Ala Ser Gly Ala Ser
   465                 470                 475                 480
   Arg Ile Thr Asp Glu Met Leu Met Ser Ala Ser Glu Thr Leu Ala Gln
                   485                 490                 495
   Tyr Ser Pro Leu Val Leu Asn Gly Glu Gly Met Val Leu Pro Glu Leu
               500                 505                 510
   Lys Asp Ile Gln Lys Val Ser Arg Ala Ile Ala Phe Ala Val Gly Lys
           515                 520                 525
   Met Ala Gln Gln Gln Gly Val Ala Val Lys Thr Ser Ala Glu Ala Leu
       530                 535                 540
   Gln Gln Ala Ile Asp Asp Asn Phe Trp Gln Ala Glu Tyr Arg Asp Tyr
   545                 550                 555                 560
   Arg Arg Thr Ser Ile
                   565
<210> 54
   <211> 2280
   <212> DNA
   <213> Escherichia coli
<220>
   <221> CDS
   <222> (1)..(2280)
<400> 54
   atg gat gac cag tta aaa caa agt gca ctt gat ttc cat gaa ttt cca        48
   Met Asp Asp Gln Leu Lys Gln Ser Ala Leu Asp Phe His Glu Phe Pro
   1               5                   10                  15
   gtt cca ggg aaa atc cag gtt tct cca acc aag cct ctg gca aca cag        96
   Val Pro Gly Lys Ile Gln Val Ser Pro Thr Lys Pro Leu Ala Thr Gln
               20                  25                  30
   cgc gat ctg gcg ctg gcc tac tca cca ggc gtt gcc gca cct tgt ctt       144
   Arg Asp Leu Ala Leu Ala Tyr Ser Pro Gly Val Ala Ala Pro Cys Leu
           35                  40                  45
   gaa atc gaa aaa gac ccg tta aaa gcc tac aaa tat acc gcc cga ggt       192
   Glu Ile Glu Lys Asp Pro Leu Lys Ala Tyr Lys Tyr Thr Ala Arg Gly
       50                  55                  60
   aac ctg gtg gcg gtg atc tct aac ggt acg gcg gtg ctg ggg tta ggc       240
   Asn Leu Val Ala Val Ile Ser Asn Gly Thr Ala Val Leu Gly Leu Gly
   65                  70                  75                  80
   aac att ggc gcg ctg gca ggc aaa ccg gtg atg gaa ggc aag ggc gtt       288
   Asn Ile Gly Ala Leu Ala Gly Lys Pro Val Met Glu Gly Lys Gly Val
                   85                  90                  95
   ctg ttt aag aaa ttc gcc ggg att gat gta ttt gac att gaa gtt gac       336
   Leu Phe Lys Lys Phe Ala Gly Ile Asp Val Phe Asp Ile Glu Val Asp
               100                 105                 110
   gaa ctc gac ccg gac aaa ttt att gaa gtt gtc gcc gcg ctc gaa cca       384
   Glu Leu Asp Pro Asp Lys Phe Ile Glu Val Val Ala Ala Leu Glu Pro
           115                 120                 125
   acc ttc ggc ggc atc aac ctc gaa gac att aaa gcg cca gaa tgt ttc       432
   Thr Phe Gly Gly Ile Asn Leu Glu Asp Ile Lys Ala Pro Glu Cys Phe
       130                 135                 140
   tat att gaa cag aaa ctg cgc gag cgg atg aat att ccg gta ttc cac       480
   Tyr Ile Glu Gln Lys Leu Arg Glu Arg Met Asn Ile Pro Val Phe His
   145                 150                 155                 160
   gac gat cag cac ggc acg gca att atc agc act gcc gcc atc ctc aac       528
   Asp Asp Gln His Gly Thr Ala Ile Ile Ser Thr Ala Ala Ile Leu Asn
                   165                 170                 175
   ggc ttg cgc gtg gtg gag aaa aac atc tcc gac gtg cgg atg gtg gtt       576
   Gly Leu Arg Val Val Glu Lys Asn Ile Ser Asp Val Arg Met Val Val
               180                 185                 190
   tcc ggc gcg ggt gcc gca gca atc gcc tgt atg aac ctg ctg gta gcg       624
   Ser Gly Ala Gly Ala Ala Ala Ile Ala Cys Met Asn Leu Leu Val Ala
           195                 200                 205
   ctg ggt ctg caa aaa cat aac atc gtg gtt tgc gat tca aaa ggc gtt       672
   Leu Gly Leu Gln Lys His Asn Ile Val Val Cys Asp Ser Lys Gly Val
       210                 215                 220
   atc tat cag ggc cgt gag cca aac atg gcg gaa acc aaa gcc gca tat       720
   Ile Tyr Gln Gly Arg Glu Pro Asn Met Ala Glu Thr Lys Ala Ala Tyr
   225                 230                 235                 240
   gcg gtg gtg gat gac ggc aaa cgt acc ctc gat gat gtg att gaa ggc       768
   Ala Val Val Asp Asp Gly Lys Arg Thr Leu Asp Asp Val Ile Glu Gly
                   245                 250                 255
   gcg gat att ttc ctg ggc tgt tcc ggc ccg aaa gtg ctg acc cag gaa       816
   Ala Asp Ile Phe Leu Gly Cys Ser Gly Pro Lys Val Leu Thr Gln Glu
               260                 265                 270
   atg gtg aag aaa atg gct cgt gcg cca atg atc ctg gcg ctg gcg aac       864
   Met Val Lys Lys Met Ala Arg Ala Pro Met Ile Leu Ala Leu Ala Asn
           275                 280                 285
   ccg gaa ccg gaa att ctg ccg ccg ctg gcg aaa gaa gtg cgt ccg gat       912
   Pro Glu Pro Glu Ile Leu Pro Pro Leu Ala Lys Glu Val Arg Pro Asp
       290                 295                 300
   gcc atc att tgc acc ggt cgt tct gac tat ccg aac cag gtg aac aac       960
   Ala Ile Ile Cys Thr Gly Arg Ser Asp Tyr Pro Asn Gln Val Asn Asn
   305                 310                 315                 320
   gtc ctg tgc ttc ccg ttc atc ttc cgt ggc gcg ctg gac gtt ggc gca      1008
   Val Leu Cys Phe Pro Phe Ile Phe Arg Gly Ala Leu Asp Val Gly Ala
                   325                 330                 335
   acc gcc atc aac gaa gag atg aaa ctg gcg gcg gta cgt gcg att gca      1056
   Thr Ala Ile Asn Glu Glu Met Lys Leu Ala Ala Val Arg Ala Ile Ala
               340                 345                 350
   gaa ctc gcc cat gcg gaa cag agc gaa gtg gtg gct tca gcg tat ggc      1104
   Glu Leu Ala His Ala Glu Gln Ser Glu Val Val Ala Ser Ala Tyr Gly
           355                 360                 365
   gat cag gat ctg agc ttt ggt ccg gaa tac atc att cca aaa ccg ttt      1152
   Asp Gln Asp Leu Ser Phe Gly Pro Glu Tyr Ile Ile Pro Lys Pro Phe
       370                 375                 380
   gat ccg cgc ttg atc gtt aag atc gct cct gcg gtc gct aaa gcc gcg      1200
   Asp Pro Arg Leu Ile Val Lys Ile Ala Pro Ala Val Ala Lys Ala Ala
   385                 390                 395                 400
   atg gag tcg ggc gtg gcg act cgt ccg att gct gat ttc gac gtc tac      1248
   Met Glu Ser Gly Val Ala Thr Arg Pro Ile Ala Asp Phe Asp Val Tyr
                   405                 410                 415
   atc gac aag ctg act gag ttc gtt tac aaa acc aac ctg ttt atg aag      1296
   Ile Asp Lys Leu Thr Glu Phe Val Tyr Lys Thr Asn Leu Phe Met Lys
               420                 425                 430
   ccg att ttc tcc cag gct cgc aaa gcg ccg aag cgc gtt gtt ctg ccg      1344
   Pro Ile Phe Ser Gln Ala Arg Lys Ala Pro Lys Arg Val Val Leu Pro
           435                 440                 445
   gaa ggg gaa gag gcg cgc gtt ctg cat gcc act cag gaa ctg gta acg      1392
   Glu Gly Glu Glu Ala Arg Val Leu His Ala Thr Gln Glu Leu Val Thr
       450                 455                 460
   ctg gga ctg gcg aaa ccg atc ctt atc ggt cgt ccg aac gtg atc gaa      1440
   Leu Gly Leu Ala Lys Pro Ile Leu Ile Gly Arg Pro Asn Val Ile Glu
   465                 470                 475                 480
   atg cgc att cag aaa ctg ggc ttg cag atc aaa gcg ggc gtt gat ttt      1488
   Met Arg Ile Gln Lys Leu Gly Leu Gln Ile Lys Ala Gly Val Asp Phe
                   485                 490                 495
   gag atc gtc aat aac gaa tcc gat ccg cgc ttt aaa gag tac tgg acc      1536
   Glu Ile Val Asn Asn Glu Ser Asp Pro Arg Phe Lys Glu Tyr Trp Thr
               500                 505                 510
   gaa tac ttc cag atc atg aag cgt cgc ggc gtc act cag gaa cag gcg      1584
   Glu Tyr Phe Gln Ile Met Lys Arg Arg Gly Val Thr Gln Glu Gln Ala
           515                 520                 525
   cag cgg gcg ctg atc agt aac ccg aca gtg atc ggc gcg atc atg gtt      1632
   Gln Arg Ala Leu Ile Ser Asn Pro Thr Val Ile Gly Ala Ile Met Val
       530                 535                 540
   cag cgt ggg gaa gcc gat gca atg att tgc ggt acg gtg ggt gat tat      1680
   Gln Arg Gly Glu Ala Asp Ala Met Ile Cys Gly Thr Val Gly Asp Tyr
   545                 550                 555                 560
   cat gaa cat ttt agc gtg gtg aaa aat gtc ttt ggt tat cgc gat ggc      1728
   His Glu His Phe Ser Val Val Lys Asn Val Phe Gly Tyr Arg Asp Gly
                   565                 570                 575
   gtt cac acc gca ggt gcc atg aac gcg ctg ctg ctg ccg agt ggt aac      1776
   Val His Thr Ala Gly Ala Met Asn Ala Leu Leu Leu Pro Ser Gly Asn
               580                 585                 590
   acc ttt att gcc gat aca tat gtt aat gat gaa ccg gat gca gaa gag      1824
   Thr Phe Ile Ala Asp Thr Tyr Val Asn Asp Glu Pro Asp Ala Glu Glu
           595                 600                 605
   ctg gcg gag atc acc ttg atg gcg gca gaa act gtc cgt cgt ttt ggt      1872
   Leu Ala Glu Ile Thr Leu Met Ala Ala Glu Thr Val Arg Arg Phe Gly
       610                 615                 620
   att gag ccg cgc gtt gct ttg ttg tcg cac tcc aac ttt ggt tct tct      1920
   Ile Glu Pro Arg Val Ala Leu Leu Ser His Ser Asn Phe Gly Ser Ser
   625                 630                 635                 640
   gac tgc ccg tcg tcg agc aaa atg cgt cag gcg ctg gaa ctg gtc agg      1968
   Asp Cys Pro Ser Ser Ser Lys Met Arg Gln Ala Leu Glu Leu Val Arg
                   645                 650                 655
   gaa cgt gca cca gaa ctg atg att gat ggt gaa atg cac ggc gat gca      2016
   Glu Arg Ala Pro Glu Leu Met Ile Asp Gly Glu Met His Gly Asp Ala
               660                 665                 670
   gcg ctg gtg gaa gcg att cgc aac gac cgt atg ccg gac agc tct ttg      2064
   Ala Leu Val Glu Ala Ile Arg Asn Asp Arg Met Pro Asp Ser Ser Leu
           675                 680                 685
   aaa ggt tcc gcc aat att ctg gtg atg ccg aac atg gaa gct gcc cgc      2112
   Lys Gly Ser Ala Asn Ile Leu Val Met Pro Asn Met Glu Ala Ala Arg
       690                 695                 700
   att agt tac aac tta ctg cgt gtt tcc agc tcg gaa ggt gtg act gtc      2160
   Ile Ser Tyr Asn Leu Leu Arg Val Ser Ser Ser Glu Gly Val Thr Val
   705                 710                 715                 720
   ggc ccg gtg ctg atg ggt gtg gcg aaa ccg gtt cac gtg tta acg ccg      2208
   Gly Pro Val Leu Met Gly Val Ala Lys Pro Val His Val Leu Thr Pro
                   725                 730                 735
   atc gca tcg gtg cgt cgt atc gtc aac atg gtg gcg ctg gcc gtg gta      2256
   Ile Ala Ser Val Arg Arg Ile Val Asn Met Val Ala Leu Ala Val Val
               740                 745                 750
   gaa gcg caa acc caa ccg ctg taa                                      2280
   Glu Ala Gln Thr Gln Pro Leu
           755
<210> 55
   <211> 759
   <212> PRT
   <213> Escherichia coli
<400> 55
   Met Asp Asp Gln Leu Lys Gln Ser Ala Leu Asp Phe His Glu Phe Pro
   1               5                   10                  15
   Val Pro Gly Lys Ile Gln Val Ser Pro Thr Lys Pro Leu Ala Thr Gln
               20                  25                  30
   Arg Asp Leu Ala Leu Ala Tyr Ser Pro Gly Val Ala Ala Pro Cys Leu
           35                  40                  45
   Glu Ile Glu Lys Asp Pro Leu Lys Ala Tyr Lys Tyr Thr Ala Arg Gly
       50                  55                  60
   Asn Leu Val Ala Val Ile Ser Asn Gly Thr Ala Val Leu Gly Leu Gly
   65                  70                  75                  80
   Asn Ile Gly Ala Leu Ala Gly Lys Pro Val Met Glu Gly Lys Gly Val
                   85                  90                  95
   Leu Phe Lys Lys Phe Ala Gly Ile Asp Val Phe Asp Ile Glu Val Asp
               100                 105                 110
   Glu Leu Asp Pro Asp Lys Phe Ile Glu Val Val Ala Ala Leu Glu Pro
           115                 120                 125
   Thr Phe Gly Gly Ile Asn Leu Glu Asp Ile Lys Ala Pro Glu Cys Phe
       130                 135                 140
   Tyr Ile Glu Gln Lys Leu Arg Glu Arg Met Asn Ile Pro Val Phe His
   145                 150                 155                 160
   Asp Asp Gln His Gly Thr Ala Ile Ile Ser Thr Ala Ala Ile Leu Asn
                   165                 170                 175
   Gly Leu Arg Val Val Glu Lys Asn Ile Ser Asp Val Arg Met Val Val
               180                 185                 190
   Ser Gly Ala Gly Ala Ala Ala Ile Ala Cys Met Asn Leu Leu Val Ala
           195                 200                 205
   Leu Gly Leu Gln Lys His Asn Ile Val Val Cys Asp Ser Lys Gly Val
       210                 215                 220
   Ile Tyr Gln Gly Arg Glu Pro Asn Met Ala Glu Thr Lys Ala Ala Tyr
   225                 230                 235                 240
   Ala Val Val Asp Asp Gly Lys Arg Thr Leu Asp Asp Val Ile Glu Gly
                   245                 250                 255
   Ala Asp Ile Phe Leu Gly Cys Ser Gly Pro Lys Val Leu Thr Gln Glu
               260                 265                 270
   Met Val Lys Lys Met Ala Arg Ala Pro Met Ile Leu Ala Leu Ala Asn
           275                 280                 285
   Pro Glu Pro Glu Ile Leu Pro Pro Leu Ala Lys Glu Val Arg Pro Asp
       290                 295                 300
   Ala Ile Ile Cys Thr Gly Arg Ser Asp Tyr Pro Asn Gln Val Asn Asn
   305                 310                 315                 320
   Val Leu Cys Phe Pro Phe Ile Phe Arg Gly Ala Leu Asp Val Gly Ala
                   325                 330                 335
   Thr Ala Ile Asn Glu Glu Met Lys Leu Ala Ala Val Arg Ala Ile Ala
               340                 345                 350
   Glu Leu Ala His Ala Glu Gln Ser Glu Val Val Ala Ser Ala Tyr Gly
           355                 360                 365
   Asp Gln Asp Leu Ser Phe Gly Pro Glu Tyr Ile Ile Pro Lys Pro Phe
       370                 375                 380
   Asp Pro Arg Leu Ile Val Lys Ile Ala Pro Ala Val Ala Lys Ala Ala
   385                 390                 395                 400
   Met Glu Ser Gly Val Ala Thr Arg Pro Ile Ala Asp Phe Asp Val Tyr
                   405                 410                 415
   Ile Asp Lys Leu Thr Glu Phe Val Tyr Lys Thr Asn Leu Phe Met Lys
               420                 425                 430
   Pro Ile Phe Ser Gln Ala Arg Lys Ala Pro Lys Arg Val Val Leu Pro
           435                 440                 445
   Glu Gly Glu Glu Ala Arg Val Leu His Ala Thr Gln Glu Leu Val Thr
       450                 455                 460
   Leu Gly Leu Ala Lys Pro Ile Leu Ile Gly Arg Pro Asn Val Ile Glu
   465                 470                 475                 480
   Met Arg Ile Gln Lys Leu Gly Leu Gln Ile Lys Ala Gly Val Asp Phe
                   485                 490                 495
   Glu Ile Val Asn Asn Glu Ser Asp Pro Arg Phe Lys Glu Tyr Trp Thr
               500                 505                 510
   Glu Tyr Phe Gln Ile Met Lys Arg Arg Gly Val Thr Gln Glu Gln Ala
           515                 520                 525
   Gln Arg Ala Leu Ile Ser Asn Pro Thr Val Ile Gly Ala Ile Met Val
       530                 535                 540
   Gln Arg Gly Glu Ala Asp Ala Met Ile Cys Gly Thr Val Gly Asp Tyr
   545                 550                 555                 560
   His Glu His Phe Ser Val Val Lys Asn Val Phe Gly Tyr Arg Asp Gly
                   565                 570                 575
   Val His Thr Ala Gly Ala Met Asn Ala Leu Leu Leu Pro Ser Gly Asn
               580                 585                 590
   Thr Phe Ile Ala Asp Thr Tyr Val Asn Asp Glu Pro Asp Ala Glu Glu
           595                 600                 605
   Leu Ala Glu Ile Thr Leu Met Ala Ala Glu Thr Val Arg Arg Phe Gly
       610                 615                 620
   Ile Glu Pro Arg Val Ala Leu Leu Ser His Ser Asn Phe Gly Ser Ser
   625                 630                 635                 640
   Asp Cys Pro Ser Ser Ser Lys Met Arg Gln Ala Leu Glu Leu Val Arg
                   645                 650                 655
   Glu Arg Ala Pro Glu Leu Met Ile Asp Gly Glu Met His Gly Asp Ala
               660                 665                 670
   Ala Leu Val Glu Ala Ile Arg Asn Asp Arg Met Pro Asp Ser Ser Leu
           675                 680                 685
   Lys Gly Ser Ala Asn Ile Leu Val Met Pro Asn Met Glu Ala Ala Arg
       690                 695                 700
   Ile Ser Tyr Asn Leu Leu Arg Val Ser Ser Ser Glu Gly Val Thr Val
   705                 710                 715                 720
   Gly Pro Val Leu Met Gly Val Ala Lys Pro Val His Val Leu Thr Pro
                   725                 730                 735
   Ile Ala Ser Val Arg Arg Ile Val Asn Met Val Ala Leu Ala Val Val
               740                 745                 750
   Glu Ala Gln Thr Gln Pro Leu
           755
<210> 56
   <211> 531
   <212> DNA
   <213> Methanococcus maripaludis
<220>
   <221> CDS
   <222> (1)..(531)
<400> 56
   atg aaa gaa gtt aga ttc cac gga aga ggg gga cag ggc gca gta aca       48
   Met Lys Glu Val Arg Phe His Gly Arg Gly Gly Gln Gly Ala Val Thr
    1               5                   10                  15
   gca gct caa att ctt gct aag gct gca ttc tac gat ggt aag ttc tgc       96
   Ala Ala Gln Ile Leu Ala Lys Ala Ala Phe Tyr Asp Gly Lys Phe Cys
               20                  25                  30
   caa gct ttt cca ttc ttt ggt gtg gaa aga aga ggg gct ccc gta atg      144
   Gln Ala Phe Pro Phe Phe Gly Val Glu Arg Arg Gly Ala Pro Val Met
           35                  40                  45
   gca ttt aca aga att gac gat gaa aaa ata aga tta aga agc cag att      192
   Ala Phe Thr Arg Ile Asp Asp Glu Lys Ile Arg Leu Arg Ser Gln Ile
       50                  55                  60
   tac gaa cca aat tac gta att gtg cag gat cca aca ttg atg gat tca      240
   Tyr Glu Pro Asn Tyr Val Ile Val Gln Asp Pro Thr Leu Met Asp Ser
   65                  70                  75                  80
   gta gat gtt aca agc ggg atc aaa gac ggc gga ata att ttg ata aac      288
   Val Asp Val Thr Ser Gly Ile Lys Asp Gly Gly Ile Ile Leu Ile Asn
                   85                  90                  95
   acg tta aaa gac ata aaa ctc aat ggc tac gac gtt aaa aca ata gac      336
   Thr Leu Lys Asp Ile Lys Leu Asn Gly Tyr Asp Val Lys Thr Ile Asp
               100                 105                 110
   gct aca gga ata gca ttg gaa gtt ctt gga gtt ccg att gta aat acg      384
   Ala Thr Gly Ile Ala Leu Glu Val Leu Gly Val Pro Ile Val Asn Thr
           115                 120                 125
   aca ctt tgt gga gct ttt gct ggg ctt act gga gaa gtt act atc gaa      432
   Thr Leu Cys Gly Ala Phe Ala Gly Leu Thr Gly Glu Val Thr Ile Glu
       130                 135                 140
   tca ctt aaa aaa tcg att ttg gaa aca ttt cct ggt aaa tta ggc cct      480
   Ser Leu Lys Lys Ser Ile Leu Glu Thr Phe Pro Gly Lys Leu Gly Pro
   145                 150                 155                 160
   aaa aat gca gat gca gct gaa aaa gcg tat aac cta aca aaa ggt ctg      528
   Lys Asn Ala Asp Ala Ala Glu Lys Ala Tyr Asn Leu Thr Lys Gly Leu
                   165                 170                 175
   taa                                                                  531
<210> 57
   <211> 176
   <212> PRT
   <213> Methanococcus maripaludis
<400> 57
   Met Lys Glu Val Arg Phe His Gly Arg Gly Gly Gln Gly Ala Val Thr
     1               5                  10                  15
   Ala Ala Gln Ile Leu Ala Lys Ala Ala Phe Tyr Asp Gly Lys Phe Cys
                20                  25                  30
   Gln Ala Phe Pro Phe Phe Gly Val Glu Arg Arg Gly Ala Pro Val Met
            35                  40                  45
   Ala Phe Thr Arg Ile Asp Asp Glu Lys Ile Arg Leu Arg Ser Gln Ile
        50                  55                  60
   Tyr Glu Pro Asn Tyr Val Ile Val Gln Asp Pro Thr Leu Met Asp Ser
    65                  70                  75                  80
   Val Asp Val Thr Ser Gly Ile Lys Asp Gly Gly Ile Ile Leu Ile Asn
                    85                  90                  95
   Thr Leu Lys Asp Ile Lys Leu Asn Gly Tyr Asp Val Lys Thr Ile Asp
               100                 105                 110
   Ala Thr Gly Ile Ala Leu Glu Val Leu Gly Val Pro Ile Val Asn Thr
           115                 120                 125
   Thr Leu Cys Gly Ala Phe Ala Gly Leu Thr Gly Glu Val Thr Ile Glu
       130                 135                 140
   Ser Leu Lys Lys Ser Ile Leu Glu Thr Phe Pro Gly Lys Leu Gly Pro
   145                 150                 155                 160
   Lys Asn Ala Asp Ala Ala Glu Lys Ala Tyr Asn Leu Thr Lys Gly Leu
                   165                 170                 175
<210> 58
   <211> 258
   <212> DNA
   <213> Methanococcus maripaludis
<220>
   <221> CDS
   <222> (1)..(258)
<400> 58
   atg gta aac aca gga aca att att tat gaa cct gga agt tca gca aac       48
   Met Val Asn Thr Gly Thr Ile Ile Tyr Glu Pro Gly Ser Ser Ala Asn
     1               5                  10                  15
   aat aaa act gga agc tgg aga gta ttc aaa cca gtt tta gat caa gac       96
   Asn Lys Thr Gly Ser Trp Arg Val Phe Lys Pro Val Leu Asp Gln Asp
                20                  25                  30
   aaa tgc gta aaa tgc gaa aat tgt tac atc ttc tgt cct gaa ggc tgt      144
   Lys Cys Val Lys Cys Glu Asn Cys Tyr Ile Phe Cys Pro Glu Gly Cys
            35                  40                  45
   atc caa gaa aaa gat gga aaa ttt gaa att gac tac gac tac tgt aaa      192
   Ile Gln Glu Lys Asp Gly Lys Phe Glu Ile Asp Tyr Asp Tyr Cys Lys
        50                  55                  60
   gga tgc cta att tgc gag aaa gaa tgt cct gta aaa gcg ata aaa gca      240
   Gly Cys Leu Ile Cys Glu Lys Glu Cys Pro Val Lys Ala Ile Lys Ala
    65                  70                  75                  80
   gaa agg gaa gaa aaa taa                                              258
   Glu Arg Glu Glu Lys
                    85
<210> 59
   <211> 85
   <212> PRT
   <213> Methanococcus maripaludis
<400> 59
   Met Val Asn Thr Gly Thr Ile Ile Tyr Glu Pro Gly Ser Ser Ala Asn
   1               5                   10                  15
   Asn Lys Thr Gly Ser Trp Arg Val Phe Lys Pro Val Leu Asp Gln Asp
               20                  25                  30
   Lys Cys Val Lys Cys Glu Asn Cys Tyr Ile Phe Cys Pro Glu Gly Cys
           35                  40                  45
   Ile Gln Glu Lys Asp Gly Lys Phe Glu Ile Asp Tyr Asp Tyr Cys Lys
       50                  55                  60
   Gly Cys Leu Ile Cys Glu Lys Glu Cys Pro Val Lys Ala Ile Lys Ala
   65                  70                  75                  80
   Glu Arg Glu Glu Lys
                   85
<210> 60
   <211> 1164
   <212> DNA
   <213> Methanococcus maripaludis
<220>
   <221> CDS
   <222> (1)..(1164)
<400> 60
   atg tgt gaa gtt aag gtt atc aca gga act tct gca gcc gct gaa gct       48
   Met Cys Glu Val Lys Val Ile Thr Gly Thr Ser Ala Ala Ala Glu Ala
    1               5                  10                  15
   gca aag ctt gca gat gta gat gtt att gca gca tat cca att acc ccc       96
   Ala Lys Leu Ala Asp Val Asp Val Ile Ala Ala Tyr Pro Ile Thr Pro
               20                  25                  30
   caa acg aca tgt gta gaa aag ctt gca gat ttt gtg gct aat ggt gaa      144
   Gln Thr Thr Cys Val Glu Lys Leu Ala Asp Phe Val Ala Asn Gly Glu
           35                  40                  45
   tta aac gca gaa tac att aaa gta gaa agt gaa cat tct gca atg agt      192
   Leu Asn Ala Glu Tyr Ile Lys Val Glu Ser Glu His Ser Ala Met Ser
       50                  55                  60
   gca agt att gga gca agt gcg gca ggt gca aga gca ttt act gca aca      240
   Ala Ser Ile Gly Ala Ser Ala Ala Gly Ala Arg Ala Phe Thr Ala Thr
    65                  70                  75                  80
   tca tca caa ggt tta gca ttg atg cac gaa gtt tta ttt tca gca gca      288
   Ser Ser Gln Gly Leu Ala Leu Met His Glu Val Leu Phe Ser Ala Ala
                   85                  90                  95
   gga atg aga gtt cca gtt gta atg atg aat gca aac agg gca tta tct      336
   Gly Met Arg Val Pro Val Val Met Met Asn Ala Asn Arg Ala Leu Ser
               100                 105                 110
   gca cca att aac att tgg aat gac cag caa gac agt tta tct caa aga      384
   Ala Pro Ile Asn Ile Trp Asn Asp Gln Gln Asp Ser Leu Ser Gln Arg
           115                 120                 125
   gat aca gga tgg att caa cta tat gct gaa gac aac cag gaa gtt tta      432
   Asp Thr Gly Trp Ile Gln Leu Tyr Ala Glu Asp Asn Gln Glu Val Leu
       130                 135                 140
   gat tta gtt att caa gca ttt aaa att gca gaa gac gaa aaa gtt ctt      480
   Asp Leu Val Ile Gln Ala Phe Lys Ile Ala Glu Asp Glu Lys Val Leu
   145                 150                 155                 160
   ctt cca gtt atg gta aac tta gat ggt ttt att tta aca cat act gta      528
   Leu Pro Val Met Val Asn Leu Asp Gly Phe Ile Leu Thr His Thr Val
                   165                 170                 175
   gaa cca gtt aca gtt cca aaa caa gaa aat gta ctc gat ttt atc gga      576
   Glu Pro Val Thr Val Pro Lys Gln Glu Asn Val Leu Asp Phe Ile Gly
               180                 185                 190
   act tat gaa cca aaa cat gcg tac ttg gac cct aaa aaa cca atg aca      624
   Thr Tyr Glu Pro Lys His Ala Tyr Leu Asp Pro Lys Lys Pro Met Thr
           195                 200                 205
   caa ggt gct ctt ggt gat cca aat tac tac atg gaa aca aga tat gca      672
   Gln Gly Ala Leu Gly Asp Pro Asn Tyr Tyr Met Glu Thr Arg Tyr Ala
       210                 215                 220
   att gaa acc gca atg aga aac gct gaa aaa gtt att gca gac gtt cac      720
   Ile Glu Thr Ala Met Arg Asn Ala Glu Lys Val Ile Ala Asp Val His
   225                 230                 235                 240
   gat gct ttt gca gaa aaa ttc aac aga gct tac gga aac ggg ctt att      768
   Asp Ala Phe Ala Glu Lys Phe Asn Arg Ala Tyr Gly Asn Gly Leu Ile
                   245                 250                 255
   gaa agt tac aat tta gaa aaa gca gaa aac gta att att gca atg ggt      816
   Glu Ser Tyr Asn Leu Glu Lys Ala Glu Asn Val Ile Ile Ala Met Gly
               260                 265                 270
   tca att tgt gga aca att aaa gat att att gat ctc aag aaa aaa gaa      864
   Ser Ile Cys Gly Thr Ile Lys Asp Ile Ile Asp Leu Lys Lys Lys Glu
           275                 280                 285
   ggc gta gaa atc ggt tta tta aaa ata aga tgc tac aga cca ctt cct      912
   Gly Val Glu Ile Gly Leu Leu Lys Ile Arg Cys Tyr Arg Pro Leu Pro
       290                 295                 300
   gtt gaa atg atc aaa gat gca ctc aaa aat gct aaa aat gtt gca att      960
   Val Glu Met Ile Lys Asp Ala Leu Lys Asn Ala Lys Asn Val Ala Ile
   305                 310                 315                 320
   ctt gat aag agc ata agc ctt ggc atg aac aaa ggt gca att tat gca     1008
   Leu Asp Lys Ser Ile Ser Leu Gly Met Asn Lys Gly Ala Ile Tyr Ala
                   325                 330                 335
   gat gtt gca tca cat ttg aaa gat aaa aag act gta aat tac atc gta     1056
   Asp Val Ala Ser His Leu Lys Asp Lys Lys Thr Val Asn Tyr Ile Val
               340                 345                 350
   ggt ctt ggt gga cga gat att act cca gaa gac att ttg gga att tac     1104
   Gly Leu Gly Gly Arg Asp Ile Thr Pro Glu Asp Ile Leu Gly Ile Tyr
           355                 360                 365
   gac gat gta gaa att tct gaa gat gga aaa aca aac tgg atc gga ttg     1152
   Asp Asp Val Glu Ile Ser Glu Asp Gly Lys Thr Asn Trp Ile Gly Leu
       370                 375                 380
   aag gaa gaa taa                                                     1164
   Lys Glu Glu
   385
<210> 61
   <211> 387
   <212> PRT
   <213> Methanococcus maripaludis
<400> 61
   Met Cys Glu Val Lys Val Ile Thr Gly Thr Ser Ala Ala Ala Glu Ala
   1 5 10 15
   Ala Lys Leu Ala Asp Val Asp Val Ile Ala Ala Tyr Pro Ile Thr Pro
   20 25 30
   Gln Thr Thr Cys Val Glu Lys Leu Ala Asp Phe Val Ala Asn Gly Glu
   35 40 45
   Leu Asn Ala Glu Tyr Ile Lys Val Glu Ser Glu His Ser Ala Met Ser
   50 55 60
   Ala Ser Ile Gly Ala Ser Ala Ala Gly Ala Arg Ala Phe Thr Ala Thr
   65 70 75 80
   Ser Ser Gln Gly Leu Ala Leu Met His Glu Val Leu Phe Ser Ala Ala
   85 90 95
   Gly Met Arg Val Pro Val Val Met Met Asn Ala Asn Arg Ala Leu Ser
   100 105 110
   Ala Pro Ile Asn Ile Trp Asn Asp Gln Gln Asp Ser Leu Ser Gln Arg
   115 120 125
   Asp Thr Gly Trp Ile Gln Leu Tyr Ala Glu Asp Asn Gln Glu Val Leu
   130 135 140
   Asp Leu Val Ile Gln Ala Phe Lys Ile Ala Glu Asp Glu Lys Val Leu
   145 150 155 160
   Leu Pro Val Met Val Asn Leu Asp Gly Phe Ile Leu Thr His Thr Val
   165 170 175
   Glu Pro Val Thr Val Pro Lys Gln Glu Asn Val Leu Asp Phe Ile Gly
   180 185 190
   Thr Tyr Glu Pro Lys His Ala Tyr Leu Asp Pro Lys Lys Pro Met Thr
   195 200 205
   Gln Gly Ala Leu Gly Asp Pro Asn Tyr Tyr Met Glu Thr Arg Tyr Ala
   210 215 220
   Ile Glu Thr Ala Met Arg Asn Ala Glu Lys Val Ile Ala Asp Val His
   225 230 235 240
   Asp Ala Phe Ala Glu Lys Phe Asn Arg Ala Tyr Gly Asn Gly Leu Ile
   245 250 255
   Glu Ser Tyr Asn Leu Glu Lys Ala Glu Asn Val Ile Ile Ala Met Gly
   260 265 270
   Ser Ile Cys Gly Thr Ile Lys Asp Ile Ile Asp Leu Lys Lys Lys Glu
   275 280 285
   Gly Val Glu Ile Gly Leu Leu Lys Ile Arg Cys Tyr Arg Pro Leu Pro
   290 295 300
   Val Glu Met Ile Lys Asp Ala Leu Lys Asn Ala Lys Asn Val Ala Ile
   305 310 315 320
   Leu Asp Lys Ser Ile Ser Leu Gly Met Asn Lys Gly Ala Ile Tyr Ala
   325 330 335
   Asp Val Ala Ser His Leu Lys Asp Lys Lys Thr Val Asn Tyr Ile Val
   340 345 350
   Gly Leu Gly Gly Arg Asp Ile Thr Pro Glu Asp Ile Leu Gly Ile Tyr
   355 360 365
   Asp Asp Val Glu Ile Ser Glu Asp Gly Lys Thr Asn Trp Ile Gly Leu
   370 375 380
   Lys Glu Glu
   385
<210> 62
   <211> 897
   <212> DNA
   <213> Methanococcus maripaludis
<220>
   <221> CDS
   <222> (1)..(897)
<400> 62
   atg act gga agc caa ttt cca aga gaa gaa tta ttt gct cca gga cac        48
   Met Thr Gly Ser Gln Phe Pro Arg Glu Glu Leu Phe Ala Pro Gly His
   1               5                   10                  15
   agg ggc tgt gca ggt tgt ggt gca gca att gtt gca aga ctc gct tta        96
   Arg Gly Cys Ala Gly Cys Gly Ala Ala Ile Val Ala Arg Leu Ala Leu
               20                  25                  30
   aaa gct gct gga aaa aac acg atc gtt gca aat gct aca gga tgt ctt       144
   Lys Ala Ala Gly Lys Asn Thr Ile Val Ala Asn Ala Thr Gly Cys Leu
           35                  40                  45
   gaa gtt atg aca acg ccg tat cct gaa act gca tgg aaa gtt cct tgg       192
   Glu Val Met Thr Thr Pro Tyr Pro Glu Thr Ala Trp Lys Val Pro Trp
       50                  55                  60
   att cac gtt gca ttc gaa aac gct gca gca gta gca agt ggt gtt gag       240
   Ile His Val Ala Phe Glu Asn Ala Ala Ala Val Ala Ser Gly Val Glu
   65                  70                  75                  80
   gga gct gta gct gcc tta aag aga aaa cgc gga aaa tac ggt gaa gaa       288
   Gly Ala Val Ala Ala Leu Lys Arg Lys Arg Gly Lys Tyr Gly Glu Glu
                   85                  90                  95
   aaa gta aac gtt att gca ttt ggt ggt gac ggc ggt acc gca gat atc       336
   Lys Val Asn Val Ile Ala Phe Gly Gly Asp Gly Gly Thr Ala Asp Ile
               100                 105                 110
   gga ttc caa gca cta agt ggt gca atg gaa agg ggc cac gat atg gtt       384
   Gly Phe Gln Ala Leu Ser Gly Ala Met Glu Arg Gly His Asp Met Val
           115                 120                 125
   tat atc atg tac gat aac gaa gct tac atg aac acc gga gtt caa aga       432
   Tyr Ile Met Tyr Asp Asn Glu Ala Tyr Met Asn Thr Gly Val Gln Arg
       130                 135                 140
   agt ggt tca aca cct tta ttg gcc tca aca acg act tca cct gcc gga        480
   Ser Gly Ser Thr Pro Leu Leu Ala Ser Thr Thr Thr Ser Pro Ala Gly
   145                 150                 155                 160
   agc ctt atc cgt ggg gaa act atg cct aaa aag aac atg cca atg atc        528
   Ser Leu Ile Arg Gly Glu Thr Met Pro Lys Lys Asn Met Pro Met Ile
                   165                 170                 175
   atg gcg gct cat gga ata cct tat gtt gca aca gca tca ata agc tat        576
   Met Ala Ala His Gly Ile Pro Tyr Val Ala Thr Ala Ser Ile Ser Tyr
               180                 185                 190
   cca gaa gac ttc atg aaa aaa gtt aaa aaa gca tgt gaa ata aac ggt        624
   Pro Glu Asp Phe Met Lys Lys Val Lys Lys Ala Cys Glu Ile Asn Gly
           195                 200                 205
   cct gca ttt att caa gta atg caa cca tgt act acc ggc tgg gga tat        672
   Pro Ala Phe Ile Gln Val Met Gln Pro Cys Thr Thr Gly Trp Gly Tyr
       210                 215                 220
   cct gct gat aaa aca att gaa ttg ggc aga ctt gca gta gaa act ggt        720
   Pro Ala Asp Lys Thr Ile Glu Leu Gly Arg Leu Ala Val Glu Thr Gly
   225                 230                 235                 240
   gtt tgg cca ctt ttc gaa att gaa aat ggt gaa ttt aag tta aac tac        768
   Val Trp Pro Leu Phe Glu Ile Glu Asn Gly Glu Phe Lys Leu Asn Tyr
                   245                 250                 255
   aag cca ctt aaa aga aaa cca att gaa gaa tac tta aaa gtg cag aaa        816
   Lys Pro Leu Lys Arg Lys Pro Ile Glu Glu Tyr Leu Lys Val Gln Lys
               260                 265                 270
   aga tac aga cac tta aat ccc gaa gag att tca gta att caa aag acc        864
   Arg Tyr Arg His Leu Asn Pro Glu Glu Ile Ser Val Ile Gln Lys Thr
           275                 280                 285
   atc gac atg aaa tgc gac gaa tac ggt ctt taa                            897
   Ile Asp Met Lys Cys Asp Glu Tyr Gly Leu
       290                 295
<210> 63
   <211> 298
   <212> PRT
   <213> Methanococcus maripaludis
<400> 63
   Met Thr Gly Ser Gln Phe Pro Arg Glu Glu Leu Phe Ala Pro Gly His
   1               5                   10                  15
   Arg Gly Cys Ala Gly Cys Gly Ala Ala Ile Val Ala Arg Leu Ala Leu
               20                  25                  30
   Lys Ala Ala Gly Lys Asn Thr Ile Val Ala Asn Ala Thr Gly Cys Leu
           35                  40                  45
   Glu Val Met Thr Thr Pro Tyr Pro Glu Thr Ala Trp Lys Val Pro Trp
       50                  55                  60
   Ile His Val Ala Phe Glu Asn Ala Ala Ala Val Ala Ser Gly Val Glu
   65                  70                  75                  80
   Gly Ala Val Ala Ala Leu Lys Arg Lys Arg Gly Lys Tyr Gly Glu Glu
                   85                  90                  95
   Lys Val Asn Val Ile Ala Phe Gly Gly Asp Gly Gly Thr Ala Asp Ile
               100                 105                 110
   Gly Phe Gln Ala Leu Ser Gly Ala Met Glu Arg Gly His Asp Met Val
           115                 120                 125
   Tyr Ile Met Tyr Asp Asn Glu Ala Tyr Met Asn Thr Gly Val Gln Arg
       130                 135                 140
   Ser Gly Ser Thr Pro Leu Leu Ala Ser Thr Thr Thr Ser Pro Ala Gly
   145                 150                 155                 160
   Ser Leu Ile Arg Gly Glu Thr Met Pro Lys Lys Asn Met Pro Met Ile
                   165                 170                 175
   Met Ala Ala His Gly Ile Pro Tyr Val Ala Thr Ala Ser Ile Ser Tyr
               180                 185                 190
   Pro Glu Asp Phe Met Lys Lys Val Lys Lys Ala Cys Glu Ile Asn Gly
           195                 200                 205
   Pro Ala Phe Ile Gln Val Met Gln Pro Cys Thr Thr Gly Trp Gly Tyr
       210                 215                 220
   Pro Ala Asp Lys Thr Ile Glu Leu Gly Arg Leu Ala Val Glu Thr Gly
   225                 230                 235                 240
   Val Trp Pro Leu Phe Glu Ile Glu Asn Gly Glu Phe Lys Leu Asn Tyr
                   245                 250                 255
   Lys Pro Leu Lys Arg Lys Pro Ile Glu Glu Tyr Leu Lys Val Gln Lys
               260                 265                 270
   Arg Tyr Arg His Leu Asn Pro Glu Glu Ile Ser Val Ile Gln Lys Thr
           275                 280                 285
   Ile Asp Met Lys Cys Asp Glu Tyr Gly Leu
       290                 295
<210> 64
   <211> 504
   <212> DNA
   <213> Methanococcus maripaludis
<220>
   <221> CDS
   <222> (1)..(504)
<400> 64
   atg aaa aaa gta atg atg gtt aac gaa gct tgt gat aac tgc ggg gat      48
   Met Lys Lys Val Met Met Val Asn Glu Ala Cys Asp Asn Cys Gly Asp
    1               5                  10                  15
   tgt gtt aaa tca tgt tct gaa gtt cac gaa gtt agt gga atc agc att      96
   Cys Val Lys Ser Cys Ser Glu Val His Glu Val Ser Gly Ile Ser Ile
               20                  25                  30
   tgg gag cac gaa gga agg tat cta cca gta gta tgc cag cac tgt act     144
   Trp Glu His Glu Gly Arg Tyr Leu Pro Val Val Cys Gln His Cys Thr
           35                  40                  45
   tca tca ccc tgt atg gaa gta tgt cct gtt tcg gct atc gaa tca aag     192
   Ser Ser Pro Cys Met Glu Val Cys Pro Val Ser Ala Ile Glu Ser Lys
       50                  55                  60
   gat ggt gta att tat ctt gac aaa gaa tcc tgc att gga tgt gga ctc     240
   Asp Gly Val Ile Tyr Leu Asp Lys Glu Ser Cys Ile Gly Cys Gly Leu
   65                  70                  75                  80
   tgt gca atg gca tgc ccc ttt ggt gca ata tac ata agc gga aag act     288
   Cys Ala Met Ala Cys Pro Phe Gly Ala Ile Tyr Ile Ser Gly Lys Thr
                   85                  90                  95
   gca cac aag tgt gat ttg tgt ttt gga aga gac gaa caa gca tgt gtt     336
   Ala His Lys Cys Asp Leu Cys Phe Gly Arg Asp Glu Gln Ala Cys Val
               100                 105                 110
   aaa gca tgc agt aaa aga tgt ctc gaa gtt gta aac gtt gat gaa tta     384
   Lys Ala Cys Ser Lys Arg Cys Leu Glu Val Val Asn Val Asp Glu Leu
           115                 120                 125
   gta atg gat aaa aag ttg aat aat att gaa aat tta aca gtt ttg ggt     432
   Val Met Asp Lys Lys Leu Asn Asn Ile Glu Asn Leu Thr Val Leu Gly
       130                 135                 140
   tct aaa gga aaa tcc aaa aag aaa aaa gga ctc ctt tct tta gtt act     480
   Ser Lys Gly Lys Ser Lys Lys Lys Lys Gly Leu Leu Ser Leu Val Thr
   145                 150                 155                 160
   gca tcg tca aga tgt aat cct taa                                     504
   Ala Ser Ser Arg Cys Asn Pro
                   165
<210> 65
   <211> 167
   <212> PRT
   <213> Methanococcus maripaludis
<400> 65
   Met Lys Lys Val Met Met Val Asn Glu Ala Cys Asp Asn Cys Gly Asp
    1               5                  10                  15
   Cys Val Lys Ser Cys Ser Glu Val His Glu Val Ser Gly Ile Ser Ile
               20                  25                  30
   Trp Glu His Glu Gly Arg Tyr Leu Pro Val Val Cys Gln His Cys Thr
           35                  40                  45
   Ser Ser Pro Cys Met Glu Val Cys Pro Val Ser Ala Ile Glu Ser Lys
       50                  55                  60
   Asp Gly Val Ile Tyr Leu Asp Lys Glu Ser Cys Ile Gly Cys Gly Leu
   65                  70                  75                  80
   Cys Ala Met Ala Cys Pro Phe Gly Ala Ile Tyr Ile Ser Gly Lys Thr
                   85                  90                  95
   Ala His Lys Cys Asp Leu Cys Phe Gly Arg Asp Glu Gln Ala Cys Val
               100                 105                 110
   Lys Ala Cys Ser Lys Arg Cys Leu Glu Val Val Asn Val Asp Glu Leu
           115                 120                 125
   Val Met Asp Lys Lys Leu Asn Asn Ile Glu Asn Leu Thr Val Leu Gly
       130                 135                 140
   Ser Lys Gly Lys Ser Lys Lys Lys Lys Gly Leu Leu Ser Leu Val Thr
   145                 150                 155                 160
   Ala Ser Ser Arg Cys Asn Pro
                   165
<210> 66
   <211> 417
   <212> DNA
   <213> Methanococcus maripaludis
<220>
   <221> CDS
   <222> (1)..(417)
<400> 66
   atg aag gta atg cca aat att gac ttg tgc gta gat tgc aaa aag tgt      48
   Met Lys Val Met Pro Asn Ile Asp Leu Cys Val Asp Cys Lys Lys Cys
    1               5                  10                  15
   gaa cgg gca tgt cct ata aac gca att cac gtt ttt gat ggg att ccc      96
   Glu Arg Ala Cys Pro Ile Asn Ala Ile His Val Phe Asp Gly Ile Pro
               20                  25                  30
   att agg tgt atg cac tgt gaa gat gca ccc tgt ctt aat gta tgt ccc     144
   Ile Arg Cys Met His Cys Glu Asp Ala Pro Cys Leu Asn Val Cys Pro
           35                  40                  45
   gaa gat gca att gag aaa ata gct gat aaa gta gta gta cac cct gaa     192
   Glu Asp Ala Ile Glu Lys Ile Ala Asp Lys Val Val Val His Pro Glu
       50                  55                  60
   aag tgt gtt gga tgt gca tta tgt gct gaa gtt tgc cct gtt ggg gcc     240
   Lys Cys Val Gly Cys Ala Leu Cys Ala Glu Val Cys Pro Val Gly Ala
   65                  70                  75                  80
   ata cag att gat aga ggt aca aaa gtc gca gta aaa tgt gat gga tgt     288
   Ile Gln Ile Asp Arg Gly Thr Lys Val Ala Val Lys Cys Asp Gly Cys
                   85                  90                  95
   atc gaa aga ggc agt gaa gta tgt ctt gaa gtg tgt cct aca aag gca     336
   Ile Glu Arg Gly Ser Glu Val Cys Leu Glu Val Cys Pro Thr Lys Ala
               100                 105                 110
   ctc gac tac tac gaa aat aca att gaa aac aaa aga gcg gaa ctt gtt      384
   Leu Asp Tyr Tyr Glu Asn Thr Ile Glu Asn Lys Arg Ala Glu Leu Val
           115                 120                 125
   tca aaa ctt aag aaa tta act tca aga aaa taa                          417
   Ser Lys Leu Lys Lys Leu Thr Ser Arg Lys
       130                 135
<210> 67
   <211> 138
   <212> PRT
   <213> Methanococcus maripaludis
<400> 67
   Met Lys Val Met Pro Asn Ile Asp Leu Cys Val Asp Cys Lys Lys Cys
   1               5                   10                  15
   Glu Arg Ala Cys Pro Ile Asn Ala Ile His Val Phe Asp Gly Ile Pro
               20                  25                  30
   Ile Arg Cys Met His Cys Glu Asp Ala Pro Cys Leu Asn Val Cys Pro
           35                  40                  45
   Glu Asp Ala Ile Glu Lys Ile Ala Asp Lys Val Val Val His Pro Glu
       50                  55                  60
   Lys Cys Val Gly Cys Ala Leu Cys Ala Glu Val Cys Pro Val Gly Ala
   65                  70                  75                  80
   Ile Gln Ile Asp Arg Gly Thr Lys Val Ala Val Lys Cys Asp Gly Cys
                   85                  90                  95
   Ile Glu Arg Gly Ser Glu Val Cys Leu Glu Val Cys Pro Thr Lys Ala
               100                 105                 110
   Leu Asp Tyr Tyr Glu Asn Thr Ile Glu Asn Lys Arg Ala Glu Leu Val
           115                 120                 125
   Ser Lys Leu Lys Lys Leu Thr Ser Arg Lys
       130                 135
<210> 68
   <211> 63
   <212> DNA
   <213> Artificial sequence
<220>
   <223> primer
<400> 68
   tatgatgagt ccaactttgt tttgctgtgt tatggaaatc tcacttgaag cctgcttttt      60
   tat                                                                    63
<210> 69
   <211> 63
   <212> DNA
   <213> Artificial sequence
<220>
   <223> primer
<400> 69
   caaaaaaccc ctcgtttgag gggtttgctc tttaaacgga agggacgctc aagttagtat 60
   aaa 63
<210> 70
   <211> 63
   <212> DNA
   <213> Artificial sequence
<220>
   <223> primer
<400> 70
   ccgacgccct ggcggtaaag caaagacgat aaaagccccc cagggtgaag cctgcttttt 60
   tat 63
<210> 71
   <211> 63
   <212> DNA
   <213> Artificial sequence
<220>
   <223> primer
<400> 71
   agggagaggg aaatagcccg gtagccttca ctaccgggcg caggccgctc aagttagtat 60
   aaa 63
<210> 72
   <211> 63
   <212> DNA
   <213> Artificial sequence
<220>
   <223> primer
<400> 72
   gcccacacac tttatttgtg aacgttacgt gaaaggaaca accaatgaag cctgcttttt 60
   tat 63
<210> 73
   <211> 63
   <212> DNA
   <213> Artificial sequence
<220>
   <223> primer
<400> 73
   ctgcttaccc tgaatattca gggtaagcgt gagagttaaa aaaaacgctc aagttagtat 60
   aaa 63
<210> 74
   <211> 60
   <212> DNA
   <213> Artificial sequence
<220>
   <223> primer
<400> 74
   ttggtaccaa taattttgtt taactttaag aaggagatat aatgattact attgacggta 60
<210> 75
   <211> 52
   <212> DNA
   <213> Artificial sequence
<220>
   <223> primer
<400> 75
   gcgacgccta gcttagggta ccttaatcgg tgttgctttt ttccgctttt cc 52
<210> 76
   <211> 46
   <212> DNA
   <213> Artificial sequence
<220>
   <223> primer
<400> 76
   catagagctc taaggaggaa tgacgtatga aagaagttag attcca 46
<210> 77
   <211> 43
   <212> DNA
   <213> Artificial sequence
<220>
   <223> primer
<400> 77
   ggtgtctgcg ataggccgag ctcttatttt cttgaagtta att 43
<210> 78
   <211> 64
   <212> DNA
   <213> Artificial sequence
<220>
   <223> primer
<400> 78
   caaaagcgaa aatgcagaag aaagccattt gctaaacgct caagttagta taaaaaagct 60
   gaac 64
<210> 79
   <211> 64
   <212> DNA
   <213> Artificial sequence
<220>
   <223> primer
<400> 79
   aagcaaccgc gccattaccg tcaatagtaa tcatatagct gtttccttct agacggccaa 60
   tgct 64
<210> 80
   <211> 21
   <212> DNA
   <213> Artificial sequence
<220>
   <223> primer
<400> 80
   cttcctctga tcttcaagcc a 21
<210> 81
   <211> 20
   <212> DNA
   <213> Artificial sequence
<220>
   <223> primer
<400> 81
   ttctgccatc gtggaactgg 20
<210> 82
   <211> 720
   <212> DNA
   <213> Escherichia coli
<220>
   <221> CDS
   <222> (1)..(720)
<400> 82
   atg gtc att aag gcg caa agc ccg gcg ggt ttc gcg gaa gag tac att      48
   Met Val Ile Lys Ala Gln Ser Pro Ala Gly Phe Ala Glu Glu Tyr Ile
   1               5                   10                  15
   att gaa agt atc tgg aat aac cgc ttc cct ccc ggg act att ttg ccc      96
   Ile Glu Ser Ile Trp Asn Asn Arg Phe Pro Pro Gly Thr Ile Leu Pro
               20                  25                  30
   gca gaa cgt gaa ctt tca gaa tta att ggc gta acg cgt act acg tta     144
   Ala Glu Arg Glu Leu Ser Glu Leu Ile Gly Val Thr Arg Thr Thr Leu
           35                  40                  45
   cgt gaa gtg tta cag cgt ctg gca cga gat ggc tgg ttg acc att caa     192
   Arg Glu Val Leu Gln Arg Leu Ala Arg Asp Gly Trp Leu Thr Ile Gln
       50                  55                  60
   cat ggc aag ccg acg aag gtg aat aat ttc tgg gaa act tcc ggt tta     240
   His Gly Lys Pro Thr Lys Val Asn Asn Phe Trp Glu Thr Ser Gly Leu
   65                  70                  75                  80
   aat atc ctt gaa aca ctg gcg cga ctg gat cac gaa agt gtg ccg cag     288
   Asn Ile Leu Glu Thr Leu Ala Arg Leu Asp His Glu Ser Val Pro Gln
                   85                  90                  95
   ctt att gat aat ttg ctg tcg gtg cgt acc aat att tcc act att ttt     336
   Leu Ile Asp Asn Leu Leu Ser Val Arg Thr Asn Ile Ser Thr Ile Phe
               100                 105                 110
   att cgc acc gcg ttt cgt cag cat ccc gat aaa gcg cag gaa gtg ctg     384
   Ile Arg Thr Ala Phe Arg Gln His Pro Asp Lys Ala Gln Glu Val Leu
           115                 120                 125
   gct acc gct aat gaa gtg gcc gat cac gcc gat gcc ttt gcc gag ctg     432
   Ala Thr Ala Asn Glu Val Ala Asp His Ala Asp Ala Phe Ala Glu Leu
       130                 135                 140
   gat tac aac ata ttc cgc ggc ctg gcg ttt gct tcc ggc aac ccg att     480
   Asp Tyr Asn Ile Phe Arg Gly Leu Ala Phe Ala Ser Gly Asn Pro Ile
   145                 150                 155                 160
   tac ggt ctg att ctt aac ggg atg aaa ggg ctg tat acg cgt att ggt     528
   Tyr Gly Leu Ile Leu Asn Gly Met Lys Gly Leu Tyr Thr Arg Ile Gly
                   165                 170                 175
   cgt cac tat ttc gcc aat ccg gaa gcg cgc agt ctg gcg ctg ggc ttc     576
   Arg His Tyr Phe Ala Asn Pro Glu Ala Arg Ser Leu Ala Leu Gly Phe
               180                 185                 190
   tac cac aaa ctg tcg gcg ttg tgc agt gaa ggc gcg cac gat cag gtg     624
   Tyr His Lys Leu Ser Ala Leu Cys Ser Glu Gly Ala His Asp Gln Val
           195                 200                 205
   tac gaa aca gtg cgt cgc tat ggg cat gag agt ggc gag att tgg cac     672
   Tyr Glu Thr Val Arg Arg Tyr Gly His Glu Ser Gly Glu Ile Trp His
       210                 215                 220
   cgg atg cag aaa aat ctg ccg ggt gat tta gcc att cag ggg cga taa     720
   Arg Met Gln Lys Asn Leu Pro Gly Asp Leu Ala Ile Gln Gly Arg
   225                 230                 235
<210> 83
   <211> 239
   <212> PRT
   <213> Escherichia coli
<400> 83
   Met Val Ile Lys Ala Gln Ser Pro Ala Gly Phe Ala Glu Glu Tyr Ile
   1               5                   10                  15
   Ile Glu Ser Ile Trp Asn Asn Arg Phe Pro Pro Gly Thr Ile Leu Pro
               20                  25                  30
   Ala Glu Arg Glu Leu Ser Glu Leu Ile Gly Val Thr Arg Thr Thr Leu
           35                  40                  45
   Arg Glu Val Leu Gln Arg Leu Ala Arg Asp Gly Trp Leu Thr Ile Gln
       50                  55                  60
   His Gly Lys Pro Thr Lys Val Asn Asn Phe Trp Glu Thr Ser Gly Leu
   65                  70                  75                  80
   Asn Ile Leu Glu Thr Leu Ala Arg Leu Asp His Glu Ser Val Pro Gln
                   85                  90                  95
   Leu Ile Asp Asn Leu Leu Ser Val Arg Thr Asn Ile Ser Thr Ile Phe
               100                 105                 110
   Ile Arg Thr Ala Phe Arg Gln His Pro Asp Lys Ala Gln Glu Val Leu
           115                 120                 125
   Ala Thr Ala Asn Glu Val Ala Asp His Ala Asp Ala Phe Ala Glu Leu
       130                 135                 140
   Asp Tyr Asn Ile Phe Arg Gly Leu Ala Phe Ala Ser Gly Asn Pro Ile
   145                 150                 155                 160
   Tyr Gly Leu Ile Leu Asn Gly Met Lys Gly Leu Tyr Thr Arg Ile Gly
                   165                 170                 175
   Arg His Tyr Phe Ala Asn Pro Glu Ala Arg Ser Leu Ala Leu Gly Phe
               180                 185                 190
   Tyr His Lys Leu Ser Ala Leu Cys Ser Glu Gly Ala His Asp Gln Val
           195                 200                 205
   Tyr Glu Thr Val Arg Arg Tyr Gly His Glu Ser Gly Glu Ile Trp His
       210                 215                 220
   Arg Met Gln Lys Asn Leu Pro Gly Asp Leu Ala Ile Gln Gly Arg
   225                 230                 235
<210> 84
   <211> 53
   <212> DNA
   <213> Artificial sequence
<220>
   <223> primer
<400> 84
   tgattacgcc aagcttagga ggtctagacg ctcaagttag tataaaaaag ctg 53
<210> 85
   <211> 68
   <212> DNA
   <213> Artificial sequence
<220>
   <223> primer
<400> 85
   atcctctaga gtcgacgcgg ccgctcctgt gtgaaattgt tatccgctca caattccaca    60
   cattatac                                                             68

## Description

### Technical Field

The present invention relates to a microorganism which produces an L-amino acid and a method for producing an L-amino acid. L-Lysine, L-threonine and L-tryptophan are widely used as feed additives etc. L-Phenylalanine is used as a raw material of sweetners. L-Valine, L-leucine and L-isoleucine are used for amino acid infusion or supplements. L-Serine is useful as a food additive, a raw material of cosmetics etc.

### Background Art

For production of a target substance such as L-amino acids by fermentation using a microorganism, there are methods of using a wild-type microorganism (wild type strain), auxotrophic strains derived from wild type strains, metabolic regulation mutant strains derived from wild type strains as mutant strains resistant to various drugs, strains having characteristics as both auxotrophic and metabolic regulation mutant, and so forth.

In recent years, recombinant DNA techniques are used for the production of target substances by fermentation. For example, it is prevalent that L-amino acid productivity of a microorganism is improved by enhancing expression of a gene encoding an L-amino acid biosynthetic enzyme or a protein involved in excretion of L-amino acid or resistance to L-amino acid, or by enhancing uptake of a carbon source to the L-amino acid biosynthesis system.

As such techniques as described above, for example, there are known, for L-lysine, enhancing expression of genes encoding enzymes such as dihydrodipicolinate synthase, aspartokinase, dihydrodipicolinate reductase, diaminopimelate decarboxylase and diaminopimelate dehydrogenase (Patent document 1), reducing the activities of the homoserine dehydrogenase and lysine decarboxylase (Patent document 2), reducing the activity of the malic enzyme (Patent document 3), and so forth.

As for L-threonine, genes imparting resistance to L-threonine and/or L-homoserine include the rhtA gene (Patent document 4), rhtB gene (Patent document 5), rhtC gene (Patent document 6), yfiK, yeaS genes (Patent document 7). Additinally, enhancing expression of genes encoding aspartokinase, aspartate semialdehyde dehydrogenease, homoserine kinase, threonine synthase, and aspartate aminotransferase (aspartate transaminase)(Patent document 8), and decreasing threonine dehydrogenase (Patent document 9), and the like are exemplified.

For L-tryptophan, desensitization to the feedback inhibition of phosphoglycerate dehydrogenase and anthranilate synthase (Patent document 10), deletion of tryptophanase (Patent document 11), and so forth are known.

For L-phenylalanine, desensitization to the feedback inhibition of chorismate mutase-prephenate dehydratase (Patent document 12), deletion of chorismate mutase-prephenate dehydrogenase and tyrosine repressor (Patent document 13), and so forth are known.

For L-valine, a mutant strain which requires lipoic acid for its growth and/or which is deficient in H⁺-ATPase (Patent document 14), and so forth are known. For L-leucine, desensitization to the feedback inhibition of isopropyl malate synthase (Patent document 15) and so forth are known, and for L-isoleucine, expression enhancement of genes encoding threonine deaminase and acetohydroxy acid synthase (Patent document 16) and so forth are known.

For L-serine, a strain containing 3-phosphoglycerate dehydrogenase which is desensitized to feedback inhibition by serine (Patent document 17), a bacterium having L-serine-producing ability, at least one of which phosphoserine phosphatase activity and phosphoserine transaminase activity is enhanced, a bacterium deficient in L-serine decomposition ability (Patent document 18), a bacterium resistant to azaserine or β-(2-thienyl)-DL-alanine and having L-serine-producing ability (Patent document 19), and so forth are known.
Patent document 1: U.S. Patent No. 6,040,160
Patent document 2: U.S. Patent No. 5,827,698
Patent document 3: WO2005/010175
Patent document 4: U.S. Patent No. 6,303,348
Patent document 5: European Patent Application Laid-open No . 0994190
Patent document 6: European Patent Application Laid-open No . 1013765
Patent document 7: European Patent Application Laid-open No . 1016710
Patent document 8: WO2006/123764
Patent document 9: U.S. Patent No. 7,179,623Patent
document 10: U.S. Patent No. 6,180,373
Patent document 11: U.S. Patent No. 4,371,614
Patent document 12: U.S. Patent No. 5,354,672
Patent document 13: WO03/044191
Patent document 14: U.S. Patent No. 5,888,783
Patent document 15: U.S. Patent No. 6,403,342
Patent document 16: U.S. Patent No. 5,998,178
Patent document 17: U.S. Patent No. 5,618,716
Patent document 18: U.S. Patent No. 6,037,154
Patent document 19: U.S. Patent No. 6,258,573

### Disclosure of the Invention

Object of the present invention is to provide a bacterial strain which can efficiently produce an L-amino acid and to provide a method for efficiently producing an L-amino acid using such a strain.

Conventional L-amino acid production is mainly based on supply of acetyl-CoA to the TCA cycle by pyruvate dehydrogenase using sugar as a carbon source. However, since the reaction catalyzed by pyruvate dehydrogenase is accompanied by decarboxylation, one molecule of CO₂ is inevitably released. Therefore, the inventors of the present invention considered that, in order to further increase the productivity, it was necessary to decrease this decarboxylation. Then, the inventors of the present invention assiduously conducted various researches in order to achieve the aforementioned objects. As a result, they found that by using a substance which could supply acetyl-CoA such as ethanol and aliphatic acids as a carbon source and increasing the enzymatic activity of pyruvate synthase, which is an enzyme catalyzing carbon dioxide fixation, or pyruvate:NADP⁺ oxidoreductase, Furthermore, by increasing the enzymatic activity of ferredoxin-NADP⁺ reductase having an activity for generating the reduced ferredoxin or reduced flavodoxin from those of oxidized type required for the enzymatic activity of pyruvate: synthase, or by increasing the ablitity to produce ferredoxin or flavodoxin, productivity of L-amino acid could be improved, and thus accomplished the present invention.

That is, the present invention provides the following.

The current invention is defined, *inter alia,* by the following items:
1. A method for producing an L-amino acid, which comprises culturing in a medium a microorganism, which has an ability to produce an L-amino acid selected from the group consisting of L-lysine, L-threonine, L-tryptophan, L-phenylalanine, L-valine, L-leucine, L-isoleucine and L-serine and has been modified so that an activity of pyruvate synthase or pyruvate:NADP⁺ oxidoreductase is increased, to produce and accumulate said L-amino acid in the medium or cells, and collecting said L-amino acid from the medium or the cells, wherein the medium contains ethanol or an aliphatic acid as a carbon source, and wherein the activity of pyruvate synthase or pyruvate:NADP⁺ oxidoreductase is increased by increasing copy number of a gene encoding pyruvate synthase or pyruvate:NADP⁺ oxidoreductase, or by modifying an expression control sequence of the gene.
2. The method according to item 1, wherein the microorganism has been modified so that the activity of pyruvate synthase is increased.
3. The method according to item 1, wherein the microorganism has been modified so that the activity of pyruvate:NADP⁺ oxidoreductase is increased.
4. The method according to any one of items 1 - 3, wherein the gene encoding pyruvate synthase encodes a polypeptide defined in any one of the following (A) to (D), or polypeptides defined in the following (E), (F), (G) and (H), or polypeptides defined in the following (E), (F), (G), (H), (I) and (J) :
   (A) a polypeptide comprising the amino acid sequence shown in SEQ ID NO: 2,
   (B) a polypeptide comprising an amino acid sequence shown in SEQ ID NO: 2 including substitution, deletion, insertion or addition of one or 1 to 20 amino acid residues and having pyruvate synthase activity,
   (C) a polypeptide comprising the amino acid sequence shown in SEQ ID NO: 4,
   (D) a polypeptide comprising an amino acid sequence shown in SEQ ID NO: 4 including substitution, deletion, insertion or addition of one or 1 to 20 amino acid residues and having pyruvate synthase activity,
   (E) a polypeptide defined in the following (E1) or (E2):
      (E1) a polypeptide comprising the amino acid sequence shown in SEQ ID NO: 57,
      (E2) a polypeptide which comprises an amino acid sequence shown in SEQ ID NO: 57 including substitution, deletion, insertion or addition of one or 1 to 20 amino acid residues and having pyruvate synthase activity, and is able to constitute a protein complex having pyruvate synthase activity with at least the polypeptides (F), (G) and (H),
   (F) a polypeptide defined in the following (F1) or (F2) :
      (F1) a polypeptide comprising the amino acid sequence shown in SEQ ID NO: 59,
      (F2) a polypeptide which comprises an amino acid sequence shown in SEQ ID NO: 59 including substitution, deletion, insertion or addition of one or 1 to 20 amino acid residues and having pyruvate synthase activity, and is able to constitute a protein complex having pyruvate synthase activity with at least the polypeptides (E), (G) and (H),
   (G) a polypeptide defined in the following (G1) or (G2):
      (G1) a polypeptide comprising the amino acid sequence shown in SEQ ID NO: 61,
      (G2) a polypeptide which comprises an amino acid sequence shown in SEQ ID NO: 61 including substitution, deletion, insertion or addition of one or 1 to 20 amino acid residues and having pyruvate synthase activity, and is able to constitute a protein complex having pyruvate synthase activity with at least the polypeptides (E), (F) and (H),
   (H) a polypeptide defined in the following (H1) or (H2):
      (H1) a polypeptide comprising the amino acid sequence shown in SEQ ID NO: 63,
      (H2) a polypeptide which comprises an amino acid sequence shown in SEQ ID NO: 63 including substitution, deletion, insertion or addition of one or 1 to 20 amino acid residues and having pyruvate synthase activity, and is able to constitute a protein complex having pyruvate synthase activity with at least the polypeptides (E), (F) and (G),
   (I) a polypeptide defined in the following (I1) or (I2):
      (I1) a polypeptide comprising the amino acid sequence shown in SEQ ID NO: 65,
      (I2) a polypeptide which comprises an amino acid sequence shown in SEQ ID NO: 65 including substitution, deletion, insertion or addition of one or 1 to 20 amino acid residues and having pyruvate synthase activity, and is able to constitute a protein complex having pyruvate synthase activity with at least the polypeptides (E), (F) and (G) and (H),
   (J) a polypeptide defined in the following (J1) or (J2):
      (J1) a polypeptide comprising the amino acid sequence shown in SEQ ID NO: 67,
      (J2) a polypeptide which comprises an amino acid sequence shown in SEQ ID NO: 67 including substitution, deletion, insertion or addition of one or 1 to 20 amino acid residues and having pyruvate synthase activity, and is able to constitute a protein complex having pyruvate synthase activity with at least the polypeptides (E), (F) and (G) and (H).
5. The method according to any one of items 1 - 3, wherein the gene encoding pyruvate synthase comprises a DNA defined in any one of the following (a) to (d), or a DNA defined in the following (e), (f), (g) and (h), or a DNA defined in the following (e), (f), (g), (h), (i) and (j) :
   (a) a DNA having the nucleotide sequence shown in SEQ ID NO: 1,
   (b) a DNA which is able to hybridize with a sequence complementary to the nucleotide sequence shown in SEQ ID NO: 1 or a probe which can be prepared from the nucleotide sequence under stringent conditions, and which encodes a polypeptide having pyruvate synthase activity,
   (c) a DNA having the nucleotide sequence shown in SEQ ID NO: 3,
   (d) a DNA which is able to hybridize with a sequence complementary to the nucleotide sequence shown in SEQ ID NO: 3 or a probe which can be prepared from the nucleotide sequence under stringent conditions, and which encodes a polypeptide having pyruvate synthase activity,
   (e) a DNA defined in the following (e1) or (e2):
      (e1) a DNA having the nucleotide sequence shown in SEQ ID NO: 56,
      (e2) a DNA which is able to hybridize with a sequence complementary to the nucleotide sequence shown in SEQ ID NO: 56 or a probe which can be prepared from the nucleotide sequence under stringent conditions, and which encodes a protein complex having pyruvate synthase activity with at least DNAs defined in (f), (g) and (h),
   (f) a DNA defined in the following (f1) or (f2):
      (f1) a DNA having the nucleotide sequence shown in SEQ ID NO: 58,
      (f2) a DNA which is able to hybridize with a sequence complementary to the nucleotide sequence shown in SEQ ID NO: 58 or a probe which can be prepared from the nucleotide sequence under stringent conditions, and which encodes a protein complex having pyruvate synthase activity with at least DNAs defined in (e), (g) and (h),
   (g) a DNA defined in the following (g1) or (g2):
      (g1) a DNA having the nucleotide sequence shown in SEQ ID NO: 60,
      (g2) a DNA which is able to hybridize with a sequence complementary to the nucleotide sequence shown in SEQ ID NO: 60 or a probe which can be prepared from the nucleotide sequence under stringent conditions, and which encodes a protein complex having pyruvate synthase activity with at least DNAs defined in (e), (f) and (h),
   (h) a DNA defined in the following (h1) or (h2):
      (h1) a DNA having the nucleotide sequence shown in SEQ ID NO: 62,
      (h2) a DNA which is able to hybridize with a sequence complementary to the nucleotide sequence shown in SEQ ID NO: 62 or a probe which can be prepared from the nucleotide sequence under stringent conditions, and which encodes a protein complex having pyruvate synthase activity with at least DNAs defined in (e), (f) and (g),
   (i) a DNA defined in the following (i1) or (i2):
      (i1) a DNA having the nucleotide sequence shown in SEQ ID NO: 64,
      (i2) a DNA which is able to hybridize with a sequence complementary to the nucleotide sequence shown in SEQ ID NO: 64 or a probe which can be prepared from the nucleotide sequence under stringent conditions, and which encodes a protein complex having pyruvate synthase activity with at least DNAs defined in (e), (f), (g) and (h),
   (j) a DNA defined in the following (j1) or (j2):
      (j1) a DNA having the nucleotide sequence shown in SEQ ID NO: 66,
      (j2) a DNA which is able to hybridize with a sequence complementary to the nucleotide sequence shown in SEQ ID NO: 66 or a probe which can be prepared from the nucleotide sequence under stringent conditions, and which encodes a protein complex having pyruvate synthase activity with at least DNAs defined in (e), (f), (g) and (h); wherein the stringent conditions are washing at 60°C, 0.1 x SSC, and 0.1% SDS.
6. The method according to any one of items 1 - 3, wherein the gene encoding pyruvate:NADP⁺ oxidoreductase encodes a polypeptide defined in the following (A) or (B):
   (A) a polypeptide comprising the amino acid sequence shown in SEQ ID NO: 6,
   (B) a polypeptide comprising an amino acid sequence shown in SEQ ID NO: 6 including substitution, deletion, insertion or addition of one or 1 to 20 amino acid residues and having pyruvate:NADP⁺ oxidoreductase activity.
7. The method according to any one of items 1 - 3, wherein the gene encoding pyruvate:NADP⁺ oxidoreductase comprises a DNA defined in the following (a) or (b):
   (a) a DNA having the nucleotide sequence shown in SEQ ID NO: 5,
   (b) a DNA which is able to hybridize with a sequence complementary to the nucleotide sequence shown in SEQ ID NO: 5 or a probe which can be prepared from the nucleotide sequence under stringent conditions and, and which encodes a polypeptide having pyruvate:NADP⁺ oxidoreductase activity; wherein the stringent conditions are washing at 60°C, 0.1 x SSC, and 0.1% SDS.
8. The method according to any one of items 1 to 5, wherein the microorganism has been modified so that an activity of ferredoxin-NADP⁺ reductase is increased.
9. The method according to any one of items 1 to 5 and 10, wherein the microorganism has been modified so that ferredoxin or flavodoxin production ability is improved.
10. The method according to any one of items 1 to 9, wherein the microorganism has been modified so that an activity of malic enzyme is decreased.
11. The method according to any one of items 1 to 10, wherein the microorganism has been modified so that pyruvate dehydrogenase activity is decreased.
12. The method according to any one of items 1 to 11, wherein the microorganism has been modified so that it can aerobically assimilate ethanol.
13. The method according to any one of items 1 to 12, wherein the microorganism is a bacterium belonging to the genus selected from the group consisting of the genera *Escherichia, Enterobacter, Pantoea*, *Klebsiella and Serratia*
14. The method according to item 13, wherein activities of both of NAD-dependent malic enzyme and NADP-dependent malic enzyme are decreased.
15. The method according to any one of items 1 to 14, wherein the microorganism is a coryneform bacterium.

### Brief Description of the Drawing

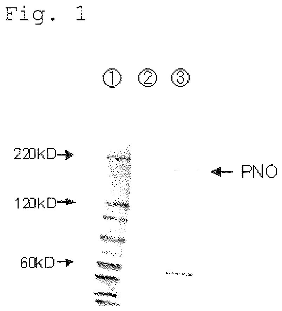

Fig. 1 is a photograph of the result of Western blotting showing expression of a pyruvate:NADP⁺ oxidoreductase (PNO) gene derived from *Euglena gracilis.*
Lane 1: Markers
Lane 2: Crude enzyme extract obtained from WC196ΔcadAΔldcC/pCABD2/pMW-Pthr
Lane 3: Crude enzyme extract obtained, from WC196ΔcadAΔldcC/pCABD2/pMW-Pthr-PNO.

### Description of the Preferred Embodiments

Hereafter, the present invention will be explained in detail.

### <1> Microorganism used for the present invention

The microorganism used for the present invention is a microorganism which has an ability to produce an L-amino acid selected from the group consisting of L-lysine, L-threonine, L-tryptophan, L-phenylalanine, L-valine, L-leucine, L-isoleucine and L-serine and has been modified so that an activity of pyruvate synthase or pyruvate:NADP⁺ oxidoreductase is increased.

In the present invention, the "L-amino acid" means L-lysine, L-threonine, L-tryptophan, L-phenylalaine, L-valine, L-leucine, L-isoleucine, and/or L-serine, unless specifically mentioned.

The term "ability to produce L-amino acid (L-amino acid-producing ability)" refers to the ability to produce L-amino acid and cause accumulation of L-amino acid in the cells of the microorganism used for the present invention or a medium to such a degree that L-amino acid can be collected from the cells or medium when the microorganism is cultured in the medium. The amino acid produced by the microorganism used for the present invention may be one amino acid or two or more amino acids. The microorganism which is able to produce L-amino acid may be a microorganism which has inherently the ability, or may be a microorganism which is imparted the ability by modifying the microorganism using mutagenesis or recombinant DNA techniques, or by introducing the gene of the present invention to the microorganism.

The expression "activity of pyruvate synthase or pyruvate:NADP⁺ oxidoreductase is increased" means that activity of at least one of pyruvate synthase and pyruvate:NADP⁺ oxidoreductase increases in a microorganism inherently having pyruvate synthase and/or pyruvate:NADP⁺ oxidoreductase, or that activity of at least one of pyruvate synthase and pyruvate:NADP⁺ oxidoreductase is imparted to a microorganism originally not having pyruvate synthase and pyruvate:NADP⁺ oxidoreductase.

### <1-1> Impartation of L-amino acid-producing ability

The microorganism used for the present invention can be obtained by using a microorganism having an L-amino acid-producing ability as a parent strain, and modifying it so that the activity of pyruvate synthase or pyruvate:NADP⁺ oxidoreductase, or the activities of these both are increased. The microorganism used for the present invention can also be obtained by using a microorganism modified so that the activity of pyruvate synthase or pyruvate:NADP⁺ oxidoreductase is increased as a parent strain, and imparting an L-amino acid-producing ability to it or enhancing an L-amino acid-producing ability thereof.

Methods for imparting L-amino acid-producing ability to a microorganism and microorganisms imparted with an L-amino acid-producing ability, which can be used for the present invention, will be exemplified below. However, so long as a method that can impart an L-amino acid-producing ability or a microorganism having an L-amino acid-producing ability is chosen, the methods and microorganisms are not limited to these.

Microorganisms used for the present invention include microorganisms belonging to γ-proteobacteria such as bacteria belonging to the genera *Escherichia, Enterobacter,* Pantoea, *Klebsiella, Serratia, Erwinia, Salmonella, Morganella,* etc.; bacteria called as coryneform bacteria such as bacteria belonging to the genera *Brevibacterium, Corynebacterium,* and *Microbacterium;* and microorganisms belonging to the genera *Alicyclobacillus, Bacillus,* and *Saccharomyces.* As γ-proteobacteria, those classified into the bacteria according to the NCBI (National Center for Biotechnology Information) taxonomy database (http://www.ncbi.nlm.nih.gov/Taxonomy/Browser/wwwtax.cgi?i d=91347) can be used.

Examples of *Escherichia* bacteria include *Escherichia coli* and so forth. When *Escherichia coli* strains are bread by using a genetic engineering technique, the *E*. *coli* K12 strain and derivatives thereof, the *Escherichia coli* MG1655 strain (ATCC 47076) and W3110 strain (ATCC 27325) can be used. The *Escherichia coli* K12 strain was isolated in the Stanford University in 1922. This strain is a lysogenic bacterium of λ phage and has the F-factor. This strain is a highly versatile strain from which genetic recombinants can be constructed by conjugation or the like. Furthermore, the genome sequence of the *Escherichia coli* K12 strain has already been determined, and the gene information thereof can also be used freely. The *Escherichia coli* K12 strain and derivatives thereof can be available from American Type Culture Collection (ATCC, Address: P.O. Box 1549, Manassas, VA 20108, United States of America).

In particular, *Pantoea* bacteria, *Erwinia* bacteria and *Enterobacter* bacteria are classified as γ-proteobacteria, and they are taxonomically very close to one another (J. Gen. Appl. Microbiol., 1997, 43, 355-361; Int. J. Syst. Bacteriol., 1997, 43, 1061-1067). In recent years, some bacteria belonging to the genus *Enterobacter* were reclassified as *Pantoea agglomerans, Pantoea dispersa,* or the like, on the basis of DNA-DNA hybridization experiments etc. (International Journal of Systematic Bacteriology, July 1989, 39:337-345). Furthermore, some bacteria belonging to the genus *Erwinia* were reclassified as *Pantoea ananas* or *Pantoea stewartii* (refer to Int. J. Syst. Bacteriol., 1993, 43:162-173).

Examples of the *Enterobacter* bacteria include, but are not limited to, *Enterobacter agglomerans, Enterobacter aerogenes,* and so forth. Specifically, the strains exemplified in European Patent Publication No. 952221 can be used. A typical strain of the genus *Enterobacter* includes the *Enterobacter agglomeranses* ATCC 12287 strain.

Typical strains of the *Pantoea* bacteria include, but are not limited to, *Pantoea ananatis, Pantoea stewartii, Pantoea agglomerans,* and *Pantoea citrea.* Specific examples include the following strains:
*Pantoea ananatis* AJ13355 (FERM BP-6614, European Patent Publication No. 0952221)
*Pantoea ananatis* AJ13356 (FERM BP-6615, European Patent Publication No. 0952221)

Although these strains are described as *Enterobacter agglomerans* in European Patent Publication No. 0952221, they are currently classified as *Pantoea ananatis* on the basis of nucleotide sequence analysis of 16S rRNA etc., as described above.

Examples of the *Erwinia* bacteria include, but are not limited to, *Erwinia amylovora* and *Erwinia carotovara,* and examples of the *Klebsiella* bacteria include *Klebsiella planticola.* Specific examples include the following strains:
*Erwinia amylovora* ATCC 15580
*Erwinia carotovora* ATCC 15713
*Klebsiella planticola* AJ13399 (FERM BP-6600, European Patent Publication No. 955368)
*Klebsiella planticola* AJ13410 (FERM BP-6617, European Patent Publication No. 955368).

The coryneform bacteria referred to in the present invention are a group of microorganisms defined in Bergey's Manual of Determinative Bacteriology, 8th Ed., p.599, 1974, and include aerobic, Gram-positive and nonacid-fast bacilli not having an ability to sporulate, which have hitherto been classified into the genus *Brevibacterium* but united into the genus *Corynebacterium* at present (Liebl, W. et al., 1991, Int. J. Syst. Bacteriol. 41:255-260), and also include bacteria belonging to the genus *Brevibacterium* or *Microbacterium* closely relative to the genus *Corynebacterium.*

Specific examples of coryneform bacteria preferably used for amino acids of L-glutamic acid family include the followings:
*Corynebacterium acetoacidophilum*
*Corynebacterium acetoglutamicum*
*Corynebacterium alkanolyticum*
*Corynebacterium callunae*
*Corynebacterium glutamicum*
*Corynebacterium lilium (Corynebacterium glutamicum)*
*Corynebacterium melassecola*
*Corynebacterium thermoaminogenes (Corynebacterium efficiens*)
*Corynebacterium herculis*
*Brevibacterium divaricatum (Corynebacterium glutamicum*)
*Brevibacterium flavum* (*Corynebacterium glutamicum)*
*Brevibacterium immariophilum*
*Brevibacterium lactofermentum (Corynebacterium* glutamicum)
*Brevibacterium roseum*
*Brevibacterium saccharolyticum*
*Brevibacterium thiogenitalis*
*Brevibacterium ammoniagenes* (*Corynebacterium ammoniagenes*)
*Brevibacterium album*
*Brevibacterium cerinum*
*Microbacterium ammoniaphilum*

Specifically, the following strains can be mentioned:
*Corynebacterium thermoaminogenes* AJ12340 (FERM BP-1539)
*Corynebacterium glutamicum* ATCC 13032
*Brevibacterium flavum* (*Corynebacterium glutamicum*) ATCC 13826, ATCC 14067
*Brevibacterium lactofermentum (Corynebacterium glutamicum)* ATCC 13665, ATCC 13869
*Brevibacterium ammoniagenes (Corynebacterium ammoniagenes*) ATCC 6871

The bacterium used for the present invention may be a bacterium having ethanol assimilability. Such a strain may be a bacterium inherently having ethanol assimilability, a recombinant strain to which ethanol assimilability is imparted, or a mutant strain of which ethanol assimilability is increased. As for *Escherichia coli,* presence of AdhE is known, which has activities of acetaldehyde dehydrogenase and alcohol dehydrogenase catalyzing reversively the reactions described below as enzymes which generate ethanol under anaerobic conditions.

Acetyl-CoA + NADH + H⁴ -> acetaldehyde + NAD⁺ + CoA

Acetaldehyde + NADH + H⁺ -> ethanol NAD⁺

Although *Escherichia coli* cannot assimilate ethanol under aerobic conditions, it is known that it comes to be able to aerobically assimilate ethanol after mutation of AdhE (Clark D.P., and Cronan, J.E. Jr., 1980, J. Bacteriol., 144:179-184; Membrillo-Hernandez, J. et al., 2000, J. Biol. Chem., 275:33869-33875). Specific examples of mutant AdhE having having a mutation such a mutation include the mutant AdhE (Glu568Lys, E568K) are known, which corresponds to *Escherichia coli* AdhE in which the glutamic acid residue at position 568 is replaced with an amino acid residue other than glutamic acid and aspartic acid residues such as lysine residue.

The aforementioned AdhE mutant may further include the following additional mutations.
A) Substitution of another amino acid residue such as lysine residue for the glutamic acid residue at position 560,
B) Substitution of another amino acid residue such as valine residue for the phenylalanine residue at position 566,
C) Substitution of other amino acid residues such as glycine residue, valine residue, cysteine residue, serine residue and valine residue for glutamic acid residue at position 22, methionine residue at position 236, tyrosine residue at position 461 position, isoleucine residue at position 554 position and alanine residue at position 786, respectively, or
D) a combination of the aforementioned mutations.

It is known that *Corynebacterium glutamicum* has two or more kinds of alcohol dehydrogenases, and can aerobically assimilate ethanol (Pelechova J et al., 1980, Folia Microbiol (Praha) 25:341-346).

The bacterium used for the present invention may be a bacterium having an ability to assimilate fat or oil or an aliphatic acid. Such a bacterium may be a bacterium inherently having an ability to assimilate fat or oil or aliphatic acid, a recombinant strain to which an ability to assimilate fat or oil or an aliphatic acid is imparted, or a mutant strain of which ability to assimilate fat or oil or aliphatic acid is increased. In is known that *Escherichia coli* can assimilate long chain aliphatic acids having a chain length of 12 or longer (Clark D.P. and Cronan J.E., 1996, In Escherichia coli and Salmonella: Cellular and Molecular Biology/Second Edition (Neidhardt, F.C. Ed.) pp.343-357). Furthermore, *Escherichia coli* mutant strains which came to be able to assimilate short-to medium-chain aliphatic acids are known (Nunn, W.D. et al., 1979, J. Biol. Chem., 254:9130-9134; Salanitro, J.P. and Wegener, W.S., 1971, J. Bacteriol., 108:885-892).

In the present invention, a bacterium having an L-amino acid-producing ability means a bacterium which can produce and accumulate an L-amino acid in a medium in such an amount that the L-amino acid can be collected from the medium when it is cultured in the medium. It preferably means a bacterium which can accumulate a target L-amino acid in the medium in an amount not less than 0.5 g/L, more preferably not less than 1.0 g/L. The "L-amino acid" encompasses L-lysine, L-threonine, L-tryptophan, L-phenylalanine, L-valine, L-leucine, L-isoleucine and L-serine. L-Lysine L-threonine, L-valine and L-tryptophan are especially preferred.

Hereafter, methods for imparting an L-amino acid-producing ability to such bacteria as mentioned above or methods for enhancing an L-amino acid-producing ability of such bacteria as described above are described.

To impart the ability to produce an L-amino acid, methods conventionally employed in the breeding of the coryneform bacteria or bacteria of the genus *Escherichia* (see "Amino Acid Fermentation", Gakkai Shuppan Center (Ltd.), 1st Edition, published May 30, 1986, pp. 77-100) can be applied. Such methods include acquisition of an auxotrophic mutant, an analogue-resistant strain, or a metabolic regulation mutant, or construction of a recombinant strain having enhanced expression of an L-amino acid biosynthesis enzyme. Here, in the breeding of an L-amino acid-producing bacteria, one or two or more properties, such as auxotrophic mutation, analogue resistance, or metabolic regulation mutation may be imparted. The expression of L-amino acid biosynthesis enzyme(s) can be enhanced singly or in combinations of two or more. Furthermore, the technique of imparting properties such as auxotrophic mutation, analogue resistance, or metabolic regulation mutation may be combined with the technique of enhancing the biosynthesis enzymes.

An auxotrophic mutant strain, L-amino acid analogue-resistant strain, or metabolic regulation mutant strain with the ability to produce an L-amino acid can be obtained by subjecting a parent strain or wild-type strain to a conventional mutatagenesis, such as exposure to X-rays or UV irradiation, or treatment with a mutagen such as N-methyl-N'-nitro-N-nitrosoguanidine, etc., then selecting those which exhibit an autotrophy, analogue resistance, or metabolic regulation mutation and which also have the ability to produce an L-amino acid.

Moreover, impartation or enhancement of L-amino acid-producing ability can also be attained by enhancing an enzymatic activity by gene recombination. Examples of the method for enhancing enzymatic activity include, for example, modifying a bacterium so that expression of a gene encoding an enzyme involved in biosynthesis of an L-amino acid is enhanced. As for the method for enhancing expression of a gene, enhancement can also be attained by introducing an amplification plasmid prepared by introducing a DNA fragment containing the gene into an appropriate plasmid, for example, a plasmid vector containing at least a gene responsible for replication and proliferation of the plasmid in a microorganism, increasing copy number of the gene on chromosome by conjugation, transfer or the like, or introducing a mutation into a promoter region of the gene (refer to International Patent Publication WO95/34672).

When a target gene is introduced into the aforementioned amplification plasmid or chromosome, any promoter may be used for expression of the gene so long as a promoter that functions in the L-amino acid-producing bacterium is chosen. The promoter may be the inherent promoter of the gene, or a modified promoter. Expression of a gene can also be controlled by suitably choosing a promoter that potently functions in the L-amino acid-producing bacterium, or by approximating -35 and -10 regions of a promoter close to the consensus sequence. The methods for enhancing expression of genes encoding the target enzymes are described in WO00/18935, European Patent Publication No. 1010755, and so forth.

Examples of methods for imparting L-amino acid-producing ability to a bacterium and bacteria imparted with an L-amino acid-producing ability will be described below.

### L-Lysine-producing bacteria

Examples of L-lysine-producing bacteria belonging to the genus *Escherichia* include mutants having resistance to an L-lysine analogue. The L-lysine analogue inhibits growth of bacteria belonging to the genus *Escherichia,* but this inhibition is fully or partially desensitized when L-lysine coexists in a medium. Examples of the L-lysine analogue include, but are not limited to, oxalysine, lysine hydroxamate, S-(2-aminoethyl)-L-cysteine (AEC), γ-methyllysine, α-chlorocaprolactam and so forth. Mutants having resistance to these lysine analogues can be obtained by subjecting bacteria belonging to the genus *Escherichia* to a conventional artificial mutagenesis treatment. Specific examples of bacterial strains useful for producing L-lysine include *Escherichia coli* AJ11442 (FERM BP-1543, NRRL B-12185; see U.S. Patent No. 4,346,170) and *Escherichia coli* VL611. In these microorganisms, feedback inhibition of aspartokinase by L-lysine is desensitized.

The strain WC196 may be used as an L-lysine-producing bacterium of *Escherichia coli.* This bacterial strain was bred by conferring AEC resistance to the strain W3110, which was derived from *Escherichia coli* K-12. The resulting strain was designated *Escherichia coli* AJ13069 strain and was deposited at the National Institute of Bioscience and Human-Technology, Agency of Industrial Science and Technology (currently National Institute of Advanced Industrial Science and Technology, International Patent Organism Depositary, Tsukuba Central 6, 1-1, Higashi 1-Chome, Tsukuba-shi, Ibaraki-ken, 305-8566, Japan) on December 6, 1994 and received an accession number of FERM P-14690. Then, it was converted to an international deposit under the provisions of the Budapest Treaty on September 29, 1995, and received an accession number of FERM BP-5252 (U.S. Patent No. 5,827,698).

Examples of L-lysine-producing bacteria and parent strains for deriving L-lysine-producing bacteria also include strains in which expression of one or more genes encoding an L-lysine biosynthetic enzyme are enhanced. Examples of such enzymes include, but are not limited to, dihydrodipicolinate synthase (*dapA*), aspartokinase (*lysC*), dihydrodipicolinate reductase (*dapB*), diaminopimelate decarboxylase *(lysA),* diaminopimelate dehydrogenase *(ddh)* (U.S. Patent No. 6,040,160), phosphoenolpyrvate carboxylase *(ppc),* aspartate aminotransferase *(aspC)* aspartate semialdehyde dehydrogenease (*asd*), diaminopimelate epimerase (*dapF*), tetrahydrodipicolinate succinylase (*dapD*), succinyl diaminopimelate deacylase (*dapE*), and aspartase (*aspA*) (EP 1253195 A). The abbreviations in parentheses represent the gene names (the same shall apply to the same occasions hereafter). Among these enzymes, dihydrodipicolinate reductase, diaminopimelate decarboxylase, diaminopimelate dehydrogenase, phosphoenolpyrvate carboxylase, aspartate aminotransferase, diaminopimelate epimerase, aspartate semialdehyde dehydrogenase, tetrahydrodipicolinate succinylase, and succinyl diaminopimelate deacylase are especially preferred. In addition, the parent strains may have an increased level of expression of the gene involved in energy efficiency (*cyo*) (EP 1170376 A), the gene encoding nicotinamide nucleotide transhydrogenase (*pntAB*) (U.S. Patent No. 5,830,716), the *ybjE* gene (WO2005/073390), or combinations thereof.

Examples of L-lysine-producing bacteria and parent strains for deriving L-lysine-producing bacteria also include strains having decreased or eliminated activity of an enzyme that catalyzes a reaction for generating a compound other than L-lysine by branching off from the biosynthetic pathway of L-lysine. Examples of the enzymes that catalyze a reaction for generating a compound other than L-lysine by branching off from the biosynthetic pathway of L-lysine include homoserine dehydrogenase, lysine decarboxylase (U.S. Patent No. 5,827,698), and the malic enzyme (WO2005/010175).

Preferred examples of L-lysine-producing bacteria include *Escherichia coli* WC196Δmez/pCABD2 (WO2005/010175), WC196ΔcadAΔldcC/pCABD2 (WO2006/078039), and so forth. The WC196Δmez/pCABD2 strain is a strain obtained by introducing the plasmid pCABD2, which is disclosed in U.S. Patent No. 6,040,160, into the strain WC196Δmez which had been obtained by disrupting *sfcA* and *b2463* genes encoding malic enzyme. The WC196Δmez is described in WO2005/010175 in detail. The nucleotide sequences of the *sfcA* gene and *b2463* gene and the amino acid sequences encoded by these genes are shown in SEQ ID NOS: 52 to 55.

The WC196ΔcadAΔldcC/pCABD2 strain is a strain obtained by introducing the plasmid pCABD2, which is disclosed in U.S. Patent No. 6,040,160, into the strain WC196 with disrupted *cadA* and *ldcC* genes encoding lysine decarboxylase. The plasmid pCABD2 contains a mutant *dapA* gene derived from *Escherichia coli* encoding a dihydrodipicolinate synthase (DDPS) having a mutation for desensitization to the feedback inhibition by L-lysine, a mutant *lysC* gene derived from *Escherichia coli* encoding aspartokinase III having a mutation for desensitization to the feedback inhibition by L-lysine, the *dapB* gene derived from *Escherichia coli* encoding dihydrodipicolinate reductase, and the *ddh* gene derived from *Brevibacterium lactofermentum* encoding diaminopimelate dehydrogenase. The *Escherichia coli* W3110(tyrA)/pCABD2 strain harboring this plasmid was designated AJ12604 and deposited at the National Institute of Advanced Industrial Science and Technology, International Patent Organism Depository (Address: Tsukuba Central 6, 1-1, Higashi 1-Chome, Tsukuba-shi, Ibaraki-ken, 305-8566, Japan) on January 28, 1991 under an accession number of FERM P-11975. The deposition was then converted to an international deposition under the provisions of Budapest Treaty on September 26, 1991, and assigned an accession number of FERM BP-3579.

### L-threonine-producing bacteria

Preferred examples of microorganisms having L-threonine-producing ability include bacteria in which one or more activities of L-threonine biosynthesis system enzymes are enhanced. Examples of L-threonine biosynthetic enzymes include aspartokinase III (*lysC*), aspartate semialdehyde dehydrogenase *(asd),* aspartokinase I (*thrA*), homoserine kinase *(thrB),* threonine synthase *(thrC),* and aspartate aminotransferase (aspartate transaminase) (*aspC*). The parentheses after the names of the enzymes are the names of the genes coding for the respective enzymes (the same shall apply throughout this specification). Among these enzymes, aspartokinase III, spartate semialdehyde dehydrogenase, aspartokinase I, homoserine kinase, aspartate aminotransferase, and threonine synthase are particularly preferred. The genes coding for the L-threonine biosynthetic enzymes may be introduced into an *Escherichia* bacterium which has a reduced ability to decompose threonine. An example of such an *Escherichia* bacterium is the TDH6 strain which is deficient in threonine dehydrogenase activity (JP 2001-346578 A).

The enzymatic activities of the L-threonine biosynthetic enzymes are inhibited by the endproduct, L-threonine. Therefore, the genes for the L-threonine biosynthetic enzymes are preferably modified so that the enzymes are desensitized to feedback inhibition by L-threonine in the L-threonine-producing strains. The aforementioned *thrA, thrB,* and *thrC* genes constitute the threonine operon, which attenuates function. The expression of the threonine operon is inhibited by isoleucine and threonine in the culture medium and also suppressed by this attenuation. Therefore, the threonine operon is preferably modified by removing the leader sequence or the sequence responsible for attenuation in the attenuation region (refer to Lynn, S.P. et al. J. Mol. Biol. 194:59-69 (1987); WO02/26993; WO2005/049808).

The native promoter of the threonine operon is present upstream of the threonine operon, and may be replaced with a non-native promoter (refer to WO98/04715), or a threonine operon which has been modified so that expression of the threonine biosynthesis gene is controlled by the repressor and promoter of λ-page (EP 0593792). Furthermore, in order to modify a bacterium so it is desensitized to feedback inhibition by L-threonine, a strain resistant to α-amino-β-hydroxyisovaleric acid (AHV) may be selected.

It is preferable to increase the copy number of the theronine operon that is modified as described above so it is desensitized to feedback inhibition by L-threonine in the host bacterium, or increase expression of such a modified operon by ligating it to a potent promoter. The copy number can also be increased by, besides amplification using a plasmid, transferring the threonine operon to a genome using a transposon, Mu-phage, or the like.

Other than increasing expression of the L-threonine biosynthetic genes, expression of the genes involved in the glycolytic pathway, TCA cycle, or respiratory chain, the genes that regulate the expression of these genes, or the genes involved in sugar uptake may also be preferably increased. Examples of these genes include the genes encoding transhydrogenase (*pntAB,* EP 733712 B), phosphoenolpyruvate carboxylase (*pepC*, WO95/06114), phosphoenolpyruvate synthase (*pps,* EP 877090 B), and pyruvate carboxylase, all of which can be derived from coryneform bacterium or *Bacillus* bacterium (WO99/18228, EP 1092776 A).

It is also preferable to enhance expression of a gene that imparts L-threonine resistance, a gene that imparts L-homoserine resistance, and/or both to the host. Examples of these genes include *rhtA* (Res. Microbiol., 154:123-135 (2003)), *rhtB* (EP 0994190 A), *rhtC* (EP 1013765 A), *yfiK,* and *yeaS* (EP 1016710 A). The methods for imparting L-threonine resistance to a host are described in EP 0994190 A and WO90/04636.

Examples of L-threonine-producing bacteria and parent strains which can be used to derive such bacteria include, but are not limited to, strains belonging to the genus *Escherichia,* such as *E. coli* TDH-6/pVIC40 (VKPM B-3996) (U.S. Patent No. 5,175,107, U.S. Patent No. 5,705,371), *E. coli* 472T23/pYN7 (ATCC 98081) (U.S. Patent No. 5,631,157), *E*. *coli* NRRL-21593 (U.S. Patent No. 5,939,307), *E. coli* FERM BP-3756 (U.S. Patent No. 5,474,918), *E. coli* FERM BP-3519 and FERM BP-3520 (U.S. Patent No. 5,376,538), *E*. *coli* MG442 (Gusyatiner et al., Genetika (in Russian), 14, 947-956 (1978)), *E. coli* VL643 and VL2055 (EP 1149911 A) and so forth.

The TDH-6 strain is deficient in the *thrC* gene, as well as being sucrose-assimilative, and the *ilvA* gene has a leaky mutation. This strain also has a mutation in the rhtA gene, which imparts resistance to high concentrations of threonine or homoserine. The B-3996 strain contains the plasmid pVIC40, which was obtained by inserting the *thrA***BC* operon, including a mutant *thrA* gene, into the RSF1010-derived vector. This mutant thrA gene encodes aspartokinase homoserine dehydrogenase I which is substantially desensitized to feedback inhibition by threonine. The B-3996 strain was deposited on November 19, 1987 in the All-Union Scientific Center of Antibiotics (Nagatinskaya Street 3-A, 117105 Moscow, Russia) under the accession number RIA 1867. The strain was also deposited at the Russian National Collection of Industrial Microorganisms (VKPM) (1 Dorozhny proezd., 1 Moscow 117545, Russia) on April 7, 1987 under the accession number VKPM B-3996.

*E. coli* VKPM B-5318 (EP 0593792 B) is also an L-threonine-producing bacterium. The B-5318 strain is prototrophic with regard to isoleucine, and a temperature-sensitive lambda-phage Cl repressor and PR promoter replace the regulatory region of the threonine operon in pVIC40. The VKPM B-5318 strain was deposited at the Russian National Collection of Industrial Microorganisms (VKPM) (1 Dorozhny proezd., 1 Moscow 117545, Russia) on May 3, 1990 under the accession number of VKPM B-5318.

The *thrA* gene which encodes aspartokinase homoserine dehydrogenase I of *Escherichia coli* has been elucidated (nucleotide positions 337 to 2799, GenBank accession NC_000913.2, gi: 49175990). The *thrA* gene is located between the *thrL* and *thrB* genes on the chromosome of *E*. *coli* K-12. The *thrB* gene which encodes homoserine kinase of *Escherichia coli* has been elucidated (nucleotide positions 2801 to 3733, GenBank accession NC 000913.2, gi: 49175990). The *thrB* gene is located between the *thrA* and *thrC* genes on the chromosome of *E*. *coli* K-12. The *thrC* gene which encodes threonine synthase of *Escherichia coli* has been elucidated (nucleotide positions 3734 to 5020, GenBank accession NC 000913.2, gi: 49175990). The *thrC* gene is located between the *thrB* gene and the *yaaX* open reading frame on the chromosome of *E. coli K*-12. All three genes function as a single threonine operon. To enhance expression of the threonine operon, the attenuator region which affects the transcription is desirably removed (WO2005/049808, WO2003/097839).

A mutant *thrA* gene which codes for aspartokinase homoserine dehydrogenase I resistant to feedback inhibition by threonine, as well as the *thrB* and *thrC* genes can be obtained as one operon from the well-known pVIC40 plasmid, which is present in the threonine-producing *E. coli* strain VKPM B-3996. pVIC40 is described in detail in U.S. Patent No. 5,705,371.

The *rhtA* gene of *E. coli* is present at 18 min on the *E. coli* chromosome, which is close to the *glnHPQ* operon, and which encodes components of the glutamine transport system. The *rhtA* gene is identical to ORFl *(ybiF* gene, nucleotide numbers 764 to 1651, GenBank accession number AAA218541, gi:440181) and is located between the *pexB* and *ompX* genes. The unit expressing the protein encoded by the ORP1 has been designated the *rhtA* gene (*rht*: resistance to homoserine and threonine). Also, it was revealed that the rhtA23 mutation is an A-for-G substitution at position -1 with respect to the ATG start codon (ABSTRACTS of the 17th International Congress of Biochemistry and Molecular Biology in conjugation with Annual Meeting of the American Society for Biochemistry and Molecular Biology, San Francisco, California August 24-29, 1997, abstract No. 457, EP 1013765 A).

The *asd* gene of *E. coli* has already been elucidated (nucleotide numbers 3572511 to 3571408, GenBank accession NC_000913.1, gi:16131307), and can be obtained by PCR (polymerase chain reaction; refer to White, T.J. et al., Trends Genet, 5, 185 (1989)) utilizing primers prepared based on the nucleotide sequence of the gene. The *asd* genes of other microorganisms can also be obtained in a similar manner.

Also, the *aspC* gene of *E. coli* has already been elucidated (nucleotide numbers 983742 to 984932, GenBank accession NC 000913.1, gi:16128895), and can be obtained by PCR. The *aspC* genes of other microorganisms can also be obtained in a similar manner.

### L-Tryptophan-producing bacteria

Examples of L-tryptophan-producing bacteria and parent strains for deriving L-tryptophan-producing bacteria include, but are not limited to, strains belonging to the genus *Escherichia,* such as *E. coli* JP4735/pMU3028 (DSM10122) and JP6015/pMU91 (DSM10123) deficient in the tryptophanyl-tRNA synthetase encoded by mutant *trpS* gene (U.S. Patent No. 5,756,345); *E. coli* SV164 (pGH5) having a *serA* allele encoding phosphoglycerate dehydrogenase free from feedback inhibition by serine and a *trpE* allele encoding anthranilate synthase free from feedback inhibition by tryptophan (U.S. Patent No. 6,180,373); *E. coli* AGX17 (pGX44) (NRRL B-12263) and AGX6(pGX50)aroP (NRRL B-12264) deficient in the enzyme tryptophanase (U.S. Patent No. 4,371,614); *E. coli* AGX17/pGX50, pACKG4-pps in which a phosphoenolpyruvate-producing ability is enhanced (WO97/08333 U.S. Patent No. 6,319,696), and so forth. L-typtophan-producing bacteria belonging to the genus *Escherichia* with an enhanced activity of the protein encoded by the *yedA* gene or the *yddG* gene may also be used (U.S. Published Patent Applications 2003/0148473 A1 and 2003/0157667 A1).

Examples of L-tryptophan-producing bacteria and parent strains for deriving L-tryptophan-producing bacteria also include strains in which one or more activities are enhanced of the following enzymes: anthranilate synthase (*trpE*), phosphoglycerate dehydrogenase (*serA*), 3-deoxy-D-arabinoheptulosonate-7-phosphate synthase *(aroG),* 3-dehydroquinate synthase (*aroB*), shikimate dehydrogenase (*aroE*), shikimate kinase (*aroL*), 5-enolpyruvylshikimate-3-phosphate synthase (*aroA*), chorismate synthase (*aroC*), prephenate dehydratase, chorismate mutase, and tryptophan synthase (*trpAB*). Prephenate dehydratase and chorismate mutase are encoded by the *pheA* gene as a bifunctional enzyme (CM-PD). Among these, phosphoglycerate dehydrogenase, 3-deoxy-D-arabinoheptulosonate-7-phosphate synthase, 3-dehydroquinate synthase, shikimate dehydratase, shikimate kinase, 5-enolpyruvylshikimate-3-phosphate synthase, chorismate synthase, prephenate dehydratase, chorismate mutase-prephenate dehydratase are especially preferred. The anthranilate synthase and phosphoglycerate dehydrogenase are both subject to feedback inhibition by L-tryptophan and L-serine, and therefore a mutation desensitizing the feedback inhibition may be introduced into these enzymes. Specific examples of strains having such a mutation include *E. coli* SV164 which harbors desensitized anthranilate synthase and a transformant strain obtained by introducing into the *E. coli* SV164 the plasmid pGH5 (WO 94/08031), which contains a mutant *serA* gene encoding feedback inhibition-desensitized phosphoglycerate dehydrogenase.

Examples of L-tryptophan-producing bacteria and parent strains for deriving L-tryptophan-producing bacteria also include strains into which the tryptophan operon which contains a gene encoding desensitized anthranilate synthase has been introduced (JP 57-71397 A, JP 62-244382 A, U.S. Patent No. 4,371,614). Moreover, L-tryptophan-producing ability may be imparted by enhancing expression of a gene which encodes tryptophan synthase, among tryptophan operons (*trpBA*). The tryptophan synthase consists of α and β subunits which are encoded by *trpA* and *trpB,* respectively. In addition, L-tryptophan-producing ability may be improved by enhancing expression of the isocitrate lyase-malate synthase operon (WO2005/103275).

### L-Phenylalanine-producing bacteria

Examples of L-phenylalanine-producing bacteria and parent strains for deriving L-phenylalanine-producing bacteria include, but are not limited to, strains belonging to the genus *Escherichia,* such as *E*. *coli* AJ12739 (tyrA::Tn10, tyrR) (VKPM-8197) (WO03/044191); E. *coli* HW1089 (ATCC 55371) harboring the *pheA34* gene encoding chorismate mutase-prephenate dehydratase desensitized to the feedback inhibition (U.S. Patent No. 5,354,672); E. *coli* MWEC101-b (KR8903681); *E. coli* NRRL B-12141, NRRL B-12145, NRRL B-12146 and NRRL B-12147 (U.S. Patent No. 4,407,952). Also, as a parent strain, *E. coli* K-12 [W3110 (tyrA)/pPHAB (FERM BP-3566) having a gene encoding chorismate mutase-prephenate dehydratase desensitized to the feedback inhibition, *E. coli* K-12 [W3110 (tyrA)/pPHAD] (FERM BP-12659), *E*. *coli* K-12 [W3110 (tyrA)/pPHATerm] (FERM BP-12662) and *E. coli* K-12 [W3110 (tyrA)/pBR-aroG4, pACMAB] named as AJ12604 (FERM BP-3579) may be used (EP 488424 B1). Furthermore, L-phenylalanine-producing bacteria belonging to the genus *Escherichia* with an enhanced activity of the protein encoded by the *yedA* gene or the *yddG* gene may also be used (U.S. Published Patent Applications 2003/0148473 A1 and 2003/0157667 A1, WO03/044192).

### L-Valine-producing bacteria

Example of L-valine-producing bacteria and parent strains for deriving L-valine-producing bacteria include, but are not limited to, strains which have been modified to overexpress the *ilvGMEDA* operon (U.S. Patent No. 5,998,178). It is desirable to remove the region of the *ilvGMEDA* operon which is required for attenuation so that expression of the operon is not attenuated by L-valine that is produced. Furthermore, the *ilvA* gene in the operon is desirably disrupted so that threonine deaminase activity is decreased.

Examples of L-valine-producing bacteria and parent strains for deriving L-valine-producing bacteria also include mutants having a mutation of amino-acyl t-RNA synthetase (U.S. Patent No. 5,658,766). For example, *E. coli* VL1970, which has a mutation in the *ileS* gene encoding isoleucine tRNA synthetase, can be used. *E. coli* VL1970 has been deposited at the Russian National Collection of Industrial Microorganisms (VKPM) (1 Dorozhny proezd., 1 Moscow 117545, Russia) on June 24, 1988 under accession number VKPM B-4411.

Furthermore, mutants requiring lipoic acid for growth and/or lacking H⁺-ATPase can also be used as parent strains (WO96/0692 U.S. Patent No. 5,888,783).

As L-valine-producing bacteria belonging to the genus Escherichia, H-81 (VKPM B- 8066), NRRL B-12287, and NRRL B-12288 (US patent No. 4,391,907), VKPM B-4411 (US patent No. 5,658,766), VKPM B-7707 (European patent application EP1016710A2), or the like can also be used. The H-81 strain was deposited at the Russian National Collection of Industrial Microorganisms (VKPM) (1 Dorozhny proezd., 1 Moscow 117545, Russia) on January 30, 2001 under the accession number VKPM B-8066, and it was then converted to an international deposit under the provisions of the Budapest Treaty on Februay 1, 2002.

### L-Leucine-producing bacteria

Examples of L-leucine-producing bacteria and parent strains for deriving L-leucine-producing bacteria include, but are not limited to, strains belonging to the genus *Escherichia,* such as *E. coli* strains resistant to leucine (for example, the strain 57 (VKPM B-7386, U.S. Patent No. 6,124,121)) or leucine analogues including β-2-thienylalanine, 3-hydroxyleucine, 4-azaleucine and 5,5,5-trifluoroleucine (JP 62-34397 B and JP 8-70879 A); *E. coli* strains obtained by the genetic engineering method described in WO96/06926; and *E*. *coli* H-9068 (JP 8-70879 A).

The bacterium used for the present invention may be improved by enhancing the expression of one or more genes involved in L-leucine biosynthesis. Examples of such genes include genes of the *leuABCD* operon, which are preferably represented by a mutant *leuA* gene encoding isopropylmalate synthase freed from feedback inhibition by L-leucine (U.S. Patent No. 6,403,342). In addition, the bacterium used for the present invention may be improved by enhancing the expression of one or more genes encoding proteins which excrete L-amino acid from the bacterial cell. Examples of such genes include the *b2682* and *b2683* genes (*ygaZH* genes) (EP 1239041 A2).

### L-Isoleucine-producing bacteria

Examples of L-isoleucine-producing bacteria and parent strains for deriving L-isoleucine-producing bacteria include, but are not limited to, mutants having resistance to 6-dimethylaminopurine (JP 5-304969 A), mutants having resistance to an isoleucine analogue such as thiaisoleucine and isoleucine hydroxamate, and mutants additionally having resistance to DL-ethionine and/or arginine hydroxamate (JP 5-130882 A). In addition, recombinant strains transformed with genes encoding proteins involved in L-isoleucine biosynthesis, such as threonine deaminase and acetohydroxy acid synthase, can also be used as parent strains (JP 2-458 A, FR 0356739, and U.S. Patent No. 5,998,178).

### L-Serine-producing bacteria

Examples of L-serine-producing bacteria and parent strains for deriving L-serine-producing bacteria include *Escherichia coli* having 3-phosphoglycerate dehydrogenase of which feedback inhibition by serine is attenuated (Japanese Patent No. 2584409, U.S. Patent No. 5,618,716). Moreover, coryneform bacteria having L-serine-producing ability in which at least one of phosphoserine phosphatase activity and phosphoserine transaminase activity is enhanced, coryneform bacteria in which L-serine decomposition ability is deleted (JP 11-253187 A, U.S. Patent No. 6,037,154), and coryneform bacteria having resistance to azaserine or β-(2-thienyl)-DL-alanine and having L-serine-producing ability (JP 11-266881 A, U.S. Patent No. 6,258,573) can also be used.

When the aforementioned L-amino acid-producing bacteria are bred by gene recombination, the genes to be used are not limited to genes having the genetic information described above or genes having known sequences, and genes having conservative mutations such as homologues or artificially modified genes can also be used so long as functions of the encoded proteins are not degraded. That is, they may be genes encoding a known amino acid sequence including substitution, deletion, insertion, addition or the like of one or several amino acid residues at one or several positions. As for the "conservative mutation", the descriptions concerning pyruvate synthase etc. described below are also applied to the aforementioned genes.

### <1-2> Enhancement of pyruvate synthase or pyruvate:NADP⁺ oxidoreductase activity

The microorganism used for the present invention is a microorganism having an L-amino acid-producing ability modified so that an activity of pyruvate synthase or pyruvate:NADP⁺ oxidoreductase is increased as described in the claims. As such modification that pyruvate synthase or pyruvate:NADP⁺ oxidoreductase activity is increased, preferred is such modification that the pyruvate synthase or pyruvate:NADP⁺ oxidoreductase activity is increased as compared to that of the parent strain, for example, a wild type strain or a non-modified strain. In addition, as described above, when the microorganism does not originally have the pyruvate synthase activity, the pyruvate synthase or pyruvate:NADP⁺ oxidoreductase activity of the microorganism, modified so that it should have that enzymatic activity, is increased compared with a non-modified strain.

The bacterium may be modified first so that the enzymatic activity of pyruvate synthase or pyruvate:NADP⁺ oxidoreductase is enhanced as described in the claims, and then imparted with an L-amino acid-producing ability. In addition, enhancement of the activity of pyruvate synthase or pyruvate:NADP⁺ oxidoreductase can be performed by such a method for enhancing expression of a gene as described above. That is, it may be attained by enhancing expression of endogenous pyruvate synthase gene or pyruvate:NADP⁺ oxidoreductase gene based on modification of expression control region such as modification of promoter or the like, or enhancing expression of exogenous pyruvate synthase gene or pyruvate:NADP⁺ oxidoreductase gene based on introduction of a plasmid containing the pyruvate synthase gene or pyruvate:NADP⁺ oxidoreductase gene into the bacterium, introduction of these genes into chromosome of the bacterium, or the like.

The "pyruvate synthase" referred to in the present invention is an enzyme catalyzing reversively the following reaction, which generates pyruvic acid from acetyl-CoA and CO₂ in the presence of an electron donor such as ferredoxin and flavodoxin (EC 1.2.7.1). Pyruvate synthase may be abbreviated as PS, and may be designated pyruvate oxidoreductase, pyruvate ferredoxin oxidoreductase, pyruvate flavodoxin oxidoreductase, or pyruvate oxidoreductase. As the electron donor, ferredoxin or flavodoxin can be used.0

Reduced ferredoxin + acetyl-CoA + CO₂ -> oxidized ferredoxin + pyruvic acid + CoA

Enhancement of the pyruvate synthase activity can be confirmed by preparing crude enzyme solutions from the microorganism before the enhancement and the microorganism after the enhancement, and comparing the pyruvate synthase activity of them. The activity of pyruvate synthase can be measured by, for example, the method of Yoon et al. (Yoon, K.S. et al., 1997. Arch. Microbiol. 167:275-279, 1997). For example, the measurement can be attained by adding pyruvic acid to a reaction mixture containing oxidized methylviologen as an electron acceptor, CoA, and crude enzyme solution, and spectroscopically measuring amount of reduced methylviologen, which increases due to the decarboxylation of pyruvic acid. One unit (U) of the enzymatic activity is defined as an activity of reducing 1 µmol of methylviologen per 1 minute. When the parent strain has the pyruvate synthase activity, the activity desirably increases, for example, preferably 1.5 times or more, more preferably 2 times or more, still more preferably 3 times or more, compared with that of the parent strain. When the parent strain does not have the pyruvate synthase activity, although it is sufficient that pyruvate synthase is produced by the introduction of the pyruvate synthase gene, the activity is preferably enhanced to such an extent that the enzymatic activity can be measured, and the activity is preferably 0.001 U/mg (cell protein) or higher, more preferably 0.005 U/mg or higher, still more preferably 0.01 U/mg or higher. Pyruvate synthase is sensitive to oxygen, and activity expression and measurement are often generally difficult (Buckel, W. and Golding, B.T., 2006, Ann. Rev. of Microbiol., 60:27-49). Therefore, as described in the examples, when the enzymatic activity is measured, it is preferable to perform the enzymatic reaction with reducing the oxygen concentration in a reaction vessel.

As the gene encoding the pyruvate synthase, it is possible to use the pyruvate synthase genes of bacteria having the reductive TCA cycle such as the pyruvate synthase genes of *Chlorobium tepidum* and *Hydrogenobacter thermophilus.* Additionally, pyruvate synthase genes of Eshcerichia coli and other bactria belonging to the *Enterobacteriaceae* can be used. Furthermore, as a gene encoding pyruvate synthase, pyruvate synthase genes of autotrophic methanogenic archaebacteria such as *Methanococcus maripaludis, Methanocaldococcus jannaschii, Methanothermobacter thermautotrophicus* and the like can be used.

Specific examples include a gene having the nucleotide sequence locating at the nucleotide numbers 1534432 to 1537989 of the genome sequence of *Chlorobium* tepidum (Genbank Accession No. NC_002932) and shown in SEQ ID NO: 1, as the pyruvate synthase gene of *Chlorobium* tepidum. The amino acid sequence encoded by this gene is shown in SEQ ID NO: 2 (Genbank Accession No. AAC76906). Furthermore, it is known that the pyruvate synthase of *Hydrogenobacter thermophilus* forms a complex of four subunits, δ-subunit (Genbank Accession No. BAA95604), α-subunit (Genbank Accession No. BAA95605), β-subunit (Genbank Accession No. BAA95606) and γ-subunit (Genbank Accession No. BAA95607) (Ikeda, T. et al., 2006, Biochem. Biophys. Res. Commun., 340:76-82). Examples of the gene further include the pyruvate synthase gene consisting of four genes, HP1108, HP1109, HP1110 and HP1111, locating at the nucleotide numbers of 1170138 to 1173296 of the genome sequence of *Helicobacter pylori* (GenBank Accession No. NC 000915), and the pyruvate synthase gene encoded by four genes, SSO1208, SSO7412, SSO1207 and SSO1206, identified by the nucleotide numbers of 1047593 to 1094711 in the genome sequence of *Sulfolobus solfata ricus* (GenBank Accession No. NC 002754). Furthermore, the pyruvate synthase gene may be those cloned from *Chlorobium, Desulfobacter, Aquifex, Hydrogenabacter, Thermoproteus, Pyrobaculum* bacteria or the like on the basis of homology to the genes exemplified above.

It is predicted that, in *Escherichia coli,* the *ydbK* gene (*b1378*) having the nucleotide sequence shown in SEQ ID NO: 3, which locates at the nucleotide numbers 1435284 to 1438808 in the genome sequence of the K-12 strain (GenBank Accession No. U00096), encodes pyruvate flavodoxin oxidoreductase, i.e., pyruvate synthase, on the basis of homology of the sequences. The amino acid sequence encoded by this gene is shown in SEQ ID NO: 4 (GenBank Accession No. AAC76906). As demonstrated in the example section, it was verified that this gene product has the pyruvate synthase activity, and enhancing expression of this gene improves an L-amino acid-producing ability. Furthermore, a gene encoding pyruvate synthase can be the pyruvate synthase gene of *Enterobacteriaceae* such as genus *Escherichia, Salmonella, Serratia, Enterobacter, Shigella, Citrobacter* or the like which has high homology to the pyruvate synthase gene of *Escherichia coli* (*ydbK*).

The pyruvate synthase gene of *Methanococcus maripaludis* is encoded by the *porCDABEF* operon which locates at the nucleotide numbers 1462535 to 1466397 in the genome sequence of Methanococcus *maripaludis* (GenBank Accession No. NC_005791) (Hendrickson, E. L. et al. 2004. J. Bacteriol. 186: 6956-6969). This pyruvate synthase includes four subunits γ, α, β, anf δ, and it has been known that PorE and PorF in addition to these subunits are important for pyruvate synthase activity (Lin, W. and Whitman, W. B. 2004. Arch. Microbiol. 181: 68-73). The γ subunit is encoded by the *porA* gene corresponding to the nucleotide numbers 1465867 to 1466397 (complementary strand) of the genome sequence. This nucleotide sequence is shown in SEQ ID NO: 56 and the amino acid sequence endoced by the gene is shown in SEQ ID NO: 57. The δ subunit is encoded by the *porB* gene corresponding to the nucleotide numbers 1465595 to 1465852 (complementary strand) of the genome sequence. This nucleotide sequence is shown in SEQ ID NO: 58 and the amino acid sequence endoced by the gene is shown in SEQ ID NO: 59 (GenBank Accession No. NP_988627). The α subunit is encoded by the *porC* gene corresponding to the nucleotide numbers 1464410 to 1465573 (complementary strand) of the genome sequence. This nucleotide sequence is shown in SEQ ID NO: 60 and the amino acid sequence endoced by the gene is shown in SEQ ID NO: 61 (GenBank Accession No. NP_988625). The β subunit is encoded by the *porD* gene corresponding to the nucleotide numbers 1463497 to 1464393 (complementary strand) of the genome sequence. This nucleotide sequence is shown in SEQ ID NO: 62 and the amino acid sequence endoced by the gene is shown in SEQ ID NO: 63 (GenBank Accession No. NP_988624). The PorE is encoded by the *porE* gene corresponding to the nucleotide numbers 1462970 to 1463473 (complementary strand) of the genome sequence. This nucleotide sequence is shown in SEQ ID NO: 64 and the amino acid sequence endoced by the gene is shown in SEQ ID NO: 65 (GenBank Accession No. NP_988623. The PorF is encoded by the *porF* gene corresponding to the nucleotide numbers 1462535 to 1462951 (complementary strand) of the genome sequence. This nucleotide sequence is shown in SEQ ID NO: 66 and the amino acid sequence endoced by the gene is shown in SEQ ID NO: 67 (GenBank Accession No. NP_988622.

It has been known that *Methanocaldococcus jannaschii* and *Methanothermobacter thermautotrophicus* and the like, which are autotrophic methanogenic archaebacteria, have a pyruvate synthase gene having the similar structure, and therefore, these genes can be used.

The "pyruvate:NADP⁺ oxidoreductase" referred to in the present invention is an enzyme catalyzing reversively the following reaction, which generates pyruvic acid from acetyl CoA and CO₂, in the presence of an electron donor such as NADPH or NADH (EC 1.2.1.15). The pyruvate:NADP⁺ oxidoreductase may be abbreviated as PNO, and may be designated pyruvate dehydrogenase. However, the "pyruvate dehydrogenase activity" referred to in the present invention is the activity of catalyzing the oxidative decarboxylation of pyruvic acid to generate acetyl-CoA, as described later, and pyruvate dehydrogenase (PDH) which catalyses this reaction is an enzyme different from pyruvate:NADP⁺ oxidoreductase. Pyruvate:NADP⁺ oxidoreductase can use NADPH or NADH as the electron donor.

NADPH + acetyl-CoA + CO₂ -> NADP⁺ + pyruvic acid + CoA

Enhancement of the pyruvate:NADP⁺ oxidoreductase activity can be confirmed by preparing crude enzyme solutions from the microorganism before the enhancement and the microorganism after the enhancement, and comparing the pyruvate:NADP⁺ oxidoreductase activity of them. The activity of pyruvate:NADP⁺ oxidoreductase can be measured by, for example, the method of Inui et al. (Inui, H. et al., J. Biol. Chem., 262:9130-9135). For example, the measurement can be attained by adding pyruvic acid to a reaction mixture containing oxidized methylviologen as an electron acceptor, CoA, and crude enzyme solution, and spectroscopically measuring amount of reduced methylviologen, which increases due to the decarboxylation of pyruvic acid. One unit (U) of the enzymatic activity is defined as an activity of reducing 1 µmol of methylviologen per 1 minute. When the parent strain has the pyruvate:NADP⁺ oxidoreductase activity, the activity desirably increases, for example, preferably 1.5 times or more, more preferably 2 times or more, still more preferably 3 times or more, as compared to that of the parent strain. When the parent strain does not have the pyruvate:NADP⁺ oxidoreductase activity, although it is sufficient that pyruvate:NADP⁺ oxidoreductase is produced by the introduction of the pyruvate:NADP⁺ oxidoreductase gene, the activity is preferably enhanced to such an extent that the enzymatic activity can be measured, and the activity is preferably 0.001 U/mg (cell protein) or higher, more preferably 0.005 U/mg or higher, still more preferably 0.01 U/mg or higher. Pyruvate:NADP⁺ oxidoreductase is sensitive to oxygen, and activity expression and measurement are often generally difficult (Inui, H. et al., 1987, J. Biol. Chem., 262: 9130-9135; Rotte, C. et al., 2001, Mol. Biol. Evol., 18:710-720). When the activity cannot be measured due to inactivation or the like, it is possible to confirm expression of the protein by Western blotting or the like, as described in the example section.

As for the gene encoding pyruvate:NADP⁺ oxidoreductase, besides the pyruvate:NADP⁺ oxidoreductase gene of *Euglena gracilis,* which is a photosynthetic eukaryotic microorganism and is also classified into protozoans (Nakazawa et al., 2000, FEBS Lett., 479:155-156), and the pyruvate:NADP⁺ oxidoreductase gene of a protist, *Cryptosporidium parvum* (Rotte, C. et al., 2001, Mol. Biol. Evol., 18:710-720), it is known that a homologous gene also exists in Bacillariophyta, *Tharassiosira pseudonana* (Ctrnacta, V. et al., 2006, J. Eukaryot. Microbiol., 53:225-231).

Specifically, a gene having the nucleotide sequence shown in SEQ ID NO: 5 (GenBank Accession No. AB021127) can be exemplified as the pyruvate:NADP⁺ oxidoreductase gene of *Euglena gracilis.* The amino acid sequence encoded by this gene is shown in SEQ ID NO: 6 (GenBank Accession No. BAB12024).

The microorganism described herein may be a microorganism modified so that the pyruvate synthase activity is increased by such a modification that the activity of recycling oxidized electron donor to reduced electron donor, which is required for the pyruvate synthase activity, is increased as compared to a parent strain, for example, a wild type strain or a non-modified strain. Example of the activity for recycling oxidized electron donor to reduced electron donor include ferredoxin NADP⁺ reductase activity. Furthermore, the microorganism may be a microorganism modified so that the activity of pyruvate synthase is increased by such a modification that pyruvate synthase activity is increased, in addition to the enhancement of the electron donor recycling activity. The aforementioned parent strain may be a strain inherently having a gene encoding the electron donor recycling activity, or a strain which does not inherently have the electron donor recycling activity, but can be imparted with the activity by introduction of a gene encoding the activity, and improve an L-amino acid-producing ability.

The "ferredoxin NADP⁺ reductase" means an enzyme that reversibly catalyzes the following reaction (EC 1.18.1.2).

Reduced ferredoxin + NADP⁺ -> Oxidized ferredoxin + NADPH + H⁺

This reaction is a reversible reaction, and can generate the reduced ferredoxin in the presence NADPH and the oxidized ferredoxin. Ferredoxin is replaceable with flavodoxin, and the enzyme has a function equivalent to that of the enzyme designated flavodoxin NADP⁺ reductase. Existence of ferredoxin NADP⁺ reductase is confirmed in a wide variety of organisms ranging from microorganisms to higher organisms (refer to Carrillo, N. and Ceccarelli, E.A., 2004, Eur. J. Biochem., 270:1900-1915; Ceccarelli, E.A. et al., 2004, Biochim. Biophys. Acta., 1698:155-165), and some of the enzymes are also named ferredoxin NADP⁺ oxidoreductase or NADPH-ferredoxin oxidoreductase.

Enhancement of the ferredoxin NADP⁺ reductase activity can be confirmed by preparing crude enzyme solutions from the microorganism before the modification and the microorganism after the modification, and comparing the ferredoxin NADP⁺ reductase activity of them. The activity of ferredoxin NADP⁺ reductase can be measured by, for example, the method of Blaschkowski et al. (Blaschkowski, H.P. et al., 1989, Eur. J. Biochem., 123:563-569). For example, the activity can be measured by using ferredoxin as a substrate to spectroscopically measure decrease of the amount of NADPH. One unit (U) of the enzymatic activity is defined as activity for oxidizing 1 µmol of NADPH per 1 minute. When the parent strain has the ferredoxin NADP⁺ reductase activity, and the activity of the parent strain is sufficiently high, it is not necessary to enhance the activity. However, the enzymatic activity is desirably increases preferably 1.5 times or more, more preferably 2 times or more, still more preferably 3 times or more, compared with that of the parent strain.

Genes encoding the ferredoxin NADP⁺ reductase are found in many biological species, and any of them showing the activity in the objective L-amino acid producing strain can be used. As for *Escherichia coli,* the *fpr* gene has been identified as a gene for flavodoxin NADP⁺ reductase (Bianchi, V. et al., 1993, 175:1590-1595). Moreover, it is known that, in *Pseudomonas putida,* an NADPH-putidaredoxin reductase gene and a putidaredoxin gene exist as an operon (Koga, H. et al., 1989, J. Biochem. (Tokyo), 106:831-836).

Examples of the flavodoxin NADP⁺ reductase gene of *Escherichia coli* include the *fpr* gene which locates at the nucleotide numbers 4111749 to 4112495 (complementary strand) of the genome sequence of the *Escherichia coli* K-12 strain (Genbank Accession No. U00096) and which has the nucleotide sequence shown in SEQ ID NO: 7. The amino acid sequence of Fpr is shown in SEQ ID NO: 8 (Genbank Accession No. AAC76906). Moreover, a ferredoxin NADP⁺ reductase gene (Genbank Accession No. BAB99777) is also found at the nucleotide numbers 2526234 to 2527211 of the genome sequence of *Corynebacterium glutamicum* (Genbank Accession No. BA00036).

The pyruvate synthase activity requires presence of ferredoxin or flavodoxin as an electron donor. Therefore, the microorganism may be a microorganism modified so that the activity of pyruvate synthase is increased by such a modification that the production ability for ferredoxin or flavodoxin is improved.

Moreover, the microorganism may also be modified so that the production ability for ferredoxin or flavodoxin is improved, in addition to being modified so that pyruvate synthase activity or flavodoxin NADP⁺ reductase and pyruvate synthase activities is enhanced.

In the present invention, the "ferredoxin" refers to a protein containing nonheme iron atoms (Fe) and sulfur atoms, bound with an iron-sulfur cluster called 4Fe-4S, 3Fe-4S or 2Fe-2S cluster, and functioning as a one-electron carrier. The "flavodoxin" refers to a protein containing FMN (flavin-mononucleotide) as a prosthetic group and functioning as a one- or two-electron carrier. Ferredoxin and flavodoxin are described in the reference of McLean et al. (McLean K.J. et al., 2005, Biochem. Soc. Trans., 33:796-801).

The parent strains to be subjected to the modification may be strains which inherently have an endogenous gene encoding ferredoxin or flavodoxin. Alternatively, the parent strains may be strains which do not inherently have a gene encoding ferredoxin or flavodoxin, but which can be imparted with the activity by introduction of a ferredoxin or flavodoxin gene to show improved L-glutamic acid-producing ability.

Improvement of ferredoxin or flavodoxin-producing ability compared with the parent strain such as a wild-type or non-modified strain can be confirmed by, for example, comparing amount of mRNA for ferredoxin or flavodoxin with that of a wild type strain or non-modified strain. Examples of the method for confirming the expression amount include Northern hybridization and RT-PCR (Sambrook, J. Et al., 1989, Molecular Cloning A Laboratory Manual/Second Edition, Cold Spring Harbor Laboratory Press, New York.). Degree of the increase of the expression is not particularly limited so long as it increases compared with that of a wild type strain or non-modified strain. However, it is desirably increases, for example, 1.5 times or more, preferably 2 times or more, more preferably 3 times or more, compared with that of a wild type strain or non-modified strain.

Improvement of the ferredoxin or flavodoxin-producing ability compared with a parent strain, for example, a wild type strain or a non-modified strain, can be detected by SDS-PAGE, two-dimensional electrophoresis, or Western blotting using antibodies (Sambrook J. et al., 1989, Molecular Cloning A Laboratory Manual/Second Edition, Cold Spring Harbor Laboratory Press, New York). Degree of the improvement of the production is not particularly limited so long as it increases compared with that of a wild type strain or non-modified strain. However, it is desirably increases, for example, 1.5 times or more, preferably 2 times or more, more preferably 3 times or more, compared with that of a wild type strain or non-modified strain.

The activities of ferredoxin and flavodoxin can be measured by adding them to a suitable oxidation-reduction reaction system. For example, a method comprising reducing produced ferredoxin with ferredoxin NADP⁺ reductase and quantifying reduction of cytochrome C by the produced reduced ferredoxin is disclosed by Boyer et al. (Boyer, M.E. et al., 2006, Biotechnol. Bioeng., 94:128-138). Furthermore, the activity of flavodoxin can be measured by the same method using flavodoxin NADP⁺ reductase.

Genes encoding ferredoxin or flavodoxin are widely distributed, and any of those can be used so long as ferredoxin or flavodoxin encoded by the genes can be utilized by pyruvate synthase and an electron donor recycling system. For example, in *Escherichia coli,* the fdx gene exists as a gene encoding ferredoxin which has a 2Fe-2S cluster (Ta, D.T. and Vickery, L.E., 1992, J. Biol. Chem., 267:11120-11125), and the *yfhL* gene is expected as a gene encoding ferredoxin which has a 4Fe-4S cluster. Furthermore, as the flavodoxin gene, the *fldA* gene (Osborne C. et al., 1991, J. Bacteriol., 173:1729-1737) and the *fldB* gene (Gaudu, P. and Weiss, B., 2000, J. Bacteriol., 182:1788-1793) are known. In the genome sequence of *Corynebacterium glutamicum* (Genbank Accession No. BA00036), multiple ferredoxin genes, *fdx* (Genbank Accession No. BAB97942) were found at the nucleotide numbers of 562643 to 562963, and the *fer* gene was found at the nucleotide numbers of 1148953 to 1149270 (Genbank Accession No. BAB98495). Furthermore, in the *Chlorobium tepidum*, many ferredoxin genes exist, and ferredoxin I and ferredoxin II have been identified as ferredoxin genes for the 4Fe-4S type which serves as the electron acceptor of pyruvate synthase (Yoon, K.S. et al., 2001, J. Biol. Chem., 276:44027-44036). Ferredoxin gene or flavodoxin gene of bacteria having the reductive TCA cycle such as the ferredoxin gene of *Hydrogenobacter thermophilus* and the like can also be used.

Specific examples of the ferredoxin gene of *Escherichia coli* include the *fdx* gene locating at the nucleotide numbers of 2654770 to 2655105 (complementary strand) of the genome sequence of the *Escherichia coli* K-12 strain (Genbank Accession No. U00096) and shown in SEQ ID NO: 9, and the *yfhL* gene locating at the nucleotide numbers of 2697685 to 2697945 of the same and shown in SEQ ID NO: 11. The amino acid sequences of Fdx and YfhL are shown in SEQ ID NOS: 10 and 12 (Genbank Accession Nos. AAC75578 and AAC75615, respectively). Examples of the flavodoxin gene of *Escherichia coli* include the *fldA* gene locating at the nucleotide numbers of 710688 to 710158 (complementary strand) of the genome sequence of the *Escherichia coli* K-12 strain (Genbank Accession No. U00096) and shown in SEQ ID NO: 13, and the *fldB* gene locating at the nucleotide numbers 3037877 to 3038398 of the same and shown in SEQ ID NO: 15. The amino acid sequences encoded by the *fldA* gene and the *fldB* gene are shown in SEQ ID NOS: 14 and 16 (Genbank Accession Nos. AAC73778 and AAC75933, respectively).

Examples of the ferredoxin gene of *Chlorobium* tepidum include the ferredoxin I gene locating at the nucleotide numbers of 1184078 to 1184266 in the genome sequence of *Chlorobium tepidum* (Genbank Accession No. NC_002932) and shown in SEQ ID NO: 17, and the ferredoxin II gene locating at the nucleotide numbers of 1184476 to 1184664 of the same and shown in SEQ ID NO: 19. The amino acid sequences of Ferredoxin I and Ferredoxin II are shown in SEQ ID NOS: 18 and 20 (Genbank Accession Nos. AAM72491 and AAM72490, respectively). Examples further include the ferredoxin gene of *Hydrogenobacter thermophilus* (Genbank Accession No. BAE02673) and the ferredoxin gene of *Sulfolobus solfataricus* indicated with the nucleotide numbers of 2345414 to 2345728 in the genome of *Sulfolobus solfataricus*. Furthermore, the gene may be those cloned from *Chlorobium, Desulfobacter, Aquifex, Hydrogenobacter, Thermoproteus, Pyrobaculum* bacteria or the like on the basis of homology to the genes exemplified above, or those cloned from γ-proteobacteria such as those of the genus *Enterobacter, Klebsiella, Serratia, Erwinia* and *Yersinia*, coryneform bacteria such as *Corynebacterium glutamicum* and *Brevibacterium lactofermentum, Pseudomonas* bacteria such as *Pseudomonas aeruginosa, Mycobacterium* bacteria such as *Mycobacterium tuberculoses,* and so forth.

As these genes encoding pyruvate synthase, ferredoxin-NADP⁺ reductase, ferredoxin, and flavodoxin (henceforth generically called the gene of the present invention), genes having conservative mutations such as homologues or artificially modified genes of the genes can also be used so long as functions of the encoded proteins are not degraded. That is, they may be those encoding a conservative variant of the proteins having amino acid sequences of known proteins or wild-type proteins including substitution, deletion, insertion or addition of one or several amino acid residues at one or several positions. Although the number of the "one or several" amino acid residues referred to herein may differ depending on positions in the three-dimensional structure or types of amino acid residues of the proteins, it is preferably 1 to 20, more preferably 1 to 10, particularly preferably 1 to 5.

These mutations are preferably conservative substitutions that are neutral mutations and preserve the function of the protein. A conservative substitution is a mutation wherein substitution takes place mutually among Phe, Trp and Tyr, if the substitution site is an aromatic amino acid; among Leu, Ile and Val, if the substitution site is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having a hydroxyl group.

Specific examples of conservative substitutions include: substitution of Ser or Thr for Ala; substitution of Gln, His or Lys for Arg; substitution of Glu, Gln, Lys, His or Asp for Asn; substitution of Asn, Glu or Gin for Asp; substitution of Ser or Ala for Cys; substitution of Asn, Glu, Lys, His, Asp or Arg for Gln; substitution of Gly, Asn, Gln, Lys or Asp for Glu; substitution of Pro for Gly; substitution of Asn, Lys, Gln, Arg or Tyr for His; substitution of Leu, Met, Val or Phe for Ile; substitution of Ile, Met, Val or Phe for Leu; substitution of Asn, Glu, Gln, His or Arg for Lys; substitution of Ile, Leu, Val or Phe for Met; substitution of Trp, Tyr, Met, Ile or Leu for Phe; substitution of Thr or Ala for Ser; substitution of Ser or Ala for Thr; substitution of Phe or Tyr for Trp; substitution of His, Phe or Trp for Tyr; and substitution of Met, Ile or Leu for Val. The above-mentioned amino acid substitution, deletion, insertion, addition, inversion etc. may be a result of a naturally-occurring mutation or variation due to an individual difference, or a difference of species of a bacterium harboring the gene of the present invention.

Furthermore, a gene with substitutions of codons that can be easily used in a host into which the gene of the present invention is introduced may be used. Similarly, so long as the gene of the present invention maintains its function, the gene may be extended or shortened at either the N-terminus and/or C-terminus. The length of the extension or shortening is, for example, 50 or less, preferably 20 or less, more preferably 10 or less, particularly preferably 5 or less, in terms of the number of amino acid residues.

A gene encoding such a conservative variant can be obtained by, for example, modifying the nucleotide sequence by site-specific mutagenesis so that amino acid residues of specific sites of the encoded protein includes substitutions, deletions, insertions or additions of amino acid residues. Furthermore, it can also be obtained by the conventionally known mutagenesis. Examples of the mutagenesis include treating the gene of the present invention with hydroxylamine or the like *in vitro,* and irradiating ultraviolet to a microorganism such as an Escherichia bacterium containing the gene, or treating the microorganism with a mutagen used for usual mutagenesis such as N-methyl-N'-nitro-N-nitrosoguanidine (NTG) or ethyl methanesulfonate (EMS). Moreover, such substitutions, deletions, insertions, additions, inversions etc. of amino acid residues as described above include those caused by a naturally occurring mutation or variation based on difference of individuals or species of the microorganism containing the gene of the present invention. Whether those gene encodes functional pyruvate synthase, ferredoxin-NADP⁺ reductase, ferredoxin, or flavodoxin can be confirmed by, for example, introducing each gene into a microorganism, and measuring the activity of expression product of each gene.

The gene of the present invention may be a DNA which is able to hybridizes with a DNA having any one of the aforementioned nucleotide sequences or a probe prepared from a DNA which has any one of these nucleotide sequences under stringent conditions and which encodes pyruvate synthase, ferredoxin-NADP⁺ reductase, ferredoxin, or flavodoxin.

The term "stringent conditions" refers to conditions where a so-called specific hybrid is formed and a nonspecific hybrid is not formed. It is difficult to clearly define the conditions by numerical value, but examples thereof include conditions where DNAs having high homology, for example, at least 70%, preferably 80%, more preferably 90%, and further more preferably 95% homology, hybridize with each other and DNAs having homology less than the value do not hybridize with each other; and specifically include conditions corresponding to salt concentration and temperature of washing conditions of typical of Southern hybridization, e.g., washing at 60°C, 1×SSC, 0.1% SDS, preferably 60°C, 0.1×SSC, 0.1% SDS, more preferably 68°C, 0.1×SSC, 0.1% SDS, once or preferably twice or three times. In the present specification, the term "homology" may mean "identity".

The probe may be one having a partial sequence of the gene of the present invention. Such a probe can be prepared by PCR using oligonucleotides prepared based on the nucleotide sequence of each gene as primers according to a method well known to a person skilled in the art, and a DNA fragment containing each gene as a template. When a DNA fragment of a length of about 300 bp is used as the probe, the conditions of washing after hybridization consist of, for example, 50°C, 2×SSC, and 0.1% SDS.

The aforementioned descriptions concerning the conservative variant is also applied to the enzymes and genes described above for impartation of L-amino acid-producing ability.

The modification for enhancing expression of the gene of the present invention can be performed in the same manner as that of the method for enhancing expression of a target gene described for the impartation of the L-Amino acid-producing ability. The gene of the present invention can be obtained by PCR using a chromosomal DNA of a microorganism having it as a template.

For example, the pyruvate synthase gene of *Chlorobium tepidum* can be obtained by PCR (polymerase chain reaction)(see White, T. J. et al., 1989, Trends Genet., 5:185-189) using primers prepared on the basis of the nucleotide sequence of SEQ ID NO: 1, for example, the primers shown in SEQ ID NOS: 35 and 36, and the chromosomal DNA of *Chlorobium tepidum* as a template.

The pyruvate synthase gene of *Escherichia coli* can be obtained by PCR using primers prepared on the basis of the nucleotide sequence of SEQ ID NO: 3, for example, the primers shown in SEQ ID NOS: 38 and 39, and the chromosomal DNA of *Escherichia coli* as a template.

The NADP⁺ oxidoreductase gene of *Euglena gracilis* can be obtained by PCR using primers prepared on the basis of the nucleotide sequence of SEQ ID NO: 5, for example, the primers shown in SEQ ID NOS: 40 and 41, and the chromosomal DNA of *Euglena gracilis* as a template.

The flavodoxin NADP⁺ reductase gene of *Escherichia coli* can be obtained by PCR using primers prepared on the basis of the nucleotide sequence of SEQ ID NO: 7, for example, the primers shown in SEQ ID NOS: 42 and 43, and the chromosomal DNA of *Escherichia coli* as a template.

The ferredoxin gene *fdx* of *Escherichia coli* can be obtained by PCR using primers prepared on the basis of the nucleotide sequence of SEQ ID NO: 9, for example, the primers shown in SEQ ID NOS: 44 and 45, and the chromosomal DNA of *Escherichia coli* as a template.

The flavodoxin gene *fldA* of *Escherichia coii* can be obtained by PCR using primers prepared on the basis of the nucleotide sequence of SEQ ID NO: 13, and the flavodoxin gene *fldB* of *Escherichia coli* can be obtained by PCR using primers prepared on the basis of the nucleotide sequence of SEQ ID NO: 15, and the chromosomal DNA of *Escherichia coli* as a template, respectively.

Furthermore, the ferredoxin I gene of *Chlorobium tepidum* can be obtained by PCR using primers prepared on the basis of the nucleotide sequence of SEQ ID NO: 17, and the ferredoxin II gene of *Chlorobium tepidum* can be obtained by PCR using primers prepared on the basis of the nucleotide sequence of SEQ ID NO: 19, with using the chromosomal DNA of *Chlorobium tepidum* as a template in both cases.

The gene of the present invention from other microorganisms can also be obtained from the chromosomal DNA or a chromosomal DNA library of the chosen microorganism by PCR using, as primers, oligonucleotides prepared based on the sequences of the aforementioned gene or sequences of genes or proteins known in the chosen microorganism; or hybridization using an oligonucleotide prepared based on such sequence as mentioned above as a probe. A chromosomal DNA can be prepared from a microorganism that serves as a DNA donor by the method of Saito and Miura (Saito H. and Miura K., 1963, Biochem. Biophys. Acta, 72:619-629;, Experiment Manual for Biotechnology, edited by The Society for Biotechnology, Japan, p97-98, Baifukan Co., Ltd., 1992) or the like.

Enhancement of expression of the gene of the present invention and genes of L-amino acid synthesis systems can be attained by increasing copy number of the gene of the present invention by performing transformation or homologous recombination or modifying an expression control sequence of the gene of the present invention in such manners as described above. Furthermore, enhancement of expression of the gene of the present invention can also be attained by amplifying an activator which increases expression of the gene of the present invention, and/or by eliminating or attenuating a regulator which reduce expression of the gene of the present invention.

Methods for enhancing a gene will be explained below.

The first method is increasing copy number of a target gene. For example, copy number of the target gene can be increased by cloning the gene on an appropriate vector and transforming a host microorganism with the obtained vector.

The vector used for transformation may be a plasmid autonomously replicable in a cell of the host microorganism. Examples of the plasmid autonomously replicable in bacteria of the *Enterobacteriaceae* include plasmid vectors pUC19, pUC18, pBR322, RSF1010, pHSG299, pHSG298, pHSG399, pHSG398, pSTV28, pSTV29 (pHSG and pSTV vectors are available from Takara Bio Inc.), pMW119, pMW118, pMW219, pMW218 (pMW vectors are available from Nippon Gene Co., Ltd.) and so forth. Furthermore, plasmids for coryneform bacteria include pAM330 (Japanese Patent Laid-open No. 58-67699), pHM1519 (Japanese Patent Laid-open No. 58-77895), pSFK6 (Japanese Patent Laid-open No. 2000-262288), pVK7 (USP2003-0175912A), pAJ655, pAJ611, pAJ1844 (Japanese Patent Laid-open No. 58-192900), pCG1 (Japanese Patent Laid-open No. 57-134500), pCG2 (Japanese Patent Laid-open No. 58-35197), pCG4, pCG11 (Japanese Patent Laid-open No. 57-183799), pHK4 (Japanese Patent Laid-open No. 5-7491) and so forth. Moreover, if a DNA fragment having an ability to make a plasmid autonomously replicable in a coryneform bacterium is cut out from these vectors and inserted into the aforementioned vectors for *Escherichia coli,* they can be used as a so-called shuttle vector autonomously replicable in both of *Escherichia coli* and coryneform bacteria. In addition, a phage DNA may also be used as the vector instead of a plasmid.

Examples of transformation methods include treating recipient cells with calcium chloride so to increase permeability of the DNA, which has been reported for *Escherichia coli* K-12 (Mandel, M. and Higa, A., 1970, J. Mol. Biol., 53:159-162), and preparing competent cells from cells which are at the growth phase, followed by transformation with DNA, which has been reported for *Bacillus subtilis* (Duncan, C.H. et al., 1977, Gene, 1:153-167). Alternatively, a method of making DNA-recipient cells into protoplasts or spheroplasts, which can easily take up recombinant DNA, followed by introducing the recombinant DNA into the cells, which is known to be applicable to *Bacillus subtilis,* actinomycetes and yeasts (Chang, S. and Choen, S.N., 1979, Mol. Gen. Genet., 168:111-115; Bibb, M.J. et al., 1978, Nature, 274:398-400; Hinnen, A. et al., 1978, Proc. Natl. Sci., USA, 75:1929-1933) can also be employed. In addition, transformation of microorganisms can also be performed by the electric pulse method (Japanese Patent Laid-open No. 2-207791).

Increasing the copy number of a target gene can also be achieved by introducing multiple copies of the gene into the chromosomal DNA of the microorganism. Introducing multiple copies of the gene into the chromosomal DNA of the microorganism may be performed by homologous recombination (MillerI, J. H. Experiments in Molecular Genetics, 1972, Cold Spring Harbor Laboratory) using a sequence whose multiple copies exist as targets in the chromosomal DNA. Sequences having multiple copies in the chromosomal DNA include, but are not limited to, repetitive DNA, and inverted repeats existing at the end of a transposable element. Also, as disclosed in Japanese Patent Laid-open No. 2-109985, it is possible to incorporate the target gene into a transposon, and allow it to be transferred to introduce multiple copies of the gene into the chromosomal DNA. Introduction of a target gene into a bacterial chromosome can be also achieved by the method using Mu phage (Japanese Patent Laid-open No. 2-109985), or the like. Transfer of a target gene to a chromosome can be confirmed by Southern hybridization using a part of the gene as a probe.

When copy number of a gene is increased, the copy number is not particularly limited so long as activity of the product of the target gene can be enhanced. However, when the microorganism originally has the target gene, the copy number is preferably 2 or more. When the microorganism does not originally have the gene of the present invention, the copy number of the gene introduced may be 1, but it may also be 2 or more.

The second method is enhancing expression of a target gene by replacing an expression regulatory sequence of the target gene such as promoter on the chromosomal DNA or plasmid with a promoter which has an appropriate strength. For example, the thr promoter, lac promoter, trp promoter, trc promoter, pL promoter, tac promoter, etc., are known as promoters frequently used. Examples of promoters with high expression activity in coryneform bacteria include promoter of the elongation factor Tu (EF-Tu) gene, *tuf*, promoters of genes that encode cochaperonin GroES-chaperonin GroEL, thioredoxin reductase, phosphoglycerate mutase, glyceraldehyde-3-phophate dehydrogenase, and the like (WO2006/028063 EP1697525). Examples of strong promoters and methods for evaluating the strength of promoters are described in an article by Goldstein and Doi (Goldstein, M. A. and Doi R. H., 1995, Biotechnol. Annu. Rev., 1:105-128), etc.

Moreover, it is also possible to substitute several nucleotides in a promoter region of a gene, so that the promoter has an appropriate strength, as disclosed in International Patent Publication WO00/18935. Substitution of the expression regulatory sequence can be performed, for example, in the same manner as in gene substitution using a temperature sensitive plasmid. Examples of vectors having a temperature sensitive replication origin which can be used for *Escherichia coli* or *Pantoea ananatis* include, for example, the temperature sensitive plasmid pMAN997 described in International Publication WO99/03988, its derivative, and so forth. Furthermore, substitution of an expression regulatory sequence can also be performed by methods which employ linear DNA, such as a method called "Red-driven integration" using Red recombinase of λ phage (Datsenko, K.A. and Wanner, B.L., 2000, Proc. Natl. Acad. Sci.. USA., 97:6640-6645), a method combining the Red-driven integration method and the λ phage excision system (Cho, E. H. et al., 2002, J. Bacteriol., 184:5200-5203) (WO2005/010175 and so forth. The modification of an expression regulatory sequence can be combined with the increasing gene copy number described above.

Furthermore, it is known that substitution of several nucleotides in a spacer between the ribosome binding site (RBS) and the start codon, in particular, the sequences immediately upstream of the start codon profoundly affect the mRNA translatability. Translation can be enhanced by modifying these sequences.

When pyruvate synthase consists of multiple subunits, expressions of genes encoding the subunits may be individually enhanced, or may be simultaneously enhanced as a polycistron. Furthermore, when the genes are introduced into a microorganism by using a vector, the genes encoding the subunits may be simultaneously carried by a single vector molecule, or may be separately carried by different vector molecules. Also when the genes encoding the subunits are inserted into a chromosome, the genes may be simultaneously inserted into the same site on the genome, or may be separately inserted on different sites.

Furthermore, in the bacterium used for the present invention, it is preferable that malic enzyme activity is decreased, in addition to that pyruvate synthase acitivity or pyruvate:NADH⁺ oxidoreductase activity is enhanced. Especially, it is preferred that malic enzyme activity is reduced when the bacterium of the present invention is a bacterium belonging to the genus *Escherichia, Enterobacter, Pantoea, Klebsiella,* or *Serattia.*

In the present invention, malic enzyme activity means an activity for catalyzing reversively the reaction of oxidatively decarboxylating malic acid to produce pyruvic acid. The aforementioned reaction is catalyzed by two kinds of enzymes, NADP-dependent malic enzyme which uses NADP as electron acceptor (also noted as "malate dehydrogenase (oxaloacetate-decarboxylating)(NADP⁺)", (EC:1.1.1.40 *b2463* gene (also noted as *maeB* gene, SEQ ID NO: 54), and NAD-dependent malic enzyme which uses NAD as electron acceptor (also noted as "malate dehydrogenase (oxaloacetate-decarboxylating) (NAP⁺)", (EC:1.1.1.38 *sfc* gene (also noted as *maeA* gene, SEQ ID NO: 52). Malic enzyme activity can be measured by the method of Bologna et al. (Bologna, F. P. et al. 2007. J. Bacteriol. 2007 189: 5937-5946).
NADP-dependent malic enzyme : NADP⁺ + malate -> NADPH + CO₂ + pyruvate
NAD-dependent malic enzyme : NAD⁺ + malate -> NADH + CO₂ + pyruvate

The enzyme activity can be decreased in the same manner as in decreasing pyruvate dehydrogenase activity as described below.

In the present invention, it is preferable that both of NADP-dependent malic enzyme and NAD-dependent malic enzyme. It is particularly preferred that activities of both type of malic enzymes are decreased when the bacterium of the present invention is a bacterium belonging to the genus *Escherichia, Enterobacter,* Pantoea, *Klebsiella,* or *Serattia.*

Furthermore, in the microorganism used for the present invention, it is preferred that pyruvate dehydrogenase activity is reduced, in addition to that pyruvate synthase acitivity or pyruvate:NADH⁺ oxidoreductase activity is enhanced.

In the present invention, pyruvate dehydrogenase (henceforth also referred to as "PDH") activity means an activity for catalyzing the reaction of oxidatively decarboxylating pyruvic acid to produce acetyl-CoA. The aforementioned reaction is catalyzed by three kinds of enzymes, PDH (E1p, pyruvate dehydrogenase, EC:1.2.4.1, *aceE* gene, SEQ ID NO: 46), dihydrolipoyl transacetylase (E2p, EC:2.3.1.12, *aceF* gene, SEQ ID NO: 48), and dihydrolipoamide dehydrogenase (E3, EC:1.8.1.4, *lpdA* gene, SEQ ID NO: 50). That is, these three kinds of subunits catalyze the following reactions, respectively, and the activity for catalyzing the reaction corresponding to the total of these three reactions is called PDH activity. As for confirmation of the PDH activity, the activity can be measured according to the method of Visser and Strating (Visser, J. and Strating, M., 1982, Methods Enzymol., 89:391-399).
Elp: Pyruvate + [dihydrolipoyllysine-residue succinyltransferase] lipoyllysine -> [dihydrolipoyllysine-residue acetyltransferase] S-acetyldihydrolipoyllysine + CO₂
E2p: CoA + enzyme N6-(S-acetyldihydrolipoyl)lysine -> acetyl-CoA + enzyme N6-(dihydrolipoyl)lysine
E3: Protein N6-(dihydrolipoyl)lysine + NAD⁺ -> protein N6-(lipoyl)lysine + NADH + H⁺

Decreasing enzyme activity may be achieved by, for example, deleting a part or entire region of the coding region of one or more genes of *aceE, aceF* and *lpdA,* or by modifying an expression control sequence such as a promoter or Shine Dargarno (SD) sequence, or the like. Expression amount of the genes can also be reduced by modifying a non-translation region other than expression control regions. Furthermore, entire gene including upstream and downstream regions of the genes on a chromosome may be deleted. In addition, an amino acid substitution (missense mutation), a stop codon (nonsense mutation), or a frame shift mutation which adds or deletes one or two nucleotides may be introduced into an enzyme coding region on a chromosome by genetic recombination (Wang, J. P. et al. 2006. J. Agric. Food Chem. 54: 9405-9410; Winkler, W. C. 2005. Curr. Opin. Chem. Biol. 9: 594-602; Qiu, Z. and Goodman, M. F. 1997. J. Biol. Chem. 272: 8611-8617; Wente, S. R. and Schachman, H. K. 1991. J. Biol. Chem. 266: 20833-20839).

In the present invention, it is preferable to delete a part or all of an expression control sequence such as promoter region, a coding region or a non-coding region of a gene on a chromosome, or insert another sequence into these regions by homologous recombination to reduce the intracellular enzymatic activity. However, the modification may be a modification based on usual mutatagenesis, such as exposure to X-rays or UV irradiation, or treatment with a mutagen such as N-methyl-N'-nitro-N-nitrosoguanidine, etc., so long as the PDH activity is reduced by the modification.

Modification of an expression control sequence is performed for preferably one or more nucleotides, more preferably two or more nucleotides, particularly preferably three or more nucleotides. When a coding region is deleted, the region to be deleted may be an N-terminus region, an internal region or a C-terminus region, or even the entire coding region, so long as the function of the enzyme protein to be produced is reduced. Deletion of a longer region can usually more surely inactivate the gene. Furthermore, it is preferred that reading frames upstream and downstream of the region to be deleted are not the same.

Also when another sequence is inserted into a coding region, the sequence may be inserted into any region of the gene, and insertion of a longer region can usually more surely inactivate the gene. It is preferred that reading frames upstream and downstream of the insertion site are not the same. The other sequence is not particularly limited so long as a sequence which reduces or deletes function of the enzyme protein is chosen, and examples include, for example, a transposon carrying an antibiotic resistance gene or a gene useful for L-amino acid production.

Such modification of a gene on a chromosome as described above can be attained by, for example, preparing a deletion type gene in which a partial sequence of a gene of interest is deleted so that a protein that can normally function is produced, and transforming a bacterium with a DNA containing the deletion type gene to cause homologous recombination between the deletion type gene and the gene on a chromosome and thereby substitute the deletion type gene for the gene on the genome. The enzyme protein encoded by the deletion type gene has a conformation different from that of a wild-type enzyme protein, even if it is produced, and thus the function thereof is reduced or deleted. Gene disruption based on gene substitution utilizing such homologous recombination can be performed by the aforementioned Red driven integration method, a method of using a linear DNA such as a method utilizing the Red driven integration method in combination with an excision system derived from λ phage, a method of using a plasmid containing a temperature sensitive replication origin, or a plasmid capable of conjugative transfer, a method of utilizing a suicide vector not having replication origin in a host (U.S. Patent No. 6,303,383, JP 05-007491 A) etc.

The aforementioned description concerning reduction of the PDH activity is also applied to "reduction of activity" of the other enzymes described above, or "destruction" of the other genes described above.

When the microorganism used for the present invention is cultured under anaerobic or microaerobic conditions, it may be a microorganism which has been modified so that it does not produce any organic acid or ethanol under the anaerobic or microaerobic conditions, in addition to that pyruvate synthase acitivity or pyruvate: NADH⁺ oxidoreductase activity is enhanced. Examples of the organic acid referred to here include lactic acid, formic acid and acetic acid. Examples of the method for modifying so that any organic acid or ethanol is not produced include a method of disrupting the gene encoding lactate dehydrogenase (Verumi, G.N. et al., 2002, J. Industrial Microbiol. Biotechnol., 28:325-332; Japanese Patent Laid-open No. 2005-95169).

Furthermore, in the bacterium used for the present invention, an assimilability of fatty acids can be increased when the bacterium is cultured in a medium containing fatty acids as carbon sources. The means for increasing the assimilability of fatty acids include, for example, attenuation or elimination of *fadR* gene expression, enhancement of expression of genes involved in fatty acid utilization, *fadL, fadE, fadD, fadB,* and/or *fadA,* or enhancement of expression of *cyoABCED* which is gene cluster encoding subunits of cytochrome bo terminal oxidase complex (Gennis, R. B. and Stewart, V. 1996. p. 217-261. In F. D. Neidhardt (ed.), Escherichia coli and Salmonella Cellular and Molecular Biology/Second Edition, American Society for Microbiology Press, Washington, D.C; Chepuri et al. 1990. J. Biol. Chem. 265: 11185-11192).

The "*fadR* gene" is a gene encoding a transcription factor FadR having a DNA binding ability to regulate fatty acid metabolism, which in found in enterobacteria (DiRusso, C. C. et al. 1992. J. Biol. Chem. 267: 8685-8691; DiRusso, C. C. et al. 1993. Mol. Microbiol. 7: 311-322). The *fadR* gene of *Eschericia coli* is exemplified by a gene having the nucleotide sequence of SEQ ID NO: 82, which is located at the nucleotide positions 1234161 to 1234880 in the genome sequence of *Escherichia coli* (GenBank Accession No. U00096). The amino acid sequence encoded by the gene is shown in SEQ ID NO: 83.

### <2> Method for producing L-amino acid of the present invention

The method of the present invention is a method for producing an L-amino acid by culturing the microorganism used for the present invention in a medium to produce and accumulate an L-amino acid in the medium or cells, and collecting the L-amino acid from the medium or cells as described in the current claims.

For the method of the present invention, batch culture, fed-batch culture and continuous culture may be used. Ethanol or an aliphatic acid in the medium may be contained in starting medium or feed medium, and may be contained in both these medium.

In the present invention, the aforementioned fed-batch culture refers to a culture method in which a medium is continuously or intermittently fed into a culture vessel, and the medium is not extracted until the end of culture. The continuous culture means a method in which a medium is continuously or intermittently fed into a culture vessel, and the medium is extracted from the vessel (usually in a volume equivalent to the volume of fed medium) at the same time. The starting medium means a medium used in batch culture in the fed-batch culture or continuous culture before feeding the feed medium (medium used at the time of the start of the culture), and feed medium means a medium which is supplied to a fermentation tank in the fed-batch culture or continuous culture. The batch culture means a method in which fresh medium is prepared for every culture, and a strain is inoculated into the medium, into which medium is not fed until harvest.

As the carbon source, a substance from which acetyl-CoA can be produced without a decarboxylation reaction is preferred, and specific examples include ethanol, aliphatic acids, aliphatic acid esters including fats and oils which generate an aliphatic acid upon decomposition, and so forth. Examples of using ethanol or an aliphatic acid as the carbon source will be described below.

Ethanol is a monohydric alcohol represented by the molecular formula C₂H₅OH, and it may be used as a pure substance, or a mixture containing ethanol, such as ethanol in a culture medium produced in ethanol fermentation etc., as it is may also be used.

Aliphatic acid is a monovalent carboxylic acid represented by the general formula CₘHₙCOOH. So long as it is assimilable by bacteria having L-amino acid-producing ability, it may have any chain length, and may contain aliphatic acids of any chain lengths at any ratio. Preferred aliphatic acids are oleic acid (C₁₇H₃₃COOH) and palmitic acid (C₁₅H₃₁COOH), and oleic acid is particularly preferred. As for oleic acid, a mixture of long chain aliphatic acids containing oleic acid can be obtained by hydrolysis of fats and oils. Oleic acid used for the present invention can be obtained as a hydrolysate of fats and oils such as palm oil, and oleic acid extracted from any of animal oils, vegetable oils, waste cooking oils, other blended fats and oils, foodstuffs containing fats such as chocolate may be used. Aliphatic acid may be used as a free acid, or as an alkali metal salt such as sodium salts and potassium salts, or an ammonium salt.

Ethanol or aliphatic acid contained in the medium used in the present invention may be contained at any concentration so long as the bacterium used for the method of the present invention can assimilate it as a carbon source. When it is used as a sole carbon source in the medium, it is preferably contained in the medium in an amount of 20% w/v or less, more preferably 10% w/v or less, still more preferably 2% w/v or less. Furthermore, ethanol or aliphatic acid may be contained in the medium at any concentration so long as it is contained at least in such an amount that it can be assimilated as a carbon source by the bacterium. When it is used as a sole carbon source in the medium, it is desirably contained in the medium in an amount of 0.001% w/v or more, preferably 0.05% w/v or more, more preferably 0.1% w/v or more.

As for feed medium, when ethanol or aliphatic acid is used as a sole carbon source, it is preferably contained in the medium in an amount of 10% w/v or less, more preferably 5% w/v or less, still more preferably 1% w/v or less, and it is preferably contained in the medium in an amount of 0.001% w/v or more, more preferably 0.05% w/v or more, still more preferably 0.1% w/v or more.

Although concentration of ethanol can be measured by various methods, measurement by the enzymatic method is convenient and common (Swift R., 2003, Addiction, 98:73-80). Concentration of aliphatic acid can be measured by usual methods such as gas chromatography and HPLC (Lehotay, S. J. and Hajssova, J. TrAC Trends Anal. Chem., 2002, 21:686-697; Lin J.T., Snyder L.R., and McKeon, T.A., 1998, J. Chromatogr. A., 808:43-49).

Furthermore, the medium used for the present invention may contain a mixture of ethanol and an aliphatic acid. Addition concentrations of ethanol and aliphatic acid may be any concentrations so long as the bacterium used for the method of the present invention can assimilate them as a carbon source. However, when a mixture of ethanol and an aliphatic acid is used as a sole carbon source in the medium, it is preferably contained in an amount of 20% w/v or less, more preferably 10% w/v or less, still more preferably 2% w/v or less, in terms of the total concentration. Furthermore, a mixture of ethanol and an aliphatic acid may be contained in the medium at any concentration so long as it is contained at least in such an amount that it can be assimilated as a carbon source by the bacterium. However, when a mixture of ethanol and an aliphatic acid is used as a sole carbon source in the medium, it is desirably contained in the medium in an amount of 0.001% w/v or more, preferably 0.05% w/v or more, more preferably 0.1% w/v or more, in terms of the total concentration of ethanol and oleic acid.

Mixing ratio of ethanol and aliphatic acid may be any ratio so long as they are at such concentrations that the bacteria used for the method of the present invention can assimilate them as a carbon source. However, the aliphatic acid is generally mixed at a ratio of about 2 or less, preferably about 1.5 or less, preferably about 1 or less, based on ethanol, which is taken as 1. Although the lower limit of the mixing ratio of the aliphatic acid is not particularly limited in the case of mixing the aliphatic acid, the aliphatic acid is preferably mixed at a ratio of 0.05 or more, desirably 0.1 or more, based on ethanol, which is taken as 1.

In addition to ethanol or aliphatic acid, or both ethanol and aliphatic acid, other carbon sources may be further added to the medium used for the method of the present invention. Preferred are saccharides such as glucose, fructose, sucrose, lactose, galactose, blackstrap molasses and starch hydrolysate, polyhydric alcohols such as glycerol, and organic acids such as fumaric acid, citric acid and succinic acid. Glucose, sucrose, fructose and glycerol are especially preferred. As glycerol, crude glycerol produced in biodiesel fuel production can also be used. The carbon source may consist of one kind of substance or a mixture of two or more kinds of substances. When other carbon sources are used, ratio of ethanol, aliphatic acid, or a mixture of ethanol and aliphatic acid in the carbon source is preferably 10% by weight or more, more preferably 30% by weight or more, still more preferably 50% by weight or more.

In the present invention, ethanol or aliphatic acid may be contained at a certain constant concentration throughout the culture process, it may be added only to the starting medium or the feed medium, or if other carbon sources are sufficient, there may be a period where ethanol or aliphatic acid temporarily runs short. The term "temporarily" means that, for example, the aliphatic acid may run short for a period corresponding to 10%, 20%, or 30% at most, of the whole fermentation period.

As for the other components to be added to the medium, usual media containing a nitrogen source, inorganic ions, and if needed, other organic components in addition to the carbon source can be used. Examples of the nitrogen source contained in the medium used for the present invention include ammonia, ammonium salts such as ammonium sulfate, ammonium carbonate, ammonium chloride, ammonium phosphate, ammonium acetate and urea, nitrates and so forth. Ammonia gas and aqueous ammonia used for pH adjustment can also be utilized as the nitrogen source. Furthermore, peptone, yeast extract, meat extract, malt extract, corn steep liquor, soybean hydrolysate and so forth can also be utilized. The medium may contain one or more types of these nitrogen sources. These nitrogen sources can also be used for both the starting medium and the feed medium. Furthermore, the same nitrogen source can be used for both the starting medium and the feed medium, or the nitrogen source of the feed medium may be different from that of the starting medium.
The medium used for the present invention preferably contains a phosphoric acid source and a sulfur source in addition to the carbon source, the nitrogen source and sulfur. As the phosphoric acid source, potassium dihydrogenphosphate, dipotassium hydrogenphosphate, phosphate polymers such as pyrophosphoric acid and so forth can be utilized. Although the sulfur source may be any substance containing sulfur atoms, sulfuric acid salts such as sulfates, thiosulfates and sulfites, and sulfur-containing amino acids such as cysteine, cystine and glutathione are desirable, and ammonium sulfate is especially desirable.

Furthermore, the medium may contain a growth promoting factor (nutrient having a growth promoting effect) in addition to the carbon source, nitrogen source and sulfur source. As the growth promoting factor, trace metals, amino acids, vitamins, nucleic acids as well as peptone, casamino acid, yeast extract, soybean protein degradation product and so forth containing the foregoing substances can be used. Examples of the trace metals include iron, manganese, magnesium, calcium and so forth. Examples of the vitamins include vitamin B₁, vitamin B₂, vitamin B₆, nicotinic acid, nicotinamide, vitamin B₁₂ and so forth. These growth promoting factors may be contained in the starting medium or the feed medium.

Furthermore, when an auxotrophic mutant that requires an amino acid or the like for growth thereof is used, it is preferable to supplement a required nutrient to the medium. In particular, since the L-lysine biosynthetic pathway is enhanced and L-lysine degrading ability is often attenuated in L-lysine-producing bacteria that can be used for the present invention as described below, one or more types of substances selected from L-threonine, L-homoserine, L-isoleucine and L-methionine are preferably added. The starting medium and the feed medium may have the same or different medium composition. Furthermore, the starting medium and the feed medium may have the same or different composition. Furthermore, when the feed medium is fed at multiple stages, the compositions of the feed medium fed at the stages may be the same or different.

The culture is preferably performed as aeration culture at a fermentation temperature of 20 to 45°C, particularly preferably at 33 to 42°C. The oxygen concentration is adjusted to 5 to 50%, desirably about 10%. Furthermore, the aeration culture is preferably performed with pH adjusted to 5 to 9. If pH is lowered during the culture, for example, calcium carbonate or an alkali such as ammonia gas and aqueous ammonia is added to neutralize the culture. When culture is performed under such conditions preferably for about 10 to 120 hours, a marked amount of L-amino acid is accumulated in the culture medium. Although the concentration of L-amino acid accumulated is not limited so long as it is higher than that observed with wild type strains and the L-amino acid can be isolated and collected from the medium, it may be 50 g/L or higher, desirably 75 g/L or higher, more desirably 100 g/L or higher.

When the target amino acid is a basic amino acid, the production thereof may be performed by a method in which fermentation is performed by a method wherein pH of the medium is controlled to be 6.5 to 9.0 during the culture and to be 7.2 to 9.0 at the end of the culture, and the internal pressure in the fermentation tank is controlled to be positive during the fermentation, or carbon dioxide gas or a mixed gas containing carbon dioxide is supplied to the medium so that there is a culture period that bicarbonate ions and/or carbonate ions exist in an amount of 2 g/L or larger in the medium, and thereby the bicarbonate ions and/or carbonate ions can be used as counter ions of cations mainly consisting of the basic amino acid, and the objective basic amino acid is collected (refer to JP 2002-065287 A).

L-Amino acid can be collected by a known collection method from the culture medium after the culture. For example, L-amino acid is collected by ion exchange resin method or precipitation method, or after bacterial cells are removed from the culture medium by centrifugation or the like, L-amino acid is collected by concentration for crystallization.

In the present invention, culture of the microorganism may be performed as seed culture and main culture in order to secure accumulation of L-amino acid higher than a certain level. The seed culture may be performed as shaking culture using a flask or the like or batch culture, and the main culture may be performed as fed-batch culture or continuous culture. Alternatively, both the seed culture and the main culture may be performed as batch culture.

When fed-batch culture or continuous culture is performed according to the present invention, the feed medium may be intermittently fed so that supply of ethanol, aliphatic acid or other carbon sources is temporarily stopped. The supply of the feed medium is preferably stopped for, at maximum, 30% or less, desirably 20% or less, particularly desirably 10% or less, of the feeding time. When the feed medium is intermittently fed, the feed medium may be initially added over a predetermined time, and the second and following additions may be controlled so that it is started when elevation of pH or dissolved oxygen concentration is detected by a computer upon depletion of the carbon source in the fermentation medium during an addition-stopped period prior to a certain medium-addition period, and thus the substrate concentration in the culture tank is always automatically maintained at a low level (U.S. Patent No..5,912,113).

The feed medium used for the fed-batch culture is preferably a medium containing ethanol or an aliphatic acid, another carbon source and a nutrient having a growth promoting effect (growth promoting factor), and may be controlled so that the concentration of the aliphatic acid in the fermentation medium is at a predetermined concentration or lower. The expression "predetermined concentration or lower" used herein means that the medium is prepared so that the aliphatic acid concentration in the medium becomes 10% w/v or lower, preferably 5% w/v or lower, more preferably 1% w/v or lower.

As the other carbon source, glucose, sucrose, fructose and glycerol are preferred. As the growth promoting factor, nitrogen sources, phosphoric acid, amino acids and so forth are preferred. As the nitrogen source, ammonia, ammonium salts such as ammonium sulfate, ammonium carbonate, ammonium chloride, ammonium phosphate, ammonium acetate and urea, nitrates and so forth can be used. Furthermore, as the phosphoric acid source, potassium dihydrogenphosphate and dipotassium hydrogenphosphate can be used. As for the amino acids, when an auxotrophic mutant strain is used, it is preferable to supplement a required nutrient. Furthermore, the feed medium may consist of one type of medium, or a mixture of two or more types of media. When two or more types of feed media are used, the media may be mixed and fed by using one feed can, or the media may be separately fed by using two or more feed cans.

When the continuous culture method is used for the present invention, the medium may be extracted and fed simultaneously, or a part of the medium may be extracted, and then the medium may be fed. Furthermore, the method may also be a continuous culture method in which the culture medium containing L-amino acid and bacterial cells is extracted, and only the cells are returned to the fermenter to reuse the cells (French Patent No. 2669935). As the method for continuously or intermittently feeding a nutrient source, the same method as used in the fed-batch culture is used.

The continuous culture method reusing bacterial cells is a method of intermittently or continuously extracting the fermentation medium when the amino acid concentration reaches a predetermined level, extracting only L-amino acid and re-circulating filtration residues containing bacterial cells into the fermenter, and it can be performed by referring to, for example, French Patent No. 2669935.

When the culture medium is intermittently extracted, it is preferred that a part of L-amino acid is extracted when the L-amino acid concentration reaches a predetermined level, and a fresh medium is fed to continue the culture. Furthermore, as for the volume of the medium to be added, the culture is preferably performed so that the final volume of the medium after the addition of the medium is equal to the volume of the culture medium before the extraction. The term "equal" used herein means that the volume after the addition of the medium corresponds to about 93 to 107% of the volume of the medium before the extraction.

When the culture medium is continuously extracted, the extraction is preferably started at the same time as or after the feeding of the nutrient medium. For example, within 5 hours, desirably 3 hours, more desirably 1 hour, after the start of the feeding, the extraction is started. Furthermore, the extraction volume of the culture medium is preferably equal to the volume of the fed medium.

### Examples

Hereafter, the present invention will be still more specifically explained with reference to examples. However, the present invention is not limited by these examples.

### Example 1: Construction of alcohol dehydrogenase (AdhE) mutated strain derived from Escherichia coli

An *Escherichia coli* strain having mutant alcohol dehydrogenase AdhE was constructed so as to obtain an aerobically ethanol assimilable *Escherichia coli* strain. The nucleotide sequence of the wild-type AdhE gene (*adhE*) derived from *Escherichia coli* and the encoded amino acid sequence are shown in SEQ ID NOS: 21 and 22, respectively.

### <1-1> Construction of Escherichia coli MG1655::P_{L-tac}adhE strain

Substitution of the P_{L-tac} promoter for the promoter region of the *Escherichia coli adhE* gene was performed by the method called "Red-driven integration", which was developed by Datsenko and Wanner (Datsenko, K.A. and Wanner, B.L., 2000, Proc. Natl. Acad. Sci. USA., 97:6640-6645) using the excision system derived from λ phage (Cho, E.H. et al., 2002, J. Bacteriol., 184:5200-5203).

By this technique, a genetic recombinant strain can be constructed in one step using a PCR product obtained by using synthetic oligonucleotides designed so as to contain a part of a target gene at the 5' end side and a part of antibiotic resistance gene at the 3' end side, respectively, as primers. By further using the excision system derived from λ phage in combination, the antibiotic resistance gene integrated into the genetic recombinant strain can be eliminated.

A fragment containing the P_{L-tac} promoter and the *cat* gene encoding a chloramphenicol resistance (Cm^{R}) gene was amplified by PCR using the genome of the *Escherichia coli* MG1655 P_{L-tac}xylE strain described in WO2006/043730 as a template and primers shown in SEQ ID NOS: 23 and 24. The primer of SEQ ID NO: 23 contained a sequence complementary to upstream region of the *adhE* gene, and the primer of SEQ ID NO: 23 contained a sequence complementary to a 5' region of the *adhE* gene.

The sequence of the P_{L-tac} promoter is shown in SEQ ID NO: 25. For PCR, Gene Amp PCR System 2700 Amplificatory (Applied Biosystems) and Taq DNA polymerase (Fermentas) were used. The obtained amplified fragment was purified and collected by agarose gel electrophoresis. This fragment was introduced into the *Escherichia coli* MG1655/pKD46 strain harboring the plasmid pKD46 having temperature sensitive replication ability by electroporation. The plasmid pKD46 (Datsenko, K.A. and Wanner, B.L., 2000, Proc. Natl. Acad. Sci. USA, 97:12:6640-45) includes a 2,154 nucleotide DNA fragment of phage λ (nucleotide positions 31088 to 33241, GenBank/EMBL accession no. J02459), and contains genes of the λ Red homologous recombination system (γ, β, exo genes) under the control of the arabinose-inducible ParaB promoter. The plasmid pKD46 is necessary for integration of the PCR product into the chromosome of strain MG1655.

The amplified PCR product was pulified by agarose gel electrophoresis, and was used to electroporation of the *E.* coli MG1655 harboring the plasmid pKD46, which contains the plasmid pKD46 having a temperature-sensitive replication ability.

Competent cells for electroporation were prepared as follows: *E*. *coli* MG1655/pKD46 was grown overnight at 30 °C in LB medium (trypotone 10g/L, yeaset extract 5g/L, NaCl 10g/L) containing ampicillin (100 mg/l), and the culture was diluted 100 times with 5 ml of LB medium containing 100mg/L of ampicillin and 10mM of L-arabinose. The diluted suspention were grown with aeration at 30 °C to an OD₆₀₀ of about 0.6 and then were made electrocompetent by concentrating 100-fold and washing three times with 10% glycerol. Electroporation was performed using 70 µl of cells and about 100 ng of the PCR product. Cells after electroporation were incubated with 1 ml of SOC medium (Sambrook, J. et al., 1989, Molecular Cloning A Laboratory Manual/Second Edition, Cold Spring Harbor Laboratory Press, New York) at 37 °C for 1 hour and then were plated onto LB agar medium (trypotone 10g/L, yeaset extract 5g/L, NaCl 10g/L, agar 15g/L) containing 40 mg/L of chloramphenicol and grown at 37 °C to select Cm^{R} recombinants.

Cm resistant strains were inoculated onto the M9 plate medium (Sambrook J. et al., 1989, Molecular Cloning A Laboratory Manual/Second Edition, Cold Spring Harbor Laboratory Press, New York) containing 2% ethanol, and about 100 clones were selected among strains grown within 2 to 3 days. Then the selected clones were cultured on the M9 plate medium containing 2% ethanol followed by selecting strains grown within 36 hours. In order to determine sequences of the Cm^{R} gene and the promoter egion of these strains, PCR amplification was performed using the primers shown in SEQ ID NOS: 26 and 27. One of the clones confirmed to contain the Cm^{R} gene in the promoter region of the *adhE* gene was cultured at 37°C to eliminate the temperature sensitive plasmid pKD46 and thereby obtain an MG1655:: P_{L-tac}adhE strain.

### <1-2> Construction of Escherichia coli MG1655ΔadhE strain

The *adhE* gene of wild-type *Escherichia coli* MG1655 strain (ATCC 700926) was replaced with inactivated *adhE* gene by the method developed by Datsenko and Wanner. A fragment containing the kan gene encoding the kanamycin resistance (Kan^{R}) gene was amplified by PCR using the plasmid pACYC177 (GenBank/EMBL accession number X06402, Fermentas) as a template and the primers shown in SEQ ID NOS: 28 and 29. The primer of SEQ ID NO: 28 contained a sequence of 40 bases complementary to the region of the 318 bp upstream of the *adhE* gene, and the primer of SEQ ID NO: 29 contained a sequence of 41 bases complementary to the region on the 3' side of the *adhE* gene. For PCR, Gene Amp PCR System 2700 Amplificatory (Applied Biosystems) and Taq DNA Polymerase (Fermentas) were used. The obtained amplified fragment was purified and collected by agarose gel electrophoresis. This fragment was introduced into the *Escherichia coli* MG1655/pKD46 strain harboring the plasmid pKD46 by electroporation.

PCR amplification was performed by using the primers shown in SEQ ID NOS: 30 and 31 for confirming the presence of the Km^{R} gene in clones grown on the LB plate medium (Sambrook, J. et al., 1989, Molecular Cloning A Laboratory Manual/Second Edition, Cold Spring Harbor Laboratory Press, New York) containing 20 µg/ml of kanamycin. One of clones confirmed to contain the Km^{R} gene in the *adhE* gene region was cultured at 37°C to remove the temperature sensitive plasmid pKD46 and thereby obtain an MG1655ΔadhE strain.

### <1-3> Construction of mutant alcohol dehydrogenase (AdhE*)

In order to introduce Glu568Lys (E568K) mutation into AdhE, PCR was performed using the primer of SEQ ID NO: 32 complementary to nucleotide sequences of 1662 to 1701 and 1703 to 1730 of the *adhE* gene and containing a g -> a mutation at the nucleotide of position 1702, the primer of SEQ ID NO: 33 homologous to a 3' end side region of the nucleotide sequence of the *adhE* gene, and the genome of the *Escherichia coli* MG1655 strain as a template. For PCR, Gene Amp PCR System 2700 Amplificatory (Applied Biosystems) and Pyrobest DNA Polymerase (Takara Shuzo) were used. The obtained amplification fragment of 1.05 kbp was purified and collected by agarose gel electrophoresis.

PCR was performed using genome of the *Escherichia coli* MG1655:: P_{L-tac}adhE strain as a template, the primer shown in SEQ ID NO: 34 and the fragment of 1.05 kbp introduced with the mutation as another primer. The primer of SEQ ID NO: 34 is a primer corresponding to the sequence 402 to 425 bp upstream from the start codon of the *adhE* gene. For PCR, Gene Amp PCR System 2700 Amplificatory (Applied Biosystems) and TaKaRa LA DNA Polymerase (Takara Shuzo) were used. The obtained amplification fragment of 4.7 kbp was purified and collected by agarose gel electrophoresis.

In order to replace the wild-type *adhE* gene region with the mutant *adhE* gene, the 4.7 kbp fragment containing the Cm^{R} gene and the mutant *adhE* gene downstream of the P_{L-tac} promoter (cat-P_{L-tac}adhE*) was introduced into the MG1655AadhE/pKD46 strain by electroporation according to the method of Datsenko and Wanner. The obtained clones were selected on the M9 plate medium containing 2% ethanol as a sole carbon source. By sequencing the *adhE* gene of a grown clone, Glu568Lys (gag-aag), Ile554Ser (atc-agc), Glu22Gly (gaa-gga), Met236Val (atg-gtg), Tyr461Cys (tac-tgc) and Ala786Val (gca-gta) were identified as mutations accompanied by amino acid substitution, and this clone was designated MG1655:: P_{L-tac}adhE* strain.

By P1 transduction (Miller, J.H., 1972, Experiments in Molecular Genetics, Cold Spring Harbor Lab. Press, Plainview, NY) for infecting MG1655Δtdh rhtA* strain with Plᵥᵢᵣ phage using the *Escherichia coli* MG1655:: P_{L-tac}adhE* strain, which is a L-threonine-producing strain having ethanol assimilability, as a donor, an MG1655Δtdh rhtA* P_{L-tac}adhE* strain was obtained. The MG1655Atdh rhtA* strain is an L-threonine-producing strain which corresponds to the MG1655 strain in which the tdh gene encoding threonine dehydrogenase has been disrupted by the method of Datsenko and Wanner and the *rhtA23* mutation derived from the VL614rhtA23 strain (Livshits, V.A. et al., 2003, Res. Microbiol., 154:123-135) which is resistant to threonine of high concentration in a minimal medium has been introduced to the *rhtA* gene.

### <1-4> Construction of alcohol dehydrogenase (AdhE) mutated strain derived from Escherichia coli WC196Δmez strain

In order to impart ethanol assimilability to an L-lysine-producing bacterium, the L-lysine-producing bacterium WC196Δmez/pCABD2 strain described in International Patent Publication WO2005/0101 was subjected to P1 transduction using MG1655Δtdh rhtA* P_{L-tac}*adhE** as a donor to obtain a WC196Δmez P_{L-tac}dhE*/pCABD2 strain which is an L-threonine-producing strain having ethanol assimilability. WC196Δmez had been obtained by disrupting sfcA and b2463 genes encoding malic enzyme of the WC196 strain (WO2005/010175) pCABD2 is the plasmid described in U.S. Patent No. 6,040,160 carrying the *dapA** gene imparting resistance to the feedback inhibition by L-lysine, the *lysC** gene imparting resistance to the feedback inhibition by L-lysine, *dapB* gene, and *ddh* gene.

### Example 2: Construction of a plasmid for expression of pyruvate synthase gene of Chlorobium tepidum and measurement of activity

### <2-1> Construction of a plasmid for expression of pyruvate synthase gene of Chlorobium tepidum

*Chlorobium tepidum* is a meso- to thermophilic autotrophic bacterium, of which optimum growth temperature is 48°C. The genome sequence of the *Chlorobium tepidum* TLS strain has been elucidated by Eisen et al. (Eisen, J.A. et al., 2002, Proc. Natl. Acad. Sci. USA, 99:9509-9514). The pyruvate synthase gene was isolated from this strain, and a plasmid expressing it was constructed.

### <2-2> Measurement of pyruvate synthase activity of a strain expressing pyruvate synthase gene of Chlorobium tepidum

PCR was performed using the chromosomal DNA of the *Chlorobium tepidum* TLS strain (ATCC 49652) as a template and the oligonucleotides shown in SEQ ID NOS: 35 and 36 to amplify a pyruvate synthase gene fragment. The obtained gene fragment was digested with *Sac*I, and the digested fragment was inserted into the *Sac*I site of pSTV28 (Takara Bio) to construct a plasmid, which was designated pSTV-PS. After it was confirmed that the pyruvate synthase gene contained no PCR error over the full length by using BigDye Terminators v1.1 Cycle Sequencing Kit, the pyruvate synthase gene was excised from pSTV-PS with *Sac*I, and inserted into the *Sac*I site of pMW-Pthr to construct a plasmid pMW-Pthr-PS for expression of the pyruvate synthase gene. pMW-Pthr is a plasmid corresponding to the vector pMW219 (Nippon Gene) having the promoter region (Pthr) of the threonine operon *(thrABC)* of the *Escherichia coli* K-12 strain between the *Hind*III site and the *Xba*I site and capable of expressing a gene cloned downstream of the promoter. The promoter sequence of the used threonine operon was shown in SEQ ID NO: 37.

pMW-Pthr-PS and the vector pMW-Pthr as a control for comparison were introduced into the WC196ΔcadALldcC/pCABD2 strain by electroporation, respectively, transformants were obtained on the basis of the kanamycin resistance, and introduction of the plasmids was confirmed. The strain expressing the pyruvate synthase gene of *Chlorobium tepidum* was designated WC196ΔcadAΔldcC/pCABD2/pMW-Pthr-PS, and the control strain was designated WC196ΔcadAΔldc/pCABD2/pMW-Pthr.

The aforementioned strains were each inoculated into the LB medium containing 20 mg/L of streptomycin and 40 mg/L of kanamycin, and cultured overnight at 37°C with shaking. The cells were collected by centrifugation and suspended in a 50 mM HEPES buffer (pH 8.0). The cells in the suspension were disrupted by using a ultrasonicator, the suspension was centrifuged at 15000 rpm for 15 minutes, and the obtained supernatant was used as a crude enzyme solution.

Protein concentration in the crude enzyme solution was measured by using Protein Assay CBB Solution (Nakalai Tesque), and the crude enzyme solution containing 250 µg of the total protein was used for the activity measurement.

Measurement of the activity was performed as follows. The reaction was performed by adding the crude enzyme solution to 2 ml of the following reaction solution. The reaction solution containing all the ingredients except for pyruvic acid serving as a substrate was first added to a cell for spectrometry, and the cell was sealed with a rubber stopper and an aluminum cap. After argon gas was injected into the cell for 5 minutes using a syringe to reduce the oxygen concentration in the cell, the cell was set on a spectrophotometer (U-3210 Spectrophotometer, Hitachi), and a pyruvic acid solution was added by using a syringe to start the reaction. The reaction was allowed at 37°C for 30 minutes, and absorbance was periodically measured at 578 nm to examine change of reduced methylviologen amount. The results are shown in Table 1. In the table, the unit of the specific activity is U/mg protein. One unit is defined as activity for reducing 1 nmol of methylviologen per 1 minute.

**[Reaction mixture]**

| | |
|---|---|
| MgCl₂ | 1 mM |
| Dithiothreitol | 1 mM |
| Methylviologen | 5 mM |
| CoA | 0.25 mM |
| Pyruvic acid | 10 mM |
| (added immediately before start of measurement) | |
| HEPES (pH 8.0) | 50 mM |

**Table 1**

| Plasmid | Specific activity |
|---|---|
| pMW-Pthr | 0.0 |
| pMW-Pthr-PS | 1.2 |

### Example 3: Construction of a plasmid for expressing pyruvate synthase gene of Chlorobium tepidum, amplifying flavodoxin NADP⁺ reductase gene of Escherichia coli and ferredoxin gene of Escherichia coli

By using the flavodoxin NADP⁺ reductase gene of *Escherichia coli* and the ferredoxin gene of *Escherichia coli* as coenzyme regenerating systems required for the pyruvate synthase activity, a plasmid simultaneously expressing three of genes including these genes and the pyruvate synthase gene was constructed. The *fpr* gene was used as the ferredoxin-NADP⁺ reductase gene of *Escherichia coli,* and the *fdx* gene was used as the ferredoxin gene of *Escherichia coli.*

### <3-1> Construction of a vector for amplifying flavodoxin NADP⁺ reductase gene of E. coli

PCR was performed using the chromosomal DNA of the E. *coli* MG1655 strain as a template and the oligonucleotides shown in SEQ ID NOS: 42 and 43 to amplify the flavodoxin NADP⁺ reductase gene fragment. The obtained gene fragment was digested with *Sma*I, and inserted into the *Sma*I site of pMW-Pthr to construct a plasmid for amplifying the flavodoxin NADP⁺ reductase gene, which was designated pMW-Pthr-fpr.

### <3-2> Construction of a plasmid for amplifying ferredoxin (fdx) gene of E. coli

PCR was performed using the chromosomal DNA of the *E*. *coli* MG1655 strain as a template and the oligonucleotides shown in SEQ ID NOS: 44 and 45 to amplify the ferredoxin (fdx) gene fragment. The gene fragment was digested with *Eco*RI, and inserted into the *Eco*RI site of pMW-Pthr to construct a plasmid for amplifying the ferredoxin (fdx) gene, pMW-Pthr-fdx.

### <3-3> Construction of a plasmid for amplifying pyruvate synthase gene of C. tepidum and flavodoxin NADP⁺ reductase gene and ferredoxin (fdx) gene of E. coli

pMW-Pthr-fpr was digested with *Sma*I, and the obtained *fpr* gene fragment was ligated with pMW-Pthr-fdx treated with *Sma*I to obtain pMW-Pthr-fpr-fdx. Then, pMW-Pthr-PS was digested with *Sac*I, and the obtained PS gene fragment was ligated with pMWPthr-fpr-fdx treated with SacI to construct a plasmid for expressing the pyruvate synthase gene of *C*. *tepidum* and enhancing expression of the flavodoxin NADP⁺ reductase gene and the ferredoxin *(fdx)* gene of E. *coli,* pMW-Pthr-fpr-PS-fdx.

In the aforementioned plasmids, the pyruvate synthase gene of *C*. *tepidum* is transcribed from Pthr, and the other genes are also transcribed by read through from Pthr.

### Example 4: Effect on L-lysine-producing ability of strain in which expression of pyruvate synthase gene of Chlorobium tepidum, flavodoxin NADP⁺ reductase gene of Escherichia coli and ferredoxin gene of Escherichia coli was enhanced in culture using oleic acid as carbon source

### <4-1> Introduction of plasmid for amplification of pyruvate synthase gene of Chlorobium tepidum, flavodoxin NADP⁺ reductase gene of Escherichia coli and ferredoxin gene of Escherichia coli into WC196Δmez strain

pMW-Pthr-fpr-PS-fdx and the vector pMW-Pthr as a control for comparison were introduced into WC196Δmez/pCABD2 by electroporation, respectively, transformants were obtained on the basis of the kanamycin resistance, and introduction of the plasmids were confirmed. The strain expressing the pyruvate synthase gene of *Chlorobium tepidum*, the flavodoxin NADP⁺ reductase gene of *Escherichia coli* and the ferredoxin gene of *Escherichia coli* was designated WC196Δmez/pCABD2/pMW-Pthr-fpr-PS-fdx, and the control strain was designated WC196Δmez/pCABD2/pMW-Pthr.

### <4-2> Effect on L-lysine-producing ability of strain in which expression of pyruvate synthase gene of Chlorobium tepidum flavodoxin NADP⁺ reductase gene of Escherichia coli and ferredoxin gene of Escherichia coli was enhanced in culture using oleic acid as carbon source

Both the strains WC196Δmez/pCABD2/pMW-Pthr and WC196vmez/pCABD2/pmW-Pthr-fpr-PS-fdx were inoculated on the LB plate medium, respectively, and precultured overnight at 37°C. The cells corresponding to 1/8 of the plate were inoculated into 20 ml of the oleic acid medium having the following composition contained in a 500 ml-volume Sakaguchi flask, and aerobically cultured at a stirring rate of 120 rpm at 37°C for 72 hours. L-Lysine accumulated in the medium was measured by using Biosensor BF-5 (Oji Scientific Instruments). The live cell count in the medium was also measured. Averages of the values obtained in the culture performed in duplicate are shown in Table 2. Improvement in L-lysine accumulation was observed for the strain in which expression of pyruvate synthase gene of *Chlorobium tepidum,* flavodoxin NADP⁺ reductase gene of *Escherichia coli* and ferredoxin gene of *Escherichia coli* was enhanced, compared with the control.

**[Composition of oleic acid medium]**

| | |
|---|---|
| Sodium oleate | 20 g/L |
| Tween80 | 5g/L |
| MgSO₄ · 7H₂O | 1.0 g/L |
| (NH₄)₂SO₄ | 12 g/L |
| KH₂PO₄ | 0.5 g/L |
| Yeast extract | 1.0 g/L |
| FeSO₄ · 7H₂O | 0.01 g/L |
| MnSO₄ · 5H₂O | 0.01 g/L |
| Kanamycin | 40 mg/L |
| Streptomycin | 20 mg/L |
| Calcium carbonate | 30 g/L |
| pH 7.0 (adjusted with KOH) | |

Sterilization conditions: 115°C, 10 minutes. Calcium carbonate was sterilized by dry-heat at 180 °C for more than 2 hours. Sodium oleate and Tween 20 were mixed and sterilized, and then added to the medium. MgSO₄ · 7H₂O and calcium carbonate were separately sterilized and then added to the medium. Kanamycin and streptomycin were separately added to the medium.

**Table 2**

| Strain | L-lysine (g/l) | Live cell count (10⁸/ml) |
|---|---|---|
| WC196Δmez/pCABD2/pMW-Pthr | 1.77 | 22.3 |
| WC196Δmez/pCABD2/pMW-Pthr-fpr-PS-fdx | 2.35 | 13.6 |

### Example 5: Construction of a plasmid for expression of pyruvate synthase gene of Escherichia coli and measurement of activity

An expression plasmid for the *ydbK* gene homologous to the pyruvate synthase gene found in the genome of *Escherichia coli* MG1655 strain was constructed, and the activity was measured.

### <5-1> Construction of a plasmid for expression of pyruvate synthase gene of Escherichia coli

PCR was performed using the chromosomal DNA of the *Escherichia coli* MG1655 strain as a template and the oligonucleotides shown in SEQ ID NOS: 38 and 39 to amplify the pyruvate synthase gene fragment. The obtained gene fragment was digested with *Kpn*I, and the digested fragment was inserted into the *Kpn*I site of pSTV28 (Takara Bio) to construct a plasmid, which was designated pSTV-ydbK. After it was confirmed that the pyruvate synthase gene contained no PCR error over the full length by using BigDye Terminators v1.1 Cycle Sequencing Kit, the pyruvate synthase gene was excised from pSTV-ydbK with *Kpn*I, and inserted into the *Kpn*I site of pMW-Pthr to construct a plasmid pMW-Pthr-ydbK for expression of the pyruvate synthase gene.

### <5-2> Measurement of pyruvate synthase activity of a strain expressing pyruvate synthase gene of Escherichia coli

pMW-Pthr-ydbK and the vector pMW-Pthr as a control for comparison were introduced into the WC196ΔcadAΔldcC/pCABD2 strain by electroporation, respectively, transformants were obtained on the basis of the kanamycin resistance, and introduction of the plasmids was confirmed. The strain expressing the pyruvate synthase gene of *Escherichia* coli was designated WC196ΔcadAΔldcC/pCABD2/pMW-Pthr-ydbK, and the control strain was designated WC196ΔcadAΔldc/pCABD2/pMW-Pthr.

The aforementioned strains were each inoculated into the LB medium containing 20 mg/l of streptomycin and 40 mg/l of kanamycin, and cultured overnight at 37°C with shaking. The cells were collected by centrifugation, and the activity was measured in the same manner as that of the activity measurement method for the strain expressing the pyruvate synthase gene of *Chlorobium tepidum* described in Example 2. The results are shown in Table 3. Whereas the activity of pyruvate synthase was not confirmed for the control strain WC196ΔcadAΔldcC/pCABD2/pMW-Pthr, 8.0 U/mg of the activity was confirmed for the strain expressing pyruvate synthase gene of *Escherichia coli,* WC196ΔcadAΔldcC/pCABD2/pMW-Pthr-ydbK. The results are shown in Table 3. The unit of the specific activity is the same as that used in Table 1.

**Table 3**

| Plasmid | Specific activity |
|---|---|
| pMW-Pthr | 0.0 |
| pMW-Pthr-ydbK | 8.0 |

### Example 6: Construction of a plasmid for expressing pyruvate synthase gene of Escherichia coli, flavodoxin NADP⁺ reductase gene of Escherichia coli and ferredoxin gene of Escherichia coli

The plasmid pMW-Pthr-fpr containing the flavodoxin NADP⁺ reductase gene of *Escherichia coli* described in Example 3 was digested with *Sma*I, and the obtained *fpr* gene fragment was ligated with the plasmid pMW-Pthr-fdx containing the ferredoxin gene of *Escherichia coli* treated with *Sma*I to obtain pMW-Pthr-fpr-fdx. Then, pMW-Pthr-ydbK was digested with *Kpn*I*,* and the obtained *ydbK* gene fragment was ligated with pMW-Pthr-fpr-fdx treated with *Kpn*I to construct a plasmid for enhancing expression of the pyruvate synthase gene of *Escherichia coli,* flavodoxin NADP⁺ reductase gene of *Escherichia coli* and ferredoxin fdx gene, pMW-Pthr-fpr-ydbK-fdx.

### Example 7: Effect on L-lysine-producing ability of a strain in which expression of pyruvate synthase gene of Escherichia coli, flavodoxin NADP⁺ reductase gene of Escherichia coli and ferredoxin gene of Escherichia coli was enhanced in culture using ethanol as carbon source

### <7-1> Introduction of plasmid for amplification of pyruvate synthase gene of Escherichia coli, flavodoxin NADP⁺ reductase gene of Escherichia coli and ferredoxin gene of Escherichia coli into WC196Δmez adhE* strain

pMW-Pthr-fpr-ydbK-fdx and the vector pMW-Pthr as a control for comparison were introduced into WC196Δmez adhE*/pCABD2 by electroporation, respectively, transformants were obtained on the basis of the kanamycin resistance, and introduction of the plasmids were confirmed. The strain expressing flavodoxin NADP⁺ reductase gene, the pyruvate synthase gene, and ferredoxin gene of *Escherichia coli* was designated WC196Δmez P_{L-tac}adhE*/pCABD2/pMW-Pthr-fpr-ydbK-fdx, and the control strain was designated WC196Δmez P_{L-tac}adhE*/pCABD2/pMW-Pthr.

### <7-2> Effect on L-lysine-producing ability of strain in which expression of pyruvate synthase gene of Escherichia coli, flavodoxin NADP⁺ reductase gene of Escherichia coli and ferredoxin gene of Escherichia coli was enhanced in culture using ethanol as carbon source>

Both the strains NC196Δmez P_{L-tac}adhE*/pCABD2/pMN-Pthr and WC196Δmez P_{L-tac}adhE*/pCABD2/pMW-Pthr-fpr-ydbK-fdx were inoculated on the LB plate medium, respectively, and cultured overnight at 37°C. The cells corresponding to 1/8 of the plate were inoculated into 20 ml of the ethanol medium having the following composition contained in a 500 ml-volume Sakaguchi flask, and aerobically cultured at a stirring rate of 120 rpm at 37°C for 96 hours. L-Lysine accumulated in the medium and residual ethanol were measured by using Biosensor BF-5 (Oji Scientific Instruments). The turbidity of the medium was also measured. Averages of the values obtained in the culture performed in duplicate are shown in Table 4. Improvement in L-lysine accumulation was observed for the strain in which expression of pyruvate synthase gene of *Escherichia* coli, flavodoxin NADP⁺ reductase gene of *Escherichia coli* and ferredoxin gene of *Escherichia coli* was enhanced, compared with the control.

**[Composition of ethanol medium]**

| | |
|---|---|
| Ethanol | 20 ml/L |
| MgSO₄ · 7H₂O | 1.0 g/L |
| (NH₄)₂SO₄ | 12 g/L |
| KH₂PO₄ | 0.5 g/L |
| Yeast extract | 1.0 g/L |
| FeSO₄·7H₂O | 0.01 g/L |
| MnSO₄ · 5H₂O | 0.01 g/L |
| Kanamycin | 40 mg/L |
| Streptomycin | 20 mg/L |
| Calcium carbonate | 30 g/L |
| pH 7.0 (adjusted with KOH) | |

Sterilization conditions: 115°C, 10 minutes. MgSO₄ · 7H₂O and calcium carbonate were separately sterilized and then added to the medium. Ethanol, kanamycin and streptomycin were separately added to the medium.

**Table 4**

| Strain | Lys (g/L) | EtOH (V/V %) | OD620 |
|---|---|---|---|
| WC196Δmez P_{L-tac}adhE*/pCABD2/pMW-Pthr | 2.47 | 0.00 | 14.7 |
| WC196Δmez P_{L-tac}adhE*/pCABD2/pMW-Pthr-fpr-ydbK-fdx | 2.89 | 0.00 | 9.3 |

### Example 8: Construction of a plasmid for expression of pyruvate:NADP⁺ oxidoreductase gene of Euglena gracilis and measurement of activity and confirmation of the expression

The *Euglena gracilis* Z train is a photosynthetic protist, of which optimum growth temperature is 27°C. The pyruvate:NADP⁺ oxidoreductase gene was isolated from this organism, and a plasmid expressing this gene was constructed.

### <8-1> Construction of a plasmid for expressing pyruvate:NADP⁺ oxidoreductase gene of Euglena gracilis

cDNA library of the *Euglena gracilis* Z strain (ATCC12894) was prepared accrding to the method of Rotte (Rotte, C. et al. 2001. Mol. Biol. Evol. 18: 710-720). PCR was performed by using the cDNA as a template and the oligonucleotides shown in SEQ ID NOS: 40 and 41 to amplify the pyruvate:NADP⁺ oxidoreductase gene fragment. The obtained gene fragment was digested with *Kpn*I, and the digested fragment was inserted into the *Kpn*I site of pUC19 (Takara Bio) to construct a plasmid, which was designated pUC-PNO. After it was confirmed that the pyruvate:NADP⁺ oxidoreductase gene contained no PCR error over the full length by using BigDye Terminators v1.1 Cycle Sequencing Kit, the pyruvate:NADP⁺ oxidoreductase gene was excised from pUC-PNO with *Kpn*I, and inserted into the *Kpn*I site of pMW-Pthr to construct a plasmid pMW-Pthr-PNO for expression of the pyruvate:NADP⁺ oxidoreductase gene.

### <8-2> Confirmation of expression of pyruvate:NADP⁺ oxidoreductase in strain expressing pyruvate:NADP⁺ oxidoreductase gene of Euglena gracilis

pMW-Pthr-PNO and the vector pMW-Pthr as a control for comparison were introduced into the WC196ΔcadAΔldcC/pCABD2 strain by electroporation, respectively, transformants were obtained on the basis of the kanamycin resistance, and introduction of the plasmids was confirmed. The strain expressing the pyruvate:NADP⁺ oxidoreductase gene of *Euglena gracilis* was designated WC196ΔcadAΔldc/pCABD2/pMW-Pthr-PNO, and the control strain was designated WC196ΔcadAΔldcC/pCABD2/pMW-Pthr.

The aforementioned strains were each inoculated into the LB medium containing 20 mg/l of streptomycin and 40 mg/l of kanamycin, and cultured overnight at 37°C with shaking. The medium in a volume of 1 ml was inoculated to 20 ml of the LB medium containing 20 mg/l of streptomycin and 40 mg/l of kanamycin, and cultured at 37°C for 5 hours with shaking. The cells were collected by centrifugation and suspended in 1 ml of PBS. The cells in the suspension were disrupted by using a ultrasonicator, the suspension was centrifuged at 15000 rpm for 15 minutes, and the obtained supernatant was used as a crude extract. Protein concentration in the crude extract was measured by using Protein Assay CBB Solution (Nakalai Tesque), and the crude extract containing 10 µg of protein was used for preparation of samples. Each sample was prepared by adding NuPAGE LDS Sample Buffer (Invitrogen) to the crude extract at a concentration of 1.1 times, then adding NuPAGE Sample Reducing Agent (Invitrogen) at a final concentration of 10%, and heating the mixture at 70°C for 10 minutes. The prepared sample was subjected to electrophoresis using NuPAGE Tris-Acetate Gel 3-8% (Invitrogen). MagicMark XP Western Protein Standard (Invitrogen) was used as markers.

The gel after the electrophoresis was transferred to a membrane by using iBlot Gel Transfer Device (Invitrogen). The operations from blocking to detection after the transfer were performed by using WesternBreeze Chemiluminescent Western Blot Immunodetection Kit (Invitrogen). First, the membrane was subjected to a blocking treatment for 30 minutes, washed twice with purified water and incubated in an anti-PNO serum solution diluted 1000 times for 1 hour. The membrane was washed 3 times with a washing solution, and incubated in a second antibody solution for 30 minutes. The membrane was washed 3 times with a washing solution and further twice with purified water, sprinkled with a detection reagent, and subjected to detection using Lumino-image Analyzer LAS-1000 (Fuji Photo Film). The results are shown in Fig. 1. A band presumed to be PNO was detected around 200 kD for the WC196ΔcadAΔldcC/pCABD2/pMW-Pthr-PNO strain, whereas such a band was not detected for the WC196ΔcadAΔldc/pCABD2/pMW-Pthr strain as the control strain.

### Example 9: Effect on L-lysine-producing ability of strain in which expression of pyruvate:NADP⁺ oxidoreductase gene of Euglena gracilis was enhanced in culture using oleic acid as carbon source

### <9-1> Introduction of plasmid for amplification of pyruvate:NADP⁺ oxidoreductase gene of Euglena gracilis into WC196Δmez strain

pMW-Pthr-PNO and the vector pMW-Pthr as a control for comparison were introduced into WC196Δmez/pCABD2 by electroporation, respectively, transformants were obtained on the basis of the kanamycin resistance, and introduction of the plasmids were confirmed. The strain expressing the pyruvate:NADP⁺ oxidoreductase gene of *Euglena gracilis* was designated WC196Δmez/pCABD2/pMW-Pthr-PNO, and the control strain was designated WC196Δmez/pCABD2/pMW-Pthr.

### <9-2> Effect on L-lysine-producing ability of strain in which expression of pyruvate:NADP⁺ oxidoreductase gene of Euglena gracilis was enhanced in culture using oleic acid as carbon source

Both the strains WC196Δmez/pCABD2/pMW-Pthr and WC196Δmez/pCABD2/pMW-Pthr-PNO were inoculated on the LB plate medium, respectively, and cultured overnight at 37°C. The cells corresponding to 1/8 of the plate were inoculated into 20 ml of the oleic acid medium having the following composition contained in a 500 ml-volume Sakaguchi flask, and aerobically cultured at a stirring rate of 120 rpm at 37°C for 72 hours. L-Lysine accumulated in the medium was measured by using Biosensor BF-5 (Oji Scientific Instruments). The live cell count in the medium was also measured. Averages of the values obtained in the culture performed in duplicate are shown in Table 5. Improvement in L-lysine accumulation was observed for the strain in which expression of pyruvate:NADP⁺ oxidoreductase gene of *Euglena gracilis* was enhanced, compared with the control.

| | |
|---|---|
| [Composition of oleic acid medium] | |
| Sodium oleate | 20 g/L |
| Tween80 | 5 g/L |
| MgSO₄ · 7H₂O | 1.0 g/L |
| (NH₄)₂SO₄ | 12 g/L |
| KH₂PO₄ | 0.5 g/L |
| Yeast extract | 1.0 g/L |
| FeSO₄ · 7H₂O | 0.01 g/L |
| MnSO₄ · 5H₂O | 0.01 g/L |
| Kanamycin | 40 mg/L |
| Streptomycin | 20 mg/L |
| Calcium carbonate | 30 g/L |
| pH 7.0 (adjusted with KOH) | |

Sterilization conditions: 115°C, 10 minutes. Sodium oleate and Tween 20 were mixed and sterilized, and then added to the medium. MgSO₄ · 7H₂O and calcium carbonate were separately sterilized and then added to the medium. Kanamycin and streptomycin were separately added to the medium.

**Table 5**

| Strain | Lys (g/l) | Live cell count (10⁸/ml) |
|---|---|---|
| WC19CΔmez/pCABD2/pMW-Pthr | 1.77 | 22.3 |
| WC196Δmez/pCABD2/pMW-Pthr-PNO | 2.41 | 15.9 |

### Example 10: Effect on L-lysine-producing ability of strain in which expression of pyruvate:NADP⁺ oxidoreductase gene of Euglena gracilis was enhanced in culture using ethanol as carbon source

### <10-1> Introduction of plasmid for amplification of pyruvate:NADP⁺ oxidoreductase gene of Euglena gracilis into WC196Δmez adhE* strain

pMW-Pthr-PNO and the vector pMW-Pthr as a control for comparison were introduced into WC196Δmez adhE*/pCABD2 by electroporation, respectively, transformants were obtained on the basis of the kanamycin resistance, and introduction of the plasmids were confirmed. The strain expressing the pyruvate:NADP⁺ oxidoreductase gene of *Euglena gracilis* was designated WC196Δmez adhE*/pCABD2/pMW-Pthr-PNO, and the control strain was designated WC196Δmez adhE*/pCABD2/pMW-Pthr.

### <10-2> Effect on L-lysine-producing ability of strain in which expression of pyruvate:NADP⁺ oxidoreductase gene of Euglena gracilis was enhanced in culture using ethanol as carbon source

Both the strains WC196Δmez P_{L-tac}adhE*/pCA13D2/pMW-Pthr and WC196Δmez P_{L-tac}adhE*/pCABD2/pMW-Pthr-PNO were each inoculated on the LB plate medium, and precultured overnight at 37°C. The cells corresponding to 1/8 of the plate were inoculated into 20 ml of the ethanol medium having the following composition contained in a 500 ml-volume Sakaguchi flask, and aerobically cultured at a stirring rate of 120 rpm at 37°C for 96 hours. After the culture for 96 hours, 1 ml of the medium was sampled, and L-Lysine accumulated in the medium was measured by using Biosensor BF-5 (Oji Scientific Instruments). The turbidity of the medium was also measured. Averages of the values obtained in the culture performed in duplicate are shown in Table 6. Improvement in L-lysine accumulation was observed in the strain in which expression of pyruvate:NADP⁺ oxidoreductase gene of *Euglena gracilis* was enhanced, compared with the control.

**[Composition of ethanol medium]**

| | |
|---|---|
| Ethanol | 20 ml/L |
| MgSO₄ · 7H₂O | 1.0 g/L |
| (NH₄)₂SO₄ | 12 g/L |
| KH₂PO₄ | 0.5 g/L |
| Yeast extract | 1.0 g/L |
| FeSO₄ · 7H₂O | 0.01 g/L |
| MnSO₄ · 5H₂O | 0.01 g/L |
| Kanamycin | 40 mg/L |
| Streptomycin | 20 mg/L |
| Calcium carbonate | 30 g/L |
| pH 7.0 (adjusted with KOH) | |

Sterilization conditions: 115°C, 10 minutes. MgSO₄ · 7H₂O and calcium carbonate were separately sterilized and then added to the medium. Ethanol, kanamycin and streptomycin were separately added to the medium.

**Table 6**

| Strain | Lys (g/l) | EtOH (V/V %) | OD620 |
|---|---|---|---|
| WC196Δmez adhE*/pCABD2/pMW-Pthr | 2.47 | 0.00 | 14.7 |
| WC196Δmez adhE*/pCABD2/pMW-Pthr-PNO | 2.63 | 0.00 | 12.9 |

### Example 11: Construction of Escherichia coli L-lysine producing strain WC196ΔcaaAΔldcCAmezΔfadR/pCABD2

### <11-1> Construction of Escherichia coli L-lysine producing strain WC196ΔcadAΔldcCΔfadR

A transcriptional factor FadR which regulates fatty acid metabolism in *Escherichia coli* is encoded by the *fadR* gene (SEQ ID NO: 82) (DiRusso, C. C. et al. 1992. J. Biol. Chem. 267: 8685-8691). A deletion mutant strain of the *fadR* gene was constructed from the WC196ΔcadAΔldcC/pCABD2 strain described in WO2006/078039 as an L-lysine producing strain of *Escherichia coli.*

Deletion of the *fadR* gene encoding the transcriptional factor which regulates fatty acid metabolism is performed according to the method of Datsenko and Wanner (Datsenko, K. A. and Wanner, B. L. 2000. Proc. Natl. Acad. Sci. USA. 97: 6640-6645) using the excision system of λ phage (Cho, E. H. et al. 2002. J. Bacteriol. 184: 5200-5203). PCR was performed using the plasmid pMM118-(λattL-Km^{r}-aλttR) (WO2006/093322) as a template, and the synthetic oligonucleotides shown in SEQ ID NOS: 68 and 69 which contain sequences corresponding to the both ends of attL and attR at the 3' terminus and sequences corresponding to a part of the *fadR* gene, the target gene, at the 5' terminus as primers.

The amplified PCR product was purified by agarose gel electrophoresis and introduced into the *Escherichia* coli WC196ΔcadAΔldcC strain harboring the pKD46 plasmid having a temperature sensitive replication ability by electroporation. Then, in order to remove the pKD46 plasmid, subculture was performed twice on LB agar medium containing kanamycin at 42°C, and the ampicillin resistance of the resulting colonies was tested to obtain ampicillin sensitive strains from which the pKD46 plasmid was eliminated. Deletion of the *fadR* gene which can be identified by the kanamycin resistant gene was confirmed by PCR among the ampicillin sensitive strains. The obtained *fadR* deletion strain was designated WC196ΔcadAΔldcCΔfadR::att-kan strain.

Then, in order to remove the att-kan gene which had been introduced within the *fadR* gene, the above helper plasmid pMW-intxis-ts (US2006/0141586) was used. pMW-intxis-ts is a plasmid which harbors a gene encoding an integrase (Int) and a gene encoding an excisionase (Xis) of λ phage and has a temperature sensitive replication ability.

Competent cells were prepared from the WC196ΔcadAΔldcCAfadR::att-kan strain obtained above according to the conventional method, transformed with the helper plasmid pMW-intxis-ts, and then plated onto LB agar medium containing 100 mg/L of ampicillin at 30°C to select an ampicillin resistant strain. Then, in order to remove the pMW-intxis-ts plasmid, subculture was performed twice on LB agar medium at 42°C, and the ampicillin resistance and kanamycin resistance of the resulting colonies were tested to obtain a kanamycin and ampicillin sensitive strain, a *fadR* disrupted strain from which att-kan and pMW-intxis-ts were eliminated. The strain was designated WC196ΔcadAΔldcCΔfadR strain.

### <11-2> Construction of Escherichia coli L-lysine producing strain WC196ΔcadAΔldcCΔfadRΔsfcA

The *sfcA* gene was deleted from WC196ΔcadAΔldcCΔfadR strain using the primers of SEQ ID NOS: 70 and 71 as primers for disruption of sfcA according to the method described above <11-1>. Thus, WC196ΔcadAΔldcCΔfadRΔsfcA strain was obtained.

### <11-3> Construction of Escherichia coli L-lysine producing strain NC196ΔcadAΔldcCΔmezΔfadR/pCABD2

The *b2463* gene was deleted from the WC196ΔcadAΔldcCΔfadRΔsfcA strain using the primers of SEQ ID NOS: 72 and 73 as primers for disruption of *b2463* according to the method described above <11-1>. Thus, WC196ΔcadΔAldcCΔfadRΔsfcAΔb2463 strain was obtained. The strain was designated WC196ΔcadAΔldcCΔmezΔfadR strain. The plasmid pCABD2 was introduced into the strain by electroporation, transformants were obtained on the basis of the streptomycin resistance, and introduction of the plasmid was confirmed. This strain was designated WC196ΔcadAΔldcCΔmezΔfadR/pCABD2 strain.

### Example 12: Construction of a plasmid for expression of pyruvate synthase gene of Escherichia coli and flavodoxin-NADP⁺ reductase gene of Escherichia coli

### <12-1> Construction of a plasmid for amplifying flavodoxin-NADP⁺ reductase gene of Escherichia coli

PCR was performed using the chromosomal DNA of the *E. coli* MG1655 strain as a template and the oligonucleotides shown in SEQ ID NOS: 42 and 43 to amplify the flavodoxin NADP⁺ reductase gene fragment of *E. coli.* The obtained gene fragment was digested with *Sma*I, and inserted into the *Sma*I site of pMW-P_{L-tac} to construct a plasmid for amplifying the flavodoxin NADP⁺ reductase gene of *Escherichia coli,* which was designated pMW-P_{L-tac}-fpr. pMW-P_{L-tac} is a plasmid constructed by amplifying a fragment containing P_{L-tac} by PCR method using the genome of the *E*. *coli* MG1655P_{L-tac}xylE strain described in WO2006/043730 as a template and the primers shown in SEQ ID NOS: 84 and 85, digesting the obtained gene fragment with *HindIII* and *SalI*, and then inserting into pMM119 (Nippongene) digested with *HindIII* and *SalI.* Using the plasmid, a gene can be cloned into the downstream of this P_{L-tac} and expressed.

### <12-2> Insertion of pyruvate synthase gene of Escherichia coli into plasmid for expression of flavodoxin-NADP⁺ reductase gene of Escherichia coli

The pyruvate synthase gene *ydbK* was excised from pSTV-ydbK described in Example 5 with *KpnI,* and inserted into the *KpnI* site of pMW-P_{L-tac}-fpr to construct the plasmid pMW-P_{L-tac}-fpr-ydbK for expression of the pyruvate synthase gene of *Escherichia coli* and the flavodoxin-NADP⁺ reductase gene of *Escherichia coli*

### Example 13: Effect on L-lysine-producing ability of a strain in which expression of pyruvate synthase gene of Eschcrichia coli and flavodoxin NADP⁺ reductase gene of Escherichia coli was enhanced in culture using oleic acid as carbon source

### <13-1> Introduction of plasmid for expression of pyruvate synthase gene of Escherichia coli and flavodoxin NADP⁺ reductase gene of Escherichia coli into WC196ΔcadAΔldcCΔmezΔfadR/pCABD2 strain

pMN-P_{L-tac}-tpr-ydbK and the vector pMW-P_{L-tac} as a control for comparison were introduced into the WC196ΔcadΔAldcCΔmezΔfadR/pCABD2 strain by electroporation, respectively, transformants were obtained on the basis of the ampicillin resistance, and introduction of the plasmid was confirmed. The strain in which expression of the pyruvate synthase gene of *Escherichia coli* and the flavodoxin-NADP⁺ reductase gene of *Escherichia coli* was enhanced was designated WC196ΔcadAΔldcCΔmezΔfadR/pCABD2/pMW-P_{L-tac}-fpr-ydbK, and the control strain was designated WC196ΔcadAΔldcCΔmezΔfadR/pCABD2/pMW-P_{L-tac}.

### <13-2> Effect on L-lysine-producing ability of a strain in which expression of pyruvate synthase gene of Escherichia coli and flavodoxin NADP⁺ reductase gene of Escherichia coli was enhanced in culture using oleic acid as carbon source

Both the strains WC196ΔcadAΔldcCΔmezΔfadR/pCABD2/pMW-P_{L-tac} and WC196ΔcadAΔldcCΔmezΔfadR/pCABD2/pMW-P_{L-tac}-fpr-ydbK were inoculated on the LB plate medium, respectively, and precultured overnight at 37°C. The cells corresponding to 1/8 of the plate were inoculated into 20 ml of the oleic acid medium having the following composition contained in a 500 ml-volume Sakaguchi flask, and aerobically cultured at a stirring rate of 120 rpm at 37°C for 72 hours. L-Lysine accumulated in the medium was measured by using Biosensor BF-5 (Oji Scientific Instruments). The turbidity of the medium was also measured. Averages of the values obtained in the culture performed in duplicate are shown in Table 7. Improvement in L-lysine accumulation was observed for the strain in which expression of the pyruvate synthase gene of *Escherichia coli* and the flavodoxin-NADP⁺ reductase gene of *Escherichia coli* was enhanced, compared with the control.

| | |
|---|---|
| [Composition of oleic acid medium] | |
| Sodium oleate | 10g /L |
| Tween80 | 5 g/L |
| MgSO₄ · 7H₂O | 1 g/L |
| (NH₄)₂SO₄ | 12 g/L |
| KH₂PO₄ | 0.5 g/L |
| Yeast extract | 1 g/L |
| FeSO₄ · 7H₂O | 0.01 g/L |
| MnSO₄ · 5H₂O | 0.01 g/L |
| Ampicillin | 100 mg/L |
| Streptomycin | 20 mg/L |
| Calcium carbonate | 30 g/L |
| pH7.0 (adjusted with KOH) | |

Sterilization conditions: 115°C, 10 minutes. Sodium oleate and Tween80 were mixed, sterilized and then added to the medium. MgSO₄ · 7H₂O and calcium carbonate were separately sterilized and then added to the medium. Ampicillin and streptomycin were also separately added to the medium.

**Table 7**

| Strain | Lys (g/L) | Live cell count (10⁸/mL) |
|---|---|---|
| WC196ΔcadAΔldcCAmezΔfadR/pCABD2/pMW-P_{L-tac} | 3.17 | 6.00 |
| WC196ΔcadAΔldcCΔmezΔfadR/pCABD2/ pMW-P_{L-tac}-fpr-ydbK | 4.11 | 0.23 |

### Example 14::Effect on L-lysine-producing ability of a strain in which expression of pyruvate synthase gene of Escherichia coli and flavodoxin NADP⁺ reductase gene of Escherichia coli was enhanced in culture using ethanol as carbon source

### <14-1> Construction of alcohol dehydrogenase (AdhE) mutated strain derived from Escherichia coli WC196ΔcadΔldcC strain

In order to impart ethanol assimilability to an L-lysine-producing bacterium, the L-lysine-producing bacterium WC196ΔcadAΔldcC strain was subjected to P1 transduction using MG1655Δtdh rhtA* P_{L-tac}adhE* as a donor to obtain the WC196ΔcadAΔldcC P_{L-tac}adhE* strain.

### <14-2> Construction of Escherichia coli L-lysine producing strain WC196ΔcadAΔldcCΔsfcA P_{L-tac}adhE*

Deletion of the *sfcA* gene from the NC196ΔcadAΔldcC adhE* strain was performed using the primers of SEQ ID NOS: 70 and 71 as primers for disruption of sfcA according to the method described above <11-1>. Thus, the WC196ΔcadAΔldcCΔsfcA P_{L-tac}adhE* strain was obtained.

### <14-3> Construction of Escherichia coli L-lysine producing strain WC196ΔcadAΔldcCΔmez P_{L-tac}adhE*/pCABD2

Deletion of the b2463 gene from the WC196ΔcadAΔldcCAsfcA adhE* strain was performed using primers of SEQ ID NOS: 72 and 73 as primers for disruption of *b2Q63* according to the method described above <11-1>. Thus, the WC196ΔcadAΔldcCΔsfcAΔb2463 P_{L-tac}adhE* strain was obtained. The strain was designated WC196ΔcadAΔldcCΔmez P_{L-tac}adhE* strain. The plasmid pCABD2 was introduced into the strain by electroporation, transformants were obtained on the basis of the streptomycin resistance, and introduction of the plasmid was confirmed. This strain was designated WC196ΔcadAΔldcCΔmez P_{L-tac}adhE*/pCABD2 strain.

### <14-4> Introduction of plasmid for expression of pyruvate synthase gene of Escherichia coli and flavodoxin NADP⁺ reductase gene of Escherichia coli into WC196ΔcadAΔldcCΔmez P_{L-tac}adhE*/pCABD2 strain

pMW-P_{L-tac}-fpr-ydbK and the vector pMW-P_{L-tac} as a control for comparison were introduced into the WC196ΔcadAΔldcCΔmez P_{L-tac}adhE*/pCABD2 strain by electroporation, respectively, transformants were obtained on the basis of the ampicillin resistance, and introduction of the plasmid was confirmed. The strain in which expression of the pyruvate synthase gene of *Escherichia coli* and the flavodoxin-NADP⁺ reductase gene of *Escherichia coli* was enhanced was designated WC196ΔcadAΔldcCΔmez P_{L-tac}adhE*/pCABD2/pMM-P_{L-tac}-fpr-ydbK, and the control strain was designated WC196ΔcadAΔldcCΔmez P_{L-tac}adhE*/pCABD2/pMW-P_{L-tac}.

### <14-5> Effect on L-lysine-producing ability of a strain in which expression of pyruvate synthase gene of Escherichia coli and flavodoxin NADP⁺ reductase gene of Escherichia coli was enhanced in culture using ethanol as carbon source

Both the strains WC196ΔcadAΔldcCΔmez P_{L-tac}adhE*/pCABD2/pMW-P_{L-tac} and WC196ΔcadAΔldcCΔmez P_{L-tac}adhE*/pCABD2/pMW-P_{L-tac}-fpr-ydbK were inoculated on the LB plate medium, respectively, and precultured overnight at 37°C. The cells corresponding to 1/8 of the plate were inoculated into 20 ml of the ethanol medium having the following composition contained in a 500 ml-volume Sakaguchi flask, and aerobically cultured at a stirring rate of 120 rpm at 37°C for 120 hours. One ml of the culture broth was sampled at 120 hr of cultivation, and L-Lysine accumulated in the medium was measured by using Biosensor BF-5 (Oji Scientific Instruments). The turbidity of the medium was also measured. Averages of the values obtained in the culture performed in duplicate are shown in Table 8. Improvement in L-lysine accumulation was observed for the strain in which expression of the pyruvate synthase gene of *Escherichia coli* and the flavodoxin-NADP⁺ reductase gene of *Escherichia coli* was enhanced, compared with the control.

| | |
|---|---|
| [Composition of ethanol medium] | |
| Ethanol | 20mL/L |
| MgSO₄·7H₂O | 1g/L |
| (NH₄)₂SO₄ | 12g/L |
| KH₂PO₄ | 0.5g/L |
| Yeast extract | 1g/L |
| FeSO₄·7H₂O | 0.01g/L |
| MnSO₄·5H₂O | 0.01g/L |
| Ampicillin | 100mg/L |
| Streptomycin | 20mg/L |
| Calcium carbonate | 30g/L |
| pH 7.0 (adjusted with KOH) | |

Sterilization conditions: 115°C, 10 minutes. MgSO₄·7H₂O and calcium carbonate were separately sterilized and then added to the medium. Ethanol, ampicillin and streptomycin were separately added to the medium.

**Table 8**

| Strain | Lys (g/L) | EtOH (%V/V) | OD600 |
|---|---|---|---|
| WC196ΔcadAΔldcCΔmez P_{L-tac}adhE*/pCABD2/ pMW - P_{L-tac} | 2.15 | 0.00 | 12.0 |
| WC196ΔcadAΔldcCΔmez P_{L-tac}adhE*/pCABD2/ pMW-P_{L-tac}-fpr-ydbK | 2.52 | 0.00 | 10.0 |

### Example 15: Construction of a plasmid for expression of pyruvate synthase gene of Escherichia coli and Effect on L-lysine-producing ability of a strain in which expression of the gene was enhanced in culture using oleic acid as carbon source

### <15-1> Construction of a plasmid for expression of pyruvate synthase gene of Escherichia coli

PCR was performed using the chromosomal DNA of the *E*. *coli* MG1655 strain as a template and the oligonucleotides shown in SEQ ID NOS: 74 and 75 to amplify the pyruvate synthase gene fragment. The obtained gene fragment was digested with *KpnI,* and inserted into the *KpnI* site of pMW-P_{L-tac} to construct the plasmid pMW-P_{L-tac}-ydbK for amplifying the pyruvate synthase gene of *Escherichia coli.*

### <15-2> Introduction of plasmid for expression of pyruvate synthase gene of Escherichia coli into WC196ΔcadAΔldcCΔmezΔfadR/pCABD2 strain

pMW-P_{L-tac}-ydbK and the vector pMW-P_{L-tac} as a control for comparison were introduced into the WC196ΔcadAΔldcCΔmezΔfadR/pCABD2 strain by electroporation, respectively, transformants were obtained on the basis of the ampicillin resistance, and introduction of the plasmid was confirmed. The strain in which expression of the pyruvate synthase gene of *Escherichia coli* was enhanced was designated WC196ΔcadAΔldcCΔmezΔfadR/pCABD2/pMW-P_{L-tac}-ydbK, and the control strain was designated WC196ΔcadAΔldcCΔmezΔfadR/pCABD2/pMW-P_{L-tac}.

### <15-3> Effect on L-lysine-producing ability of a strain in which expression of pyruvate synthase gene of Escherichia coli was enhanced in culture using oleic acid as carbon source

Both the strains WC196ΔcadAΔldcCΔmezΔfadR/pCABD2/pMW-P_{L-tac} and WC196ΔcadAΔldcCΔmezΔfadR/pCABD2/pMW-P_{L-tac}-ydbK were inoculated on the LB plate medium, respectively, and precultured overnight at 37°C. The cells corresponding to 1/8 of the plate were inoculated into 20 ml of the oleic acid medium having the following composition contained in a 500 ml-volume Sakaguchi flask, and aerobically cultured at a stirring rate of 120 rpm at 37°C for 72 hours. L-Lysine accumulated in the medium was measured by using Biosensor BF-5 (Oji Scientific Instruments). Averages of the values obtained in the culture performed in triplicate are shown in Table 9. Improvement in L-lysine accumulation was observed for the strain in which expression of the pyruvate synthase gene of *Escherichia coli* was enhanced, compared with the control.

**[Composition of oleic acid medium]**

| | |
|---|---|
| Sodium oleate | 10g/L |
| Tween80 | 5 g/L |
| MgSO₄·7H₂O | 1 g /L |
| (NH₄)₂SO₄ | 12 g/L |
| KH₂PO₄ | 0.5 g/L |
| Yeast extract | 1 g/L |
| FeSO₄·7H₂O | 0.01 g/L |
| MnSO₄·5H₂O | 0.01 g/L |
| Ampicillin | 100 mg/L |
| Streptomycin | 20 mg/L |
| Calcium carbonate | 30 g/L |
| pH7.0 (adjusted with KOH) | |

Sterilization conditions: 115°C, 10 minutes.
Sodium oleate and Tween80 were mixed, sterilized and then added to the medium. MgSO₄·7H₂O and calcium carbonate were separately sterilized and then added to the medium. Ampicillin and streptomycin were also separately added to the medium.

**Table 9**

| Strain | Lys (g/L) |
|---|---|
| WC196ΔcadAΔldcCΔmezΔfadR/pCABD2/PMW-P_{L-tac} | 2.95 |
| WC196ΔcadAΔldcCΔmezΔfadR/pCABD2/pMW-P_{L-tac}-ydbK | 4.05 |

### Example 16: Construction of a plasmid for expression of pyruvate synthase gene of Methanococcus maripaludis and measurement of activity

### <16-1> Construction of a plasmid for expression of pyruvate synthase gene of Methanococcus maripaludis

PCR was performed using the chromosomal DNA of the *Methanococcus maripaludis* ATCC43000 strain as a template and the oligonucleotides shown in SEQ ID NOS: 76 and 77 to amplify the pyruvate synthase gene fragment. The obtained gene fragment was digested with *SacI,* and the digested fragment was inserted into the *SacI* site of pMW-P_{L-tac} to construct the plasmid pMW-P_{L-tac}-MmPS for expression of the pyruvate synthase gene of *Methanococcus maripaludis.*

### <16-2> Measurement of pyruvate synthase activity of a strain expressing pyruvate synthase gene of Methanococcus maripaludis

pMW-P_{L-tac}-MmPS and the vector pMW-P_{L-tac} as a control for comparison were introduced into the *Escherichia coli* JM109 strain (TAKARA BIO) by electroporation, respectively, transformants were obtained on the basis of the ampicillin resistance, and introduction of the plasmids was confirmed. The strain expressing the pyruvate synthase gene of *Methanococcus maripaludis* was designated JM109/pMW-P_{L-tac}-MmPS, and the control strain was designated JM109/pMW-P_{L-tac}.

The aforementioned strains were each inoculated into the LB medium containing 100 mg/l of ampicillin, and cultured overnight at 37°C with shaking. The cells were collected by centrifugation, and the activity was measured in the same manner as that of the activity measurement method for the strain expressing the pyruvate synthase gene of *Chlorobium tepidum* described in Example 2. The results are shown in Table 10. Whereas the activity of pyruvate synthase was not confirmed for the control strain JM109/pMW-P_{L-tac}, 7.1 U/mg of the activity was confirmed for the strain expressing pyruvate synthase gene of *Methanococcus maripaludis,* JM109/pMW-P_{L-tac}-MmPS. The unit of the specific activity is the same as that used in Table 1.

**Table 10**

| Plasmid | Specific activity |
|---|---|
| pMW-P_{L-tac} | 0.0 |
| pMW-P_{L-tac}-MmPS | 7.1 |

### Example 17: Effect on L-lysine-producing ability of a strain in which expression of pyruvate synthase gene of Methanococcus maripaludis was enhanced in culture using oleic acid as carbon source

### <17-1> Introduction of plasmid for expression of pyruvate synthase gene of Methanococcus maripaludis into WC196ΔcadAΔldcCΔmezΔfadR/pCABD2 strain

pMN-P_{L-tac}-MmPS and the vector pMW-P_{L-tac} as a control for comparison were introduced into WC196ΔcadAΔldcCΔmezΔfadR/pCABD2 strain by electroporation, respectively, transformants were obtained on the basis of the ampicillin resistance, and introduction of the plasmid was confirmed. The strain in which expression of the pyruvate synthase gene of *Methanococcus maripaludis* was enhanced was designated WC196ΔcadAΔldcCΔmezΔfadR/pCABD2/pMW-P_{L-tac}-MmPS, and the control strain was designated WC196ΔcadAΔldcCΔmezΔfadR/pCABD2/pMW-P_{L-tac}.

### <17-2> Effect on L-lysine-producing ability of a strain in which expression of pyruvate synthase gene of Methanococcus maripaludis was enhanced in culture using oleic acid as carbon source

Both the strain WCl96ΔcadAΔldcCΔmezΔfadR/pCABD2/pMW-P_{L-tac} and WC196ΔcadAΔldcCΔmezΔfadR/pCABD2/pMW-P_{L-tac}-MmPS were inoculated on the LB plate medium, respectively, and precultured overnight at 37°C. The cells corresponding to 1/8 of the plate were inoculated into 20 ml of the oleic acid medium having the following composition contained in a 500 ml-volume Sakaguchi flask, and aerobically cultured at a stirring rate of 120 rpm at 37°C for 72 hours. L-Lysine accumulated in the medium was measured by using Biosensor BF-5 (Oji Scientific Instruments). Averages of the values obtained in the culture performed in triplicate are shown in Table 11. Improvement in L-lysine accumulation was observed for the strain in which expression of the pyruvate synthase gene of *Methanococcus maripaludis* was enhanced, compared with the control.

**[Composition of oleic acid medium]**

| | |
|---|---|
| Sodium oleate | 10g /L |
| Tween80 | 5 g/L |
| MgSO₄·7H₂O | 1 g/L |
| (NH₄)₂SO₄ | 12 g/L |
| KH₂PO₄ | 0.5 g/L |
| Yeast extract | 1 g/L |
| FeSO₄·7H₂O | 0.01 g/L |
| MnS0₄·5H₂O | 0.01 g/L |
| Ampicillin | 100 mg/L |
| Streptomycin | 20 mg/L |
| Calcium carbonate | 30 g/L |
| (pH7.0 (adjusted with KOH) | |

Sterilization conditions:115°C, 10 minutes.
Sodium oleate and Tween80 were mixed, sterilized and then added to the medium. MgSO₄·7H₂O and calcium carbonate were separately sterilized and then added to the medium. Ampicillin and streptomycin were also separately added to the medium.

**Table 11**

| Strain | Lys (g/L) |
|---|---|
| WC196ΔcadAΔldcCΔmezΔfadR/pCABD2/PMW-P_{L-tac} | 2.90 |
| WC196ΔcadAΔldcCΔmezΔfadR/pCABD2/pMW-P_{L-tac}-MmPS | 4.22 |

### Example 18: Effect on L-lysine-producing ability of a strain in which expression of pyruvate synthase gene of Methanococcus maripaludis was enhanced in culture using ethanol as carbon source

### <18-1> Introduction of plasmid for expression of pyruvate synthase gene of Methanococcus maripaludis into WC196ΔcadAΔldcCΔmez adhE*/pCABD2 strain

pMW-P_{L-tac}-MmPS and the vector pMN-P_{L-tac} as a control for comparison were introduced into WC196ΔcadAΔldcCΔmez adhE*/pCABD2 strain by electroporation, respectively, transformants were obtained on the basis of the ampicillin resistance, and introduction of the plasmid was confirmed. The strain in which expression of the pyruvate synthase gene of *Methanococcus maripaludis* was enhanced was designated WC196ΔcadAΔldcCΔmez P_{L-tac}adhE*/pCABD2/pMW-P_{L-tac}-MmPS, and the control strain was designated WC196ΔcadAΔldcCΔmez P_{L-tac}adhE*/pCABD2/pMW-P_{L-tac}.

### <18-2> Effect on L-lysine-producing ability of a strain in which expression of pyruvate synthase gene of Methanococcus maripaludis was enhanced in culture using ethanol as carbon source

Both the strains WC196ΔcadAΔldcCΔmez P_{L-tac}-adhE*/pCABD2/pMW-P_{L-tac} and WC196ΔcadAΔldcCΔmez P_{L-tac}adhE*/pCABD2/pMW-P_{L-tac}-MmPS were inoculated on the LB plate medium, respectively, and precultured overnight at 37°C. The cells corresponding to 1/8 of the plate were inoculated into 20 ml of the ethanol medium having the following composition contained in a 500 ml-volume Sakaguchi flask, and aerobically cultured at a stirring rate of 120 rpm at 37°C for 96 hours. One ml of the culture broth was sampled at 96 hr of cultivation, and L-Lysine accumulated in the medium was measured by using Biosensor BF-5 (Oji Scientific Instruments). The turbidity of the medium was also measured. Averages of the values obtained in the culture performed in duplicate are shown in Table 12. Improvement in L-lysine accumulation was observed for the strain in which expression of the pyruvate synthase gene of *Methanococcus maripaludis* was enhanced, compared with the control.

**[Composition of ethanol medium]**

| | |
|---|---|
| Ethanol | 20 mL/L |
| MgSO₄·7H₂O | 1 g/L |
| (NH₄)₂SO₄ | 12 g/L |
| KH₂PO₄ | 0.5 g/L |
| Yeast extract | 1 g/L |
| FeSO₄·7H₂O | 0.01 g/L |
| MnSO₄·5H₂O | 0.01 g/L |
| Ampicillin | 100 mg/L |
| Streptomycin | 20 mg/L |
| Calcium carbonate | 30 g/L |
| pH 7.0 (adjusted with KOH) | |

Sterilization conditions: 115°C, 10 minutes.
MgSO₄·7H₂O and calcium carbonate were separately sterilized and then added to the medium. Ethanol, amplicillin and streptomycin were separately added to the medium.

**Table 12**

| Strain | Lys (g/L) | EtOH (%V/V) | OD620 |
|---|---|---|---|
| WC196ΔcadAΔldcCΔmez P_{L-tac}adhE*/ pCABD2/pMW-P_{L-tac} | 2.19 | 0.10 | 13.9 |
| WC196ΔcadAΔldcCΔmez P_{L-tac}adhE*/ pCABD2/pMW-P_{L-tac}-MmPS | 2.67 | 0.14 | 12.0 |

### Example 19: Construction of a plasmid for hyperexpression of pyruvate synthase gene of Escherichia coli

### <19-1> Construction of Escherichia coli MG1655 cat-PL-ydbK strain

In order to prepare a plasmid for hyperexpression of pyruvate synthase gene of *Escherichia coli,* the promoter of the *ydbK* gene was replaced with the PL promoter. The promoter replacement was performed according to the method of Datsenko and Wanner (Datsenko, K. A. and Wanner, B. L. 2000. Proc. Natl. Acad. Sci. USA. 97: 6640-6645) using the excision system of λ phage (Cho, E. H. et al. 2002. J. Bacteriol. 184: 5200-5203). ydbK-attR (SEQ ID NO: 78) containing the sequence of attR, an upstream sequence of the *ydbK* gene and a sequence of a region adjacent to the Cm^{r} gene on the genome of the BW25113 cat-PL-yddG strain and PL-ydbK (SEQ ID NO: 79) which contains sequences of an upstream region of the *ydbK* gene and a downstream region of the PL promoter on the genome of the BW25113 cat-PL-yddG strain were prepared as primers. The BW25113 cat-PL-yddG strain is described in EP1449918A1 or RU2222596 in detail. PCR was performed using the genome of the BW25113 cat-PL-yddG strain as a template and the synthetic oligonucleotides shown in SEQ ID NOS: 78 and 79. The resulting PCR product was introduced into the *Escherichia coli* MG1655/pKD46 strain by electroporation, and Cm resistant transformants were selected. In order to remove the pKD46 plasmid, the transformants were plated onto the LB plate medium containing Cm, and subculture was performed twice at 42°C to select an ampicillin sensitive strain. The strain obtained above was designated MG1655 cat-PL-ydbK strain.

### <19-2> Construction of the plasmid pPL-ydbK3

The genome of the MG1655 cat-PL-ydbK strain was prepared using GenElute Bacterial Genomic DNA Kit (Sigma-Aldrich Corp.). The genome DNA and the plasmid pMW119 were digested with *SalI,* and then mixed and ligated. The MG1655 strain was transformed with the reaction solution, and a Cm resistant strain was selected. Construction of cat-PL-ydbK was confirmed by PCR using the plasmid DNA as a template and ydbKC1 (SEQ ID NO: 80) and ydbK CH2 (SEQ ID NO: 81) as primers. The plasmid pMW119 cat-PL-ydbK big-1 containing a insert of 11.2 kb in length was obtained. The plasmid pMW119 cat-PL-ydbK big-1 was digested with a restriction enzyme *SmlI* (New England Biolabs, Inc), and then normally treated with the Klenow fragment (Sambrook, J. et al. 1989. Molecular CloningA Laboratory Manual/Second Edition, Cold Spring Harbor Laboratory Press, New York). After digesting pMW119 with *SmaI,* both digested plasmids were mixed and ligated. The MG1655 strain was transformed with the reaction solution, and a Cm resistant strain was selected. A plasmid was prepared from the Cm resistant strain, and the length was confirmed by digestion with *Bam*HI. Thus, the plasmid pPLydbK3 containing a 6.7 kb cat-PL-ydbK insert was obtained.

### Example 20: Effect on L-threonine-producing ability of a strain in which expression of pyruvate synthase gene of Escherichia coli was enhanced in culture using ethanol as carbon source

In order to evaluate the effect of amplifying the pyruvate synthase gene of *Escherichia coli* on L-threonine-producing ability, MG1655Atdh rhtA* P_{L-tac} adhE* constructed in Example 1 was transformed with the plasmid pVIC40. The plasmid pVIC40 is a plasmid for L-threonine production described in US5, 705, 371. MG1655Δtdh rhtA* P_{L-tac}adhE* /pVIC40 was further transformed with pPLydbK3 to obtain the MG1655Δtdh rhtA* P_{L-tac}adhE*/pVIC40/pPLydbK3 strain. After culturing these strains in the nutrient broth at 37°C for 18 hours, 0.1 ml of the culture broth was inoculated into 2 ml of the ethanol medium for L-threonine production contained in a 20×200mm test tube, and rotation culture was performed at 32°C for 72 hours. L-threonine concentration in the medium at 48 and 72 hr was measured by paper chromatography using a mobile phase consisting of butanol : acetic acid : water = 4 : 1 : 1 (v/v). Two percent ninhydrin in acetone solution was used as a visualization reagent. A spot containing L-threonine was excised, L-threonine was eluted within a 0.5 % solution of CdCl₂, and then quantified by measuring the absorbance at 540 nm. Results of the culture using 10 test tubes are shown in Table 13. MC1655Δtdh rhtA* P_{L-tac}adhE*/pVIC40/pPLydbK3 in which expression of the pyruvate synthase gene of *Escherichia coli* was enhanced showed higher level of L-threonine accumulation than the non-enhanced strain, MG1655Δtdh rhtA* P_{L-tac}adhE*/pVIC40.

**[Composition of ethanol medium for L-threonine production]**

| | |
|---|---|
| Ethanol | 20g/L |
| (NH4)₂SO₄ | 22g/L |
| K₂HPO₄ | 2g/L |
| MgSO₄·7H₂O | 0.8g/L |
| MnSO₄·5H₂O | 0.02g/L |
| FeSO₄·7H₂O | 0.02g/L |
| Thiamine hydrochloride | 0.002g/L |
| Yeast extract | 1.0g/L |
| CaCO₃ | 30g/L |
| pH 7.0 | |

K₂HPO₄ and CaCO₃ were separately sterilized and then added to the medium. Ethanol was separately added to the medium.

**Table 13**

| Strain | 48 hr | | 72 hr | |
|---|---|---|---|---|
| | OD₅₅₀ | Thr (g/l) | OD₅₅₀ | Thr (g/l) |
| MG1655 Δtdh rhtA* cat-P_{Ltac}-adhE*/ pVIC40 | 13.8±0.5 | 2.4±0.3 | 12.5 | 2.5±0.2 |
| MG1655 Δtdh rhtA* cat-P_{Ltac}-adhE*/ pVIC40 /pPLydbK3 | 13.9±0.7 | 3.8±0.5 | 12.7 | 3.6±0.5 |

### Example 21: Effect on L-valine-producing ability of a strain in which expression of pyruvate synthase gene of Escherichia coli was enhanced in culture using ethanol as carbon source

In order to evaluate the effect of amplifying the pyruvate synthase gene of *Escherichia coli* on L-valine-producing ability,
the L-valine-producing bacterium H-81 strain was subjected to P1 transduction using the MG1655::P_{L-tac}adhE* strain as a donor to obtain the H-81 P_{L-tac}adhE* strain. The H-81 P_{L-tac}adhE* strain was further transformed with pPLydbK3 to obtain the H-81 P_{L-tac}adhE*/pPLydbK3 strain. After culturing these strains in the nutrient broth at 37°C for 18 hours, 0.1 ml of the culture broth was inoculated into 2 ml of the ethanol medium for L-valine, production contained in a 20×200mm test tube, and gyratory culture was performed at 32°C for 72 hours. L-valine concentration in the medium at 48hr and 72 hr was quantified by TLC. Using 10×15 cm TLC plates containing no fluorescent reagent coated with 0.11 mm Sorbfil silica gel (Stock Company Sorbpolymer, Krasnodar, Russia), samples were developed with a mobile phase consisting of propan-2-ol : ethylacetate : 25 % aqueous ammonia : water = 40 : 40 : 7 : 16 (v/v), and then quantified with 2% ninhydrin in acetone solution as a visualization reagent. Results of the culture using 10 test tubes are shown in Table 14. The H-81 P_{L-tac}adhE*/pPLydbK3 strain in which expression of the pyruvate synthase gene of *Escherichia coli* was enhanced showed higher level of L-valine accumulation than the not enhanced strain, H-81 P_{L-tac}adhE*.

**[Composition of ethanol medium for L-valine production]**

| | |
|---|---|
| Ethanol | 20 g/L |
| (NH₄)₂SO₄ | 15 g/L |
| KH₂PO₄ | 1.5 g/L |
| MgSO₄·7H₂O | 1 g/L |
| Mameno (as total nitrogen) | 0.4 g/L |
| CaCO₃ | 25 g/L |
| pH 7.0 | |

CaCO₃ was separately sterilized. Ethanol was separately added to the medium.

**Table 14**

| Strain | 48 hr | | 72 hr | |
|---|---|---|---|---|
| | OD₅₅₀ | Val (g/l) | OD₅₅₀ | Val (g/l) |
| H-81 P_{Ltac}adhE* | 5.5±0.5 | 1.1±0.1 | 12.4±0.6 | 1.6±0.1 |
| H-81 P_{Ltac}adhE*/ pPLydbK3 | 16.8±0.4 | 2.1±0.2 | 16.1+0.4 | 2.0±0.1 |

### [Explanation of Sequence Listing]

SEQ ID NO: 1: Nucleotide sequence of *Chlorobium tepidum* pyruvate synthase gene
SEQ ID NO: 2: Amino acid sequence of *Chlorobium tepidum* pyruvate synthase
SEQ ID NO: 3: Nucleotide sequence of *Escherichia coli* pyruvate synthase gene
SEQ ID NO: 4: Amino acid sequence of *Escherichia coli* pyruvate synthase
SEQ ID NO: 5: Nucleotide sequence of *Escherichia g-gracilis* pyruvate: NADP⁺ oxidoreductase gene
SEQ ID NO: 6: Amino acid sequence of *Escherichia gracilis* pyruvate: NADP⁺ oxidoreductase gene
SEQ ID NO: 7: Nucleotide sequence of *Escherichia coli* flavodoxin NADP⁺ reductase(*fpr*) gene
SEQ ID NO: 8: Amino acid sequence encoded by *E*. *coli E*. *coli* flavodoxin NADP⁺ reductase (*fpr*) gene
SEQ ID NO: 9: Nucleotide sequence of *E*. *coli* ferredoxin (*fdx*) gene
SEQ ID NO: 10: Amino acid sequence encoded by *E*. *coli* ferredoxin (*fdx*) gene
SEQ ID NO: 11: Nucleotide sequence of *E. coli* ferredoxin(*yfhL*) gene
SEQ ID NO: 12: Amino acid sequence encoded by *E. coli* ferredoxin(*yfhL*) gene
SEQ ID NO: 13: Nucleotide sequence of *E. coli* flavodoxin(*fldA*) gene
SEQ ID NO: 14: Amino acid sequence encoded by *E. coli* flavodoxin(*fldA*) gene
SEQ ID NO: 15: Nucleotide sequence of *E. coli* flavodoxin(*fldB*) gene
SEQ ID NO: 16: Amino acid sequence encoded by *E. coli* flavodoxin(*fldB*) gene
SEQ ID NO: 17: Nucleotide sequence of *Chlorobium tepidum* ferredoxin I gene
SEQ ID NO: 18: Amino acid sequence encoded by *Chlorobium tepidum* ferredoxin, I gene
SEQ ID NO: 19: Nucleotide sequence of *Chlorobium tepidum* ferredoxin II gene
SEQ ID NO: 20: Amino acid sequence encoded by *Chlorobium tepidum* ferredoxin II gene
SEQ ID NO: 21: Nucleotide sequence of *E. coli* alcohol dehydrogenase gene
SEQ ID NO: 22: Amino acid sequence encoded by *E*. *coli* alcohol dehydrogenase gene
SEQ ID NO: 23: P_{L-tac} and chloramphenicol resistance (Cm^{R}) gene amplification primer 1
SEQ ID NO: 24: P_{L-tac} and chloramphenicol resistance (Cm^{R}) gene amplification primer 2
SEQ ID NO: 25: Nucleotide sequence of P_{L-tac}
SEQ ID NO: 26: P_{L-tac} and chloramphenicol resistance (Cm^{R}) gene amplification primer 3
SEQ ID NO: 27: P_{L-tac} and chloramphenicol resistance (Cm^{R}) gene amplification primer 4
SEQ ID NO: 28: Kanamycin resistance (Km^{R}) gene amplification primer 1
SEQ ID NO: 29: Kanamycin resistance (Km^{R}) gene amplification primer 2
SEQ ID NO: 30: Kanamycin resistance (Km^{R}) gene amplification primer 3
SEQ ID NO: 31: Kanamycin resistance (Km^{R}) gene amplification primer 4
SEQ ID NO: 32: *Escherichia coli* mutant alcohol dehydrogenase gene amplification primer 1
SEQ ID NO: 33: *Escherichia coli* mutant alcohol dehydrogenase gene amplification primer 2
SEQ ID NO: 34: *Escherichia coli* mutant alcohol dehydrogenase gene amplification primer 3
SEQ ID NO: 35: *Chlorobium tepidum* pyruvate synthase gene amplification primer 1
SEQ ID NO: 36: *Chlorobium tepidum* pyruvate synthase gene amplification primer 2
SEQ ID NO: 37: Threonine operon promoter sequence
SEQ ID NO: 38: *Escherichia coli* pyruvate synthase gene amplification primer 1
SEQ ID NO: 39: *Escherichia coli* pyruvate synthase gene amplification primer 2
SEQ ID NO: 40: *Escherichia gracilis* pyruvate:NADP⁺ oxidoreductase gene amplification primer 1
SEQ ID NO: 41: *Escherichia gracilis* pyruvate:NADP⁺ oxidoreductase gene amplification primer 2
SEQ ID NO: 42: *Escherichia coli* flavodoxin NADP⁺ reductase gene amplification primer 1
SEQ ID NO: 43: *Escherichia coli* flavodoxin NADP⁺ reductase gene amplification primer 2
SEQ ID NO: 44: *Escherichia coli* fdx gene amplification primer 1
SEQ ID NO: 45: *Escherichia coli* fdx gene amplification primer 2
SEQ ID NO: 46: Nucleotide sequence of *Escherichia coli* pyruvate dehydrogenase Ep1 subunit gene (*aceE*)
SEQ ID NO: 47: Amino acid sequence of *Escherichia coli* pyruvate dehydrogenase Ep1 subunit
SEQ ID NO: 48: Nucleotide sequence of *Escherichia coli* pyruvate dehydrogenase E2 subunit gene (*aceF*)
SEQ ID NO: 49: Amino acid sequence of *Escherichia coli* pyruvate dehydrogenase E2 subunit
SEQ ID NO: 50: Nucleotide sequence of *Escherichia coli* pyruvate dehydrogenase E3 subunit gene (*lpdA*)
SEQ ID NO: 51: Amino acid sequence of *Escherichia coli* pyruvate dehydrogenase E3 subunit
SEQ ID NO: 52: Nucleotide sequence of gene (*sfcA*) encoding *Escherichia coli* malic enzyme
SEQ ID NO: 53: Amino acid sequence of malic enzyme encoded by *Escherichia coli sfcA* gene
SEQ ID NO: 54: Nucleotide sequence of gene (*b2463*) encoding *Escherichia coli* malic enzyme
SEQ. ID NO: 55: Amino acid sequence of malic enzyme encoded by *Escherichia coli b2463* gene
SEQ ID NO: 56: Nucleotide sequence of *Methanococcus maripaludis* pyruvate synthase γ subunit gene (*porA*)
SEQ ID NO: 57: Amino acid sequence of *Methanococcus maxipaludis* pyruvate synthase γ subunit
SEQ ID NO: 58: Nucleotide sequence of *Methanococcus maripaludis* pyruvate synthase δ subunit gene (*porB*)
SEQ ID NO: 59: Amino acid sequence of *Methanococcus maripaludis* pyruvate synthase δ subunit
SEQ ID NO: 60: Nucleotide sequence of *Methanococcus maripaludis* pyruvate synthase α subunit gene (*porC*)
SEQ ID NO: 61: Amino acid sequence of *Methanococcus maripaludis* pyruvate synthase α subunit
SEQ ID NO: 62: Nucleotide sequence of *Methanococcus maripaludis* pyruvate synthase β subunit gene (*porD*)
SEQ ID NO: 63: Amino acid sequence of *Methanococcus maripaludis* pyruvate synthase β subunit
SEQ ID NO: 64: Nucleotide sequence of *Methanococcus maripaludis* pyruvate synthase *porE* gene
SEQ ID NO: 65: Amino acid sequence of *Methanococcus maripaludis* pyruvate synthase PorE
SEQ ID NO: 66: Nucleotide sequence of *Methanococcus maripaludis* pyruvate synthase *porF* gene
SEQ ID NO: 67: Amino acid sequence of *Methanococcus maripaludis* pyruvate synthase PorF
SEQ ID NO: 68: *fadR* gene disruption primer 1
SEQ ID NO: 69: *fadR* gene disruption primer 2
SEQ ID NO: 70: *sfcA* gene disruption primer 1
SEQ ID NO: 71: *sfcA* gene disruption primer 2
SEQ ID NO: 72: *b2463* gene disruption primer 1
SEQ ID NO: 73: *b2463* gene disruption primer 2
SEQ ID NO: 74: *Escherichia coli* pyruvate synthase gene amplification primer 1
SEQ ID NO: 75: *Escherichia coli* pyruvate synthase gene amplification primer 2
SEQ ID NO: 76: *Methanacoccus maripaludis* pyruvate synthase gene amplification primer 1
SEQ ID NO: 77: *Methanococcus maripaludis* pyruvate synthase gene amplification primer 2
SEQ ID NO: 78: amplification primer 1 for promoter replacement of *Escherichia coli* pyruvate synthase gene
SEQ ID NO: 79: amplification primer 2 for promoter replacement of *Escherichia coli* pyruvate synthase gene
SEQ ID NO: 80: cat-PL-ydbK structure confirmation primer 1
SEQ ID NO: 81: cat-PL-ydbK structure confirmation primer 2
SEQ ID NO: 82: Nucleotide sequence of *Escherichia coli fadR* gene
SEQ ID NO: 83: Amino acid sequence of *Escherichia coli* FadR
SEQ ID NO: 84: P_{L-tac} amplification primer 1
SEQ ID NO: 85: P_{L-tac} amplification primer 2

### Industrial Applicability

By using the microorganism of the present invention, an L-amino acid can be efficiently produced by fermentation. Moreover, according to a preferred embodiment of the method of the present invention, the method of the present invention is an environment-friendly method which can reduce carbon dioxide emission by suppressing decarboxylation and utilizing carbon dioxide fixation.

## Claims

1. A method for producing an L-amino acid, which comprises culturing in a medium a microorganism, which has an ability to produce an L-amino acid selected from the group consisting of L-lysine, L-threonine, L-tryptophan, L-phenylalanine, L-valine, L-leucine, L-isoleucine and L-serine and has been modified so that an activity of pyruvate synthase or pyruvate:NADP⁺ oxidoreductase is increased, to produce and accumulate said L-amino acid in the medium or cells, and collecting said L-amino acid from the medium or the cells, wherein the medium contains ethanol or an aliphatic acid as a carbon source, and wherein the activity of pyruvate synthase or pyruvate:NADP⁺ oxidoreductase is increased by increasing copy number of a gene encoding pyruvate synthase or pyruvate:NADP⁺ oxidoreductase, or by modifying an expression control sequence of the gene.

2. The method according to claim 1, wherein the microorganism has been modified so that the activity of pyruvate synthase is increased.

3. The method according to claim 1, wherein the microorganism has been modified so that the activity of pyruvate:NADP⁺ oxidoreductase is increased.

4. The method according to any one of claims 1 - 3, wherein the gene encoding pyruvate synthase encodes a polypeptide defined in any one of the following (A) to (D), or polypeptides defined in the following (E), (F), (G) and (H), or polypeptides defined in the following (E), (F), (G), (H), (I) and (J) :
(A) a polypeptide comprising the amino acid sequence shown in SEQ ID NO: 2,
(B) a polypeptide comprising an amino acid sequence shown in SEQ ID NO: 2 including substitution, deletion, insertion or addition of one or 1 to 20 amino acid residues and having pyruvate synthase activity,
(C) a polypeptide comprising the amino acid sequence shown in SEQ ID NO: 4,
(D) a polypeptide comprising an amino acid sequence shown in SEQ ID NO: 4 including substitution, deletion, insertion or addition of one or 1 to 20 amino acid residues and having pyruvate synthase activity,
(E) a polypeptide defined in the following (E1) or (E2):
(E1) a polypeptide comprising the amino acid sequence shown in SEQ ID NO: 57,
(E2) a polypeptide which comprises an amino acid sequence shown in SEQ ID NO: 57 including substitution, deletion, insertion or addition of one or 1 to 20 amino acid residues and having pyruvate synthase activity, and is able to constitute a protein complex having pyruvate synthase activity with at least the polypeptides (F), (G) and (H),
(F) a polypeptide defined in the following (F1) or (F2) :
(F1) a polypeptide comprising the amino acid sequence shown in SEQ ID NO: 59,
(F2) a polypeptide which comprises an amino acid sequence shown in SEQ ID NO: 59 including substitution, deletion, insertion or addition of one or 1 to 20 amino acid residues and having pyruvate synthase activity, and is able to constitute a protein complex having pyruvate synthase activity with at least the polypeptides (E), (G) and (H),
(G) a polypeptide defined in the following (G1) or (G2):
(G1) a polypeptide comprising the amino acid sequence shown in SEQ ID NO: 61,
(G2) a polypeptide which comprises an amino acid sequence shown in SEQ ID NO: 61 including substitution, deletion, insertion or addition of one or 1 to 20 amino acid residues and having pyruvate synthase activity, and is able to constitute a protein complex having pyruvate synthase activity with at least the polypeptides (E), (F) and (H),
(H) a polypeptide defined in the following (H1) or (H2):
(H1) a polypeptide comprising the amino acid sequence shown in SEQ ID NO: 63,
(H2) a polypeptide which comprises an amino acid sequence shown in SEQ ID NO: 63 including substitution, deletion, insertion or addition of one or 1 to 20 amino acid residues and having pyruvate synthase activity, and is able to constitute a protein complex having pyruvate synthase activity with at least the polypeptides (E), (F) and (G),
(I) a polypeptide defined in the following (I1) or (I2):
(I1) a polypeptide comprising the amino acid sequence shown in SEQ ID NO: 65,
(I2) a polypeptide which comprises an amino acid sequence shown in SEQ ID NO: 65 including substitution, deletion, insertion or addition of one or 1 to 20 amino acid residues and having pyruvate synthase activity, and is able to constitute a protein complex having pyruvate synthase activity with at least the polypeptides (E), (F) and (G) and (H),
(J) a polypeptide defined in the following (J1) or (J2):
(J1) a polypeptide comprising the amino acid sequence shown in SEQ ID NO: 67,
(J2) a polypeptide which comprises an amino acid sequence shown in SEQ ID NO: 67 including substitution, deletion, insertion or addition of one or 1 to 20 amino acid residues and having pyruvate synthase activity, and is able to constitute a protein complex having pyruvate synthase activity with at least the polypeptides (E), (F) and (G) and (H).

5. The method according to any one of claims 1 - 3, wherein the gene encoding pyruvate synthase comprises a DNA defined in any one of the following (a) to (d), or a DNA defined in the following (e), (f), (g) and (h), or a DNA defined in the following (e), (f), (g), (h), (i) and (j) :
(a) a DNA having the nucleotide sequence shown in SEQ ID NO: 1,
(b) a DNA which is able to hybridize with a sequence complementary to the nucleotide sequence shown in SEQ ID NO: 1 or a probe which can be prepared from the nucleotide sequence under stringent conditions, and which encodes a polypeptide having pyruvate synthase activity,
(c) a DNA having the nucleotide sequence shown in SEQ ID NO: 3,
(d) a DNA which is able to hybridize with a sequence complementary to the nucleotide sequence shown in SEQ ID NO: 3 or a probe which can be prepared from the nucleotide sequence under stringent conditions, and which encodes a polypeptide having pyruvate synthase activity,
(e) a DNA defined in the following (e1) or (e2):
(e1) a DNA having the nucleotide sequence shown in SEQ ID NO: 56,
(e2) a DNA which is able to hybridize with a sequence complementary to the nucleotide sequence shown in SEQ ID NO: 56 or a probe which can be prepared from the nucleotide sequence under stringent conditions, and which encodes a protein complex having pyruvate synthase activity with at least DNAs defined in (f), (g) and (h),
(f) a DNA defined in the following (f1) or (f2):
(f1) a DNA having the nucleotide sequence shown in SEQ ID NO: 58,
(f2) a DNA which is able to hybridize with a sequence complementary to the nucleotide sequence shown in SEQ ID NO: 58 or a probe which can be prepared from the nucleotide sequence under stringent conditions, and which encodes a protein complex having pyruvate synthase activity with at least DNAs defined in (e), (g) and (h),
(g) a DNA defined in the following (g1) or (g2):
(g1) a DNA having the nucleotide sequence shown in SEQ ID NO: 60,
(g2) a DNA which is able to hybridize with a sequence complementary to the nucleotide sequence shown in SEQ ID NO: 60 or a probe which can be prepared from the nucleotide sequence under stringent conditions, and which encodes a protein complex having pyruvate synthase activity with at least DNAs defined in (e), (f) and (h),
(h) a DNA defined in the following (h1) or (h2):
(h1) a DNA having the nucleotide sequence shown in SEQ ID NO: 62,
(h2) a DNA which is able to hybridize with a sequence complementary to the nucleotide sequence shown in SEQ ID NO: 62 or a probe which can be prepared from the nucleotide sequence under stringent conditions, and which encodes a protein complex having pyruvate synthase activity with at least DNAs defined in (e), (f) and (g),
(i) a DNA defined in the following (i1) or (i2):
(i1) a DNA having the nucleotide sequence shown in SEQ ID NO: 64,
(i2) a DNA which is able to hybridize with a sequence complementary to the nucleotide sequence shown in SEQ ID NO: 64 or a probe which can be prepared from the nucleotide sequence under stringent conditions, and which encodes a protein complex having pyruvate synthase activity with at least DNAs defined in (e), (f), (g) and (h),
(j) a DNA defined in the following (j1) or (j2):
(j1) a DNA having the nucleotide sequence shown in SEQ ID NO: 66,
(j2) a DNA which is able to hybridize with a sequence complementary to the nucleotide sequence shown in SEQ ID NO: 66 or a probe which can be prepared from the nucleotide sequence under stringent conditions, and which encodes a protein complex having pyruvate synthase activity with at least DNAs defined in (e), (f), (g) and (h); wherein the stringent conditions are washing at 60°C, 0.1 x SSC, and 0.1% SDS.

6. The method according to any one of claims 1 - 3, wherein the gene encoding pyruvate:NADP⁺ oxidoreductase encodes a polypeptide defined in the following (A) or (B):
(A) a polypeptide comprising the amino acid sequence shown in SEQ ID NO: 6,
(B) a polypeptide comprising an amino acid sequence shown in SEQ ID NO: 6 including substitution, deletion, insertion or addition of one or 1 to 20 amino acid residues and having pyruvate:NADP⁺ oxidoreductase activity.

7. The method according to any one of claims 1 - 3, wherein the gene encoding pyruvate:NADP⁺ oxidoreductase comprises a DNA defined in the following (a) or (b):
(a) a DNA having the nucleotide sequence shown in SEQ ID NO: 5,
(b) a DNA which is able to hybridize with a sequence complementary to the nucleotide sequence shown in SEQ ID NO: 5 or a probe which can be prepared from the nucleotide sequence under stringent conditions and, and which encodes a polypeptide having pyruvate:NADP⁺ oxidoreductase activity; wherein the stringent conditions are washing at 60°C, 0.1 x SSC, and 0.1% SDS.

8. The method according to any one of claims 1 to 5, wherein the microorganism has been modified so that an activity of ferredoxin-NADP⁺ reductase is increased.

9. The method according to any one of claims 1 to 5 and 8, wherein the microorganism has been modified so that ferredoxin or flavodoxin production ability is improved.

10. The method according to any one of claims 1 to 9, wherein the microorganism has been modified so that an activity of malic enzyme is decreased.

11. The method according to any one of claims 1 to 10, wherein the microorganism has been modified so that pyruvate dehydrogenase activity is decreased.

12. The method according to any one of claims 1 to 11, wherein the microorganism has been modified so that it can aerobically assimilate ethanol.

13. The method according to any one of claims 1 to 12, wherein the microorganism is a bacterium belonging to the genus selected from the group consisting of the genera *Escherirhia, Enterobacter*, *Pantoea, Klebsiella and Serratia*.

14. The method according to claim 13, wherein activities of both of NAD-dependent malic enzyme and NADP-dependent malic enzyme are decreased.

15. The method according to any one of claims 1 to 14, wherein the microorganism is a coryneform bacterium.

## Patentansprüche

1. Verfahren zum Produzieren einer L-Aminosäure, welches das Kultivieren eines Mikroorganismus in einem Medium umfasst, wobei der Mikroorganismus eine Fähigkeit zur Produktion einer L-Aminosäure, ausgewählt aus der Gruppe, bestehend aus L-Lysin, L-Threonin, L-Tryptophan, L-Phenylalanin, L-Valin, L-Leucin, L-Isoleucin und L-Serin, hat und modifiziert wurde, so dass eine Aktivität von Pyruvatsynthase oder Pyruvat:NADP⁺-Oxidoreduktase gesteigert wird, um die L-Aminosäure in dem Medium oder Zellen zu produzieren und zu akkumulieren, und das Gewinnen der L-Aminosäure aus dem Medium oder den Zellen, wobei das Medium Ethanol oder eine aliphatische Säure als eine Kohlenstoffquelle enthält, und wobei die Aktivität von Pyruvatsynthase oder Pyruvat:NADP⁺-Oxidoreduktase gesteigert wird durch Steigern der Kopienzahl eines Gens, welches Pyruvatsynthase oder Pyruvat:NADP⁺-Oxidoreduktase codiert, oder durch Modifizieren einer Expressionskontrollsequenz des Gens.

2. Verfahren gemäß Anspruch 1, wobei der Mikroorganismus modifiziert wurde, so dass die Aktivität von Pyruvatsynthase gesteigert wird.

3. Verfahren gemäß Anspruch 1, wobei der Mikroorganismus modifiziert wurde, so dass die Aktivität von Pyruvat:NADP⁺-Oxidoreduktase gesteigert wird.

4. Verfahren gemäß irgendeinem der Ansprüche 1-3, wobei das Gen, welches Pyruvatsynthase codiert, ein Polypeptid, welches in irgendeinem der folgenden (A) bis (D) definiert ist, oder Polypeptide, die in den folgenden (E), (F), (G) und (H) definiert sind, oder Polypeptide, die in den folgenden (E), (F), (G), (H), (I) und (J) definiert sind, codiert:
(A) ein Polypeptid, welches die in SEQ ID NO: 2 gezeigte Aminosäuresequenz umfasst,
(B) ein Polypeptid, welches eine in SEQ ID NO: 2 gezeigte Aminosäuresequenz, einschließlich Substitution, Deletion, Insertion oder Addition eines oder von 1 bis 20 Aminosäure-Resten, umfasst und Pyruvatsynthase-Aktivität hat,
(C) ein Polypeptid, welches die in SEQ ID NO: 4 gezeigte Aminosäuresequenz umfasst,
(D) ein Polypeptid, welches eine in SEQ ID NO: 4 gezeigte Aminosäuresequenz, einschließlich Substitution, Deletion, Insertion oder Addition eines oder von 1 bis 20 Aminosäure-Resten, umfasst und Pyruvatsynthase-Aktivität hat,
(E) ein in den folgenden (E1) oder (E2) definiertes Polypeptid:
(E1) ein Polypeptid, welches die in SEQ ID NO: 57 gezeigte Aminosäuresequenz umfasst,
(E2) ein Polypeptid, welches eine in SEQ ID NO: 57 gezeigte Aminosäuresequenz, einschließlich Substitution, Deletion, Insertion oder Addition eines oder von 1 bis 20 Aminosäure-Resten, umfasst und Pyruvatsynthase-Aktivität hat, und dazu fähig ist, einen Proteinkomplex, der Pyruvatsynthase-Aktivität hat, mit wenigstens den Polypeptiden (F), (G) und (H) zu bilden,
(F) ein in den folgenden (F1) oder (F2) definiertes Polypeptid:
(F1) ein Polypeptid, welches die in SEQ ID NO: 59 gezeigte Aminosäuresequenz umfasst,
(F2) ein Polypeptid, welches eine in SEQ ID NO: 59 gezeigte Aminosäuresequenz, einschließlich Substitution, Deletion, Insertion oder Addition eines oder von 1 bis 20 Aminosäure-Resten, umfasst und Pyruvatsynthase-Aktivität hat, und dazu fähig ist, einen Proteinkomplex, der Pyruvatsynthase-Aktivität hat, mit wenigstens den Polypeptiden (E), (G) und (H) zu bilden,
(G) ein in den folgenden (G1) oder (G2) definiertes Polypeptid:
(G1) ein Polypeptid, welches die in SEQ ID NO: 61 gezeigte Aminosäuresequenz umfasst,
(G2) ein Polypeptid, welches eine in SEQ ID NO: 61 gezeigte Aminosäuresequenz, einschließlich Substitution, Deletion, Insertion oder Addition eines oder von 1 bis 20 Aminosäure-Resten, umfasst und Pyruvatsynthase-Aktivität hat, und dazu fähig ist, einen Proteinkomplex, der Pyruvatsynthase-Aktivität hat, mit wenigstens den Polypeptiden (E), (F) und (H) zu bilden,
(H) ein in den folgenden (H1) oder (H2) definiertes Polypeptid:
(H1) ein Polypeptid, welches die in SEQ ID NO: 63 gezeigte Aminosäuresequenz umfasst,
(H2) ein Polypeptid, welches eine in SEQ ID NO: 63 gezeigte Aminosäuresequenz, einschließlich Substitution, Deletion, Insertion oder Addition eines oder von 1 bis 20 Aminosäure-Resten, umfasst und Pyruvatsynthase-Aktivität hat, und dazu fähig ist, einen Proteinkomplex, der Pyruvatsynthase-Aktivität hat, mit wenigstens den Polypeptiden (E), (F) und (G) zu bilden,
(I) ein in den folgenden (I1) oder (I2) definiertes Polypeptid:
(I1) ein Polypeptid, welches die in SEQ ID NO: 65 gezeigte Aminosäuresequenz umfasst,
(I2) ein Polypeptid, welches eine in SEQ ID NO: 65 gezeigte Aminosäuresequenz, einschließlich Substitution, Deletion, Insertion oder Addition eines oder von 1 bis 20 Aminosäure-Resten, umfasst und Pyruvatsynthase-Aktivität hat, und dazu fähig ist, einen Proteinkomplex, der Pyruvatsynthase-Aktivität hat, mit wenigstens den Polypeptiden (E), (F), (G) und (H) zu bilden,
(J) ein in den folgenden (J1) oder (J2) definiertes Polypeptid:
(J1) ein Polypeptid, welches die in SEQ ID NO: 67 gezeigte Aminosäuresequenz umfasst,
(J2) ein Polypeptid, welches eine in SEQ ID NO: 67 gezeigte Aminosäuresequenz, einschließlich Substitution, Deletion, Insertion oder Addition eines oder von 1 bis 20 Aminosäure-Resten, umfasst und Pyruvatsynthase-Aktivität hat, und dazu fähig ist, einen Proteinkomplex, der Pyruvatsynthase-Aktivität hat, mit wenigstens den Polypeptiden (E), (F) und (G) und (H) zu bilden.

5. Verfahren gemäß irgendeinem der Ansprüche 1-3, wobei das Gen, welches Pyruvatsynthase codiert, eine DNA, die in irgendeinem der folgenden (a) bis (d) definiert ist, oder eine DNA, die in den folgenden (e), (f), (g) und (h) definiert ist, oder eine DNA, die in den folgenden (e), (f), (g), (h), (i) und (j) definiert ist, umfasst:
(a) eine DNA, die die in SEQ ID NO: 1 gezeigte Nukleotidsequenz hat,
(b) eine DNA, die dazu fähig ist, mit einer Sequenz, die komplementär zu der in SEQ ID NO: 1 gezeigten Nukleotidsequenz ist, oder einer Sonde, die aus der Nukleotidsequenz hergestellt werden kann, unter stringenten Bedingungen zu hybridisieren, und die ein Polypeptid codiert, welches Pyruvatsynthase-Aktivität hat,
(c) eine DNA, die die in SEQ ID NO: 3 gezeigte Nukleotidsequenz hat,
(d) eine DNA, die dazu fähig ist, mit einer Sequenz, die komplementär zu der in SEQ ID NO: 3 gezeigten Nukleotidsequenz ist, oder einer Sonde, die aus der Nukleotidsequenz hergestellt werden kann, unter stringenten Bedingungen zu hybridisieren, und die ein Polypeptid codiert, welches Pyruvatsynthase-Aktivität hat,
(e) eine DNA, die in den folgenden (e1) oder (e2) definiert ist:
(e1) eine DNA, die die in SEQ ID NO: 56 gezeigte Nukleotidsequenz hat,
(e2) eine DNA, die dazu fähig ist, mit einer Sequenz, die komplementär zu der in SEQ ID NO: 56 gezeigten Nukleotidsequenz ist, oder einer Sonde, die aus der Nukleotidsequenz hergestellt werden kann, unter stringenten Bedingungen zu hybridisieren, und die einen Proteinkomplex, welcher Pyruvatsynthase-Aktivität hat, mit wenigstens den in (f), (g) und (h) definierten DNAs codiert,
(f) eine DNA, die in den folgenden (f1) oder (f2) definiert ist:
(f1) eine DNA, die die in SEQ ID NO: 58 gezeigte Nukleotidsequenz hat,
(f2) eine DNA, die dazu fähig ist, mit einer Sequenz, die komplementär zu der in SEQ ID NO: 58 gezeigten Nukleotidsequenz ist, oder einer Sonde, die aus der Nukleotidsequenz hergestellt werden kann, unter stringenten Bedingungen zu hybridisieren, und die einen Proteinkomplex, welcher Pyruvatsynthase-Aktivität hat, mit wenigstens den in (e), (g) und (h) definierten DNAs codiert,
(g) eine DNA, die in den folgenden (g1) oder (g2) definiert ist:
(g1) eine DNA, die die in SEQ ID NO: 60 gezeigte Nukleotidsequenz hat,
(g2) eine DNA, die dazu fähig ist, mit einer Sequenz, die komplementär zu der in SEQ ID NO: 60 gezeigten Nukleotidsequenz ist, oder einer Sonde, die aus der Nukleotidsequenz hergestellt werden kann, unter stringenten Bedingungen zu hybridisieren, und die einen Proteinkomplex, welcher Pyruvatsynthase-Aktivität hat, mit wenigstens den in (e), (f) und (h) definierten DNAs codiert,
(h) eine DNA, die in den folgenden (h1) oder (h2) definiert ist:
(h1) eine DNA, die die in SEQ ID NO: 62 gezeigte Nukleotidsequenz hat,
(h2) eine DNA, die dazu fähig ist, mit einer Sequenz, die komplementär zu der in SEQ ID NO: 62 gezeigten Nukleotidsequenz ist, oder einer Sonde, die aus der Nukleotidsequenz hergestellt werden kann, unter stringenten Bedingungen zu hybridisieren, und die einen Proteinkomplex, welcher Pyruvatsynthase-Aktivität hat, mit wenigstens den in (e), (f) und (g) definierten DNAs codiert,
(i) eine DNA, die in den folgenden (i1) oder (i2) definiert ist:
(i1) eine DNA, die die in SEQ ID NO: 64 gezeigte Nukleotidsequenz hat,
(i2) eine DNA, die dazu fähig ist, mit einer Sequenz, die komplementär zu der in SEQ ID NO: 64 gezeigten Nukleotidsequenz ist, oder einer Sonde, die unter stringenten Bedingungen aus der Nukleotidsequenz hergestellt werden kann, zu hybridisieren, und die einen Proteinkomplex, welcher Pyruvatsynthase-Aktivität hat, mit wenigstens den in (e), (f), (g) und (h) definierten DNAs codiert,
(j) eine DNA, die in den folgenden (j1) oder (j2) definiert ist:
(j1) eine DNA, die die in SEQ ID NO: 66 gezeigte Nukleotidsequenz hat,
(j2) eine DNA, die dazu fähig ist, mit einer Sequenz, die komplementär zu der in SEQ ID NO: 66 gezeigten Nukleotidsequenz ist, oder einer Sonde, die aus der Nukleotidsequenz hergestellt werden kann, unter stringenten Bedingungen zu hybridisieren, und die einen Proteinkomplex, welcher Pyruvatsynthase-Aktivität hat, mit wenigstens den in (e), (f), (g) und (h) definierten DNAs codiert; wobei die stringenten Bedingungen Waschen bei 60°C, 0,1 x SSC und 0,1% SDS sind.

6. Verfahren gemäß irgendeinem der Ansprüche 1-3, wobei das Gen, welches Pyruvat:NADP⁺-Oxidoreduktase codiert, ein Polypeptid codiert, welches in den folgenden (A) oder (B) definiert ist:
(A) ein Polypeptid, welches die in SEQ ID NO: 6 gezeigte Aminosäuresequenz umfasst,
(B) ein Polypeptid, welches eine in SEQ ID NO: 6 gezeigte Aminosäuresequenz, einschließlich Substitution, Deletion, Insertion oder Addition eines oder von 1 bis 20 Aminosäure-Resten, umfasst und Pyruvat:NADP⁺-Oxidoreduktase-Aktivität hat.

7. Verfahren gemäß irgendeinem der Ansprüche 1-3, wobei das Gen, welches Pyruvat:NADP⁺-Oxidoreduktase codiert, eine DNA umfasst, die in den folgenden (a) oder (b) definiert ist:
(a) eine DNA, die die in SEQ ID NO: 5 gezeigte Nukleotidsequenz hat,
(b) eine DNA, die dazu fähig ist, mit einer Sequenz, die komplementär zu der in SEQ ID NO: 5 gezeigten Nukleotidsequenz ist, oder einer Sonde, die aus der Nukleotidsequenz hergestellt werden kann, unter stringenten Bedingungen zu hybridisieren, und die ein Polypeptid codiert, welches Pyruvat:NADP⁺-Oxidoreduktase-Aktivität hat; wobei die stringenten Bedingungen Waschen bei 60°C, 0,1 x SSC und 0,1% SDS sind.

8. Verfahren gemäß irgendeinem der Ansprüche 1 bis 5, wobei der Mikroorganismus modifiziert wurde, so dass eine Aktivität von Ferredoxin-NADP⁺-Reduktase gesteigert wird.

9. Verfahren gemäß irgendeinem der Ansprüche 1 bis 5 und 8, wobei der Mikroorganismus modifiziert wurde, so dass Ferredoxin- oder Flavodoxin-Produktionsfähigkeit verbessert wird.

10. Verfahren gemäß irgendeinem der Ansprüche 1 bis 9, wobei der Mikroorganismus modifiziert wurde, so dass eine Aktivität von Malatenzym verringert wird.

11. Verfahren gemäß irgendeinem der Ansprüche 1 bis 10, wobei der Mikroorganismus modifiziert wurde, so dass Pyruvatdehydrogenase-Aktivität verringert wird.

12. Verfahren gemäß irgendeinem der Ansprüche 1 bis 11, wobei der Mikroorganismus modifiziert wurde, so dass er aerob Ethanol assimilieren kann.

13. Verfahren gemäß irgendeinem der Ansprüche 1 bis 12, wobei der Mikroorganismus ein Bakterium ist, welches zu der Gattung gehört, ausgewählt aus der Gruppe, bestehend aus den Gattungen *Escherichia, Enterobacter, Pantoea, Klebsiella* und *Serratia.*

14. Verfahren gemäß Anspruch 13, wobei die Aktivitäten sowohl des NAD-abhängigen Malatenzyms als auch des NADPabhängigen Malatenzyms verringert sind.

15. Verfahren gemäß irgendeinem der Ansprüche 1 bis 14, wobei der Mikroorganismus ein coryneformes Bakterium ist.

## Revendications

1. Procédé de production d'un acide L-aminé, qui comprend la mise en culture dans un milieu d'un microorganisme, qui a la capacité de produire un acide L-aminé choisi dans le groupe constitué de la L-lysine, la L-thréonine, le L-tryptophane, la L-phénylalanine, la L-valine, la L-leucine, la L-isoleucine et la L-sérine, et qui a été modifié de façon à ce qu'une activité pyruvate synthase ou pyruvate:NADP⁺ oxydoréductase soit augmentée, de produire et accumuler ledit acide L-aminé dans le milieu ou les cellules, et le recueil dudit acide L-aminé dans le milieu ou les cellules, dans lequel le milieu contient de l'éthanol ou un acide aliphatique comme source de carbone, et dans lequel l'activité pyruvate synthase ou de pyruvate:NADP⁺ oxydoréductase est augmentée en augmentant un nombre de copies d'un gène codant la pyruvate synthase ou la pyruvate:NADP⁺ oxydoréductase, ou en modifiant une séquence de contrôle d'expression du gène.

2. Procédé selon la revendication 1, dans lequel le microorganisme a été modifié de façon à ce que l'activité pyruvate synthase soit augmentée.

3. Procédé selon la revendication 1, dans lequel le microorganisme a été modifié de façon à ce que l'activité pyruvate:NADP⁺ oxydoréductase soit augmentée.

4. Procédé selon l'une quelconque des revendications 1 à 3, dans lequel le gène codant la pyruvate synthase code un polypeptide défini à l'un quelconque des points (A) à (D) suivants, ou des polypeptides définis aux points (E), (F), (G) et (H) suivants, ou des polypeptides définis aux points (E), (F), (G), (H), (I) et (J) suivants :
(A) un polypeptide comprenant la séquence d'acides aminés représentée dans SEQ ID N° 2,
(B) un polypeptide comprenant une séquence d'acides aminés représentée dans SEQ ID N° 2, comportant la substitution, la délétion, l'insertion ou l'addition d'un ou de 1 à 20 résidus d'acides aminés et ayant une activité pyruvate synthase,
(C) un polypeptide comprenant la séquence d'acides aminés représentée dans SEQ ID N° 4,
(D) un polypeptide comprenant une séquence d'acides aminés représentée dans SEQ ID N° 4, comportant la substitution, la délétion, l'insertion ou l'addition d'un ou de 1 à 20 résidus d'acides aminés et ayant une activité pyruvate synthase,
(E) un polypeptide défini aux points (E1) ou (E2) suivants :
(E1) un polypeptide comprenant la séquence d'acides aminés représentée dans SEQ ID N° 57,
(E2) un polypeptide qui comprend une séquence d'acides aminés représentée dans SEQ ID N° 57, comportant la substitution, la délétion, l'insertion ou l'addition d'un ou de 1 à 20 résidus d'acides aminés et ayant une activité pyruvate synthase, et est capable de constituer un complexe de protéines ayant une activité pyruvate synthase avec au moins les polypeptides (F), (G) et (H),
(F) un polypeptide défini aux points (F1) ou (F2) suivants :
(F1) un polypeptide comprenant la séquence d'acides aminés représentée dans SEQ ID N° 59,
(F2) un polypeptide qui comprend une séquence d'acides aminés représentée dans SEQ ID N° 59, comportant la substitution, la délétion, l'insertion ou l'addition d'un ou de 1 à 20 résidus d'acides aminés et ayant une activité pyruvate synthase, et est capable de constituer un complexe de protéines ayant une activité pyruvate synthase avec au moins les polypeptides (E), (G) et (H),
(G) un polypeptide défini aux points (G1) ou (G2) suivants :
(G1) un polypeptide comprenant la séquence d'acides aminés représentée dans SEQ ID N° 61,
(G2) un polypeptide qui comprend une séquence d'acides aminés représentée dans SEQ ID N° 61, comportant la substitution, la délétion, l'insertion ou l'addition d'un ou de 1 à 20 résidus d'acides aminés et ayant une activité pyruvate synthase, et est capable de constituer un complexe de protéines ayant une activité pyruvate synthase avec au moins les polypeptides (E), (F) et (H),
(H) un polypeptide défini aux points (H1) ou (H2) suivants :
(H1) un polypeptide comprenant la séquence d'acides aminés représentée dans SEQ ID N° 63,
(H2) un polypeptide qui comprend une séquence d'acides aminés représentée dans SEQ ID N° 63, comportant la substitution, la délétion, l'insertion ou l'addition d'un ou de 1 à 20 résidus d'acides aminés et ayant une activité pyruvate synthase, et est capable de constituer un complexe de protéines ayant une activité pyruvate synthase avec au moins les polypeptides (E), (F) et (G),
(I) un polypeptide défini aux points (I1) ou (I2) suivants :
(I1) un polypeptide comprenant la séquence d'acides aminés représentée dans SEQ ID N° 65,
(I2) un polypeptide qui comprend une séquence d'acides aminés représentée dans SEQ ID N° 65, comportant la substitution, la délétion, l'insertion ou l'addition d'un ou de 1 à 20 résidus d'acides aminés et ayant une activité pyruvate synthase, et est capable de constituer un complexe de protéines ayant une activité pyruvate synthase avec au moins les polypeptides (E), (F) et (G) et (H),
(J) un polypeptide défini aux points (J1) ou (J2) suivants :
(J1) un polypeptide comprenant la séquence d'acides aminés représentée dans SEQ ID N° 67,
(J2) un polypeptide qui comprend une séquence d'acides aminés représentée dans SEQ ID N° 67, comportant la substitution, la délétion, l'insertion ou l'addition d'un ou de 1 à 20 résidus d'acides aminés et ayant une activité pyruvate synthase, et est capable de constituer un complexe de protéines ayant une activité pyruvate synthase avec au moins les polypeptides (E), (F) et (G) et (H).

5. Procédé selon l'une quelconque des revendications 1 à 3, dans lequel le gène codant la pyruvate synthase comprend un ADN défini à l'un quelconque des points (a) à (d) suivants, ou un ADN défini aux points (e), (f), (g) et (h) suivants, ou un ADN défini aux points (e), (f), (g), (h), (i) et (j) suivants :
(a) un ADN ayant la séquence de nucléotides représentée dans SEQ ID N° 1,
(b) un ADN qui est capable de s'hybrider avec une séquence complémentaire de la séquence de nucléotides représentée dans SEQ ID N° 1 ou une sonde qui peut être préparée à partir de la séquence de nucléotides dans des conditions stringentes, et qui code un polypeptide ayant une activité pyruvate synthase,
(c) un ADN ayant la séquence de nucléotides représentée dans SEQ ID N° 3,
(d) un ADN qui est capable de s'hybrider avec une séquence complémentaire de la séquence de nucléotides représentée dans SEQ ID N° 3 ou une sonde qui peut être préparée à partir de la séquence de nucléotides dans des conditions stringentes, et qui code un polypeptide ayant une activité pyruvate synthase,
(e) un ADN défini aux points (e1) ou (e2) suivants :
(e1) un ADN ayant la séquence de nucléotides représentée dans SEQ ID N° 56,
(e2) un ADN qui est capable de s'hybrider avec une séquence complémentaire de la séquence de nucléotides représentée dans SEQ ID N° 56 ou une sonde qui peut être préparée à partir de la séquence de nucléotides dans des conditions stringentes, et qui code un complexe de protéines ayant une activité pyruvate synthase avec au moins les ADN définis aux points (f), (g) et (h),
(f) un ADN défini aux points (f1) ou (f2) suivants :
(f1) un ADN ayant la séquence de nucléotides représentée dans SEQ ID N° 58,
(f2) un ADN qui est capable de s'hybrider avec une séquence complémentaire de la séquence de nucléotides représentée dans SEQ ID N° 58 ou une sonde qui peut être préparée à partir de la séquence de nucléotides dans des conditions stringentes, et qui code un complexe de protéines ayant une activité pyruvate synthase avec au moins les ADN définis aux points (e), (g) et (h),
(g) un ADN défini aux points (g1) ou (g2) suivants :
(g1) un ADN ayant la séquence de nucléotides représentée dans SEQ ID N° 60,
(g2) un ADN qui est capable de s'hybrider avec une séquence complémentaire de la séquence de nucléotides représentée dans SEQ ID N° 60 ou une sonde qui peut être préparée à partir de la séquence de nucléotides dans des conditions stringentes, et qui code un complexe de protéines ayant une activité pyruvate synthase avec au moins les ADN définis aux points (e), (f) et (h),(h) un ADN défini aux points (h1) ou (h2) suivants : (h1) un ADN ayant la séquence de nucléotides représentée dans SEQ ID N° 62, (h2) un ADN qui est capable de s'hybrider avec une séquence complémentaire de la séquence de nucléotides représentée dans SEQ ID N° 62 ou une sonde qui peut être préparée à partir de la séquence de nucléotides dans des conditions stringentes, et qui code un complexe de protéines ayant une activité pyruvate synthase avec au moins les ADN définis aux points (e), (f) et (g),
(i) un ADN défini aux points (i1) ou (i2) suivants :
(i1) un ADN ayant la séquence de nucléotides représentée dans SEQ ID N° 64,
(i2) un ADN qui est capable de s'hybrider avec une séquence complémentaire de la séquence de nucléotides représentée dans SEQ ID N° 64 ou une sonde qui peut être préparée à partir de la séquence de nucléotides dans des conditions stringentes, et qui code un complexe de protéines ayant une activité pyruvate synthase avec au moins les ADN définis aux points (e), (f), (g) et (h),
(j) un ADN défini aux points (j1) ou (j2) suivants :
(j1) un ADN ayant la séquence de nucléotides représentée dans SEQ ID N° 66,
(j2) un ADN qui est capable de s'hybrider avec une séquence complémentaire de la séquence de nucléotides représentée dans SEQ ID N° 66 ou une sonde qui peut être préparée à partir de la séquence de nucléotides dans des conditions stringentes, et qui code un complexe de protéines ayant une activité pyruvate synthase avec au moins les ADN définis aux points (e), (f), (g) et (h) ; dans lequel les conditions stringentes sont un lavage à 60 °C, 0,1 x SSC et SDS 0,1 %.

6. Procédé selon l'une quelconque des revendications 1 à 3, dans lequel le gène codant la ρyruvate:NADP⁺ oxydoréductase code un polypeptide défini aux points (A) ou (B) suivants :
(A) un polypeptide comprenant la séquence d'acides aminés représentée dans SEQ ID N° 6,
(B) un polypeptide comprenant une séquence d'acides aminés représentée dans SEQ ID N° 6, comportant la substitution, la délétion, l'insertion ou l'addition d'un ou de 1 à 20 résidus d'acides aminés et ayant une activité pyruvate:NADP⁺ oxydoréductase.

7. Procédé selon l'une quelconque des revendications 1 à 3, dans lequel le gène codant la pyruvate:NADP⁺ oxydoréductase comprend un ADN défini aux points (a) ou (b) suivants :
(a) un ADN ayant la séquence de nucléotides représentée dans SEQ ID N° 5,
(b) un ADN qui est capable de s'hybrider avec une séquence complémentaire de la séquence de nucléotides représentée dans SEQ ID N° 5 ou une sonde qui peut être préparée à partir de la séquence de nucléotides dans des conditions stringentes, et qui code un polypeptide ayant une activité pyruvate:NADP⁺ oxydoréductase ; dans lequel les conditions stringentes sont un lavage à 60 °C, 0,1 x SSC et SDS 0,1 %.

8. Procédé selon l'une quelconque des revendications 1 à 5, dans lequel le microorganisme a été modifié de façon à ce qu'une activité ferrédoxine-NADP⁺ réductase soit augmentée.

9. Procédé selon l'une quelconque des revendications 1 à 5 et 8, dans lequel le microorganisme a été modifié de façon à ce que la capacité de production de ferrédoxine ou de flavodoxine soit améliorée.

10. Procédé selon l'une quelconque des revendications 1 à 9, dans lequel le microorganisme a été modifié de façon à ce qu'une activité d'enzyme malique soit diminuée.

11. Procédé selon l'une quelconque des revendications 1 à 10, dans lequel le microorganisme a été modifié de façon à ce que l'activité pyruvate déshydrogénase soit diminuée.

12. Procédé selon l'une quelconque des revendications 1 à 11, dans lequel le microorganisme a été modifié de façon à permettre l'assimilation aérobie de l'éthanol.

13. Procédé selon l'une quelconque des revendications 1 à 12, dans lequel le microorganisme est une bactérie appartenant au genre sélectionné dans le groupe constitué des genres Escherichia, Enterobacter, Pantoea, Klebsiella et Serratia.

14. Procédé selon la revendication 13, dans lequel les activités de l'enzyme malique dépendante du NAD et de l'enzyme malique dépendante du NADP sont diminuées.

15. Procédé selon l'une quelconque des revendications 1 à 14, dans lequel le microorganisme est une bactérie corynéforme.
